# EUROPEAN PATENT APPLICATION

(11) **EP 4 328 222 A1**
(43) Date of publication of application: **28.02.2024**
(21) Application number: 22791938.8
(22) Date of filing: 08.04.2022
(51) Int. Cl.: C07D 403/12, A61K 31/506, A61P 35/00, C07D 405/14, C07D 409/14, C07D 471/04, C07D 401/14

(54) **HETEROARYL DERIVATIVE COMPOUND AND USE THEREOF**

(30) Priority: 22.04.2021 KR 20210052559
(71) Applicant: Voronoi Inc., Incheon 21984 (KR)
(72) Inventor: KO, Yi Kyung, Incheon 21984 (KR); PARK, Jin Hee, Incheon 21984 (KR); LEE, Yeong Deok, Incheon 21984 (KR); IM, Hye Rim, Incheon 21984 (KR); KIM, Kyun Eun, Incheon 21984 (KR); HWANG, Dong Keun, Incheon 21984 (KR); HAN, Ah Reum, Incheon 21984 (KR); NAM, Su Been, Incheon 21984 (KR); HEO, Myung Hoe, Incheon 21984 (KR); KO, Eun Hwa, Incheon 21984 (KR); CHOI, Hwan Geun, Incheon 21984 (KR); KIM, Sung Hwan, Incheon 21984 (KR); JUNG, Hong Ryul, Incheon 21984 (KR); YOO, Ji Hye, Incheon 21984 (KR)
(74) Representative: Viering, Jentschura & Partner mbB Patent- und Rechtsanwälte
(86) International application number: PCT/KR2022/005163
(87) International publication number: WO 2022/225238

(57) **Abstract**

The present invention relates to a heteroaryl derivative compound and a use thereof. The heteroaryl derivative of the present invention exhibits excellent inhibitory activity against HER2 and EGFR, and thus can be effectively used as a therapeutic agent for HER2- and/or EGFR-associated diseases.

## Description

### [Technical Field]

The present disclosure relates to a heteroaryl derivative compound and medicinal uses thereof. Specifically, the present disclosure relates to a heteroaryl derivative compound having HER2 and/or EGFR inhibitory activity.

### [Background Art]

Protein kinases act as molecular switches to participate in signal transduction pathways, and the transition between active and inactive states of target proteins by kinases in cells should be smoothly controlled. If the transition between the active and inactive states is abnormally controlled, intracellular signal transduction is excessively activated or deactivated to induce uncontrollable cell division and proliferation. In particular, abnormal activation by mutation, amplification and/or overexpression of protein kinase genes causes the development and progression of various tumors or plays a crucial role in the development of various diseases such as inflammatory diseases, degenerative brain diseases, and autoimmune diseases.

In particular, HER2 (ErbB2), which is a receptor tyrosine kinase of the ErbB family, forms homodimers or heterodimers with other EGFR receptors such as HER1 (EGFR, ErbB1), HER3 (ErbB3), or HER4 (ErbB4) and is activated by autophosphorylation at intracellular tyrosine residues to play an important role in cell proliferation, differentiation and survival in normal cells and cancer cells (Di Fore PP, et al., Science. 1987; 237: 178-182). HER2 is known to be overexpressed in various carcinomas such as breast cancer, gastric cancer and ovarian cancer (Hardwick R, et al., Eur. J Surg Oncol. 1997 (23):30-35; Korkaya H, et al., Oncogene. 2008;27(47):6120-6130;).

In addition, it is known that epidermal growth factor receptor (EGFR), which is another ErbB family, is abnormally active in many epithelial cell tumors, including non-small cell lung carcinoma (NSCLC), breast cancer, glioma, squamous cell carcinoma of the head and neck, colorectal cancer, rectal adenocarcinoma, head and neck cancer, stomach cancer and prostate cancer, and the activation of the EGFR-tyrosine kinase causes continuous cell proliferation, invasion of surrounding tissues, distant metastasis, and angiogenesis, and increases cell survival.

There are afatinib (GILOTRIF) and trastuzumab (HERCEPTIN), etc., as anticancer drugs that target HER2 and EGFR, but these anticancer drugs have problems in that cancer recurs after treatment or only some patient groups show reactivity. For example, in the case of trastuzumab, the following cases have been reported that about 11.6% of patients with HER2-overexpressing breast cancer who received conventional anticancer treatment are responsive to trastuzumab, and the remaining 88.4% of patients have no or weak response to trastuzumab (Baselga et al., J. Clin. Oncol. 14:737-744 (1996)).

As described above, there is an increasing unmet need for novel compounds capable of being usefully utilized in the treatment of HER2- and EGFR-related diseases by modulating HER2 and EGFR activities.

### [Disclosure]

### [Technical Problem]

An object of the present disclosure is to provide a heteroaryl derivative having a novel structure, an optical isomer thereof, or a pharmaceutically acceptable salt thereof.

Another object of the present disclosure is to provide a method for preparing the heteroaryl derivative compound.

Still another object of the present disclosure is to provide a pharmaceutical use of the heteroaryl derivative compound, and specifically, to a pharmaceutical composition for the treatment or prevention of HER2- and/or EGFR-related diseases comprising the heteroaryl derivative compound as an active ingredient, use of the compound for the treatment or prevention of HER2- and/or EGFR-related diseases, or a method for treating or preventing HER2- and/or EGFR-related diseases comprising administering the compound.

### [Technical Solution]

In order to achieve the above object, the present inventors have made research efforts, and as a result, completed the present invention by confirming that a heteroaryl derivative compound represented by the following Chemical Formula 1 inhibited the proliferation of HER2- and/or EGFR-activated cells.

### Heteroaryl Derivative Compound

The present invention provides a compound represented by the following Chemical Formula 1, an optical isomer thereof, or a pharmaceutically acceptable salt thereof: 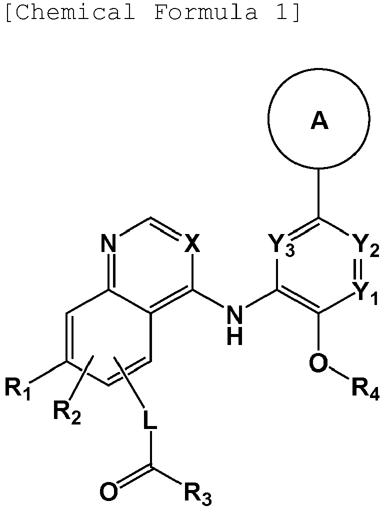 in Chemical Formula 1 above,
X is N or CR_{X1};
R₁ is -H, -OR_{X2,} or -NR_{X3}R_{X4};
R_{X1} is -H, -C₁₋₆alkyl, -C₁₋₆haloalkyl, -CN, or -halo;
R_{X2} is -H, -C₁₋₆alkyl, -C₁₋₆hydroxyalkyl, -C₁₋₆aminoalkyl, -C₁₋₆haloalkyl, -C₁₋₆alkyl-O(C₁₋₆alkyl), -C₁₋₆alkyl-O(C₁₋₆alkyl) -C₁₋₆aminoalkyl, -(CH₂)n-cycloalkyl, or -(CH₂)n-heterocycloalkyl {wherein at least one H of the -(CH₂)n-cycloalkyl or -(CH₂)n-heterocycloalkyl ring may be substituted with -C₁₋₆alkyl, -C₁₋₆hydroxyalkyl, -C₁₋₆aminoalkyl, -C₁₋₆haloalkyl, -C₁₋₆alkyl-O(C₁₋₆alkyl), -CN, -NH₂, -NH-C₁₋₆alkyl, -N(C₁₋₆alkyl)(C₁₋₆alkyl), -NO₂, -OH, -halo, or heterocycloalkyl};
n is 0, 1, 2, 3, or 4;
R_{X3} and R_{X4} are each independently -H or -C₁₋₆alkyl, or R_{X3} and R_{X4} are linked to each other to form 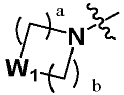 or 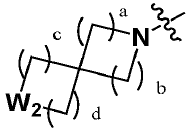 ring together with an N atom, and the W₁ and W₂ are each independently CH₂, NH, O, or S {wherein at least one H of the 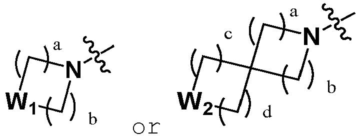 heterocycloalkyl ring may be substituted with -C₁₋₆alkyl, -C₁₋₆hydroxyalkyl, -C₁₋₆aminoalkyl, -C₁₋₆haloalkyl, -CN, -NH₂, -NH-C₁₋₆alkyl, -N(C₁₋₆alkyl) (C₁₋₆alkyl), -NO₂, -OH, -halo, or heterocycloalkyl};
a to d are each independently 1, 2, or 3;
R₂ is -H, -C₁₋₆alkyl, -C₁₋₆haloalkyl, -CN, or -halo;
L is -NH-, 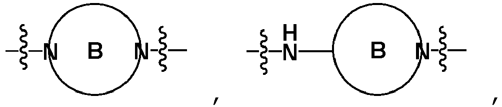 or 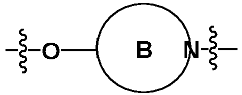 {wherein the ring B is a single ring or multiple rings containing an N atom in the ring, and at least one H of the ring B may be substituted with -C₁₋₆alkyl, -C₁-₆hydroxyalkyl, -C₁₋₆aminoalkyl, -C₁₋₆haloalkyl, -CN, -NH₂, - NH-C₁₋₆alkyl, -N(C₁₋₆alkyl)(C₁₋₆alkyl), -NO₂, -OH, =O, or - halo};
R₃ is -CZ₁=Z₂Z₃, -C₁₋₆alkyl, -C₁₋₆haloalkyl, or cycloalkyl;
Z₁ is -H, -C₁₋₆alkyl, -C₁₋₆aminoalkyl, -C₁₋₆hydroxyalkyl, -C₁₋₆haloalkyl, -CN, or -halo;
Z₂ and Z₃ are each independently -H, -C₁₋₆alkyl, -C₁-₆hydroxyalkyl, -C₁₋₆aminoalkyl, -C₁₋₆haloalkyl, -C₁₋₆alkyl-O(C₁₋₆alkyl), -halo, cycloalkyl, heterocycloalkyl, -C₁₋₆alkylcycloalkyl, or -C₁₋₆alkyl-heterocycloalkyl {wherein at least one H of the cycloalkyl, heterocycloalkyl, -C₁₋₆alkylcycloalkyl, or -C₁₋₆alkyl-heterocycloalkyl may be substituted with -C₁₋₆alkyl, -C₁₋₆hydroxyalkyl, -C₁₋₆aminoalkyl, -C₁₋₆haloalkyl, -CN, -NH₂, -NH-C₁₋₆alkyl, -N(C₁₋₆alkyl)(C₁₋₆alkyl), -NO₂, -OH, =O, or heterocycloalkyl};
Y₁ to Y₃ are each independently N or CR_{Y};
R_{Y} is -H, -C₁₋₆alkyl, -C₁₋₆aminoalkyl, -C₁₋₆hydroxyalkyl, -C₁₋₆haloalkyl, -CN, -NH₂, -NH-C₁₋₆alkyl, -N(C₁₋₆alkyl)(C₁₋₆alkyl), -NO₂, -OH, or -halo;
R₄ is -C₁₋₆alkyl, -C₁₋₆alkyl-O-C₁₋₆alkyl, -C₁₋₆haloalkyl, cycloalkyl, heterocycloalkyl, -C₁₋₆alkyl-cycloalkyl, or -C₁₋₆alkyl-heterocycloalkyl {wherein at least one H of the cycloalkyl, heterocycloalkyl, -C₁₋₆alkyl-cycloalkyl, or -C₁₋₆alkyl-heterocycloalkyl may be substituted with -C₁₋₆alkyl};
ring A is aryl, heteroaryl, cycloalkyl, cycloalkenyl, heterocycloalkyl, or heterocycloalkenyl {wherein at least one H of the aryl, heteroaryl, cycloalkyl, cycloalkenyl, heterocycloalkyl, or heterocycloalkenyl ring may be substituted with -C₁₋₆alkyl, -C₁₋₆aminoalkyl, -C₁₋₆hydroxyalkyl, -C₁₋₆haloalkyl, -C₁₋₆alkenyl, -CN, -C(=O)-R_{A1}, -NO₂, -NR_{A2}R_{A3}, - OR_{A4}, -S-C₁₋₆alkyl, -halo, or heterocycloalkyl [here, at least one H of the heterocycloalkyl ring may be substituted with -C₁₋₆alkyl, -C₁₋₆haloalkyl, -halo, or heterocycloalkyl], and substituents on the aryl or heteroaryl ring may be linked to each other to form a 5-6 membered cycloalkyl or a 5-6 membered heterocycloalkyl};
R_{A1} is -H, -C₁₋₆alkyl, -NH₂, -NH-C₁₋₆alkyl, -N(C₁-₆alkyl) (C₁₋₆alkyl), -NH-(CH₂)m-aryl, -OH, or -O-C₁₋₆alkyl {wherein at least one H of the -NH-(CH₂)m-aryl ring may be substituted with -C₁₋₆alkyl, -C₁₋₆haloalkyl, or -halo};
m is 0, 1, 2, 3, or 4;
R_{A2} and R_{A3} are each independently -H, -C₁₋₆alkyl, - C(=O)-C₁₋₆alkyl, -C(=O)-C₁₋₆alkenyl, -C(=O)-cycloalkyl,-C(=O)-heterocycloalkyl, or -C(=O)-aryl {wherein at least one H of the -C(=O)-cycloalkyl, -C(=O)-heterocycloalkyl, or - C(=O)-aryl ring may be substituted with -C₁₋₆alkyl, -C₁₋₆haloalkyl, =O, -halo, or aryl}; and
R_{A4} is -H, -C₁₋₆alkyl, or -C₁₋₆haloalkyl.

According to an embodiment of the present invention, the compound represented by Chemical Formula 1, the optical isomer thereof, or the pharmaceutically acceptable salt thereof may be in the following ranges:
X is N or CR_{X1};
R₁ is -H, -OR_{X2,} or -NR_{X3}R_{X4};
R_{X1} is -H or -CN;
R_{X2} is -C₁₋₆alkyl, -C₁₋₆aminoalkyl, -C₁₋₆haloalkyl, -C₁₋₆alkyl-O(C₁₋₆alkyl), -C₁₋₆alkyl-O(C₁₋₆alkyl)-C₁₋₆aminoalkyl, or -(CH₂)n-heterocycloalkyl {wherein at least one H of the - (CH₂)n-heterocycloalkyl ring may be substituted with -C₁-₆alkyl, -C₁₋₆alkyl-O(C₁₋₆alkyl), or heterocycloalkyl};
n is 0, 1, 2, 3, or 4;
R_{X3} and R_{X4} are each independently -H or -C₁₋₆alkyl, or R_{X3} and R_{X4} are linked to each other to form 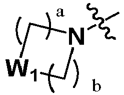 or 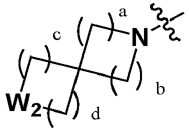 ring together with an N atom, and W₁ and W₂ are each independently CH₂, NH, or O {wherein at least one H of the 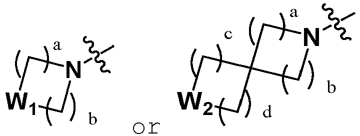 ring may be substituted with - halo, or heterocycloalkyl};
a to d are each independently 1 or 2;
R₂ is -H or -halo;
L is -NH-, 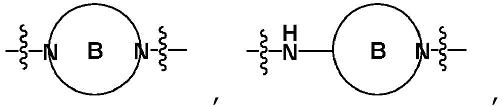 or 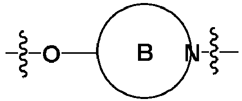 {wherein the ring B is a single ring or multiple rings containing an N atom in the ring, and at least one H of the ring B may be substituted with -C₁₋₆alkyl, -C₁-₆haloalkyl, =O, or -halo};
R₃ is -CZ₁=Z₂Z₃, -C₁₋₆haloalkyl, or cycloalkyl;
Z₁ is -H, -C₁₋₆aminoalkyl, -CN, or -halo;
Z₂ and Z₃ are each independently -H, -C₁₋₆alkyl, -C₁-₆aminoalkyl, -C₁₋₆alkyl-O(C₁₋₆alkyl), -halo, -heterocycloalkyl, or -C₁₋₆alkyl-heterocycloalkyl {wherein at least one H of the -heterocycloalkyl, or -C₁₋₆alkyl-heterocycloalkyl may be substituted with -C₁₋₆alkyl or -heterocycloalkyl};
Y₁ to Y₃ are each independently N or CR_{Y};
Ry is -H, -C₁₋₆alkyl, or -halo; and
R₄ is -C₁₋₆alkyl, -C₁₋₆alkyl-O-C₁₋₆alkyl, -C₁₋₆haloalkyl, cycloalkyl, or heterocycloalkyl {wherein at least one H of the cycloalkyl or heterocycloalkyl may be substituted with -C₁₋₆alkyl};
ring A is aryl, heteroaryl, heterocycloalkyl, or heterocycloalkenyl {wherein at least one H of the aryl, heteroaryl, cycloalkyl, cycloalkenyl, heterocycloalkyl, or heterocycloalkenyl ring may be substituted with -C₁₋₆alkyl, -C₁₋₆haloalkyl, -C₁₋₆alkenyl, -CN, -C(=O)-R_{A1}, -NO₂, -NR_{A2}R_{A3}, - OR_{A4}, -S-C₁₋₆alkyl, -halo, or heterocycloalkyl [here, at least one H of the heterocycloalkyl ring may be substituted with -C₁₋₆alkyl, -C₁₋₆haloalkyl, -halo, or heterocycloalkyl], and substituents on the aryl or heteroaryl ring may be linked with each other to form a 5-6 membered cycloalkyl or a 5-6 membered heterocycloalkyl};
R_{A1} is -H, -NH-(CH₂)m-aryl, -OH, or -O-C₁₋₆alkyl {wherein at least one H of the -NH-(CH₂)m-aryl ring may be substituted with -halo};
m is 0, 1, 2, 3, or 4;
R_{A2} and R_{A3} are each independently -H, -C₁₋₆alkyl, - C(=O)-C₁₋₆alkyl, -C(=O)-C₁₋₆alkenyl, -C(=O)-cycloalkyl, - C(=O)-heterocycloalkyl, or -C(=O)-aryl {wherein at least one H of the -C(=O)-cycloalkyl, -C(=O)-heterocycloalkyl, or - C(=O)-aryl ring may be substituted with =O, -halo, or aryl}; and
R_{A4} is -H, -C₁₋₆alkyl, or -C₁₋₆haloalkyl.

According to an embodiment of the present invention, the compound represented by Chemical Formula 1, the optical isomer thereof, or the pharmaceutically acceptable salt thereof may be in the following ranges:
X is N or CR_{X1};
R₁ is -H, -OR_{X2}, or -NR_{X3}R_{X4};
R_{X1} is -H or -CN;
R_{X2} is -C₁₋₆alkyl, -C₁₋₆aminoalkyl, -C₁₋₆haloalkyl, -C₁₋₆alkyl-O(C₁₋₆alkyl), -C₁₋₆alkyl-O(C₁₋₆alkyl)-C₁₋₆aminoalkyl, or - (CH₂)n-heterocycloalkyl {wherein the -(CH₂)n-heterocycloalkyl ring is 4-6 membered, and at least one H of the -(CH₂)n-heterocycloalkyl ring may be substituted with - C₁₋₆alkyl, -C₁₋₆alkyl-O(C₁₋₆alkyl), or 4-6 membered heterocycloalkyl};
n is 0, 1, 2, or 3;
R_{X3} and R_{X4} are each independently -H or -C₁₋₆alkyl, or R_{X3} and R_{X4} are linked to each other to form 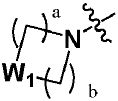 or 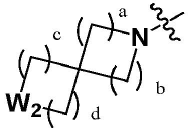 together with an N atom, and W₁ and W₂ are each independently CH₂ or O {wherein at least one H of the 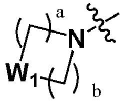 or 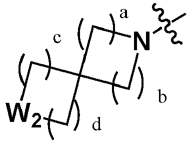 ring may be substituted with -halo, or 4-6 membered heterocycloalkyl};
a to d are each independently 1 or 2; and
R₂ is -H or -halo.

According to an embodiment of the present invention, the compound represented by Chemical Formula 1, the optical isomer thereof, or the pharmaceutically acceptable salt thereof may be in the following ranges:
L is -NH-, 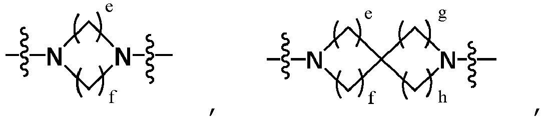 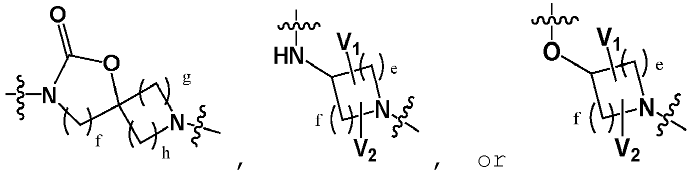 {wherein at least one H of the 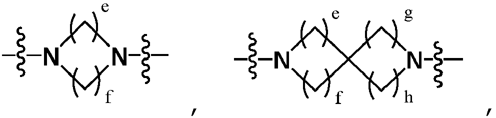 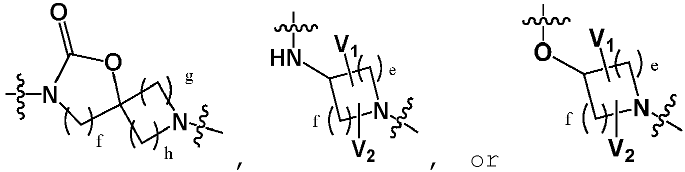 ring may be substituted with -C₁₋₆alkyl, -C₁₋₆haloalkyl, =O, or -halo};
V₁ and V₂ are linked together by a single bond, C₁alkyl, C₂alkyl, or C₃alkyl to form a bridged double ring or nothing (null); and
e to h are each independently 1, 2, or 3.

According to an embodiment of the present invention, the compound represented by Chemical Formula 1, the optical isomer thereof, or the pharmaceutically acceptable salt thereof may be in the following ranges:
R₃ is -CZ₁=Z₂Z₃, -C₁₋₆haloalkyl, or cycloalkyl {wherein the cycloalkyl ring is a single ring or multiple rings};
Z₁ is -H, -C₁₋₆aminoalkyl, -CN, or -halo; and
Z₂ and Z₃ are each independently -H, -C₁₋₆alkyl, -C₁-₆aminoalkyl, -C₁₋₆alkyl-O(C₁₋₆alkyl), -halo, -heterocycloalkyl, or -C₁₋₆alkyl-heterocycloalkyl {wherein the heterocycloalkyl or -C₁₋₆alkyl-heterocycloalkyl ring is 4-6 membered, and at least one H of the heterocycloalkyl, -C₁₋₆alkyl-cycloalkyl, or -C₁₋₆alkyl-heterocycloalkyl may be substituted with -C₁-₆alkyl, or 4-6 membered heterocycloalkyl}.

According to an embodiment of the present invention, the compound represented by Chemical Formula 1, the optical isomer thereof, or the pharmaceutically acceptable salt thereof may be in the following ranges:
Y₁ to Y₃ are each independently N or CR_{Y};
R_{Y} is -H, -C₁₋₆alkyl, or -halo; and
R₄ is -C₁₋₆alkyl, -C₁₋₆alkyl-O-C₁₋₆alkyl, -C₁₋₆haloalkyl, 3-7 membered cycloalkyl, or 4-6 membered heterocycloalkyl {wherein at least one H of the 3-7 membered cycloalkyl or 4-6 membered heterocycloalkyl may be substituted with -C₁-₆alkyl}.

According to an embodiment of the present invention, the compound represented by Chemical Formula 1, the optical isomer thereof, or the pharmaceutically acceptable salt thereof may be in the following ranges:
ring A is phenyl, 5-10 membered heteroaryl, 4-6 membered heterocycloalkyl, or 4-6 membered heterocycloalkenyl {wherein at least one H of the phenyl, 5-10 membered heteroaryl, 4-6 membered heterocycloalkyl, or 4-6 membered heterocycloalkenyl ring may be substituted with -C₁₋₆alkyl, -C₁₋₆haloalkyl, -C₁₋₆alkenyl, -CN, -C(=O)-R_{A1}, -NO₂, -NR_{A2}R_{A3}, -OR_{A4}, -S-C₁₋₆alkyl, -halo, or 4-6 membered heterocycloalkyl [here, at least one H of the 4-6 membered heterocycloalkyl ring may be substituted with -C₁₋₆alkyl, - C₁₋₆haloalkyl, -halo, or heterocycloalkyl], and substituents on the phenyl or 5-10 membered heteroaryl ring may be linked to each other to form a 5-6 membered cycloalkyl or a 5-6 membered heterocycloalkyl};
R_{A1} is -H, -NH-(CH₂)m-phenyl, -OH, or -O-C₁₋₆alkyl {wherein at least one H of the -NH-(CH₂)m-phenyl ring may be substituted with -halo};
m is 0, 1, or 2;
R_{A2} and R_{A3} are each independently -H, -C₁₋₆alkyl, - C(=O)-C₁₋₆alkyl, -C(=O)-C₁₋₆alkenyl, -C(=O)-cycloalkyl,-C(=O)-heterocycloalkyl, or -C(=O)-phenyl {wherein at least one H of the -C(=O)-cycloalkyl, -C(=O)-heterocycloalkyl, or -C(=O)-phenyl ring may be substituted with -C₁₋₆alkyl, -C₁₋₆haloalkyl, =O, -halo, or phenyl}; and
R_{A4} is -H, -C₁₋₆alkyl, or -C₁₋₆haloalkyl.

According to another embodiment of the present invention, the compound represented by Chemical Formula 1 may be selected from the group consisting of compounds listed in Table 1 described below.

In the present disclosure, unless otherwise specified, the term "alkyl" may refer to a straight or branched chain acyclic, cyclic, or saturated hydrocarbon to which they are bonded. For example, "C₁₋₆alkyl" may indicate an alkyl containing 1 to 6 carbon atoms. As an example, acyclic alkyl may include, but is not limited to, methyl, ethyl, n-propyl, n-butyl, isopropyl, sec-butyl, isobutyl, tert-butyl, or the like. Cyclic alkyl may be used interchangeably with "cycloalkyl" as used herein, and as an example, may include, but is not limited to, cyclopropyl, cyclobutyl, cyclopentyl, cyclohexyl, cycloheptyl, cyclooctyl, or the like.

In the present disclosure, "alkoxy" may indicate -(O-alkyl) as an alkyl ether group, wherein alkyl is the same as defined above. For example, "C₁₋₆alkoxy" may mean alkoxy containing C₁₋₆alkyl, that is, - (O-C₁₋₆alkyl), and as an example, may include, but is not limited to, methoxy, ethoxy, *n*-propoxy, isopropoxy, *n*-butoxy, isobutoxy, *sec*-butoxy, *tert*-butoxy, or the like.

In the present disclosure, "halo" may be F, Cl, Br, or I.

As used herein, "haloalkyl" may mean a straight or branched chain alkyl (hydrocarbon) having one or more halo-substituted carbon atoms as defined herein. Examples of the haloalkyl may include, but are not limited to, methyl, ethyl, propyl, isopropyl, isobutyl or *n*-butyl independently substituted with one or more halogens, such as F, Cl, Br, or I.

In the present disclosure, "hydroxyalkyl" may indicate a straight or branched chain alkyl (hydrocarbon) having a carbon atom substituted with -hydroxy (-OH). Examples of the haloalkyl may include, but are not limited to, methyl, ethyl, propyl, isopropyl, isobutyl or n-butyl independently substituted with one or more halogens, for example, -OH.

As used herein, "aminoalkyl" may mean a straight or branched chain alkyl (hydrocarbon) having a carbon atom substituted with amino (NR'R"). Here, R' and R" may be each independently selected from the group consisting of hydrogen and C₁₋₆alkyl, and the selected R' and R" may be each independently substituted or unsubstituted.

In the present disclosure, "heterocycloalkyl" may mean a ring containing 1 to 5 heteroatoms selected from N, O, and S, which atoms form the ring, and may be saturated or partially unsaturated. Here, when unsaturated, it may be referred to as a heterocycloalkene. Unless otherwise stated, heterocycloalkyl may be a single ring or a multiple ring such as a spiro ring, a bridged ring or a fused ring. In addition, "3- to 12-membered heterocycloalkyl" may indicate a heterocycloalkyl containing 3 to 12 atoms forming a ring. As an example, the heterocycloalkyl may include, but is not limited to, pyrrolidine, piperidine, imidazolidine, pyrazolidine, butyrolactam, valerolactam, imidazolidinone, hydantoin, dioxolane, phthalimide, piperidine, pyrimidin-2,4(1*H*,3*H*)-dione, 1,4-dioxane, morpholine, thiomorpholine, thiomorpholine-*S*-oxide, thiomorpholine-*S,S*-oxide, piperazine, pyran, pyridone, 3-pyrroline, thiopyran, pyrone, tetrahydrofuran, tetrahydrothiophene, quinuclidine, tropane, 2-azaspiro[3.3]heptane, (1*R*,5*S*)-3-azabicyclo[3.2.1]octane, (1*s*,4*s*)-2-azabicyclo[2.2.2]octane, or (1*R*,4*R*)-2-oxa-5-azabicyclo[2.2.2]octane, and the like.

In the present disclosure, "arene" may mean an aromatic hydrocarbon ring. The arene may be a monocyclic arene or a polycyclic arene. The number of ring-forming carbons in the arene may be 5 or more and 30 or less, 5 or more and 20 or less, or 5 or more and 15 or less. Examples of the arene may include, but are not limited to, benzene, naphthalene, fluorene, anthracene, phenanthrene, bibenzene, terbenzene, quaterbenzene, quinquebenzene, sexibenzene, triphenylene, pyrene, benzofluoranthene, chrysene, and the like. In the present specification, the residue obtained by removing one hydrogen atom from "arene" is referred to as "aryl".

In the present disclosure, "heteroarene" may be a ring containing at least one of O, N, P, Si, and S as a heterogeneous element. The number of ring-forming carbons in the heteroarene may be 2 or more and 30 or less, or 2 or more and 20 or less. The heteroarene may be a monocyclic heteroarene or a polycyclic heteroarene. The polycyclic heteroarene may have, for example, a bicyclic or tricyclic structure. Examples of the heteroarene may include thiophene, purine, pyrrole, pyrazole, imidazole, thiazole, oxazole, isothiazole, oxadiazole, triazole, pyridine, bipyridyl, triazine, acridyl, pyridazine, pyrazine, quinoline, quinazoline, quinoxaline, phenoxazine, phthalazine, pyrimidine, pyridopyrimidine, pyridopyrazine, pyrazinopyrazine, isoquinoline, indole, carbazole, imidazopyridazine, imidazopyridine, imidazopyrimidine, pyrazolopyrimidine, imidazopyrazine or pyrazolopyridine, triazolopyridine, triazolopyrimidine, *N*-arylcarbazole, *N-*heteroarylcarbazole, *N*-alkylcarbazole, benzoxazole, benzoimidazole, benzothiazole, benzocarbazole, benzothiophene, dibenzothiophene, thienothiophene, benzofuran, phenanthroline, isoxazole, oxadiazole, thiadiazole, benzothiazole, tetrazole, phenothiazine, dibenzosilole, dibenzofuran, and the like, but are not limited thereto. In an embodiment of the present disclosure, heteroarene may also include bicyclic heterocyclo-arene containing heteroarene fused to an arene ring or a cycloalkyl ring fused to heterocycloalkyl rings. In the present specification, the residue obtained by removing one hydrogen atom from the "heteroarene" is referred to as "heteroaryl".

In the present disclosure, "ring" may be a single ring or a multiple ring, and the multiple ring may be in the form of a spiro ring, a bridged ring, a fused ring, or the like.

In the present disclosure, the term "optical isomer (enantiomer)" means compounds of the present disclosure or salts thereof that have the same chemical formula or molecular formula but are different in stereostructure. Each of these enantiomers and mixtures thereof are also included within the scope of the present disclosure. Unless otherwise specified, the straight solid-line bond (-) connecting an asymmetric carbon atom may include a wedge-shaped solid-line bond ( 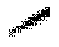 ) or a wedge-shaped dashed-line bond ( 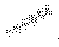 ) indicating the absolute configuration of the stereocenter.

In the present disclosure, the term "cis" refers to a case in which the binding directions of two substituents in a ring are the same, and the term "trans" refers to a case in which the binding directions of two substituents in the ring are different.

The compound of Chemical Formula 1 of the present disclosure may exist in the form of a "pharmaceutically acceptable salt". As the salt, an acid addition salt formed by a pharmaceutically acceptable free acid is useful. The term "pharmaceutically acceptable salt" as used herein means any and all organic or inorganic acid addition salts of the compound represented by Chemical Formula 1 of which side effects caused by the salt do not reduce the beneficial efficacy of the compound at concentrations having an effective action that is relatively non-toxic and harmless to a patient.

Acid addition salts are prepared by conventional methods, for example by dissolving the compound in an excess of aqueous acid solution and precipitating the salt using a water-miscible organic solvent such as methanol, ethanol, acetone or acetonitrile. An acid or alcohol in an equimolar amount of the compound and water may be heated, and the mixture may then be evaporated to dryness, or the precipitated salt may be filtered off with suction.

Here, an organic acid and an inorganic acid may be used as the free acid, wherein the inorganic acid may be hydrochloric acid, phosphoric acid, sulfuric acid, nitric acid, or the like, and the organic acid may be methanesulfonic acid, p-toluenesulfonic acid, acetic acid, trifluoroacetic acid, maleic acid, succinic acid, oxalic acid, benzoic acid, tartaric acid, fumaric acid, mandelic acid, propionic acid, citric acid, lactic acid, glycolic acid, gluconic acid, galacturonic acid, glutamic acid, glutaric acid, glucuronic acid, aspartic acid, ascorbic acid, carbonic acid, vanillic acid, hydroiodic acid, or the like. However, the present disclosure is not limited thereto.

In addition, it is possible to prepare a pharmaceutically acceptable metal salt using a base. The alkali metal salt or alkaline earth metal salt is obtained, for example, by dissolving a compound in an excess of alkali metal hydroxide or alkaline earth metal hydroxide solution, filtering the undissolved compound salt, and then evaporating and drying the filtrate. Here, it is pharmaceutically suitable to prepare a sodium, potassium, or calcium salt as the metal salt, but the present disclosure is not limited thereto. Further, the corresponding silver salt may be obtained by reacting an alkali metal or alkaline earth metal salt with a suitable silver salt (for example, silver nitrate).

Unless otherwise indicated, the pharmaceutically acceptable salt of the present disclosure includes salts of acidic or basic groups that may be present in the compound of Chemical Formula 1. For example, the pharmaceutically acceptable salt may include sodium, calcium and potassium salts of hydroxyl groups, and the like, and as other pharmaceutically acceptable salts of amino groups, may include hydrobromide, sulfate, hydrogen sulfate, phosphate, hydrogen phosphate, dihydrogen phosphate, acetate, succinate, citrate, tartrate, lactate, mandelate, methanesulfonate (mesylate), and p-toluenesulfonate (tosylate) salts, and the like, and may be prepared by a method for preparing a salt known in the art.

### Use of heteroaryl derivative compound

The present invention provides use of a compound represented by the following Chemical Formula 1, an optical isomer thereof, or a pharmaceutically acceptable salt thereof: 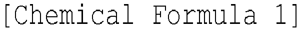 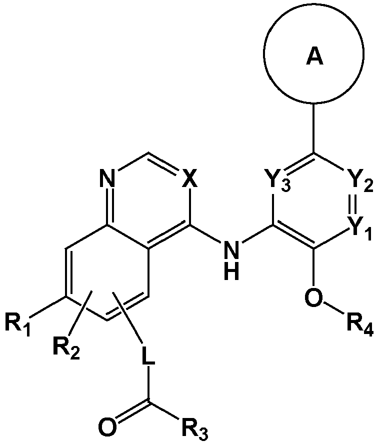 wherein the Chemical Formula 1 is the same as defined above.

The compound represented by Chemical Formula 1 of the present invention, the optical isomer thereof, or the pharmaceutically acceptable salt thereof exhibits inhibitory activity against various kinases.

According to an embodiment of the present invention, the heteroaryl derivative represented by Chemical Formula 1 may exhibit excellent inhibitory activity against HER2 and EGFR kinases, thereby being usefully employed for treatment or prevention of HER2- and/or EGFR-related diseases, in particular, cancer. In particular, the heteroaryl derivative compound of the present invention may exhibit excellent inhibitory activity against HER2 mutations (for example, HER2 L869R, HER2 L755S, HER2 T798I, HER2 T862A, and the like) and EGFR mutations (for example, EGFR G719A, EGFR L861Q, EGFR S768I, EGFR G719A/S768I, EGFR Del19/T790M, and the like), which may be usefully employed for the treatment or prevention of carcinomas induced by HER2 and/or EGFR.

In the present disclosure, the cancer includes any cancer capable of exhibiting therapeutic or prophylactic efficacy due to inhibition of HER2 and/or EGFR kinase activity, and may be a solid cancer or a hematologic cancer. For example, the cancer may be one or more selected from the group consisting of pseudomyxoma, intrahepatic cholangiocarcinoma, hepatoblastoma, liver cancer, thyroid cancer, colon cancer, testicular cancer, myelodysplastic syndrome, glioblastoma, oral cancer, lip cancer, mycosis fungoides, acute myeloid leukemia, acute lymphoblastic leukemia, basal cell carcinoma, ovarian epithelial cancer, ovarian germ cell tumor, male breast cancer, brain cancer, pituitary adenoma, multiple myeloma, gallbladder cancer, biliary tract cancer, colorectal cancer, chronic myelogenous leukemia, chronic lymphocytic leukemia, retinoblastoma, choroidal melanoma, ampulla of vater cancer, bladder cancer, peritoneal cancer, parathyroid cancer, adrenal cancer, sinonasal cancer, non-small cell lung cancer, tongue cancer, astrocytoma, small cell lung cancer, pediatric brain cancer, pediatric lymphoma, pediatric leukemia, small bowel cancer, meningioma, esophageal cancer, glioma, renal pelvis cancer, renal cancer, heart cancer, duodenal cancer, malignant soft tissue tumor, malignant bone tumor, malignant lymphoma, malignant mesothelioma, malignant melanoma, eye cancer, vulvar cancer, ureteral cancer, urethral cancer, cancer of unknown primary site, gastric lymphoma, gastric cancer, gastric carcinoid tumor, gastrointestinal stromal tumor, Wilms' tumor, breast cancer, sarcoma, penile cancer, pharyngeal cancer, gestational choriocarcinoma, cervical cancer, endometrial cancer, uterine sarcoma, prostate cancer, metastatic bone cancer, metastatic brain cancer, mediastinal cancer, rectal cancer, rectal carcinoid tumor, vaginal cancer, spinal cord cancer, vestibular schwannoma, pancreatic cancer, salivary gland cancer, Kaposi's sarcoma, Paget's disease, tonsil cancer, squamous cell carcinoma, lung adenocarcinoma, lung cancer, lung squamous cell carcinoma, skin cancer, anal cancer, rhabdomyosarcoma, laryngeal cancer, pleura cancer, hematological cancer, and thymic carcinoma, but are not limited thereto. The cancer includes not only primary cancer but also metastatic cancer.

According to an embodiment of the present disclosure, the present disclosure provides a pharmaceutical composition for treatment or prevention of HER2- and/or EGFR-related diseases containing the compound represented by Chemical Formula 1, the optical isomer thereof, or the pharmaceutically acceptable salt thereof as an active ingredient. Specifically, the HER2- and/or EGFR-related disease may be cancer. The types of cancer are the same as described above.

The pharmaceutical composition of the present disclosure may further include one or more active ingredients exhibiting the same or similar drug efficacy in addition to the compound represented by Chemical Formula 1 above, the optical isomer thereof, or the pharmaceutically acceptable salt thereof.

Further, according to an embodiment of the present disclosure, there is provided a method for treating or preventing HER2- and/or EGFR-related diseases, comprising administering to a subject in need thereof a therapeutically effective amount of the compound represented by Chemical Formula 1, the optical isomer thereof, or the pharmaceutically acceptable salt thereof. The subject may be a mammal including a human.

The term "therapeutically effective amount" as used herein refers to an amount of the compound represented by Chemical Formula 1 that is effective for the treatment or prevention of HER2- and/or EGFR-related diseases. Specifically, "therapeutically effective amount" indicates an amount sufficient to treat a disease with a reasonable benefit/risk ratio applicable to medical treatment, and the effective dose level may be determined depending on factors including the subject type and severity, age, sex, type of disease, drug activity, drug sensitivity, administration time, administration route and excretion rate, treatment period, drugs used at the same time, and other factors well-known in medical fields. The pharmaceutical composition of the present disclosure may be administered as an individual therapeutic agent or may be administered in combination with other therapeutic agents, and may be administered sequentially or simultaneously with commercially available therapeutic agents. In addition, the pharmaceutical composition of the present disclosure may be administered in a single dose or multiple doses. It is important to administer the minimum amount capable of obtaining the maximum effect without side effects in consideration of all of the above factors, and the amount may be readily determined by those skilled in the art. The dosage of the pharmaceutical composition of the present disclosure may be determined by a medical specialist according to various factors such as the patient's condition, age, sex, complications, and the like. Since the active ingredient of the pharmaceutical composition of the present disclosure has excellent safety, it may be used at a dose higher than the determined dosage.

Further, according to an embodiment of the present disclosure, the present disclosure provides use of the compound represented by Chemical Formula 1, the optical isomer thereof, or the pharmaceutically acceptable salt thereof for use in preparation of a medicament to treat or prevent HER2- and/or EGFR-related diseases. The compound represented by Chemical Formula 1 for preparing the medicament may be mixed with acceptable adjuvants, diluents, carriers, and the like, and may have a synergistic effect of active ingredients by being prepared as a complex formulation with other active agents.

Matters mentioned in the uses, compositions and treatment methods of the present disclosure are applied equally except to the extent that they are inconsistent with each other.

### [Advantageous Effects]

The heteroaryl derivative compound of the present disclosure exhibits excellent inhibitory activity against HER2 and/or EGFR, and thus may be usefully employed for the treatment or prevention of HER2- and/or EGFR-related diseases.

### [Best Mode]

Hereinafter, the present invention will be described in detail by Examples and Experimental Examples. However, the following Examples and Experimental Examples are only provided to illustrate the present invention, and the scope of the present invention is not limited to these Examples.

### <Analysis and purification conditions>

### 1. HPLC analysis conditions

(a) Instrument: Waters e2695
   Column: Xbridge C18, 4.6 x 150 mm, 5 µm, 40 °C
   Mobile phase: 20% -> 95% acetonitrile/H₂O + 0.1% TFA
   Analysis time: 10 minutes, flow rate: 1 mL/min
   UV detector: 254 nm
(b) Instrument: Waters e2695
   Column: YMC-Pack ODS-AM, 150 x 4.6 mm, 3 um, 12 nm, 40 °C
   Mobile phase: 10% -> 90% acetonitrile/H₂O + 0.1% TFA
   Analysis time: 20 minutes, flow rate: 1 mL/min
   UV detector: 254 nm

### 2. LC-MS analysis conditions

Instrument: Waters ACQUITY UPLC
Column: ACQUITY UPLC^{®} BEH C18, 50 x 2.1 mm, 5 µm, 40 °C
Mobile phase: acetonitrile/H₂O + 0.1% TFA
Flow rate: 0.6 mL/min
UV detector: 254 nm

### 3. MPLC analysis conditions

Instrument: CombiFlash^{®} Rf+
UV detector: 254 nm

### 4. Prep-HPLC purification conditions (A)

Instrument: ACCQPrep HP125
Column: XBridge^{®} Prep Shield RP18, 250 x 19 mm, 10 um
Mobile phase: acetonitrile/0.1% TFA H₂O
Flow rate: 25 mL/min
UV detector: 254 nm

### 5. Prep-HPLC purification conditions (B)

Instrument: ACCQPrep HP125
Column: XBridge^{®} Prep Shield RP18, 250 x 19 mm, 10 um
Mobile phase: acetonitrile/0.1% FA H₂O
Flow rate: 25 mL/min
UV detector: 254 nm

### 6. ¹H NMR

Instrument: Bruker Ascend^{™} 400 (400 MHz)
Commercial reagents used in the experiments were used without further purification. In the present invention, room temperature means a temperature of 15 ~ 25 °C. Concentration under reduced pressure or solvent distillation removal was performed using a rotary evaporator.

### Preparation Example 1. Preparation of N-(2,6-difluoro-3-(4,4,5,5-tetramethyl-1,3,2-dioxaborolan-2-yl)phenyl)acetamide

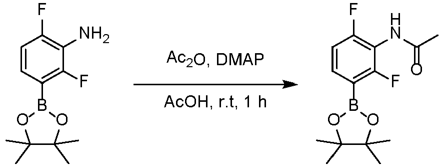

In acetic acid (15.4 equiv.), 2,6-difluoro-3-(4,4,5,5-tetramethyl-1,3,2-dioxaborolan-2-yl)aniline (1.0 equiv.) and acetic anhydride (2.81 equiv.) were dissolved, DMAP (0.02 equiv.) was added, and the mixture was stirred at room temperature for 1 hour. The reaction mixture was concentrated to obtain the target compound as an ivory solid, which was used in the next reaction without further purification (yield: 100%, MS (ESI): m/z 298 [M+1]⁺).

### Example 1. Preparation of N-(3'-((6-((1-acryloylpiperidin-4-yl)oxy)-7-methoxyquinazolin-4-yl)amino)-2,4-difluoro-4'-methoxy-[1,1'-biphenyl]-3-yl)acetamide

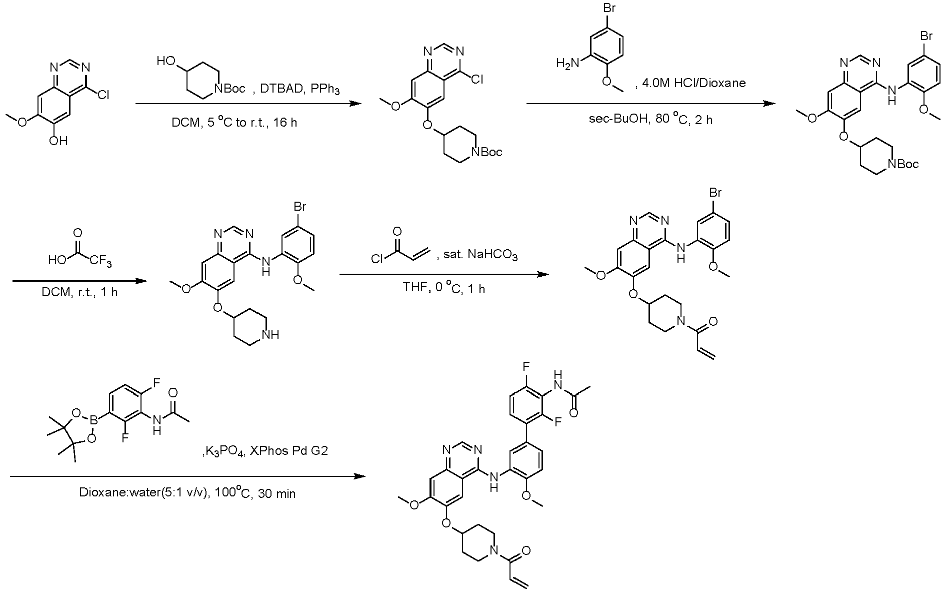

### [Step-1] Preparation of tert-butyl 4-((4-chloro-7-methoxyquinazolin-6-yl)oxy)piperidine-1-carboxylate

In DCM, 4-chloro-7-methoxyquinazolin-6-ol (1.0 equiv.), tert-butyl 4-hydroxypiperidine-1-carboxylate (2.0 equiv.), and triphenylphosphine (1.5 equiv.) were dissolved. Then, DTBAD (1.5 equiv.) was slowly added at 5 °C, followed by stirring at room temperature for 16 hours. The reaction mixture was concentrated and purified by MPLC (DCM:MeOH) to obtain the target compound as a white solid (yield: 52%, MS (ESI): m/z 394 [M+1]⁺).

### [Step-2] Preparation of tert-butyl 4-((4-((5-bromo-2-methoxyphenyl)amino)-7-methoxyquinazolin-6-yl)oxy)piperidine-1-carboxylate

In sec-BuOH, tert-butyl 4-((4-chloro-7-methoxyquinazolin-6-yl)oxy)piperidine-1-carboxylate (1.0 equiv.) obtained in Step-1 above and 5-bromo-2-methoxyaniline (1.2 equiv.) were dissolved. To the reaction mixture, 4.0M HCl dissolved in dioxane was added, and stirred at 80 °C for 2 hours. The reaction mixture was cooled to room temperature, and ethyl ether was then added to produce a solid. The solid was filtered through a filter to obtain the target compound as an ivory solid (yield: 96%, MS (ESI): m/z 559 [M+1]⁺).

### [Step-3] Preparation of N-(5-bromo-2-methoxyphenyl)-7-methoxy-6-(piperidin-4-yloxy)quinazolin-4-amine

In DCM, tert-butyl 4-((4-((5-bromo-2-methoxyphenyl)amino)-7-methoxyquinazolin-6-yl)oxy)piperidine-1-carboxylate (1.0 equiv.) obtained in Step-2 above was dissolved, and then trifluoroacetic acid (50 equiv.) was added and stirred at room temperature for 1 hour. The reaction mixture was concentrated and purified by MPLC (DCM:MeOH) to obtain the target compound as a pale yellow solid (yield: 97%, MS (ESI): m/z 459 [M+1]⁺).

### [Step-4] Preparation of 1-(4-((4-((5-bromo-2-methoxyphenyl)amino)-7-methoxyquinazolin-6-yl)oxy)piperidin-1-yl)prop-2-en-1-one

In THF, N-(5-bromo-2-methoxyphenyl)-7-methoxy-6-(piperidin-4-yloxy)quinazolin-4-amine (1.0 equiv.) obtained in Step-3 above and saturated NaHCO₃ aqueous solution (5.0 equiv.) were dissolved, and then acryloyl chloride (1.0 equiv.) was added at 0 °C and stirred for 1 hour. The reaction mixture was concentrated, water was added thereto, and organic materials were extracted with DCM. The collected organic layer was concentrated by removing remaining water using MgSO₄. The reaction mixture was purified by MPLC (DCM:MeOH) to obtain the target compound as a white solid (yield: 60%, MS (ESI) : m/z 513 [M+1]⁺).

### [Step-5] Preparation of N-(3'-((6-((1-acryloylpiperidin-4-yl)oxy)-7-methoxyquinazolin-4-yl)amino)-2,4-difluoro-4'-methoxy-[1,1'-biphenyl]-3-yl)acetamide

In dioxane:water (5:1), 1-(4-((4-((5-bromo-2-methoxyphenyl)amino)-7-methoxyquinazolin-6-yl)oxy)piperidin-1-yl)prop-2-en-1-one (1.0 equiv.) obtained in Step-4 above, N-(2,6-difluoro-3-(4,4,5,5-tetramethyl-1,3,2-dioxaborolan-2-yl)phenyl)acetamide (2.3 equiv.) obtained in Preparation Example 1, and K₃PO₄ (3.5 equiv.) were dissolved. The reaction mixture was degassed using nitro gas, and Xphos Pd G2 (0.1 equiv.) was added at 80 °C, followed by stirring at 100 °C for 30 minutes. The reaction product was filtered through a celite filter, and concentrated. The reaction mixture was purified by prep-HPLC to obtain the target compound as a yellow solid (yield: 96%, MS (ESI): m/z 604 [M+1]⁺).

### Preparation of Compounds of Examples 2 to 231

Compounds of Examples 2 to 231 according to the present invention were prepared in a similar manner to Example 1 above. Chemical structures, compound names, ¹H NMR, MS, HPLC data and yields of respective Examples are summarized in Table 1 below.

**[Table 1]**

| Exa mpl e No. | Structure | Compound Name | ¹H NMR; MS | Yie ld (%) | HPLC r.t.(min ) Purity(% ) (method) |
|---|---|---|---|---|---|
| 1 | 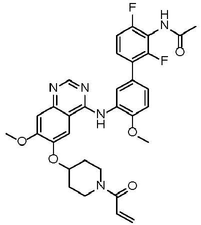 | N-(3'-((6-((1-acryloylpiper idin-4-yl)oxy)-7-methoxyquinaz olin-4-yl)amino)-2,4-difluoro-4'-methoxy-[1,1'-biphenyl]-3-yl)acetamide | ¹H NMR (400 MHz, TFA salt, Methanol-*d*₄) δ 8.56 (d, *J* = 3.2 Hz, 1H), 8.02 (s, 1H), 7.70 (s, 1H), 7.58 (d, *J* = 8.6 Hz, 1H), 7.47-7.39 (m, 1H), 7.29 (d, *J* = 8.7 Hz, 1H), 7.23 (s, 1H), 7.13 (t, *J* = 8.9 Hz, 1H), 6.82 (dd, *J* = 16.8, 10.7 Hz, 1H), 6.23 (dd, *J* = 16.8, 1.9 Hz, 1H), 5.77 (dd, *J* = 10.7, 1.9 Hz, 1H), 4.90-4.86 (m, 1H), 4.08 (s, 3H), 3.99-3.93 (m, 2H), 3.91 (s, 3H), 3.71-3.61 (m, 2H), 2.19 (s, 3H), 2.17-2.05 (m, 2H), 1.98-1.84 (m, 2H); MS (ESI): m/z=501 [M+1] ⁺ | 96 | 8.84 97 (b) |
| 2 | 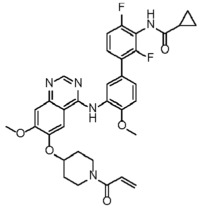 | N-(3'-((6-((1-acryloylpiper idin-4-yl)oxy)-7-methoxyquinaz olin-4-yl)amino)- 2,4-difluoro-4'-methoxy-[1,1'-biphenyl]-3-yl)cyclopropa ncarboxamide | ¹H NMR (400 MHz, FA salt, Methanol-*d*₄) δ 8.34 (s, 1H), 8.19 (s, 1H), 7.85 (s, 1H), 7.81 (s, 1H), 7.49-7.41 (m, 2H), 7.24-7.20 (t, 2H), 7.13-7.08 (t, 1H), 6.85-6.78 (m, 1H), 6.24-6.19 (dd, *J* = 16.81 Hz, 1H), 5.77-5.74 (dd, *J* = 10.66 Hz, 1H), 4.02 (s, 3H), 3.98-3.93 (m, 2H), 3.91 (s, 3H), 3.71-3.61 (m, 2H), 2.13-2.05 (m, 2H), 1.96-1.84 (m, 3H), 0.99-0.96 (m, 2H), 0.92-0.90 (m, 2H); MS (ESI): m/z=630 [M+1] ⁺ | 88 | 9.50 99 (b) |
| 3 | 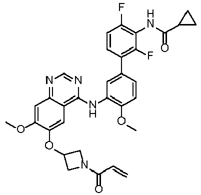 | N-(3'-((6-((1-acryloylazeti din-3-yl)oxy)-7-methoxyquinaz olin-4-yl)amino)-2,4-difluoro-4'-methoxy-[1,1'-biphenyl]-3-yl)cyclopropa ncarboxamide | ¹H NMR (400MHz, FA salt, Methanol-*d*₄) δ 9.30 (s, 1H), 8.64 (s, 1H), 7.78 (s, 1H), 7.59-7.50 (m, 2H) 7.32-7.27 (m, 2H), 7.09-7.03 (m, 2H), 6.99-6.93 (m, 1H), 6.87 (d, *J* = 15.2 Hz, 1H), 4.18 (s, 3H), 4.14-4.08 (m, 1H), 3.93 (s, 3H), 3.84-3.78 (m, 1H), 3.28-3.23 (m, 2H), 2.49-2.44 (m, 1H), 2.31-2.07 (m, 3H); MS (ESI): m/z 602 [M+1]⁺ | 8 | 9.19 100 (b) |
| 4 | 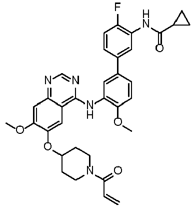 | N-(3'-((6-((1-acryloylpiper idin-4-yl)oxy)-7-methoxyquinaz olin-4-yl)amino)-4-fluoro-4'-methoxy-[1,1'-biphenyl]-3-yl)cyclopropa ncarboxamide | ¹H NMR (400 MHz, FA salt, Methanol-*d₄*) δ 8.31 (s, 1H), 8.20 (dd, *J* = 7.4, 2.0 Hz, 1H), 7.88-7.83 (m, 2H), 7.53 (dd, *J* = 8.6, 2.3 Hz, 1H), 7.41-7.36 (m, 1H), 7.24-7.18 (m, 3H), 6.86-6.78 (m, 1H), 6.24-6.18 (m, 1H), 5.76 (dd, *J* = 10.6, 1.9 Hz, 1H), 4.86-4.83 (m, 1H), 4.02 (s, 3H), 3.98-3.93 (m, 2H), 3.90 (s, 3H), 3.71-3.61 (m, 2H), 2.14-2.04 (m, 2H), 1. 93-1. 84 (m, 3H), 0.99-0.95 (m, 2H), 0.91-0.86 (m, 2H); MS (ESI): m/z=612 [M+1]⁺ | 30 | 9.91 99 (b) |
| 5 | 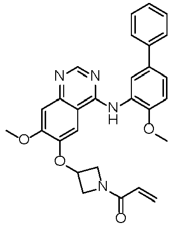 | 1-(3-((7-methoxy-4-((4-methoxy-[1,1'-biphenyl]-3-yl)amino)quin azolin-6-yl)oxy)azetid in-1-yl)prop-2-en-1-one | ¹H NMR (400MHz, free base, Methanol-*d*₄) δ 8.36 (s, 1H), 8.03 (d, *J* = 2.0 Hz, 1H), 7.63-7.61 (m, 2H), 7.53 (d, *J* = 8.8 Hz, 1H), 7.44-7.36 (m, 3H), 7.32-7.28 (m, 1H), 7.18 (s, 1H), 7.16 (s, 1H), 6.41-6.26 (m, 2H), 5.78 (d, *J* = 7.6 Hz, 1H), 5.22-5.19 (m, 1H), 4.78-4.76 (m, 1H), 4.57-4.55 (m, 1H), 4.41-4.39 (m, 1H). 4.17-3.91 (m, 1H), 4.01 (s, 3H), 3.91 (s, 3H); MS (ESI): m/z 483 [M+1]⁺ | 52 | 5.99 100 (a) |
| 6 | 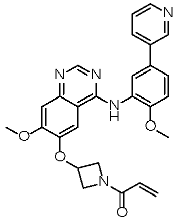 | 1-(3-((7-methoxy-4-((2-methoxy-5-(pyridin-3-yl)phenyl)ami no)quinazolin -6-yl)oxy)azetid in-1-yl)prop-2-en-1-one | ¹H NMR (400MHz, TFA salt, Methanol-*d*₄) δ 8.84 (d, *J* = 1.6 Hz, 1H), 8.51 (d, *J* = 4.8 Hz, 1H), 8.40 (s, 1H), 8.14-8.12 (m, 1H), 8.09 (d, *J* = 2.4 Hz, 1H), 7.65-7.62 (m, 1H), 7.55-7.52 (m, 1H), 7.49 (s, 1H), 7.31 (d, *J* = 8.8 Hz, 1H), 7.25 (s, 1H), 6.43-6.39 (m, 1H), 6.34-6.28 (m 1H), 5.81-5.78 (m, 1H), 5.32-5.27 (m, 1H), 4.86-4.85 (m, 1H), 4.68-4.61 (m, 1H), 4.59-4.53 (m, 1H), 4.23-4.19 (m, 1H), 4.06 (s, 3H), 3.96 (s, 3H); MS (ESI): m/z 484 [M+1]⁺ | 37 | 100* |
| 7 | 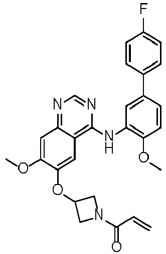 | 1-(3-((4-((4'-fluoro-4-methoxy-[1,1'-biphenyl]-3-yl)amino)-7-methoxyquinaz olin-6-yl)oxy)azetid in-1-yl)prop-2-en-1-one | ¹H NMR (400 MHz, free base, Methanol-*d*₄) δ 8.61 (s, 1H), 7.76 (d, *J* = 2.4 Hz, 1H), 7.69-7.60 (m, 4H), 7.29-7.26 (m, 2H), 7.20-7.14 (m, 2H), 6.43-6.27 (m, 2H), 5.80-5.77 (m, 1H), 5.29 (s, 1H), 4.65-4.60 (m, 1H), 4.47-4.45 (m, 1H), 4.18-4.11 (m, 4H), 3.91 (s, 3H); MS (ESI): m/z=508 [M+1]⁺ | 78 | 6.08 99 (a) |
| 8 | 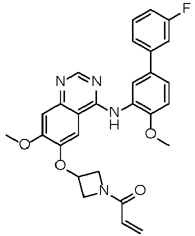 | 1-(3-((4-((3'-fluoro-4-methoxy-[1,1'-biphenyl]-3-yl)amino)-7-methoxyquinaz olin-6-yl)oxy)azetid in-1-yl)prop-2-en-1-one | ¹H NMR (400MHz, TFA salt, Methanol-*d*₄) δ 8.38 (s, 1H), 8.01-8.00 (m, 1H), 7.60-7.57 (m, 1H), 7.48-7.40 (m, 3H), 7.39-7.36 (m, 1H), 7.25-7.24 (m, 2H), 7.06-7.00 (m, 1H), 6.45-6.38 (m, 1H), 6.33-6.28 (m, 1H), 5.81-5.79 (m, 1H), 5.34-5.28 (m, 1H), 4.61-4.60 (m, 2H), 4.49-4.43 (m, 1H), 4.22-4.18 (m, 1H), 4.05 (s, 3H), 3.94 (s, 3H); MS (ESI): m/z 501 [M+1]⁺ | 78 | 6.06 100 (a) |
| 9 | 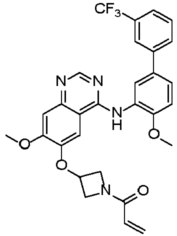 | 1-(3-((7-methoxy-4-((4-methoxy-3'-(trifluoromet hyl)-[1,1'-biphenyl]-3-yl)amino)quin azolin-6-yl)oxy)azetid in-1-yl)prop-2-en-1-one | ¹H NMR (400MHz, TFA salt, Methanol-*d*₄) δ 8.37 (s, 1H), 8.06 (d, *J* = 2.4 Hz, 1H), 7.90 (s, 2H), 7.64-7.58 (m, 3H), 7.44 (s, 1H), 7.26-7.22 (m, 2H), 6.44-6.37 (m, 1H), 6.32-6.27 (m, 1H), 5.80-5.77 (m, 1H), 5.27-5.24 (m, 1H), 4.84-4.81 (m, 1H), 4.63-4.59 (m, 1H), 4.45-4.42 (m, 1H), 4.19-4.15 (m, 1H), 4.04 (s, 3H), 3.95 (s, 3H); MS (ESI): m/z 551 [M+1] ⁺ | 19 | 6.69 100 (a) |
| 10 | 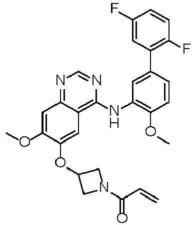 | 1-(3-((4-((2',5'-difluoro-4-methoxy-[1,1'-biphenyl]-3-yl)amino)-7-methoxyquinaz olin-6-yl)oxy)azetid in-1-yl)prop-2-en-1-one | ¹H NMR (400 MHz, TFA salt, Methanol-*d*₄) δ 8.34 (s, 1H), 7.90 (tr, *J* = 2 Hz, 1H), 7.52-7.49 (m, 1H), 7.46 (s, 1H), 7.30-7.24 (m, 1H), 7.22-7.17 (m, 3H), 7.10-7.04 (m, 1H), 6.44-6.37 (m, 1H), 6.31-6.25 (m, 1H), 5.79-5.76 (m, 1H), 5.29-5.24 (m, 1H), 4.63-4.59 (m, 3H), 4.45-4.41 (m, 1H), 4.18-4.15 (m, 1H), 4.03 (s, 3H), 3.93 (s, 3H); MS (ESI): m/z=519 [M+1] ⁺ | 47 | 6.16 98 (a) |
| 11 | 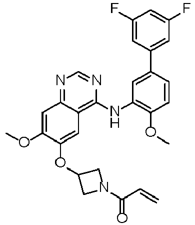 | 1-(3-((4-((3',5'-difluoro-4-methoxy-[1,1'-biphenyl]-3-yl)amino)-7-methoxyquinaz olin-6-yl)oxy)azetid in-1-yl)prop-2-en-1-one | ¹H NMR (400 MHz, free base, DMSO-*d*₆) δ 9.60 (br, 1H), 8.41 (s, 1H), 7.90 (d, *J* = 2.4 Hz, 1H), 7.74-7.71 (m, 1H), 7.58 (s, 1H), 7.47-7.41 (m, 2H), 7.27-7.24 (m, 2H), 7.20-7.14 (m, 1H), 6.41-6.34 (m, 1H), 6.16-6.12 (m, 1H), 5.23-5.18 (m, 1H), 4.82-4.78 (m, 1H), 4.59-4.55 (m, 1H), 4.32-4.28 (m, 1H), 3.97-3.94 (m, 4H), 3.85 (s, 3H), 1.23 (s, 2H); MS (ESI): m/z=519 [M+1]⁺ | 31 | 6.37 99 (a) |
| 12 | 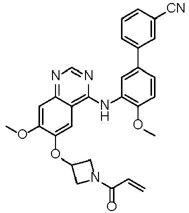 | 3'-((6-((1-acryloylazeti din-3-yl)oxy)-7-methoxyquinaz olin-4-yl)amino)-4'-methoxy-[1,1'-biphenyl]-3-carbonitrile | ¹H NMR (400MHz, free base, Methanol-*d*₄) δ 9.07-9.06 (m, 1H), 8.78 (s, 1H), 7.94-7.87 (m, 3H), 7.63-7.55 (m, 2H), 7.33 (s, 1H), 7.30-7.26 (m, 1H), 7.08-7.05 (m, 1H), 6.89 (s, 1H), 6.42-6.38 (m, 1H), 6.28-6.21 (m, 1H), 5.76-5.73 (m, 1H), 5.19-5.18 (m, 1H), 4.72-4.50 (m, 3H), 4.34-4.28 (m, 1H), 4.08 (s, 3H), 4.04 (s, 3H); MS (ESI): m/z 508 [M+1]⁺ | 21 | 5.71 100 (a) |
| 13 | 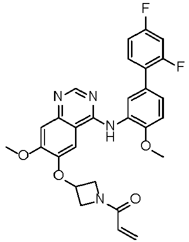 | 1-(3-((4-((2'4'-difluoro-4-methoxy-[1,1'-biphenyl]-3-yl)amino)-7-methoxyquinaz olin-6-yl)oxy)azetid in-1-yl)prop-2-en-1-one | ¹H NMR (400MHz, TFA salt, CDCl₃) δ 8.88 (s, 1H), 8.72 (s, 1H), 7.89 (s, 1H), 7.49-7.45 (m, 1H), 7.32 (s, 1H), 7.26-7.22 (m, 1H), 7.05-6.88 (m, 4H), 6.41-6.37 (m, 1H), 6.27-6.20 (m, 1H), 5.75-5.72 (m, 1H), 5.17-5.15 (m, 1H), 4.56-4.48 (m, 3H), 4.32-4.30 (m, 1H), 4.05-4.00 (m, 6H); MS (ESI): m/z 519 [M+1]⁺ | 23 | 6.20 100 (a) |
| 14 | 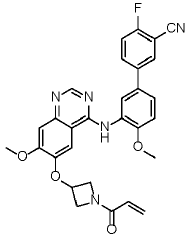 | 3'-((6-((1-acryloylazeti din-3-yl)oxy)-7-methoxyquinaz olin-4-yl)amino)-4-fluoro-4'-methoxy-[1,1'-biphenyl]-3-carbonitrile | ¹H NMR (400MHz, free base, Methanol-*d*₄) δ 8.38 (s, 1H), 8.05-7.99 (m, 3H), 7.60-7.57 (m, 1H), 7.49-7.43 (m, 2H), 7.28 (s, 1H), 7.25 (s, 1H), 6.46-6.39 (m, 1H), 6.33-6.28 (m, 1H), 5.81-5.78 (m, 1H), 5.29-5.28 (m, 1H), 4.61-4.60 (m, 2H), 4.51-4.49 (m, 1H), 4.25-4.24 (m, 1H), 4.05 (s, 3H), 3.96 (s, 3H); MS (ESI): m/z 526 [M+1]⁺ | 35 | 5.92 91 (a) |
| 15 | 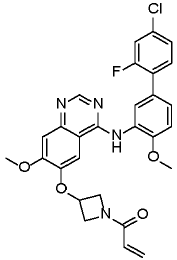 | 1-(3-((4-((4'-chloro-2'-fluoro-4-methoxy-[1,1'-biphenyl]-3-yl)amino)-7-methoxyquinaz olin-6-yl)oxy)azetid in-1-yl)prop-2-en-1-one | ¹H NMR (400MHz, TFA salt, CDCl₃) δ 8.92-8.91 (m, 1H), 8.72 (s, 1H), 8.02-7.88 (m, 1H), 7.48-7.46 (m, 1H), 7.44 (s, 1H), 7.31-7.21 (m, 3H), 7.18-7.03 (m, 1H), 6.88 (s, 1H), 6.42-6.37 (m, 1H), 6.28-6.21 (m, 1H), 5.75-5.73 (m, 1H), 5.17-5.16 (m, 1H), 4.69-4.48 (m, 3H), 4.27-4.33 (m, 1H), 4.06 (s, 3H), 4.04 (s, 3H); MS (ESI): m/z 535 [M+1]⁺ | 24 | 6.73 100 (a) |
| 16 | 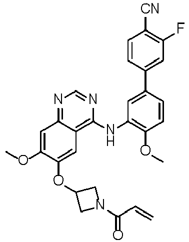 | 3'-((6-((1-acryloylazeti din-3-yl)oxy)-7-methoxyquinaz olin-4-yl)amino)-3-fluoro-4'-methoxy-[1,1'-biphenyl]-4-carbonitrile | ¹H NMR (400 MHz, free base, Methanol-*d*₄) δ 8.62 (s, 1H), 7.94 (d, *J* = 2.4 Hz, 1H), 7.83-7.80 (m, 2H), 7.68-7.65 (m, 3H), 7.34 (d, *J* = 8.8 Hz, 1H), 7.27 (s, 1H), 6.43-6.36 (m, 1H), 6.32-6.27 (m, 1H), 5.80-5.77 (m, 1H), 5.30-5.28 (m, 1H), 4.65-4.60 (m, 1H), 4.48-4.45 (m, 1H), 4.19-4.11 (m, 4H), 3.94 (s, 3H); MS (ESI): m/z=526 [M+1]⁺ | 24 | 5.92 95 (a) |
| 17 | 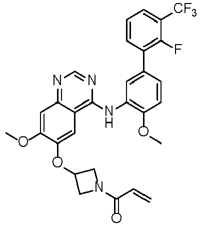 | 1-(3-((4-((2'-fluoro-4-methoxy-3'-(trifluoromet hyl)-[1,1'-biphenyl]-3-yl)amino)-7-methoxyquinaz olin-6-yl)oxy)azetid in-1-yl)prop-2-en-1-one | ¹H NMR (400MHz, free base, Methanol-*d*₄) δ 8.36 (s, 1H), 7.94 (s, 1H), 7.83-7.82 (m, 1H), 7.67-7.66 (m, 1H), 7.54-7.51 (m, 1H), 7.48-7.44 (m, 2H), 7.29-7.24 (m, 2H), 6.45-6.39 (m, 1H), 6.33-6.28 (m, 1H), 5.81-5.78 (m, 1H), 5.28-5.27 (m, 1H), 4.85-4.83 (m, 1H), 4.62-4.60 (m, 1H), 4.47-4.62 (m, 1H), 4.20-4.19 (m, 1H), 4.09 (s, 3H), 4.01 (s, 3H); MS (ESI): m/z 569 [M+1]⁺ | 39 | 6.71 100 (a) |
| 18 | 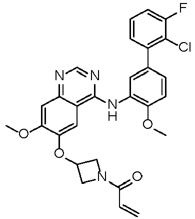 | 1-(3-((4-((2'-chloro-3'-fluoro-4-methoxy-[1,1'-biphenyl]-3-yl)amino)-7-methoxyquinaz olin-6-yl)oxy)azetid in-1-yl)prop-2-en-1-one | ¹H NMR (400MHz, FA salt, Methanol-*d*₄) δ 8.35 (s, 1H), 7.83 (d, *J* = 2.0 Hz, 1H), 7.48 (s, 1H), 7.42-7.39 (m, 2H), 7.30-7.22 (m, 4H), 6.45-6.38 (m, 1H), 6.32-6.28 (m, 1H), 5.81-5.77 (m, 1H), 5.30-5.27 (m, 1H), 4.61-4.60 (m, 2H), 4.47-4.43 (m, 1H), 4.20-4.16 (m, 1H), 4.04 (s, 3H), 3.95 (s, 3H), 2.05-2.03 (m, 1H), 1. 70-1. 68 (m, 1H); MS (ESI): m/z 535 [M+1] ⁺ | 39 | 6.34 100 (a) |
| 19 | 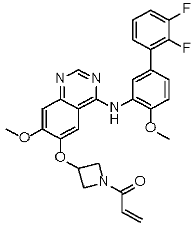 | 1-(3-((4-((2',3'-difluoro-4-methoxy-[1,1'-biphenyl]-3-yl)amino)-7-methoxyquinaz olin-6-yl)oxy)azetid in-1-yl)prop-2-en-1-one | ¹H NMR (400MHz, free base, CDCl₃) δ 8.94 (s, 1H), 8.73 (s, 1H), 7.92 (s, 1H), 7.31-7.26 (m, 3H), 7.16-7.13 (m, 2H), 7.12-7.04 (m, 1H), 6.89 (s, 1H), 6.42-6.37 (m, 1H), 6.28-6.21 (m, 1H), 5.76-5.73 (m, 1H), 5.20-5.17 (m, 1H), 4.62-4.38 (m, 3H), 4.26-4.21 (m, 1H), 4.06 (s, 3H), 4.03 (s, 3H); MS (ESI): m/z 519 [M+1] ⁺ | 39 | 6.11 100 (a) |
| 20 | 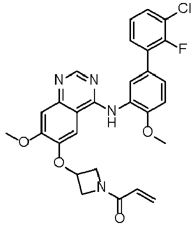 | 1-(3-((4-((3'-chloro-2'-fluoro-4-methoxy-[1,1'-biphenyl]-3-yl)amino)-7-methoxyquinaz olin-6-yl)oxy)azetid in-1-yl)prop-2-en-1-one | ¹H NMR (400 MHz, Free base, Methanol-*d*₄) δ 8.34 (s, 1H), 7.89 (S, 1H), 7.50-7.41 (m, 4H), 7.25-7.21 (m, 3H), 6.43-6.37 (m, 1H), 6.31-6.26 (m, 1H), 5.79-5.76 (m, 1H), 5.29-5.25 (m, 1H), 4.62-4.58 (m, 2H), 4.45-4.42 (m, 1H), 4.18-4.13 (m, 1H), 4.08-4.03 (m, 3H), 3.93 (s, 3H), 2.03-2.02 (m, 1H), 1.62-160 (m, 1H), 0.92-0.88 (m, 2H); MS (ESI): m/z=535 [M+1] ⁺ | 3 | 6.42 96 (a) |
| 21 | 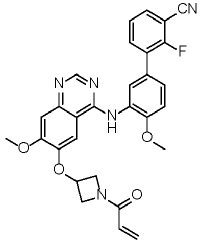 | 3'-((6-((1-acryloylazeti din-3-yl)oxy)-7-methoxyquinaz olin-4-yl)amino)-2-fluoro-4'-methoxy-[1,1'-biphenyl]-3-carbonitrile | ¹H NMR (400MHz, free base, DMSO-*d*₆) δ 9.26 (s, 1H), 8.32 (s, 1H), 7.93-7.88 (m, 2H), 7.75 (s, 1H), 7.53-7.47 (m, 3H), 7.30 (d, *J* = 8.4, 1H), 7.23 (s, 1H), 6.39-6.35 (m, 1H), 6.16-6.12 (m, 1H), 5.75-5.69 (m, 1H), 5.23-5.21 (m, 1H), 4.99-4.98 (m, 1H) 4.79-4.78 (m, 1H), 4.57-4.55 (m, 1H), 4.02-4.00 (m, 1H), 3.96 (s, 3H), 3.86 (s, 3H); MS (ESI): m/z 524 [M+1]⁺ | 22 | 5.78 93 (a) |
| 22 | 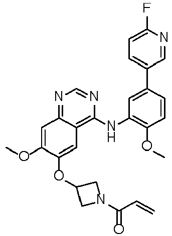 | 1-(3-((4-((5-(6-fluoropyridin -3-yl)-2-methoxyphenyl )amino)-7-methoxyquinaz olin-6-yl)oxy)azetid in-1-yl)prop-2-en-1-one | ¹H NMR (400 MHz, FA salt, CDCl₃) δ 9.01 (s, 1H), 8.74 (s, 1H), 8.47 (s, 1H), 8.05-8.01 (m, 2H), 7.36 (s, 1H), 7.07-7.00 (m, 2H), 6.89 (s, 1H), 6.40 (d, *J* = 19.2, 1H), 6.27-6.21 (m, 1H), 5.74 (d, *J* = 10 Hz, 1H), 5.18-5.17 (m, 1H), 4.69-4.65 (m, 1H), 4.59-4.50 (m, 2H), 4.31-4.29 (m, 1H), 4.06 (s, 3H), 4.04 (s, 3H), 3.49 (s, 1H), 2.00 (s, 3H), 1.25 (s, 1H); MS (ESI): m/z=519 [M+1]⁺ | 14 | 5.10 99 (a) |
| 23 | 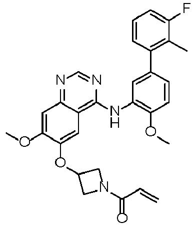 | 1-(3-((4-((3'-fluoro-4-methoxy-2'-methyl-[1,1'-biphenyl]-3-yl)amino)-7-methoxyquinaz olin-6-yl)oxy)azetid in-1-yl)prop-2-en-1-one | ¹H NMR (400MHz, free base, *Methanol-d*₄) 8.36 (s, 1H), 7.71 (s, 1H), 7.27 (s, 1H), 7.26-7.21 (m, 4H), 7.13-7.02 (m, 2H), 6.46-6.41 (m, 1H), 6.39-6.29 (m, 1H), 5.81-5.78 (m, 1H), 5.32-5.31 (m, 1H), 4.85-4.83 (m, 1H), 4.62-4.61 (m, 1H), 4.49-4.48 (m, 1H), 4.25-4.23 (m, 1H), 4.05 (s, 3H), 3.95 (s, 3H), 2.27 (s, 3H); MS (ESI): m/z 515 [M+1]⁺ | 33 | 4.46 100 (a) |
| 24 | 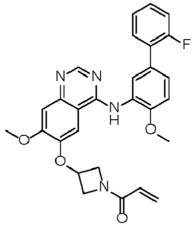 | 1-(3-((4-((2'-fluoro-4-methoxy-[1,1'-biphenyl]-3-yl)amino)-7-methoxyquinaz olin-6-yl)oxy)azetid in-1-yl)prop-2-en-1-one | ¹H NMR (400 MHz, Free base, CDCl₃) δ 8.88 (s, 1H), 8.71 (s, 1H), 7.95 (s, 1H), 7.54-7.49 (m, 1H), 7.33-7.13 (m, 5H), 7.04 (d, *J* = 8.4 Hz, 1H), 6.90 (s, 1H), 6.41-6.36 (m, 1H), 6.27-6.20 (m, 1H), 5.75-5.72 (m, 1H), 5.19-5.13 (m, 1H), 4.67-4.63 (m, 1H), 4.59-4.54 (m, 1H), 4.50-4.47 (m, 1H), 4.32-4.28 (m, 1H), 2.01-2.00 (m, 1H), 1. 70-1. 60 (m, 4H), 1.43 (s, 1H), 1.22-1.10 (m, 3H), 0.89-0.83 (m, 4H); MS (ESI): m/z=501 [M+1]⁺ | 5 | 6.09 95 (a) |
| 25 | 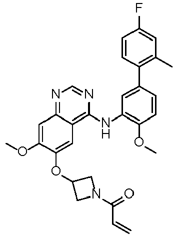 | 1-(3-((4-((4'-fluoro-4-methoxy-2'-methyl-[1,1'-biphenyl]-3-yl)amino)-7-methoxyquinaz olin-6-yl)oxy)azetid in-1-yl)prop-2-en-1-one | ¹H NMR (400MHz, FA salt, CDCl₃) δ 8.74 (d, *J* = 1.6 Hz, 1H), 8.68 (s, 1H), 7.93 (s, 1H), 7.29 (s, 1H), 7.03-6.89 (m, 5H), 6.43-6.38 (m, 1H), 6.28-6.24 (m, 1H), 5.76-5.74 (m, 1H), 5.20-5.18 (m, 1H), 4.58-4.56 (m, 1H), 4.53-4.50 (m, 2H), 4.34-4.30 (m, 1H), 4.06 (s, 3H), 4.03 (s, 3H), 2.36 (s, 3H); MS (ESI): m/z 515 [M+1]⁺ | 18 | 6. 62 100 (a) |
| 26 | 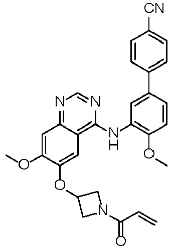 | 3'-((6-((1-acryloylazeti din-3-yl)oxy)-7-methoxyquinaz olin-4-yl)amino)-4'- methoxy-[1,1'-biphenyl]-4-carbonitrile | ¹H NMR (400MHz, TFA salt, DMSO-*d*₆) δ 9.30 (s, 1H), 8.33 (s, 1H), 8.16-8.15 (m, 1H), 8.04-8.01 (m, 1H), 7.91-7.90 (m, 1H), 7.78-7.70 (m, 1H), 7.15-7.10 (m, 1H), 7.69-7.62 (m, 1H), 7.55 (s, 1H), 7.28-7.23 (m, 2H), 6.42-6.35 (m, 1H), 6.17-6.12 (m, 1H), 5.72-5.69 (m, 1H), 5.25-5.18 (m, 1H), 4.82-4.76 (m, 1H), 4.58-4.52 (m, 1H), 4.33-4.28 (m, 1H), 4.01-3.99 (m, 1H), 3.96 (s, 3H), 3.85 (s, 3H); MS (ESI): m/z 508 [M+1]⁺ | 30 | 5.64 100 (a) |
| 27 | 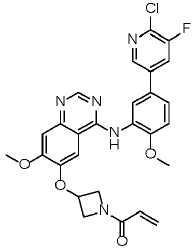 | 1-(3-((4-((5-(6-chloro-5-fluoropyridin -3-yl)-2-methoxyphenyl )amino)-7-methoxyquinaz olin-6-yl)oxy)azetid in-1-yl)prop-2-en-1-one | ¹H NMR (400MHz, TFA salt, DMSO-*d*₆) δ 9.29 (s, 1H), 8.66 (s, 1H), 8.32 (s, 1H), 8.29 (s, 1H), 7.97 (s, 1H), 7.77-7.74 (m, 1H), 7.55 (s, 1H), 7.29 (d, *J* = 8.8 Hz, 1H), 7.23 (s, 1H), 6.41-6.35 (m, 1H), 6.16-6.12 (m, 1H), 5.72-5.69 (m, 1H), 5.22-5.20 (m, 1H), 4.86-4.82 (m, 1H), 4.62-4.59 (m, 1H), 4.33-4.32 (m, 1H), 4.01-3.99 (m, 1H), 3.96 (s, 3H), 3.85 (s, 3H); MS (ESI): m/z 536 [M+1]⁺ | 15 | 5. 60 100 (a) |
| 28 | 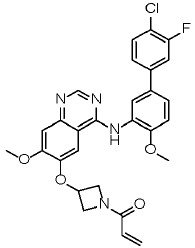 | 1-(3-((4-((4'-chloro-3'-fluoro-4-methoxy-[1,1'-biphenyl]-3-yl)amino)-7-methoxyquinaz olin-6-yl)oxy)azetid in-1-yl)prop-2-en-1-one | ¹H NMR (400MHz, TFA salt, *Methanol-d*₄) δ 8.54 (s, 1H), 7.74-7.73 (m, 1H), 7.65-7.61 (m, 2H), 7.47-7.43 (m, 2H), 7.39-7.38 (m, 1H), 7.36-7.35 (m, 2H), 7.23-7.20 (m, 2H), 6.31-6.28 (m, 1H), 6.24-6.20 (m, 1H), 5.73-5.70 (m, 1H), 5.21-5.20 (m, 1H), 4.58-4.51 (m, 1H), 4.40-4.39 (m, 1H), 4.10-4.01 (m, 2H), 4.00 (s, 3H), 3.84 (s, 3H); MS (ESI): m/z 535 [M+1]⁺ | 10 | 6.75 100 (a) |
| 29 | 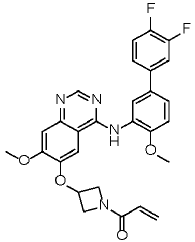 | 1-(3-((4-((3',4'-difluoro-4-methoxy-[1,1'-biphenyl]-3-yl)amino)-7-methoxyquinaz olin-6-yl) oxy) azetid in-1-yl)prop-2-en-1-one | ¹H NMR (400MHz, free base, *Methanol-d*₄) δ 8.38 (s, 1H), 7.99 (s, 1H), 7.57-7.52 (m, 2H), 7.48 (s, 1H), 7.45-7.43 (m, 1H), 7.37-7.32 (m, 1H), 7.25 (s, 1H), 7.23 (s, 1H), 6.45-6.39 (m, 1H), 6.33-6.28 (m, 1H), 5.81-5.78 (m, 1H), 5.29-5.27 (m, 1H), 4.99-4.97 (m, 1H), 4.61-4.60 (m, 1H), 4.46-4.45 (m, 1H), 4.19-4.18 (m, 1H), 4.05 (s, 3H), 3.93 (s, 3H); MS (ESI): m/z 519 [M+1] ⁺ | 25 | 6.33 98 (a) |
| 30 | 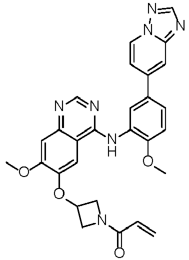 | 1-(3-((4-((5-([1,2,4]triaz olo[1,5-a]pyridin-7-yl)-2-methoxyphenyl )amino)-7-methoxyquinaz olin-6-yl)oxy)azetid in-1-yl)prop-2-en-1-one | ¹H NMR (400MHz, TFA salt, DMSO-*d*₆) δ 9.31 (s, 1H), 8.98 (d, *J* = 7.2 Hz, 1H), 8.50 (s, 1H), 8.34 (s, 1H), 8.12 (d, *J* = 1.2 Hz, 1H), 8.06 (d, *J* = 2.4 Hz, 1H), 7.86-7.83 (m, 1H), 7.59-7.56 (m, 2H), 7.31 (d, *J* = 8.4 Hz, 1H), 7.24 (s, 1H), 6.42-6.35 (m, 1H), 6.17-6.12 (m, 1H), 5.73-5.70 (m, 1H), 5.28-5.22 (m, 1H), 4.84-4.81 (m, 1H), 4.49-4.48 (m, 1H), 4.32-4.30 (m, 1H), 4.08-4.00 (m, 1H), 3.96(s, 3H), 3.87 (s, 3H); MS (ESI): m/z 524 [M+1]⁺ | 53 | 4.04 92 (a) |
| 31 | 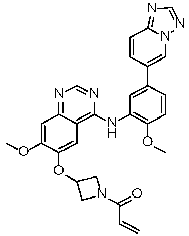 | 1-(3-((4-((5-([1,2,4]triaz olo[1,5-a] pyridin-6-yl)-2-methoxyphenyl )amino)-7-methoxyquinaz olin-6-yl)oxy)azetid in-1-yl)prop-2-en-1-one | ¹H NMR (400MHz, TFA salt, *Methanol-d*₄) δ 9.08 (s, 1H), 8.66 (s, 1H), 8.49 (s, 1H), 8.07 (d, *J* = 8.0 Hz, 1H), 7.95 (d, *J* = 2.4 Hz, 1H), 7.90 (d, *J* = 9.2 Hz, 1H), 7.87-7.81 (m, 1H), 7.73 (s, 1H), 7.38 (d, *J* = 8.8 Hz, 1H), 7.31 (s, 1H), 6.44-6.29 (m, 2H), 5.81-5.79 (m, 1H), 5.31-5.30 (m, 1H), 4.67-4.62 (m, 1H), 4.49-4.46 (m, 1H), 4.18-4.13 (m, 4H), 3.96 (s, 3H); MS (ESI): m/z 524 [M+1] ⁺ | 15 | 4.05 100 (a) |
| 32 | 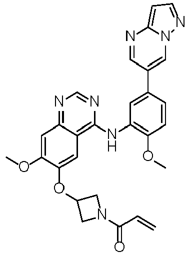 | 1-(3-((7-methoxy-4-((2-methoxy-5-(pyrazolo[1,5 -a]pyrimidin-6-yl)phenyl) ami no)quinazolin -6-yl) oxy) azetid in-1-yl)prop-2-en-1-one | ¹H NMR (400MHz, free base, DMSO-*d*₆) δ 9.41 (s, 1H), 9.33 (s, 1H), 8.95 (s, 1H), 8.33 (s, 1H), 8.24 (d, *J* = 2.4 Hz, 1H), 7.99 (d, *J* = 2.4 Hz, 1H), 7.79-7.76 (m, 1H), 7.56 (s, 1H), 7.30 (d, *J* = 8.8 Hz, 1H), 7.23 (s, 1H), 6.77 (d, *J* = 1.6 Hz, 1H), 6.38-6.35 (m, 1H), 6.17-6.12 (m, 1H), 5.73-5.70 (m, 1H), 5.28-5.25 (m, 1H), 4.88-4.82 (m, 1H), 4.62-4.57 (m, 1H), 4.35-4.32 (m, 1H), 4.09-4.02 (m, 1H), 4.96 (s, 3H), 3.88 (s, 3H); MS (ESI): m/z 524 [M+1]⁺ | 22 | 4.56 97 (a) |
| 33 | 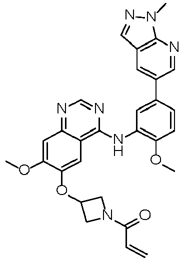 | 1-(3-((7-methoxy-4-((2-methoxy-5-(1-methyl-1H-pyrazolo[3,4-b]pyridin-5-yl)phenyl)ami no)quinazolin -6-yl)oxy)azetid in-1-yl)prop-2-en-1-one | ¹H NMR (400MHz, FA salt, CDCl₃) δ 9.12-9.11 (m, 1H), 9.10-9.09 (m, 1H), 8.88-8.87 (m, 1H), 8.27-8.26 (m, 1H), 8.07 (s, 1H), 7.34-7.31 (m, 2H), 7.11-7.09 (m, 1H), 6.90 (s, 1H), 6.43-6.42 (m, 1H), 6.39-6.38 (m, 1H), 5.77-5.74 (m, 1H), 5.18-5.17 (m, 1H), 4.68-4.66 (m, 1H), 4.65-4.53 (m, 2H), 4.36-4.34 (m, 1H), 4.33 (s, 3H), 4.08 (s, 3H), 4.04 (s, 3H); MS (ESI): m/z 538 [M+1]⁺ | 3 | 4.86 100 (a) |
| 34 | 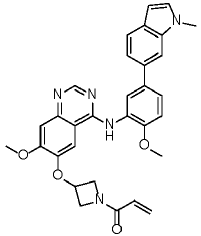 | 1-(3-((7-methoxy-4-((2-methoxy-5-(1-methyl-1H-indol-6-yl)phenyl)ami no)quinazolin -6-yl)oxy)azetid in-1-yl)prop-2-en-1-one | ¹H NMR (400MHz, TFA salt, *Methanol-d*₄) δ 8.63 (s, 1H), 7.86 (s, 1H), 7.80-7.72 (m, 1H), 7.70 (s, 1H), 7.62 (s, 1H), 7.60 (s, 1H), 7.35-7.20 (m, 3H), 7.18 (d, *J* = 2.8 Hz, 1H), 6.46-6.29 (m, 2H), 5.81-5.79 (m, 1H), 5.35-5.28 (m, 1H), 5.11-5.02 (m, 1H), 4.67-4.64 (m, 1H), 4.48-4.46 (m, 1H), 4.18-4.15 (m, 1H), 4.13 (s, 3H), 3.93 (s, 3H), 3.89 (s, 3H); MS (ESI): m/z 536 [M+1]⁺ | 10 | 6.38 100 (a) |
| 35 | 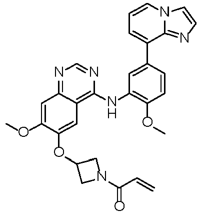 | 1-(3-((4-((5-(imidazo[1,2-a]pyridin-8-yl)-2-methoxyphenyl )amino)-7-methoxyquinaz olin-6-yl)oxy)azetid in-1-yl)prop-2-en-1-one | ¹H NMR (400MHz, free base, *Methanol-d*₄) δ 8.45 (d, *J* = 6.4 Hz, 1H), 8.34 (s, 1H), 8.25 (d, *J* = 2.0 Hz, 1H), 7.94 (S, 1H), 7.89 (d, *J* = 8.2 Hz, 1H), 7.62 (d, *J* = 1.6 Hz, 1H), 7.50 (s, 1H), 7.45-7.43 (m, 1H), 7.29 (d, *J* = 8.4 Hz, 1H), 7.24 (s, 1H), 7.06 (t, *J* = 7.2 Hz, 1H), 6.41-6.38 (m, 1H), 6.33-6.32 (m, 1H), 5.81-5.78 (m, 1H), 5.35-5.29 (m, 1H), 4.62-4.61 (m, 2H), 4.45-4.42 (m, 1H), 4.28-4.22 (m, 1H), 4.05 (s, 3H), 3.96 (s, 3H); MS (ESI): m/z 523 [M+1]⁺ | | 98 52 (a) |
| 36 | 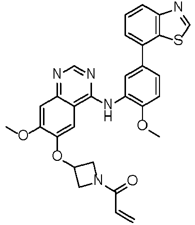 | 1-(3-((4-((5-(benzo[d]thia zol-7-yl)-2-methoxyphenyl )amino)-7-methoxyquinaz olin-6-yl)oxy)azetid in-1-yl)prop-2-en-1-one | ¹H NMR (400MHz, free base, DMSO-*d*₆) δ 9.43 (s, 1H), 9.28 (s, 1H), 8.34 (s, 1H), 8.23 (d, *J* = 8.8 Hz, 1H), 7.94-7.93 (m, 1H), 7.82-7.80 (m, 1H), 7.74-7.71 (m, 1H), 7.60 (s, 1H), 7.27 (d, *J* = 8.4 Hz, 1H), 7.23 (s, 1H), 6.42-6.35 (m, 1H), 6.17-6.12 (m, 1H), 5.72-5.70 (m, 1H), 5.22-5.21 (m, 1H), 4.58-4.57 (m, 1H), 4.31-4.29 (m, 1H), 4.11-4.10 (m, 1H), 3.96 (s, 3H), 3.86 (s, 3H); MS (ESI): m/z 540 [M+1] ⁺ | 14 | 5.48 100 (a) |
| 37 | 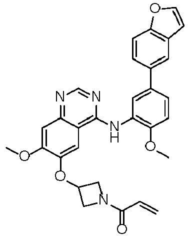 | 1-(3-((4-((5-(benzofuran-5-yl)-2-methoxyphenyl )amino)-7-methoxyquinaz olin-6-yl)oxy)azetid in-1-yl)prop-2-en-1-one | ¹H NMR (400MHz, free base, CDCl₃) δ 8.96 (d, *J* = 2.4 Hz, 1H), 8.74 (s, 1H), 7.82 (s, 1H), 7.65 (d, *J* = 2.4 Hz, 1H), 7.56 (s, 2H), 7.33-7.26 (m, 2H), 7.03 (d, *J* = 8.4 Hz, 1H), 6.87 (s, 1H), 6.83 (d, *J* = 2.0 Hz, 1H), 6.40-6.35 (m, 1H), 6.24-6.17 (m, 1H), 5.74-5.71 (m, 1H), 5.13-5.12 (m, 1H), 4.61-4.52 (m, 2H), 4.47-4.464 (m, 1H), 4.30-4.26 (m, 1H), 4.04 (s, 6H); MS (ESI): m/z 523 [M+1] ⁺ | 69 | 6.16 100 (a) |
| 38 | 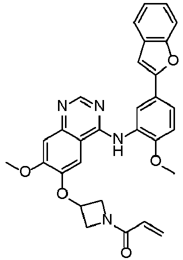 | 1-(3-((4-((5-(benzofuran-2-yl)-2-methoxyphenyl )amino)-7-methoxyquinaz olin-6-yl)oxy)azetid in-1-yl)prop-2-en-1-one | ¹H NMR (400 MHz, TFA salt, *Methanol-d*₄) δ 8.62 (s, 1H), 8.06 (d, *J* = 2.4 Hz 1H), 7.97-7.94 (m, 1H), 7.67 (s, 1H), 7.59-7.57 (m, 1H), 7.49-7.47 (m, 1H), 7.32-7.20 (m, 4H), 7.11 (d, *J* = 0.8 Hz, 1H), 6.43-6.36 (m, 1H), 6.31-6.26 (m, 1H), 5.80-5.77 (m, 1H), 5.30-5.27 (m, 1H), 4.65-4.61 (m, 1H), 4.47-4.44 (m, 1H), 4.17-4.13 (m, 1H) 4.11 (s, 3H), 3.93 (s, 3H); MS (ESI): m/z=523 [M+1]⁺ | 93 | 6.58 98 (a) |
| 39 | 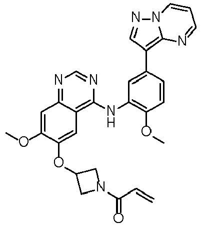 | 1-(3-((7-methoxy-4-((2-methoxy-5-(pyrazolo[1,5 -a]pyrimidin-3-yl)phenyl)ami no)quinazolin -6-yl)oxy)azetid in-1-yl)prop-2-en-1-one | ¹H NMR (400MHz, free base, *Methanol-d*₄) δ 8.93-8.91 (m, 1H), 8.60-8.59 (m, 1H), 8.55 (s, 1H), 8.41 (d, *J* = 2.4 Hz, 1H), 8.34 (s, 1H), 8.07-8.04 (m, 1H), 7.51 (s, 1H), 7.25 (s, 1H), 7.23 (s, 1H), 7.04-7.02 (m, 1H), 6.41-6.38 (m, 1H), 6.32-6.31 (m, 1H), 5.80-5.78 (m, 1H), 5.31-5.28 (m, 1H), 4.72-4.68 (m, 2H), 4.55-4.51 (m, 1H), 4.25-4.19 (m, 1H), 4.06 (s, 3H), 3.92 (s, 3H); MS (ESI): m/z 524 [M+1]⁺ | 13 | 4.62 91 (a) |
| 40 | 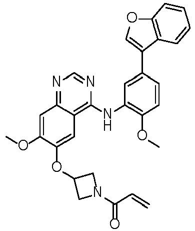 | 1-(3-((4-((5-(benzofuran-3-yl)-2-methoxyphenyl )amino)-7-methoxyquinaz olin-6-yl)oxy)azetid in-1-yl)prop-2-en-1-one | ¹H NMR (400MHz, free base, *Methanol-d*₄) δ 8.41 (s, 1H), 8.13 (d, *J* = 2.0 Hz, 1H), 8.00 (s, 1H), 7.96-7.94 (m, 1H), 7.65-7.62 (m, 1H), 7.57-7.55 (m, 1H), 7.50 (s, 1H), 7.38-7.34 (m, 2H), 7.29 (s, 1H), 7.27 (d, *J* = 4.4 Hz, 1H), 6.43-6.33 (m, 1H), 6.32-6.29 (m, 1H), 5.81-5.78 (m, 1H), 5.31-5.29 (m, 1H), 4.91-4.88 (m, 1H), 4.72-4.69 (m, 1H), 4.48-4.45 (m, 1H), 4.21-4.17 (m, 1H), 4.06 (s, 3H), 3.97 (s, 3H); MS (ESI): m/z 523 [M+1]⁺ | 45 | 6.37 100 (a) |
| 41 | 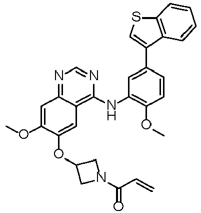 | 1-(3-((4-((5-(benzo[b]thio phen-3-yl)-2-methoxyphenyl )amino)-7-methoxyquinaz olin-6-yl)oxy)azetid in-1-yl)prop-2-en-1-one | ¹H NMR (400MHz, free base, DMSO-*d*₆) δ 9.29 (s, 1H), 8.38 (s, 1H), 8.08 (t, *J* = 8.4 Hz, 2H), 7.82 (d, *J* = 2.0 Hz, 1H), 7.79 (s, 1H), 7.57 (s, 1H), 7.53-7.44 (m, 3H), 7.31 (d, *J* = 8.8 Hz, 1H), 7.29 (s, 1H), 6.42-6.35 (m, 1H), 6.17-6.15 (m, 1H), 5.73-5.70 (m, 1H), 5.28-5.24 (m, 1H), 4.82-4.81 (m, 1H), 4.61-4.50 (m, 1H), 4.32-4.29 (m, 1H), 4.01-4.00 (m, 1H), 3.96 (s, 3H), 3.89 (s, 3H); MS (ESI): m/z 539 [M+1] ⁺ | 37 | 6. 63 100 (a) |
| 42 | 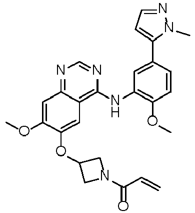 | 1-(3-((7-methoxy-4-((2-methoxy-5-(1-methyl-1H-pyrazol-5-yl)phenyl)ami no)quinazolin -6-yl)oxy)azetid in-1-yl)prop-2-en-1-one | ¹H NMR (400MHz, TFA salt, Methanol-*d*₄) δ 8.39 (s, 1H), 7.94 (d, *J* = 2.0 Hz, 1H), 7.51 (d, *J* = 2.0 Hz, 1H), 7.48 (s, 1H), 7.42-7.40 (m, 1H), 7.28 (s, 1H), 7.26 (d, *J* = 4.0 Hz, 1H), 6.46-6.39 (m, 2H), 6.33-6.28 (m, 1H), 5.81-5.78 (m, 1H), 5.29-5.27 (m, 1H), 4.85-4.83 (m, 1H), 4.62-4.60 (m, 1H), 4.48-4.45 (m, 1H), 4.20-4.19 (m, 1H), 3.97 (s, 3H), 3.34 (s, 3H), 3.32 (s, 3H); MS (ESI): m/z 487 [M+1]⁺ | 48 | 4.28 100 (a) |
| 43 | 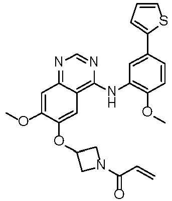 | 1-(3-((7-methoxy-4-((2-methoxy-5-(thiophen-2-yl)phenyl)ami no)quinazolin -6-yl)oxy)azetid in-1-yl)prop-2-en-1-one | ¹H NMR (400MHz, free base, Methanol-*d*₄) δ 8.62 (s, 1H), 7.64-7.61 (m, 1H), 7.35-7.31 (m, 2H), 7.24 (s, 1H), 7.21 (d, *J* = 8.8 Hz, 1H), 7.09-7.07 (m, 1H), 6.68 (s, 1H), 6.32-6.16 (m, 2H), 5.70 (d, *J* = 10.0 Hz, 1H), 4.80-4.68 (m, 2H), 4.53-4.46 (m, 1H), 4.38-4.28 (m, 1H), 4.10-4.02 (m, 1H), 3.99 (s, 3H), 3.77 (s, 3H); MS (ESI): m/z 489 [M+1]⁺ | 29 | 5.94 98 (a) |
| 44 | 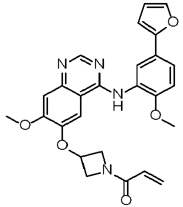 | 1-(3-((4-((5-(furan-2-yl)-2-methoxyphenyl )amino)-7-methoxyquinaz olin-6-yl)oxy)azetid in-1-yl)prop-2-en-1-one | ¹H NMR (400 MHz, FA salt, DMSO-*d*₆) δ 9.22 (s, 1H), 8.49 (s, 1H, FA), 8.33 (s, 1H), 7.81 (d, *J* = 2.2 Hz, 1H), 7.72-7.67 (m, 1H), 7.64-7.57 (m, 1H), 7.52 (s, 1H), 7.26-7.15 (m, 2H), 6.86-6.81 (m, 1H), 6.59-6.53 (m, 1H), 6.38 (dd, *J* = 17.0, 10.3 Hz, 1H), 6.14 (dd, *J* = 17.0, 2.1 Hz, 1H), 5.70 (dd, *J* = 10.3, 2.1 Hz, 1H), 5.24-5.16 (m, 1H), 4.84-4.75 (m, 1H), 4.60-4.51 (m, 1H), 4.33-4.25 (m, 1H), 4.02-3.91 (m, 4H), 3.81 (s, 3H); MS (ESI): m/z 473[M+H]⁺ | 22 | 5.54 98 (a) |
| 45 | 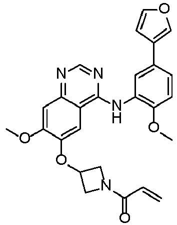 | 1-(3-((4-((5-(furan-3-yl)-2-methoxyphenyl )amino)-7-methoxyquinaz olin-6-yl)oxy)azetid in-1-yl)prop-2-en-1-one | ¹H NMR (400MHz, free base, Methanol-*d*₄) δ 8.45 (s, 1H), 7.75 (s, 1H), 7.62 (s, 1H), 7.52 (s, 1H), 7.49-7.44 (m, 2H), 7.16 (s, 1H), 7.10 (d, *J* = 8.4 Hz, 1H), 6.68 (s, 1H), 6.32-6.16 (m, 2H), 5.70 (d, *J* = 10.0 Hz, 1H), 4.80-4.68 (m, 2H), 4.53-4.46 (m, 1H), 4.38-4.28 (m, 1H), 4.10-4.02 (m, 1H), 3.99 (s, 3H), 3.77 (s, 3H); MS (ESI): m/z 473 [M+1]⁺ | 38 | 5.37 100 (a) |
| 46 | 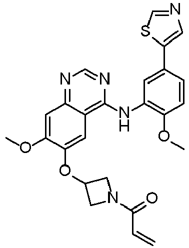 | 1-(3-((7-methoxy-4-((2-methoxy-5-(thiazol-5-yl)phenyl)ami no)quinazolin -6-yl)oxy)azetid in-1-yl)prop-2-en-1-one | ¹H NMR (400MHz, FA salt, CDCl₃) δ 9.10 (d, *J* = 2.4 Hz, 1H), 8.78 (s, 1H), 8.74 (s, 1H), 8.09 (s, 1H), 7.34 (s, 1H), 7.30-7.28 (m, 1H), 7.01-7.00 (m, 1H), 6.88 (s, 1H), 6.42-6.37 (m, 1H), 6.28-6.21 (m, 1H), 5.76-5.74 (m, 1H), 5.19-5.16 (m, 1H), 4.67-4.52 (m, 3H), 4.37-4.36 (m, 1H), 4.06 (s, 3H), 4.04 (s, 3H); MS (ESI): m/z 490 [M+1]⁺ | 17 | 4.32 100 (a) |
| 47 | 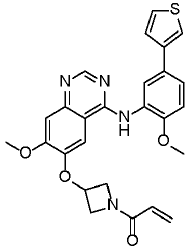 | 1-(3-((7-methoxy-4-((2-methoxy-5-(thiophen-3-yl)phenyl)ami no)quinazolin -6-yl) oxy) azetid in-1-yl)prop-2-en-1-one | ¹H NMR (400 MHz, TFA salt, Methanol-*d*₄) δ 8.62 (s, 1H), 7.82 (d, *J* = 2.4 Hz, 1H), 7.76-7.70 (m, 2H), 7.60-7.59 (m, 1H), 7.52-7.50 (m, 1H), 7.46-7.44 (m, 1H), 7.29-7.24 (m, 2H), 6.44-6.38 (m, 1H), 6.33-6.29 (m, 1H), 5.82-5.79 (m, 1H), 5.30 (s, 1H), 4.66-4.62 (m, 1H), 4.48-4.46 (m, 1H), 4.18-4.09 (m, 4H), 3.91 (s, 3H); MS (ESI): m/z=489 [M+1]⁺ | 17 | 5.80 98 (a) |
| 48 | 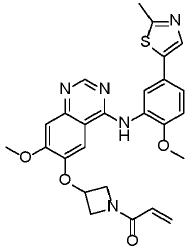 | 1-(3-((7-methoxy-4-((2-methoxy-5-(2-methylthiazol -5-yl))amino)qui nazolin-6-yl)oxy)azetid in-1-yl)prop-2-en-1-one | ¹H NMR (400MHz, FA salt, Methanol-*d*₄) δ 8.50 (s, 1H), 8.39 (s, 1H), 8.02-8.01 (m, 1H), 7.82 (s, 1H), 7.54-7.53 (m, 1H), 7.52-7.51 (m, 1H), 7.47 (s, 1H), 7.25-7.19 (m, 1H), 6.46-6.39 (m, 1H), 6.33-6.28 (m, 1H), 5.81-5.78 (m, 1H), 5.29-5.28 (m, 1H), 4.88-4.87 (m, 1H), 4.20-4.19 (m, 1H), 4.05 (s, 3H), 3.94 (s, 3H); MS (ESI): m/z 504 [M+1]⁺ | 3 | 4.34 100 (a) |
| 49 | 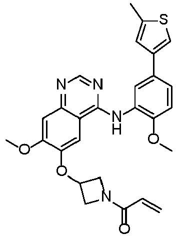 | 1-(3-((7-methoxy-4-((2-methoxy-5-(5-methylthiophe n-3-yl)phenyl)ami no)quinazolin -6-yl)oxy)azetid in-1-yl)prop-2-en-1-one | ¹H NMR (400MHz, free base, Methanol-*d*₄) δ 8.36 (s, 1H), 7.94 (d, *J* = 2.0 Hz, 1H), 7.56-7.54 (m, 1H), 7.46 (s, 1H), 7.28 (d, *J* = 1.6 Hz, 1H), 7.24 (s, 1H), 7.16-7.13 (m, 2H), 6.44-6.32 (m, 1H), 6.31-6.28 (m, 1H), 5.81-5.78 (m, 1H), 5.31-5.26 (m, 1H), 4.92-4.85 (m, 1H), 4.75-4.60 (m, 1H), 4.46-4.42 (m, 1H), 4.21-4.17 (m, 1H), 4.05 (s, 3H), 3.90 (s, 3H), 2.51 (s, 3H); MS (ESI): m/z 503 [M+1]⁺ | 54 | 6.24 98 (a) |
| 50 | 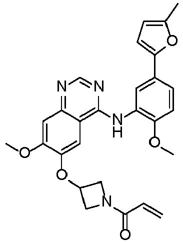 | 1-(3-((7-methoxy-4-((2-methoxy-5-(5-methylfuran-2-yl)phenyl)ami no)quinazolin -6-yl)oxy)azetid in-1-yl)prop-2-en-1-one | ¹H NMR (400 MHz, TFA salt, Methanol-*d*₄) δ 8.60 (s, 1H), 7.75 (d, *J* = 2 Hz, 1H), 7.69-7.66 (m, 2H), 7.27 (s, 1H), 7.20 (d, *J* = 8.4 Hz, 1H), 6.55 (d, *J* = 3.2 Hz, 1H), 6.43-6.36 (m, 1H), 6.31-6.27 (m, 1H), 6.09-6.08 (m, 1H), 5.80-5.77 (m, 1H), 5.28 (s, 1H), 4.64-4.60 (m, 1H), 4.47-4.44 (m, 1H), 4.16-4.11 (m, 4H), 3.87 (s, 3H), 2.33 (d, *J* = 0.8 Hz, 3H); MS (ESI): m/z=501 [M+1]⁺ | 4 | 5.93 90 (a) |
| 51 | 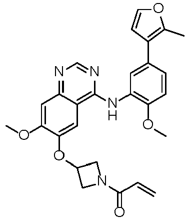 | 1-(3-((7-methoxy-4-((2-methoxy-5-(2-methylfuran-3-yl)phenyl)ami no)quinazolin -6-yl)oxy)azetid in-1-yl)prop-2-en-1-one | ¹H NMR (400 MHz, TFA salt, Methanol-*d*₄) δ 8.55 (s, 1H), 7.64-7.60 (m, 2H), 7.45-7.39 (m, 2H), 7.26-7.21 (m, 2H), 6.55 (d, *J* = 2 Hz, 1H), 6.43-6.36 (m, 1H), 6.32-6.27 (m, 1H), 5.80-5.77 (m, 1H), 5.29-5.25 (m, 1H), 4.64-4.60 (m, 1H), 4.47-4.44 (m, 1H), 4.17-4.13 (m, 1H), 4.09 (s, 3H), 3.89 (s, 3H), 3.60 (s, 2H), 2.44 (s, 3H); MS (ESI): m/z=508 [M+1]⁺ | 30 | 5.79 98 (a) |
| 52 | 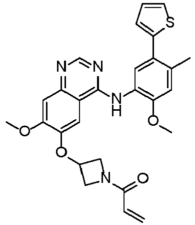 | 1-(3-((7-methoxy-4-((2-methoxy-4-methyl-5-(thiophen-2-yl)phenyl)ami no)quinazolin -6-yl)oxy)azetid in-1-yl)prop-2-en-1-one | ¹H NMR (400MHz, TFA salt, Methanol-*d*₄) δ 8.52 (s, 1H), 7.56 (s, 1H), 7.42 (s, 1H), 7.36-7.34 (m, 1H), 7.17 (s, 1H), 7.05-7.00 (m, 3H), 6.35-6.29 (m, 1H), 6.24-6.19 (m, 1H), 5.73-5.70 (m, 1H), 5.20-5.19 (m, 1H), 4.78-4.76 (m, 1H), 4.54-4.53 (m, 1H), 4.38-4.36 (m, 1H), 4.08-4.07 (m, 1H), 4.02 (s, 3H), 3.81 (s, 3H); MS (ESI): m/z 503 [M+1]⁺ | 16 | 6.19 100 (a) |
| 53 | 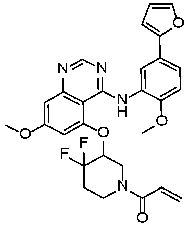 | 1-(4,4-difluoro-3-((4-((5-(furan-2-yl)- 2-methoxyphenyl )amino)-7-methoxyquinaz olin-5-yl)oxy)piperi din-1-yl)prop-2-en-1-one | ¹H NMR (400 MHz, TFA salt, Methanol-*d*₄) δ8.73 (s, 1H), 8.43 (s, 1H), 7.68-7.66 (m, 1H), 7.58 (s, 1H), 7.29 (s, 1H), 7.23 (d, *J* = 8.8 Hz, 1H), 6.91 (s, 1H), 6.72-6.70 (m, 2H), 6.54-6.53 (m, 1H), 6.07-6.03 (m, 1H), 5.67-5.64 (m, 2H), 4.20-4.19 (m, 1H), 4.12-4.11 (m, 4H), 3.98 (s, 3H), 3.89-3.87 (m, 2H), 2.41-2.35 (m, 2H); MS (ESI): m/z 537 [M+H]⁺ | 41 | 11.02 100 (b) |
| 54 | 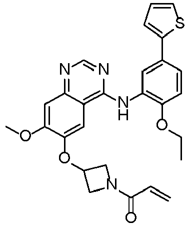 | 1-(3-((4-((2-ethoxy-5-(thiophen-2-yl)phenyl)ami no)-7-methoxyquinaz olin-6-yl)oxy)azetid in-1-yl)prop-2-en-1-one | ¹H NMR (400 MHz, TFA salt, Methanol-*d*₄) δ 8.61 (s, 1H), 7.81 (d, *J* = 2.4 Hz, 1H), 7.67-7.64 (m, 2H), 7.35-7.32 (m, 2H), 7.27 (s, 1H), 7.20 (d, *J* = 8.8 Hz, 1H), 7.09-7.07 (m, 1H), 6.42-6.35 (m, 1H), 6.31-6.27 (m, 1H), 5.80-5.77 (m, 1H), 5.28 (br, 1H), 4.64-4.60 (m, 1H), 4.47-4.44 (m, 1H), 4.19-4.14 (m, 3H), 4.11 (s, 3H), 1. 33-1. 30 (m, 3H); MS (ESI): m/z=508 [M+1]⁺ | 27 | 6.21 99 (a) |
| 55 | 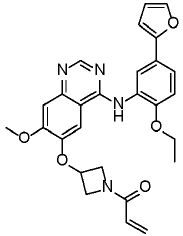 | 1-(3-((4-((2-ethoxy-5-(furan-2-yl)phenyl)ami no)-7-methoxyquinaz olin-6-yl)oxy)azetid in-1-yl)prop-2-en-1-one | ¹H NMR (400MHz, TFA salt, Methanol-*d*₄) δ 8.64 (s, 1H), 7.88-7.87 (m, 1H), 7.75-7.74 (m, 1H), 7.72-7.67 (m, 1H), 7.55-7.54 (m, 1H), 7.30 (s, 1H), 7.23 (d, *J* = 8.4 Hz, 1H), 6.71-6.70 (m, 1H), 6.53-6.52 (m, 1H), 6.44-6.41 (m, 1H), 6.40-6.29 (m, 1H), 5.82-5.79 (m, 1H), 5.30-5.28 (m, 1H), 4.70-4.63 (m, 2H), 4.49-4.48 (m, 2H), 4.21-4.17 (m, 1H), 4.15 (s, 3H), 4.13 (s, 3H); MS (ESI): m/z 487 [M+1]⁺ | 24 | 5.85 100 (a) |
| 56 | 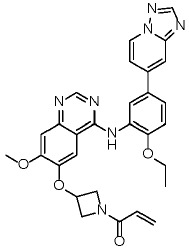 | 1-(3-((4-((5-([1,2,4]triaz olo[1,5-a] pyridin-7-yl)-2-ethoxyphenyl) amino)-7-methoxyquinaz olin-6-yl)oxy)azetid in-1-yl)prop-2-en-1-one | ¹H NMR (400MHz, free base, Methanol-*d*₄) δ 8.84(d, *J* = 7.2 Hz, 1H), 8.43 (s, 1H), 8.41 (s, 1H), 8.32 (d, *J* = 2.4 Hz, 1H), 8.01 (d, *J* = 0.8 Hz, 1H), 7.75-7.73 (m, 1H), 7.61-7.58 (m, 1H), 7.46 (s, 1H), 7.30 (d, *J* = 8.8 Hz, 1H), 7.26 (s, 1H), 6.41-6.38 (m, 1H), 6.33-6.32 (m, 1H), 5.81 (m, 1H), 5.30-5.28 (m, 1H), 4.63-4.60 (m, 1H), 4.51-4.47 (m, 1H), 4.27-4.22 (m, 4H), 4.06 (s, 3H), 1.43 (t, *J* = 6.8 Hz, 3H); MS (ESI) : m/z 538 [M+1]⁺ | 28 | 4.50 99 (a) |
| 57 | 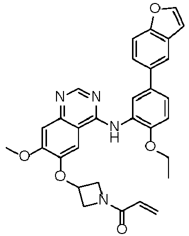 | 1-(3-((4-((5-(benzofuran-5-yl)-2-ethoxyphenyl) amino)-7-methoxyquinaz olin-6-yl)oxy)azetid in-1-yl)prop-2-en-1-one | ¹H NMR (400MHz, FA salt, DMSO-*d*₆) δ 9.15 (s, 1H), 8.36 (s, 1H), 8.02 (d, *J* = 2.0 Hz, 1H), 7.94-7.90 (m, 3H), 7.66 (d, *J* = 8.4 Hz, 1H), 7.60-7.52 (m, 3H), 7.23-7.20 (m, 2H), 7.00 (s, 1H), 6.40-6.33 (m, 1H), 6.16-6.11 (m, 1H), 5.72-5.69 (m, 1H), 5.30-5.25 (m, 1H), 4.84-4.80 (m, 1H), 4.58-4.54 (m, 1H), 4.32-4.29 (m, 1H), 4.14-4.08 (m, 2H), 3.17 (d, *J* = 4.8 Hz, 1H), 1.28 (t, *J* = 7.2 Hz, 1H); MS (ESI): m/z 537 [M+1]⁺ | 17 | 6.50 100 (a) |
| 58 | 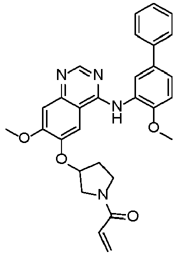 | 1-(3-((7-methoxy-4-((4-methoxy-[1,1'-biphenyl]-3-yl) amino) quin azolin-6-yl)oxy)pyrrol idin-1-yl)prop-2-en-1-one | ¹H NMR (400MHz, free base, CDCl₃) δ 8.99-8.97 (m, 1H), 8.74 (d, *J* = 5.2 Hz, 1H), 7.91 (s, 1H), 7.67-7.64 (m, 2H), 7.47-7.43 (m, 2H), 7.35-7.26 (m, 3H), 7.21 (d, *J* = 12.8 Hz, 1H), 7.04-7.01 (m, 1H), 6.53-6.39 (m, 2H), 5.75-5.68 (m, 1H), 5.19-5.08 (m, 1H), 4.04-3.79 (m, 10H), 2.48-2.26 (m, 3H); MS (ESI) : m/z 497 [M+1]⁺ | 25 | 6.08 100 (a) |
| 59 | 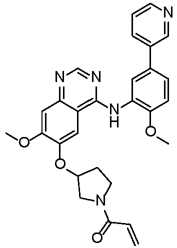 | 1-(3-((7-methoxy-4-((2-methoxy-5-(pyridin-3-yl)phenyl)ami no)quinazolin -6-yl)oxy)pyrrol idin-1-yl)prop-2-en-1-one | ¹H NMR (400MHz, free base, Methanol-*d*₄) δ 8.85 (d, *J* = 2.0 Hz, 1H), 8.51-8.50 (m, 1H), 8.38 (d, *J* = 4.0 Hz, 1H), 8.18-8.11 (m, 1H), 8.09 (d, *J* = 2.4 Hz, 1H), 7.78 (d, *J* = 11.6 Hz, 1H), 7.54-7.51 (m, 1H), 7.29-7.26 (m, 1H), 7.22 (d, *J* = 7.6 Hz, 1H), 6.67-6.64 (m, 1H), 6.34-6.30 (m, 1H), 5.81-5.78 (m, 1H), 5.31-5.28 (m, 1H), 3.99 (s, 3H), 3.83 (s, 3H), 3.82-3.78 (m, 4H), 2.56-2.34 (m, 2H); MS (ESI): m/z 498 [M+1]⁺ | 45 | 98* |
| 60 | 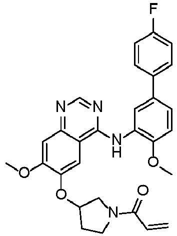 | 1-(3-((4-((4'-fluoro-4-methoxy-[1,1'-biphenyl]-3-yl)amino)-7-methoxyquinaz olin-6-yl)oxy)pyrrol idin-1-yl)prop-2-en-1-one | ¹H NMR (400 MHz, Free base, Methanol-*d*₄) δ 8.62 (d, *J* = 2.4 Hz 1H), 8.03 (s, 1H), 7.78 (d, *J =* 2.4 Hz, 1H), 7.69-7.62 (m, 3H), 7.31-7.26 (m, 2H), 7.22-7.16 (m, 2H), 6.73-6.59 (m, 1H), 6.36-6.31 (m, 1H), 5.83-5.77 (m, 1H), 5.33 (d, *J* = 21.6 Hz, 1H), 4.11-3.81 (m, 11H), 2.48-2.30 (m, 2H), 1.31 (s, 7H), 0.94-0.87 (m, 1H); MS (ESI): m/z=515 [M+1] ⁺ | 58 | 6.19 99 (a) |
| 61 | 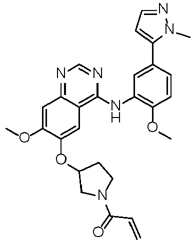 | 1-(3-((7-methoxy-4-((2-methoxy-5-(1-methyl-1H-pyrazol-5-yl)phenyl)ami no)quinazolin -6-yl)oxy)pyrrol idin-1-yl)prop-2-en-1-one | ¹H NMR (400MHz, TFA salt, Methanol-*d*₄) δ 8.38(d, *J* = 2.0 Hz, 1H), 7.93 (d, *J* = 2.0 Hz, 1H), 7.78 (d, *J* = 8.0 Hz, 1H), 7.51 (d, *J* = 2.0 Hz, 1H), 7.41-7.39 (m, 1H), 7.27-7.21 (m, 2H), 6.69-6.66 (m, 1H), 6.39-6.31 (m, 1H), 5.82-5.79 (m, 2H), 5.32-5.27 (m, 1H), 4.01 (s, 3H), 3.99 (s, 3H), 3.98-3.95 (m, 4H), 2.56-2.28 (m, 2H); MS (ESI): m/z 501 [M+1]⁺ | 50 | 4.44 98 (a) |
| 62 | 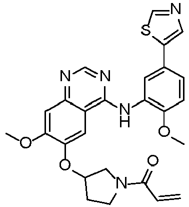 | 1-(3-((7-methoxy-4-((2-methoxy-5-(thiazol-5-yl)phenyl) ami no) quinazolin -6-yl)oxy)pyrrol idin-1-yl)prop-2-en-1-one | ¹H NMR (400MHz, free base, Methanol-*d*₄) δ 8.94 (s, 1H), 8.42 (s, 1H), 8.12-8.11 (m, 1H), 8.07-8.06 (m, 1H), 7.80-7.79 (m, 1H), 7.63-7.61 (m, 1H), 7.24-7.22 (m, 2H), 6.78-6.61 (m, 1H), 6.36-6.31 (m, 1H), 5.84-5.71 (m, 1H), 5.36-5.27 (m, 1H), 4.02-3.73 (m, 6H), 1.91 (s, 3H), 1.89 (s, 3H); MS (ESI): m/z 504 [M+1] ⁺ | 10 | 4.48 100 (a) |
| 63 | 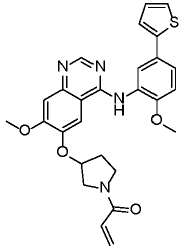 | 1-(3-((7-methoxy-4-((2-methoxy-5-(thiophen-2-yl)phenyl)ami no)quinazolin -6-yl)oxy)pyrrol idin-1-yl)prop-2-en-1-one | ¹H NMR (400MHz, free base, Methanol-*d*₄) δ 8.36 (s, 1H), 7.99 (d, *J* = 1.2 Hz, 1H), 7.78 (d, *J* = 7.2 Hz, 1H), 7.58-7.55 (m, 1H), 7.34-7.31 (m, 2H), 7.22-7.15 (m, 2H), 7.09 (t, *J* = 8.0 Hz, 1H), 6.65-6.59 (m, 1H), 6.34-6.29 (m, 1H), 5.81-5.75 (m, 1H), 5.31-5.26 (m, 1H), 4.01-3.70 (m, 9H); MS (ESI): m/z 503 [M+1]⁺ | 40 | 5.86 100 (a) |
| 64 | 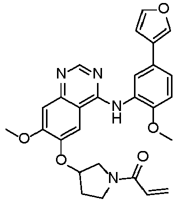 | 1-(3-((4-((5-(furan-3-yl)-2-methoxyphenyl )amino)-7-methoxyquinaz olin-6-yl)oxy)pyrrol idin-1-yl)prop-2-en-1-one | ¹H NMR (400MHz, free base, Methanol-*d*₄) δ 8.60 (s, 1H), 8.00 (s, 1H), 7.88 (s, 1H), 7.71 (s, 1H), 7.64 (d, *J* = 8.8 Hz, 1H), 7.58 (s, 1H), 7.25 (s, 1H), 7.23 (d, *J* = 8.4 Hz, 1H), 6.80 (s, 1H), 6.78-6.66 (m, 1H), 6.36-6.31 (m, 1H), 5.83-5.80 (m, 1H), 4.16-3.60 (m, 11H), 2.55-2.29 (m, 2H); MS (ESI) : m/z 487 [M+1]⁺ | 48 | 5.21 97 (a) |
| 65 | 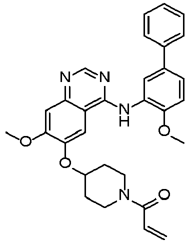 | 1-(4-((7-methoxy-4-((4-methoxy-[1,1'-biphenyl]-3-yl)amino)quin azolin-6-yl)oxy)piperi din-1-yl)prop-2-en-1-one | ¹H NMR (400MHz, free base, DMSO-*d*₆) δ 8.39 (s, 1H), 7.97 (s, 1H), 7.80 (d, *J* = 2.4 Hz, 1H), 7.67-7.60 (m, 3H), 7.46-7.44 (t, *J* = 7.6 Hz, 2H), 7.34 (t, *J* = 7.2 Hz, 1H), 7.32-7.21 (m, 2H), 6.88-6.82 (m, 1H), 6.14-6.09 (m, 1H), 5.70-5.67 (m, 1H), 4.81-4.78 (m, 1H), 3.95-3.92 (m, 5H), 3.50-3.38 (m, 2H), 2.04-2.01 (m, 2H), 1.69-1.65 (m, 2H); MS (ESI): m/z 511 [M+1] ⁺ | 50 | 6.32 98 (a) |
| 66 | 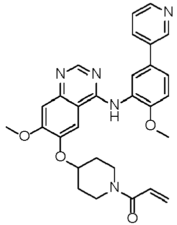 | 1-(4-((7-methoxy-4-((2-methoxy-5-(pyridin-3-yl)phenyl)ami no)quinazolin -6-yl)oxy)piperi din-1-yl)prop-2-en-1-one | ¹H NMR (400MHz, free base, Methanol-*d*₄) δ 8.84 (d, *J* = 1.6 Hz, 1H), 8.51-8.50 (m, 1H), 8.36 (s, 1H), 8.14-8.11 (m, 1H), 8.07 (d, *J* = 2.4 Hz, 1H), 7.85 (s, 1H), 7.64-7.61 (m, 1H), 7.54-7.51 (m, 1H), 7.30 (d, *J* = 8.4 Hz, 1H), 7.23 (s, 1H), 6.88-6.81 (s, 1H), 6.26-6.21 (m, 1H), 5.80-5.76 (m, 1H), 5.00-4.98 (m, 1H), 4.08 (s, 3H), 4.02-3.99 (m, 2H), 3.98 (s, 3H), 3.82-3.79 (m, 2H), 2.21-2.10 (m, 2H), 2.02-1.95 (m, 2H); MS (ESI): m/z 521 [M+1]⁺ | 47 | 3.57 100 (a) |
| 67 | 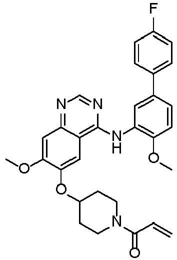 | 1-(4-((4-((4'-fluoro-4-methoxy-[1,1'-biphenyl]-3-yl)amino)-7-methoxyquinaz olin-6-yl)oxy)piperi din-1-yl)prop-2-en-1-one | ¹H NMR (400 MHz, free base, Methanol-*d*₄) δ 8.59 (s, 1H), 8.05 (br, 1H), 7.74 (d, *J* = 2.4 Hz, 1H), 7.67-7.60 (m, 3H), 7.28-7.23 (m, 2H), 7.18-7.14 (m, 2H), 6.85-6.78 (m, 1H), 6.25-6.20 (m, 1H), 5.78-5.75 (m, 1H), 4.90 (s, 1H), 4.08 (s, 3H), 3.98-3.90 (m, 5H), 3.63 (br, 2H), 2.11 (br, 2H), 1.91 (br, 2H); MS (ESI) : m/z=529 [M+1]⁺ | 63 | 6.39 99 (a) |
| 68 | 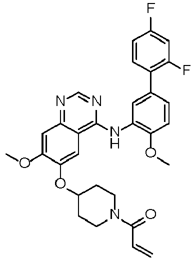 | 1-(4-((4-((2',4'-difluoro-4-methoxy-[1,1'-biphenyl]-3-yl)amino)-7-methoxyquinaz olin-6-yl)oxy)piperi din-1-yl)prop-2-en-1-one | ¹H NMR (400MHz, FA salt, Methanol-*d*₄) δ 8.33 (s, 1H), 7.85-7.84 (m, 1H), 7.79 (s, 1H), 7.54-7.41 (m, 2H), 7.19 (s, 1H), 7.17(s, 1H), 7.04-7.02 (m, 2H), 6.84-6.80 (m, 1H), 6.25-6.20 (m, 1H), 5.77-5.74 (m, 1H), 4.84-4.80 (m, 1H), 4.00-3.90 (m, 8H), 3.69-3.60 (m, 2H), 2.07-2.06 (m, 2H), 1.90-1.87 (m, 2H), 1. 92-1. 89 (m, 2H); MS (ESI): m/z 547 [M+1]⁺ | 50 | 6.48 100 (a) |
| 69 | 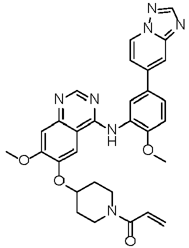 | 1-(4-((4-((5-([1,2,4]triaz olo[1,5-a]pyridin-7-yl)-2-methoxyphenyl )amino)-7-methoxyquinaz olin-6-yl)oxy)piperi din-1-yl)prop-2-en-1-one | ¹H NMR (400MHz, TFA salt, Methanol-*d*₄) δ 8.84 (d, *J* = 7.2 Hz, 1H), 8.43 (s, 1H), 8.38 (s, 1H), 8.23 (d, *J* = 2.4 Hz, 1H), 8.00 (s, 1H), 7.86 (s, 1H), 7.79-7.77 (m, 1H), 7.60-7.58 (m, 1H), 7.33 (d, *J* = 8.8 Hz, 1H), 7.24 (s, 1H), 6.88-6.81 (m, 1H), 6.26-6.22 (m, 1H), 5.80-5.77 (m, 1H), 4.66-4.61 (m, 1H), 4.04 (s, 3H), 4.00-3.99 (m, 2H), 3.98 (s, 3H), 3.71-3.68 (m, 2H), 2.11-2.10 (m, 2H), 1. 94-1. 91 (m, 2H); MS (ESI): m/z 552 [M+1]⁺ | 48 | 4.38 100 (a) |
| 70 | 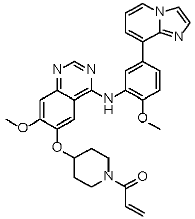 | 1-(4-((4-((5-(imidazo[1,2-a]pyridin-8-yl)-2-methoxyphenyl )amino)-7-methoxyquinaz olin-6-yl)oxy)piperi din-1-yl)prop-2-en-1-one | ¹H NMR (400MHz, TFA salt, Methanol-*d*₄) δ 8.44-8.42 (m, 1H), 8.31 (s, 1H), 8.23-8.22 (m, 1H), 7.93-7.92 (m, 1H), 7.88-7.85 (m, 2H), 7.61-7.60 (m, 1H), 7.44-7.42 (m, 1H), 7.28-7.25 (m, 1H), 7.03-7.02 (m, 1H), 6.83-6.80 (m, 2H), 6.26-6.25 (m, 1H), 6.22-6.21 (m, 1H), 5.76-5.75 (m, 1H), 4.89 (s, 3H), 4.88-4.03 (m, 2H), 4.02 (s, 3H), 3.71-3.60 (m, 2H), 2.16-2.03 (m, 2H), 1.99-1.86 (m, 2H); MS (ESI): m/z 551 [M+1]⁺ | 1 | 3.48 100 (a) |
| 71 | 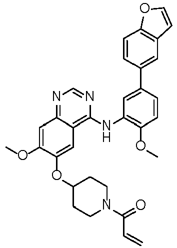 | 1-(4-((4-((5-(benzofuran-5-yl)-2-methoxyphenyl )amino)-7-methoxyquinaz olin-6-yl)oxy)piperi din-1-yl)prop-2-en-1-one | ¹H NMR (400MHz, TFA salt, Methanol-*d*₄) δ 8.59 (s, 1H), 8.05 (s, 1H), 7.82-7.78 (m, 3H), 7.70-7.67 (m, 1H), 7.54 (s, 2H), 7.28-7.25 (m, 2H), 6.88-6.79 (m, 2H), 6.26-6.21 (m, 1H), 5.79-5.76 (m, 1H), 4.08 (s, 3H), 3.99-3.92 (m, 5H), 3.68-3.60 (m, 2H), 2.11-2.08 (m, 2H), 1.91-1.89 (m, 2H); MS (ESI): m/z 551 [M+1] ⁺ | 52 | 6.56 100 (a) |
| 72 | 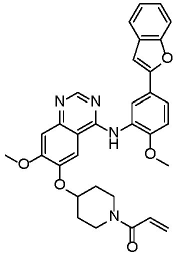 | 1-(4-((4-((5-(benzofuran-2-yl)-2-methoxyphenyl )amino)-7-methoxyquinaz olin-6-yl)oxy)piperi din-1-yl)prop-2-en-1-one | ¹H NMR (400 MHz, free base, Methanol-*d*₄) δ 8.50 (s, 1H), 8.12 (d, *J* = 2 Hz, 1H), 7.95 (s, 1H), 7.92-7.89 (m, 1H), 7.59-7.56 (m, 1H), 7.50-7.48 (m, 1H), 7.29-7.19 (m, 4H), 7.10 (d, *J* = 0.8, 1H), 6.85-6.78 (m, 1H), 6.24-6.20 (m, 1H), 5.78-5.75 (m, 1H), 4.06 (s, 3H), 3.98-3.95 (m, 5H), 3.68-3.59 (m, 2H), 2.10 (s, 2H), 1.92 (s, 2H), 1.33-1.29 (m, 4H); MS (ESI): m/z=551 [M+1]⁺ | 36 | 6.79 99 (a) |
| 73 | 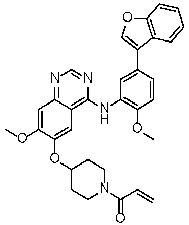 | 1-(4-((4-((5-(benzofuran-3-yl)-2-methoxyphenyl ) amino) -7-methoxyquinaz olin-6-yl)oxy)piperi din-1-yl)prop-2-en-1-one | ¹H NMR (400MHz, free base, Methanol-*d*₄) δ 8.38 (s, 1H), 8.09 (d, *J* = 2.4 Hz, 1H), 7.99 (s, 1H), 7.94 (d, *J* = 1.6 Hz, 1H), 7.85 (s, 1H), 7.64-7.61 (m, 1H), 7.57 (d, *J* = 7.2 Hz, 1H), 7.38-7.34 (m, 2H), 7.28 (d, *J* = 8.4 Hz, 1H), 7.23 (s, 1H), 6.87-6.80 (m, 1H), 6.26-6.21 (m, 1H), 5.79-5.76 (m, 1H), 4.98-4.91 (m, 1H), 4.03 (s, 3H), 4.01-3.98 (m, 2H), 3.95 (s, 3H), 3.79-3.72 (m, 2H), 2.21-2.18 (m, 2H), 2.00-1.95 (m, 2H); MS (ESI) : m/z 551 [M+1]⁺ | 51 | 6. 63 98 (a) |
| 74 | 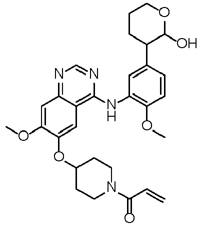 | 1-(4-((4-((5-(2-hydroxytetrah ydro-2H-pyran-3-yl)-2-methoxyphenyl )amino)-7-methoxyquinaz olin-6-yl)oxy)piperi din-1-yl)prop-2-en-1-one | ¹H NMR (400MHz, TFA salt, Methanol-*d*₄) 8.34 (s, 2H), 8.03-8.00 (m, 1H), 7.83 (s, 1H), 7.25 (s, 1H), 7.23 (s, 1H), 6.87-6.80 (m, 1H), 6.26-6.21 (m, 1H), 5.79-5.76 (m, 1H), 4.82-4.81 (m, 1H), 4.04 (s, 3H), 4.01-3.99 (m, 2H), 3.70-3.68 (m, 2H), 3.64 (t, *J =* 6.4 Hz, 2H), 3.08 (t, *J =* 6.4 Hz, 2H), 2.11-2.09 (m, 2H), 1.93-1.92 (m, 2H), 1. 84-1. 79 (m, 2H), 1.66-1.63 (m, 2H); MS (ESI) : m/z 535 [M+1]⁺ | 5 | 4.47 98 (a) |
| 75 | 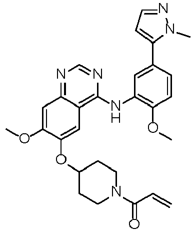 | 1-(4-((7-methoxy-4-((2-methoxy-5-(1-methyl-1H-pyrazol-5-yl)phenyl) ami no)quinazolin -6-yl)oxy)piperi din-1-yl)prop-2-en-1-one | ¹H NMR (400MHz, free base, Methanol-*d*₄) δ 8.36 (s, 1H), 7.90 (d, *J* = 2.0 Hz, 1H), 7.84 (s, 1H), 7.51 (s, 1H), 7.42-7.40 (m, 1H), 7.28 (d, *J =* 8.8 Hz, 1H), 7.23 (s, 1H), 6.88-6.81 (m, 1H), 6.39 (d, *J =* 2.0 Hz, 1H), 6.26-6.22 (m, 1H), 5.80-5.76 (m, 1H), 4.98-4.95 (m, 1H), 4.09 (s, 3H), 4.01-3.98 (m, 2H), 3.94 (s, 3H), 3.93 (s, 3H), 3.71-3.69 (m, 2H), 2.11-2.09 (m, 2H), 1. 94-1. 92 (m, 2H); MS (ESI): m/z 515 [M+1]⁺ | 39 | 4.79 92 (a) |
| 76 | 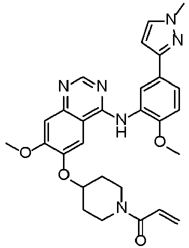 | 1-(4-((7-methoxy-4-((2-methoxy-5-(1-methyl-1H-pyrazol-3-yl)phenyl)ami no)quinazolin -6-yl)oxy)piperi din-1-yl)prop-2-en-1-one | ¹H NMR (400 MHz, free base, Methanol-*d*₄) δ 8.32 (s, 1H), 8.05 (s, 1H), 7.83 (s, 1H), 7.72 (d, *J* = 8.4 Hz, 1H), 7.59 (s, 1H), 7.21 (s, 1H), 7.18 (d, *J =* 8.8 Hz, 1H), 6.86-6.79 (m, 1H), 6.59 (s, 1H), 6.25 (d, *J* = 16.8 Hz, 1H), 5.78 (d, *J* = 10.8 Hz, 1H), 4.02 (s, 3H), 4.00-3.90 (m, 9H), 3.71-3.59 (m, 2H), 2.16-2.03 (m, 2H), 1.95-1.85 (m, 2H); MS (ESI): m/z=515 [M+1]⁺ | 47 | 4.76 100 (a) |
| 77 | 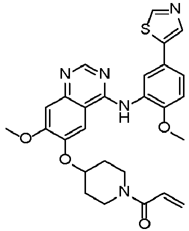 | 1-(4-((7-methoxy-4-((2-methoxy-5-(thiazol-5-yl)phenyl)ami no)quinazolin -6-yl)oxy)piperi din-1-yl)prop-2-en-1-one | ¹H NMR (400 MHz, TFA salt, Methanol-*d*₄) δ 8.92 (s, 1H), 8.37 (s, 2H), 8.09 (s, 1H), 8.02 (d, *J* = 2.4 Hz, 1H), 7.82 (s, 1H), 7.59-7.57 (m, 1H), 7.22-7.20 (m, 2H), 6.85-6.78 (m, 1H), 6.24-6.19 (m, 1H), 5.77-5.74 (m, 1H), 4.02 (s, 3H), 3.98-3.92 (m, 5H), 3.71-3.63 (m, 2H), 2.09-2.08 (m, 2H), 1.93-1.89 (m, 2H); MS (ESI): m/z=518 [M+1] ⁺ | 7 | 4.83 100 (a) |
| 78 | 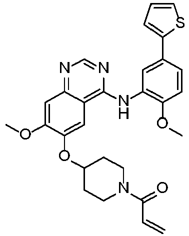 | 1-(4-((7-methoxy-4-((2-methoxy-5-(thiophen-2-yl)phenyl)ami no)quinazolin -6-yl)oxy)piperi din-1-yl)prop-2-en-1-one | ¹H NMR (400MHz, free base, Methanol-*d*₄) δ 8.52 (s, 1H), 7.95 (s, 1H), 7.85 (d, *J =* 2.4 Hz, 1H), 7.64-7.61 (m, 1H), 7.35-7.31 (m, 2H), 7.24 (s, 1H), 7.21 (d, *J* = 8.8 Hz, 1H), 7.09-7.07 (m, 1H), 6.86-6.79 (m, 1H), 6.26-6.21 (m, 1H), 5.79 (d, *J* = 8.8 Hz, 1H), 4.07 (s, 3H), 3.99-3.95 (m, 2H), 3.90 (s, 2H), 3.70-3.61 (m, 2H), 2.12-2.10 (m, 2H), 1. 92-1.91 (m, 2H); MS (ESI): m/z 517 [M+1]⁺ | 53 | 6.09 100 (a) |
| 79 | 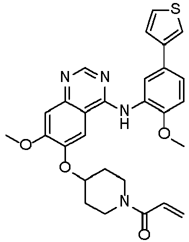 | 1-(4-((7-methoxy-4-((2-methoxy-5-(thiophen-3-yl)phenyl)ami no)quinazolin -6-yl)oxy)piperi din-1-yl)prop-2-en-1-one | ¹H NMR (400 MHz, Free base, Methanol-*d*₄) δ 8.30 (s, 1H), 7.94 (d, *J* = 2.4 Hz, 1H), 7.82 (s, 1H), 7.60-7.57 (m, 1H), 7.55-7.53 (m, 1H), 7.47-7.42 (m, 2H), 7.20-7.15 (m, 2H), 6.85-6.78 (m, 1H), 6.24-6.19 (m, 1H), 5.77-5.74 (m, 1H), 4.01-3.89 (m, 9H), 3.70-3.61 (m, 2H), 2.08-2.07 (m, 2H), 1.96-1.88 (m, 2H), 1.39-1.31 (m, 4H); MS (ESI): m/z=517 [M+1]⁺ | 39 | 6.07 94 (a) |
| 80 | 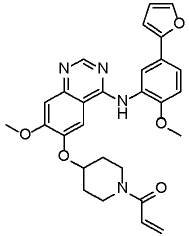 | 1-(4-((4-((5-(furan-2-yl)-2-methoxyphenyl )amino)-7-methoxyquinaz olin-6-yl)oxy)piperi din-1-yl)prop-2-en-1-one | ¹H NMR (400 MHz, HCl salt, DMSO-*d*₆) δ 10.91 (s, 1H), 8.72 (s, 1H), 8.15 (s, 1H), 7.76-7.72 (m, 3H), 7.30-7.27 (m, 2H), 6.89-6.82 (m, 2H), 6.59-6.58 (m, 1H), 6.14-6.10 (m, 1H), 5.71-5.68 (m, 1H), 4.85 (s, 1H), 4.01 (s, 3H), 3.91-3.88 (m, 2H), 3.83 (s, 3H), 3.51-3.50 (m, 2H), 2.08-2.07 (m, 2H), 1.72-1.71 (m, 2H); MS (ESI): m/z=501 [M+1]⁺ | 85 | 5.87 100 (a) |
| 81 | 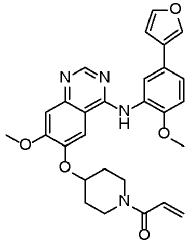 | 1-(4-((4-((5-(furan-3-yl)-2-methoxyphenyl )amino)-7-methoxyquinaz olin-6-yl)oxy)piperi din-1-yl)prop-2-en-1-one | ¹H NMR (400 MHz, free base, Methanol-*d*₄) δ 8.59 (s, 1H), 8.04 (s, 1H), 7.88 (s, 1H), 7.70 (s, 1H), 7.63 (d, *J =* 8.8 Hz, 1H), 7.57 (s, 1H), 7.27 (s, 1H), 7.23 (d, *J =* 8.4 Hz, 1H), 6.87-6.79 (m, 2H), 6.27 (d, *J =* 16.8 Hz, 1H), 5.80 (d, *J* = 10.4 Hz, 1H), 4.10 (s, 3H), 4.00-3.90 (m, 2H), 3.89 (s, 3H), 3.75-3.60 (m, 2H), 2.21-2.08 (m, 2H), 2.00-1.88 (m, 2H); MS (ESI): m/z=501 [M+1]⁺ | 52 | 5.69 100 (a) |
| 82 | 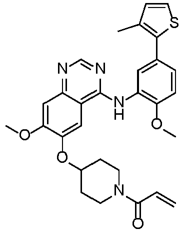 | 1-(4-((7-methoxy-4-((2-methoxy-5-(3-methylthiophe n-2-yl)phenyl)ami no)quinazolin -6-yl)oxy)piperi din-1-yl)prop-2-en-1-one | ¹H NMR (400MHz, free base, CDCl₃) δ 8.93 (s, 1H), 8.69 (s, 1H), 7.89 (s, 1H), 7.30 (s, 1H), 7.20 (s, 1H), 7.19-7.17 (m, 2H), 7.01 (d, *J* = 8.4 Hz, 1H), 6.95 (d, *J* = 5.2 Hz, 1H), 6.66-6.62 (m, 1H), 6.34-6.29 (m, 1H), 5.74-5.71 (m, 1H), 4.69-4.68 (m, 1H), 3.99-3.91 (m, 2H), 3.87-3.85 (m, 1H), 3.52-3.48 (m, 1H), 2.41 (s, 3H), 2.03-1.97 (m, 4H); MS (ESI) : m/z 531 [M+1]⁺ | 38 | 6.40 100 (a) |
| 83 | 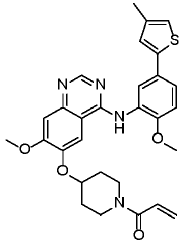 | 1-(4-((7-methoxy-4-((2-methoxy-5-(4-methylthiophe n-2-yl)phenyl)ami no)quinazolin -6-yl)oxy)piperi din-1-yl)prop-2-en-1-one | ¹H NMR (400MHz, free base, CDCl₃) δ 9.03 (s, 1H), 8.76 (s, 1H), 7.87 (s, 1H), 7.30-7.24 (m, 3H), 7.12 (s, 1H), 6.96 (d, *J =* 8.4 Hz, 1H), 6.83 (s, 1H), 6.66-6.59 (m, 1H), 6.34-6.29 (m, 1H), 5.74-5.71 (m, 1H), 4.66-4.65 (m, 1H), 4.01 (s, 3H), 3.99-3.98 (m, 2H), 3.72-3.69 (m, 1H), 3.59-3.55 (m, 1H), 2.31 (s, 3H), 2.04-1.96 (m, 4H), 1.56 (s, 3H); MS (ESI) : m/z 531 [M+1]⁺ | 34 | 6.48 99 (a) |
| 84 | 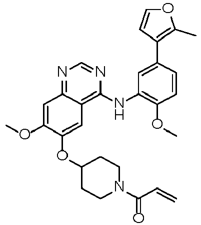 | 1-(4-((7-methoxy-4-((2-methoxy-5-(2-methylfuran-3-yl)phenyl)ami no)quinazolin -6-yl)oxy)piperi din-1-yl)prop-2-en-1-one | ¹H NMR (400 MHz, TFA salt, Methanol-*d*₄) δ 8.58 (s, 1H), 8.04 (s, 1H), 7.55 (d, *J* = 2 Hz, 1H), 7.47-7.45 (m, 1H), 7.39 (d, *J =* 1.6 Hz, 1H), 7.23-7.22 (m, 2H), 6.85-6.79 (m, 1H), 6.55 (d, *J* = 2 Hz, 1H), 6.25-6.20 (m, 1H), 5.78-5.75 (m, 1H) 4.08 (s, 3H), 3.98-3.92 (m, 2H), 3.88 (s, 3H), 3.68 (s, 2H), 2.43 (s, 3H), 2.11 (s, 2H), 1.91 (s, 2H); MS (ESI): m/z=515 [M+1] ⁺ | 24 | 6.19 100 (a) |
| 85 | 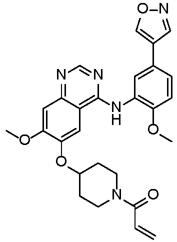 | 1-(4-((4-((5-(isoxazol-4-yl)-2-methoxyphenyl )amino)-7-methoxyquinaz olin-6-yl)oxy)piperi din-1-yl)prop-2-en-1-one | ¹H NMR (400MHz, TFA salt, Methanol-*d*₄) δ 9.01 (s, 1H), 8.81 (s, 1H), 8.32 (s, 1H), 7.94 (d, *J* = 2.0 Hz, 1H), 7.82 (s, 1H), 7.56-7.53 (m, 1H), 7.21-7.18 (m, 2H), 6.85-6.78 (m, 1H), 6.24-6.19 (m, 1H), 5.77-5.74 (m, 1H), 4.01-3.90 (m, 8H), 3.68-3.65 (m, 2H), 2.07-2.03 (m, 2H), 1. 92-1. 89 (m, 2H); MS (ESI): m/z 502 [M+1]⁺ | 5 | 5.01 100 (a) |
| 86 | 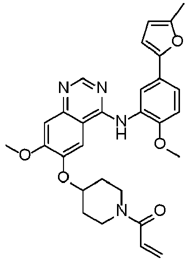 | 1-(4-((7-methoxy-4-((2-methoxy-5-(5-methylfuran-2-yl)phenyl)ami no)quinazolin -6-yl)oxy)piperi din-1-yl)prop-2-en-1-one | ¹H NMR (400MHz, TFA salt, Methanol-*d*₄) δ 8.49 (s, 1H), 7.99-7.91 (m, 1H), 7.65-7.64 (m, 1H), 7.58-7.56 (m, 1H), 7.15 (s, 1H), 7.10 (d, *J* = 8.8 Hz, 1H), 6.75-6.69 (m, 1H), 6.45-6.44 (m, 1H), 6.14 (d, *J* = 1.6 Hz, 1H), 6.11-5.98 (m, 1H), 5.69-5.65 (m, 1H), 4.06 (s, 3H), 3.99-3.84 (m, 2H), 3.77 (s, 3H), 3.22-3.20 (m, 2H), 2.23 (s, 3H), 2.11-2.00 (m, 2H), 1.89-1.78 (m, 2H); MS (ESI): m/z 515 [M+1]⁺ | 38 | 6.27 100 (a) |
| 87 | 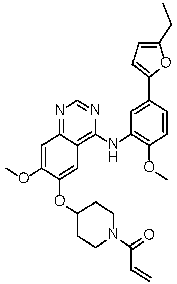 | 1-(4-((4-((5-(5-ethylfuran-2-yl)-2-methoxyphenyl )amino)-7-methoxyquinaz olin-6-yl)oxy)piperi din-1-yl)prop-2-en-1-one | ¹H NMR (400MHz, TFA salt, Methanol-*d*₄) δ 8.32 (s, 1H), 7.91-7.90 (m, 1H), 7.84 (s, 1H), 7.60-7.57 (m, 1H), 7.23 (s, 1H), 7.16 (d, *J* = 8.8 Hz, 1H), 6.87-6.80 (m, 1H), 6.55-6.54 (m, 1H), 6.26 (d, *J =* 2.0 Hz, 1H), 6.11-6.10 (m, 1H), 5.79-5.76 (m, 1H), 4.18-4.10 (m, 1H), 4.03 (s, 3H), 3.89 (s, 3H), 2.72 (q, *J* = 7.16, 7.56, 7.48 Hz, 1H), 2.16-2.00 (m, 3H), 1. 98-1. 79 (m, 2H), 1.69-1.61 (m, 2H); MS (ESI): m/z 529 [M+1]⁺ | 18 | 6.66 95 (a) |
| 88 | 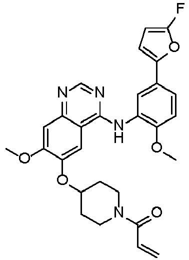 | 1-(4-((4-((5-(5-fluorofuran-2-yl)-2-methoxyphenyl )amino)-7-methoxyquinaz olin-6-yl)oxy)piperi din-1-yl)prop-2-en-1-one | ¹H NMR (400MHz, FA salt, Methanol-*d*₄) δ 8.33 (s, 1H), 7.90 (d, *J* = 2.4 Hz, 1H), 7.80 (s, 1H), 7.52-7.49 (m, 1H), 7.20 (s, 1H), 7.15 (d, *J* = 8.8 Hz, 1H), 6.85-6.78 (m, 1H), 6.59 (tr, *J* = 3.2 Hz, 1H), 6.24-6.19 (m, 1H), 5.77-5.74 (m, 1H), 5.62-5.59 (m, 1H), 4.01 (s, 3H), 3.99-3.93 (m, 2H), 3.88 (s, 3H), 3.70-3.63 (m, 2H), 2.08-2.07 (m, 2H), 1. 91-1. 90 (m, 2H); MS (ESI): m/z 519 [M+1]⁺ | 10 | 10.03 94 (b) |
| 89 | 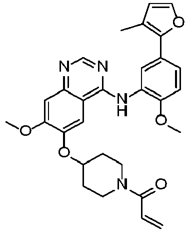 | 1-(4-((7-methoxy-4-((2-methoxy-5-(3-methylfuran-2-yl)phenyl)ami no)quinazolin -6-yl)oxy)piperi din-1-yl)prop-2-en-1-one | MS (ESI): m/z 515 [M+1]⁺ | | |
| 90 | 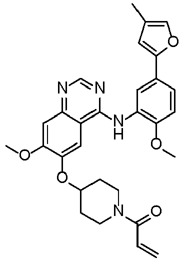 | 1-(4-((7-methoxy-4-((2-methoxy-5-(4-methylfuran-2-yl)phenyl)ami no)quinazolin -6-yl)oxy)piperi din-1-yl)prop-2-en-1-one | MS (ESI): m/z 515 [M+1]⁺ | 49 | 10.30 99 (b) |
| 91 | 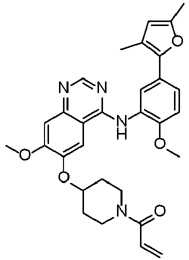 | 1-(4-((4-((5-(3,5-dimethylfuran -2-yl)-2-methoxyphenyl )amino)-7-methoxyquinaz olin-6-yl)oxy)piperi din-1-yl)prop-2-en-1-one | MS (ESI): m/z 529 [M+1]⁺ | | |
| 92 | 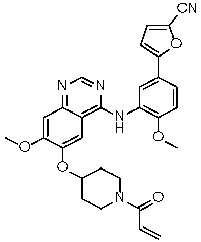 | 5-(3-((6-((1-acryloylpiper idin-4-yl)oxy)-7-methoxyquinaz olin-4-yl)amino)-4-methoxyphenyl )furan-2-carbonitrile | MS (ESI): m/z 526 [M+1] ⁺ | | |
| 93 | 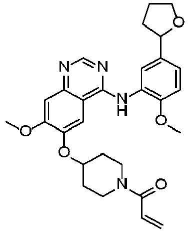 | 1-(4-((7-methoxy-4-((2-methoxy-5-(tetrahydrofu ran-2-yl)phenyl)ami no)quinazolin -6-yl)oxy)piperi din-1-yl)prop-2-en-1-one | MS (ESI): m/z 505 [M+1]⁺ | | |
| 94 | 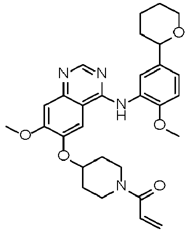 | 1-(4-((7-methoxy-4-((2-methoxy-5-(tetrahydro-2H-pyran-2-yl)phenyl)ami no)quinazolin -6-yl)oxy)piperi din-1-yl)prop-2-en-1-one | MS (ESI): m/z 519 [M+1]⁺ | | |
| 95 | 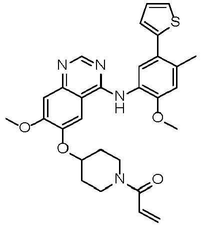 | 1-(4-((7-methoxy-4-((2-methoxy-4-methyl-5-(thiophen-2-yl))amino)qui nazolin-6-yl)oxy)piperi din-1-yl)prop-2-en-1-one | ¹H NMR (400MHz, TFA salt, Methanol-d4) δ 8.44 (s, 1H), 7.90 (s, 1H), 7.55 (s, 1H), 7.42 (d, *J* = 4.4 Hz, 1H), 7.20 (s, 1H), 7.11-7.08 (m, 3H), 6.86-6.79 (m, 1H), 6.26-6.21 (m, 1H), 5.79-5.76 (m, 1H), 4.87-4.83 (m, 1H), 4.04 (s, 3H), 3.96-3.94 (m, 2H), 3.88 (s, 3H), 3.67-3.61 (m, 2H), 3.36 (s, 1H), 2.46(s, 3H), 2.11-2.08 (m, 2H), 1.91-1.87 (m, 2H); MS (ESI): m/z 531 [M+1]⁺ | 43 | 6.46 100 (a) |
| 96 | 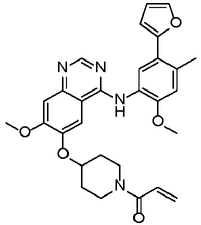 | 1-(4-((4-((5-(furan-2-yl)- 2-methoxy-4-methylphenyl) amino)-7-methoxyquinaz olin-6-yl)oxy)piperi din-1-yl)prop-2-en-1-one | ¹H NMR (400MHz, free base, Methanol-*d*₄) δ 8.29 (s, 1H), 7.85 (s, 1H), 7.84 (s, 1H), 7.57 (s, 1H), 7.20 (s, 1H), 7.05 (s, 1H), 6.88-6.81 (m, 1H), 6.57-6.54 (m, 2H), 6.26-6.22 (m, 1H), 5.79-5.76 (m, 1H), 4.85-4.84 (m, 1H), 4.03 (s, 3H), 4.00-3.96 (m, 2H), 3.88 (s, 3H), 3.68-3.66 (m, 2H), 2.55 (s, 3H), 2.10-2.09 (m, 2H), 1.92-1.90 (m, 2H); MS (ESI) : m/z 515 [M+1]⁺ | 5 | 6.11 95 (a) |
| 97 | 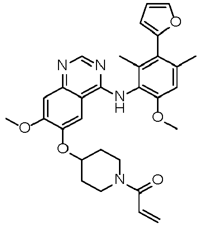 | 1-(4-((4-((3-(furan-2-yl)-6-methoxy-2,4-dimethylpheny 1)amino)-7-methoxyquinaz olin-6-yl)oxy)piperi din-1-yl)prop-2-en-1-one | MS (ESI): m/z 529 [M+1]⁺ | | |
| 98 | 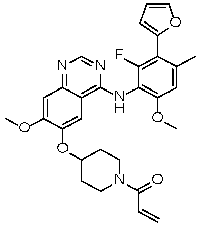 | 1-(4-((4-((2-fluoro-3-(furan-2-yl)-6-methoxy-4-methylphenyl) amino)-7-methoxyquinaz olin-6-yl)oxy)piperi din-1-yl)prop-2-en-1-one | MS (ESI): m/z 533 [M+1]⁺ | | |
| 99 | 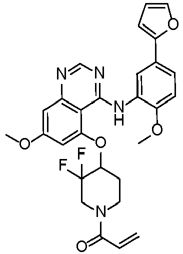 | 1-(3,3-difluoro-4-((4-((5-(furan-2-yl)-2-methoxyphenyl )amino)-7-methoxyquinaz olin-5-yl)oxy)piperi din-1-yl)prop-2-en-1-one | ¹H NMR (400 MHz, TFA salt, Methanol-*d*₄) δ8.76 (s, 2H), 7.65-7.62 (m, 1H), 7.55 (s, 1H), 7.24-7.19 (m, 2H), 6.89-6.88 (m, 2H), 6.89 (s, 1H), 6.71 (s, 1H), 6.54-6.52 (m, 1H), 6.29-6.28 (m, 1H), 5.87-5.84 (m, 1H), 4.72-4.71 (m, 1H), 4.33-4.32 (m, 1H), 4.01 (s, 3H), 3.93 (s, 3H), 3.90-3.89 (m, 1H), 3.42-3.41 (m, 1H), 2.52-2.50 (m, 1H), 2.15-2.13 (m, 1H); MS (ESI): m/z 537 [M+H]⁺ | 51 | 10.95 100 (b) |
| 100 | 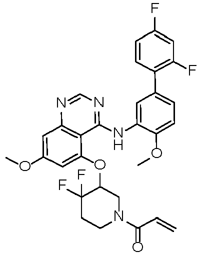 | 1-(3-((4-((2',4'-difluoro-4-methoxy-[1,1'-biphenyl]-3-yl)amino)-7-methoxyquinaz olin-5-yl)oxy)-4,4-difluoropiper idin-1-yl)prop-2-en-1-one | ¹H NMR (400 MHz, TFA salt, Methanol-*d*₄) δ8.68 (s, 1H), 8.28 (s, 1H), 7.58-7.49 (m, 2H), 7.29-7.26 (m, 2H), 7.11-7.06 (m, 2H), 6.91 (s, 1H), 6.78-6.76 (m, 1H), 6.06-6.02 (m, 1H), 5.68-5.43 (m, 2H), 4.21-4.20 (m, 1H), 4.11-4.08 (m, 4H), 4.01 (s, 3H), 3.99-3.81 (m, 2H), 2.45-2.42 (m, 2H); MS (ESI): m/z 583 [M+H]⁺ | 47 | 11.59 100 (b) |
| 101 | 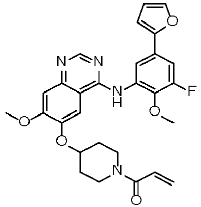 | 1-(4-((4-((3-fluoro-5-(furan-2-yl)-2-methoxyphenyl )amino)-7-methoxyquinaz olin-6-yl)oxy)piperi din-1-yl)prop-2-en-1-one | ¹H NMR (400 MHz, free base, DMSO-*d*₆) δ 9.12 (s, 1H), 8.42 (s, 1H), 7.90 (s, 1H), 7.85 (s, 1H), 7.79 (d, *J =* 12.4 Hz, 1H), 7.40 (d, *J* = 6.8 Hz, 1H), 7.22 (s, 1H), 6.88-6.82 (m, 2H), 6.69-6.68 (m, 1H), 6.14-6.09 (m, 1H), 5.70-5.67 (m, 1H), 4.82 (s, 1H), 4.12-4.08 (m, 1H), 3.94-3.90 (m, 9H), 3.17 (d, *J* = 5.2 Hz, 3H), 2.07-2.03 (m, 3H), 1.68 (s, 3H); MS (ESI): m/z=519 [M+1]⁺ | 1 | 6.19 99 (a) |
| 102 | 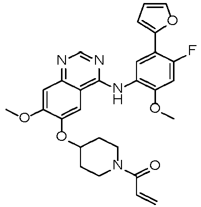 | 1-(4-((4-((4-fluoro-5-(furan-2-yl)-2-methoxyphenyl )amino)-7-methoxyquinaz olin-6-yl)oxy)piperi din-1-yl)prop-2-en-1-one | ¹H NMR (400MHz, free base, Methanol-*d*₄) δ 8.42 (s, 1H), 7.98 (d, *J* = 8.0 Hz, 1H), 7.92 (s, 1H), 7.58 (s, 1H), 7.23 (s, 1H), 7.11 (d, *J* = 12.8 Hz, 1H), 6.87-6.77 (m, 2H), 6.58-6.57 (m, 1H), 6.26-6.22 (m, 1H), 5.79-5.77 (m, 1H), 5.00-4.99 (m, 1H), 4.06 (s, 3H), 4.00-3.95 (m, 2H), 3.89 (s, 3H), 3.68-3.53 (m, 2H), 2.19-2.11 (m, 2H), 2.00-1.89 (m, 2H); MS (ESI): m/z 519 [M+1]⁺ | 67 | 6.07 100 (a) |
| 103 | 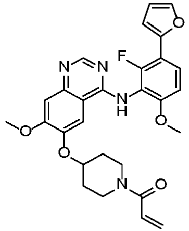 | 1-(4-((4-((2-fluoro-3-(furan-2-yl)-6-methoxyphenyl )amino)-7-methoxyquinaz olin-6-yl)oxy)piperi din-1-yl)prop-2-en-1-one | MS (ESI): m/z 519 [M+1] ⁺ | | |
| 104 | 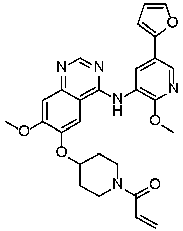 | 1-(4-((4-((5-(furan-2-yl)- 2-methoxypyridi n-3-yl)amino)-7-methoxyquinaz olin-6-yl)oxy)piperi din-1-yl)prop-2-en-1-one | ¹H NMR (400MHz, TFA salt, Methanol-*d*₄) δ 8.67 (s, 1H), 8.47 (d, *J* = 2.0 Hz, 1H), 8.20 (d, *J* = 1.6 Hz, 1H), 8.04 (s, 1H), 7.58 (s, 1H), 7.30 (s, 1H), 6.86-6.76 (m, 2H), 6.54 (s, 1H), 6.25-6.21 (m, 1H), 5.79-5.76 (m, 1H), 4.10 (s, 3H), 4.00-3.92 (m, 6H), 3.73-3.70 (m, 2H), 3.36-3.20 (m, 2H), 2.13-2.11 (m, 2H), 1.94-1.92 (m, 2H); MS (ESI): m/z 502 [M+1] ⁺ | 85 | 5.64 98 (a) |
| 105 | 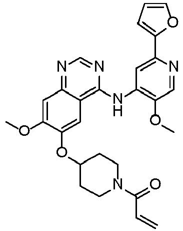 | 1-(4-((4-((2-(furan-2-yl)- 5-methoxypyridi n-4-yl)amino)-7-methoxyquinaz olin-6-yl)oxy)piperi din-1-yl)prop-2-en-1-one | ¹H NMR (400 MHz, TFA salt, Methanol-*d*₄) δ8.82 (s, 1H), 8.66 (s, 1H), 8.27 (s, 1H), 7.73 (s, 1H), 7.66 (s, 1H), 7.30 (s, 1H), 7.01 (d, *J* = 3.6 Hz, 1H), 6.88-6.81 (m, 1H), 6.61-6.60 (m, 1H), 6.27-6.22 (m, 1H), 5.80-6.77 (m ,1H), 4.93-4.91 (m, 1H), 4.11 (s, 3H), 4.05 (s, 3H), 3.99-3.96 (m, 2H), 3.82-3.79 (m, 2H), 2.11-2.09 (m, 2H), 1.95-1.93 (m, 2H); MS (ESI): m/z 502 [M+H]⁺ | 14 | 4.31 100 (a) |
| 106 | 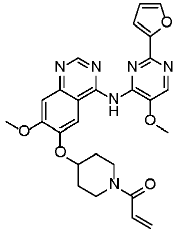 | 1-(4-((4-((2-(furan-2-yl)-5-methoxypyrimi din-4-yl)amino)-7-methoxyquinaz olin-6-yl)oxy)piperi din-1-yl)prop-2-en-1-one | MS (ESI): m/z 503 [M+1]⁺ | | |
| 107 | 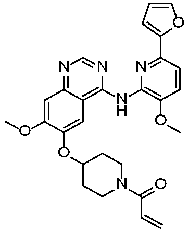 | 1-(4-((4-((6-(furan-2-yl)-3-methoxypyridi n-2-yl)amino)-7-methoxyquinaz olin-6-yl)oxy)piperi din-1-yl)prop-2-en-1-one | MS (ESI): m/z 502 [M+1]⁺ | | |
| 108 | 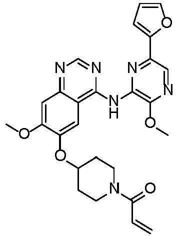 | 1-(4-((4-((6-(furan-2-yl)-3-methoxypyrazi n-2-yl)amino)-7-methoxyquinaz olin-6-yl)oxy)piperi din-1-yl)prop-2-en-1-one | MS (ESI): m/z 503 [M+1]⁺ | | |
| 109 | 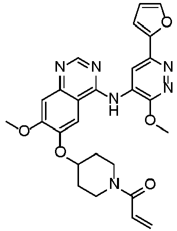 | 1-(4-((4-((6-(furan-2-yl)-3-methoxypyrida zin-4-yl)amino)-7-methoxyquinaz olin-6-yl)oxy)piperi din-1-yl)prop-2-en-1-one | MS (ESI): m/z 503 [M+1]⁺ | | |
| 110 | 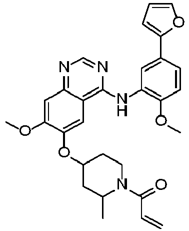 | 1-(4-((4-((5-(furan-2-yl)-2-methoxyphenyl )amino)-7-methoxyquinaz olin-6-yl)oxy)-2-methylpiperid in-1-yl)prop-2-en-1-one | MS (ESI): m/z 515 [M+1]⁺ | | |
| 111 | 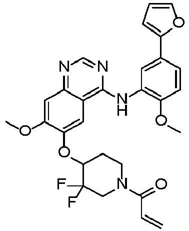 | 1-(3,3-difluoro-4-((4-((5-(furan-2-yl)-2-methoxyphenyl )amino)-7-methoxyquinaz olin-6-yl)oxy)piperi din-1-yl)prop-2-en-1-one | ¹H NMR (400 MHz, TFA salt, Methanol-*d*₄) δ8.37 (s, 1H), 8.04 (d, *J* = 2.0 Hz, 1H), 7.99 (s, 1H), 7.63-7.61 (m, 1H), 7.53-7.52 (m, 1H), 7.25 (s, 1H), 7.18 (d, *J* = 8.8 Hz, 1H), 6.88-6.81 (m, 1H), 6.51-6.50 (m, 1H), 6.31-6.27 (m, 1H), 5.85-5.82 (m, 1H), 5.01-4.96 (m, 1H), 4.21-4.19 (m, 3H), 4.08 (s, 3H), 3.99 (s, 3H), 3.97-3.96 (m, 2H), 2.23-2.19 (m, 2H); MS (ESI): m/z 537 [M+H]⁺ | 37 | 6.37 99 (a) |
| 112 | 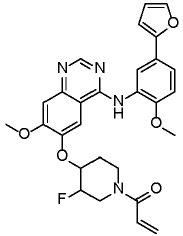 | 1-(3-fluoro-4-((4-((5-(furan-2-yl)-2-methoxyphenyl )amino)-7-methoxyquinaz olin-6-yl)oxy)piperi din-1-yl)prop-2-en-1-one | MS (ESI): m/z 519 [M+1] ⁺ | | |
| 113 | 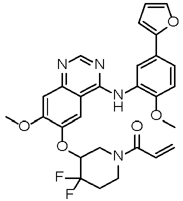 | 1-(4,4-difluoro-3-((4-((5-(furan-2-yl)- 2-methoxyphenyl )amino)-7-methoxyquinaz olin-6-yl)oxy)piperi din-1-yl)prop-2-en-1-one | ¹H NMR (400MHz, FA salt, Methanol-*d*₄) δ 8.42 (s, 1H), 8.20 (s, 1H), br), 8.04-8.00 (m, 2H), 7.66-7.63 (m, 1H), 7.54-7.53 (m, 1H), 7.23 (s, 1H), 7.20-7.18 (m, 1H), 6.68-6.54 (m, 2H), 6.51-6.50 (m, 1H), 6.19-6.14 (m, 1H), 5.85-5.55 (m, 1H), 4.88-4.55 (m, 2H), 4.02 (s, 3H), 3.92 (s, 3H), 3.78-3.50 (m, 2H), 3.20-3.10 (m, 1H), 2.60-2.08 (m, 2H); MS (ESI): m/z 537 [M+1]⁺ | 25 | 6.56 99 (a) |
| 114 | 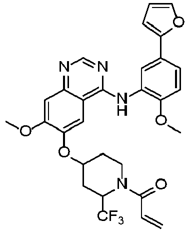 | 1-(4-((4-((5-(furan-2-yl)-2-methoxyphenyl )amino)-7-methoxyquinaz olin-6-yl)oxy)-2-(trifluoromet hyl)piperidin -1-yl)prop-2-en-1-one | MS (ESI): m/z 569 [M+1] ⁺ | | |
| 115 | 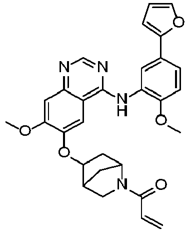 | 1-(5-((4-((5-(furan-2-yl)- 2-methoxyphenyl )amino)-7-methoxyquinaz olin-6-yl)oxy)-2-azabicyclo[2. 2.1]heptan-2-yl)prop-2-en-1-one | ¹H NMR (400MHz, TFA salt, Methanol-*d*₄) δ8.79 (s, 1H), 8.41 (s, 1H), 7.57-7.52 (m, 1H), 7.45-7.43 (m, 1H), 7.38-7.34 (m, 2H), 7.10-7.09 (m, 1H), 6.99-6.97 (m, 1H), 6.65-6.56 (m, 2H), 6.06-5.99 (m, 1H), 5.72-5.55 (m, 1H), 5.33-5.25 (m, 1H), 5.10-5.01 (m, 2H), 4.34-4.30 (m, 2H), 3.91-3.90 (m, 2H), 3.88 (s, 3H), 3.85 (s, 3H), 2.37-2.32 (m, 2H); MS (ESI): m/z 513 [M+1]⁺ | 23 | 5.98 100 (a) |
| 116 | 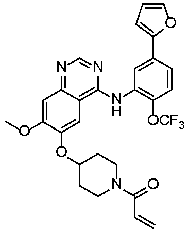 | 1-(4-((4-((5-(furan-2-yl)-2-(trifluoromet hoxy) phenyl) a mino)-7-methoxyquinaz olin-6-yl)oxy)piperi din-1-yl) prop-2-en-1-one | ¹H NMR (400MHz, TFA salt, Methanol-*d*₄) δ 8.38 (s, 1H), 8.03 (s, 1H), 7.87 (s, 1H), 7.74-7.72 (m, 1H), 7.62-7.61 (m, 1H), 6.89-6.87 (m, 1H), 6.85-6.81 (m, 1H), 6.58-6.56 (m, 1H), 6.26-6.21 (m, 1H), 5.79-5.76 (m, 1H), 4.05 (s, 3H), 4.04-3.93 (m, 2H), 3.70-3.69 (m, 3H), 2.15-2.03 (m, 2H), 1.97-1.89 (m, 2H); MS (ESI): m/z 555 [M+1] ⁺ | 19 | 6.50 92 (a) |
| 117 | 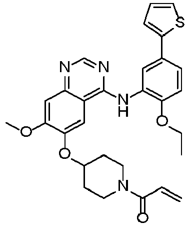 | 1-(4-((4-((2-ethoxy-5-(thiophen-2-yl) phenyl) ami no)-7-methoxyquinaz olin-6-yl)oxy)piperi din-1-yl) prop-2-en-1-one | ¹H NMR (400 MHz, HCl salt, Methanol-*d*₄) δ 8.62 (s, 1H), 8.04 (s, 1H), 7.78 (d, *J* = 2 Hz, 1H), 7.67-7.64 (m, 1H), 7.35-7.31 (m, 2H), 7.24-7.19 (m, 2H), 7.09-7.06 (m, 1H), 6.86-6.79 (m, 1H), 6.25-6.20 (m, 1H), 5.78-5.75 (m, 1H), 4.18-4.13 (m, 2H), 4.09 (s, 3H), 3.97-3.93 (m, 2H), 3.67-3.66 (m, 2H), 3.51-3.46 (m, 1H), 2.12 (s, 2H), 1.92 (s, 2H), 1. 32-1. 29 (m, 3H), 1.19-1.16 (m, 1H); MS (ESI): m/z=531 [M+1] ⁺ | 69 | 6.52 100 (a) |
| 118 | 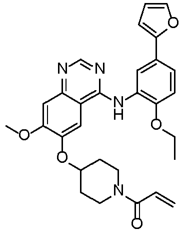 | 1-(4-((4-((2-ethoxy-5-(furan-2-yl)phenyl) ami no)-7-methoxyquinaz olin-6-yl)oxy)piperi din-1-yl)prop-2-en-1-one | ¹H NMR (400MHz, HCl salt, CDCl₃) δ 9.13-9.12 (m, 1H), 8.79 (s, 1H), 8.11 (s, 1H), 7.48-7.47 (m, 1H), 7.39-7.36 (m, 1H), 7.26 (s, 1H), 7.20 (s, 1H), 6.97-6.95 (m, 1H), 6.65-6.63 (m, 2H), 6.62-6.60 (m, 1H), 6.49-6.48 (m, 1H), 5.74-5.74 (m, 1H), 4.71-4.62 (m, 1H), 4.23-4.21 (m, 2H), 4.02 (s, 3H), 3.60-3.50 (m, 1H), 2.03-2.01 (m, 4H), 1.55 (t, J = 7.2, 6.8 Hz, 3H); MS (ESI): m/z 515 [M+1]⁺ | 2 | 6.05 100 (a) |
| 119 | 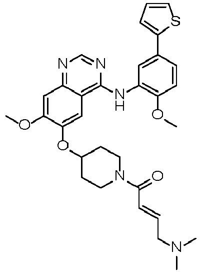 | (E)-4-(dimethylamin o)-1-(4-((7-methoxy-4-((2-methoxy-5-(thiophen-2-yl)phenyl) ami no) quinazolin -6-yl) oxy) piperi din-1-yl) but-2-en-1-one | ¹H NMR (400MHz, FA salt, Methanol-*d*₄) δ 8.57 (s, 1H), 8.32 (s, 1H), 7.94 (d, *J* = 2.4Hz, 1H), 7.78 (s, 1H), 7.54-7.52 (m, 1H), 7.31-7.28 (m, 2H), 7.19 (s, 1H), 7.14 (d, *J* = 8.8 Hz, 1H), 7.06-7.04 (m, 1H), 6.60-6.57(m, 1H), 6.15-6.09 (m, 1H), 3.99-3.84 (m, 8H), 3.66-3.56 (m, 4H), 2.57 (s, 6H), 2.14-2.06 (m, 2H), 1.92-1.86 (m, 2H); MS (ESI): m/z 574 [M+1]⁺ | 5 | 5.08 99 (a) |
| 120 | 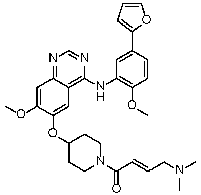 | (E)-4-(dimethylamin o)-1-(4-((4-((5-(furan-2-yl)-2-methoxyphenyl )amino)-7-methoxyquinaz olin-6-yl)oxy)piperi din-1-yl)but-2-en-1-one | ¹H NMR (400MHz, TFA salt, Methanol-*d*₄) 8.42 (s, 1H), 7.96 (d, *J* = 2.4 Hz, 1H), 7.89 (s, 1H), 7.68-7.66 (m, 1H), 7.54-7.53 (m, 1H), 7.24 (s, 1H), 7.21 (d, *J* = 8.4 Hz, 1H), 6.93 (d, *J* = 11.6 Hz, 1H), 6.69-6.68 (m, 1H), 6.52-6.51 (m, 1H), 6.27-6.24 (m, 1H), 4.98-4.97 (m, 1H), 4.05 (s, 3H), 4.02-4.01 (m, 2H), 4.00-3.99 (m, 2H), 3.98 (s, 3H), 3.78-3.77 (m, 2H), 2.99 (s, 6H), 2.14-2.11 (m, 2H), 1. 97-1. 95 (m, 2H); MS (ESI): m/z 558 [M+1]⁺ | 33 | 5.10 100 (a) |
| 121 | 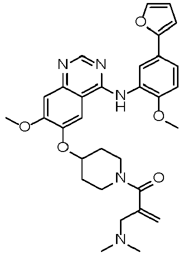 | 2-((dimethylami no)methyl)-1-(4-((4-((5-(furan-2-yl)-2-methoxyphenyl )amino)-7-methoxyquinaz olin-6-yl)oxy)piperi din-1-yl)prop-2-en-1-one | ¹H NMR (400MHz, FA salt, Methanol-*d*₄) δ 8.20 (s, 1H), 7.88 (d, *J* = 2.0 Hz, 1H), 7.62 (s, 1H), 7.51-7.49 (m, 1H), 7.41 (d, *J =* 1.6 Hz, 1H), 7.07-7.04 (m, 2H), 6.55 (d, *J* = 3.2 Hz, 1H), 6.39-6.37 (m, 1H), 5.38 (s, 1H), 5.19 (s, 1H), 4.52-4.48 (m, 1H), 3.89 (s, 3H), 3.77 (s, 3H), 3.16 (s, 2H), 3.03 (s, 3H), 2.90 (s, 3H), 2.76-2.75 (m, 2H), 2.34 (t, *J* = 8.8 Hz, 2H), 2.00-1.96 (m, 2H), 1. 82-1.79 (m, 2H); MS (ESI): m/z 558 [M+1]⁺ | 22 | 4.85 99 (a) |
| 122 | 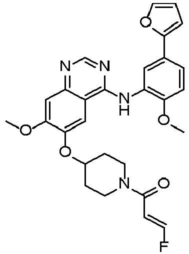 | (E)-3-fluoro-1-(4-((4-((5-(furan-2-yl)-2-methoxyphenyl )amino)-7-methoxyquinaz olin-6-yl)oxy)piperi din-1-yl)prop-2-en-1-one | MS (ESI): m/z 519 [M+1]⁺ | | |
| 123 | 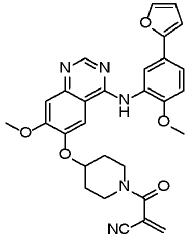 | 2-(4-((4-((5-(furan-2-yl)-2-methoxyphenyl )amino)-7-methoxyquinaz olin-6-yl)oxy)piperi din-1-carbonyl)acry lonitrile | MS (ESI): m/z 526 [M+1]⁺ | | |
| 124 | 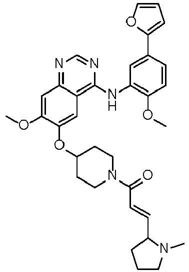 | (E)-1-(4-((4-((5-(furan-2-yl)-2-methoxyphenyl )amino)-7-methoxyquinaz olin-6-yl)oxy)piperi din-1-yl)-3-(1-methylpyrroli din-2-yl)prop-2-en-1-one | MS (ESI): m/z 584 [M+1]⁺ | | |
| 125 | 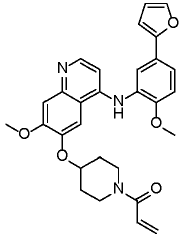 | 1-(4-((4-((5-(furan-2-yl)-2-methoxyphenyl )amino)-7-methoxyquinol in-6-yl)oxy)piperi din-1-yl)prop-2-en-1-one | ¹H NMR (400MHz, FA salt, Methanol-*d*₄) δ 8.18 (d, *J* = 6.4 Hz, 1H), 7.91 (s, 1H), 7.73-7.70 (m, 1H), 7.68 (d, *J* = 2.4 Hz, 1H), 7.52 (d, *J* = 1.6 Hz, 1H), 7.29 (s, 1H), 7.25 (d, *J =* 8.8 Hz, 1H), 6.84-6.78 (m, 1H), 6.68 (d, *J* = 3.6 Hz, 1H), 6.50-6.49 (m, 1H), 6.45 (d, *J* = 6.4 Hz, 1H), 6.24-6.19 (m, 1H), 5.77-5.74 (m, 1H), 4.04 (s, 3H), 3.98-3.94 (m, 2H), 3.87 (s, 3H), 3.67-3.61 (m, 2H), 2.08-2.07 (m, 2H), 1. 92-1. 89 (m, 2H); MS (ESI): m/z=500 [M+1]⁺ | | |
| 126 | 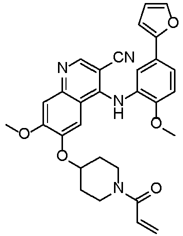 | 6-((1-acryloylpiper idin-4-yl)oxy)-4-((5-(furan-2-yl)-2-methoxyphenyl )amino)-7-methoxyquinol in-3-carbonitrile | MS (ESI): m/z=525 [M+1]⁺ | | |
| 127 | 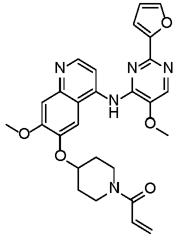 | 1-(4-((4-((2-(furan-2-yl)-5-methoxypyrimi din-4-yl)amino)-7-methoxyquinol in-6-yl)oxy)piperi din-1-yl)prop-2-en-1-one | MS (ESI): m/z=502 [M+1]⁺ | | |
| 128 | 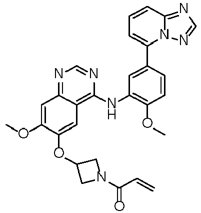 | 1-(3-((4-((5-([1,2,4]triaz olo[1,5-a]pyridin-5-yl)-2-methoxyphenyl )amino)-7-methoxyquinaz olin-6-yl)oxy)azetid in-1-yl)prop-2-en-1-one | ¹H NMR (400MHz, free base, MeOD-*d*₄) δ 8.64 (s, 1H), 8.49 (s, 1H), 8.32 (d, *J =* 2.0 Hz, 1H), 8.14-8.11 (m, 1H), 7.85-7.81 (m, 2H), 7.73 (s, 1H), 7.44-7.42 (m, 2H), 7.30 (s, 1H), 6.41-6.29 (m, 2H), 5.82-5.79 (m, 1H), 5.36-5.31 (m, 1H), 5.03-4.99 (m, 1H), 4.71-4.68 (m, 1H), 4.66-4.65 (m, 1H), 4.19-4.18 (m, 1H), 4.15 (s, 3H), 4.13 (s, 3H); MS (ESI): m/z 524 [M+1]⁺ | 10 | 4.02 100 |
| 129 | 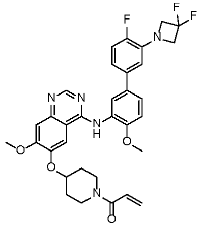 | 1-(4-((4-((3'-(3,3-difluoroazeti din-1-yl)-4'-fluoro-4-methoxy-[1,1'-biphenyl]-3-yl)amino)-7-methoxyquinaz olin-6-yl)oxy)piperi din-1-yl)prop-2-en-1-one | ¹H NMR (400 MHz, TFA salt, Methanol-*d*₄) δ8.32 (s, 1H), 7.87-7.85 (m, 2H), 7.55-7.53 (m, 1H), 7.22 (s, 1H), 7.20 (s, 1H), 7.11-7.08 (m, 2H), 6.87-6.84 (m, 2H), 6.26-6.22 (m, 1H), 5.80-5.77 (m, 1H), 4.88-4.87 (m, 1H), 4.40-4.36 (m, 4H), 4.03-4.02 (m, 4H), 3.91 (s, 3H), 3.72-3.70 (m, 2H), 2.13-2.11 (m, 2H), 1.98-1.96 (m, 2H); MS (ESI): m/z 620 [M+H] ⁺ | 17 | 11.40 100 (b) |
| 130 | 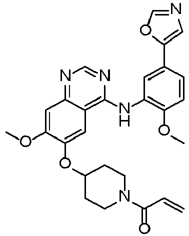 | 1-(4-((7-methoxy-4-((2-methoxy-5- (oxazol-5-yl)phenyl) ami no)quinazolin -6-yl)oxy)piperi din-1-yl)prop-2-en-1-one | ¹H NMR (400MHz, FA salt, Methanol-*d*₄) δ 8.34 (s, 1H), 8.21 (s, 1H), 8.07 (d, *J* = 2.4 Hz, 1H), 7.83 (s, 1H), 7.68-7.65 (m, 1H), 7.42 (s, 1H), 7.24-7.18 (m, 2H), 6.85-6.78 (m, 1H), 6.24-6.19 (m, 1H), 5.77-5.74 (m, 1H), 4.02 (s, 3H), 3.98-3.92 (m, 5H), 3.68-3.59 (m, 2H), 2.03 (brs, 2H), 1.93-1.90 (m, 2H); MS (ESI): m/z 502 [M+1] ⁺ | 1 | 4.80 90 (a) |
| 131 | 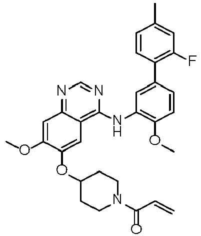 | 1-(4-((4-((2'-fluoro-4-methoxy-4'-methyl-[1,1'-biphenyl]-3-yl)amino)-7-methoxyquinaz olin-6-yl)oxy)piperi din-1-yl)prop-2-en-1-one | ¹H NMR (400 MHz, free base, Methanol-*d*₄) δ8.32 (s, 1H), 7.84 (s, 1H), 7.82 (s, 1H), 7.51-7.48 (m, 1H), 7.43-7.39 (m, 1H), 7.22-7.20 (m, 2H), 7.09 (d, *J* = 8.0 Hz, 1H), 7.03 (d, *J* = 12.0 Hz, 1H), 6.87-6.80 (m, 1H), 6.26-6.21 (m, 1H), 5.79-5.76 (m, 1H), 4.62-4.61 (m, 1H), 4.01 (s, 3H), 3.99-3.98 (m, 2H), 3.91 (s, 3H), 3.69-3.67 (m, 2H), 2.39 (s, 3H), 2.11-2.09 (m, 2H), 1.92-1.90 (m, 2H); MS (ESI): m/z 543 [M+H]⁺ | 33 | 11.02 98 (b) |
| 132 | 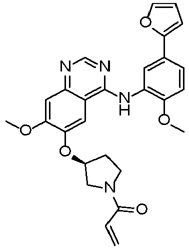 | (S)-1-(3-((4-((5-(furan-2-yl)-2-methoxyphenyl )amino)-7-methoxyquinaz olin-6-yl)oxy)pyrrol idin-1-yl)prop-2-en-1-one | MS (ESI): m/z 487 [M+H]⁺ | | |
| 133 | 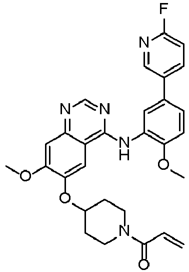 | 1-(4-((4-((5-(6-fluoropyridin -3-yl)-2-methoxyphenyl )amino)-7-methoxyquinaz olin-6-yl)oxy)piperi din-1-yl)prop-2-en-1-one | ¹H NMR (400MHz, FA salt, Methanol-*d*₄) δ 8.44 (d, *J* = 2.4 Hz, 1H), 8.21-8.16 (m, 2H), 7.98 (d, *J* = 2.4 Hz, 1H), 7.84 (s, 1H), 7.58-7.56 (m, 1H), 7.25 (d, *J* = 8.4 Hz, 1H), 7.21 (s, 1H), 7.15-7.12 (m, 1H), 6.85-6.78 (m, 1H), 6.24-6.19 (m, 1H), 5.77-5.74 (m, 1H), 4.02 (s, 3H), 3.98-3.92 (m, 5H), 3.71-3.63 (m, 2H), 2.09-2.08 (m, 2H), 1.93-1.91 (m, 2H); MS (ESI): m/z 530 [M+1]⁺ | 83 | 5.69 100 (a) |
| 134 | 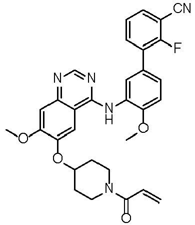 | 3'-((6-((1-acryloylpiper idin-4-yl)oxy)-7-methoxyquinaz olin-4-yl)amino)-2-fluoro-4'-methoxy-[1,1'- biphenyl]-3-carbonitrile | ¹H NMR (400 MHz, free base, Methanol-*d*₄) δ 8.34 (s, 1H), 7.94 (s, 1H), 7.89-7.85 (m, 2H), 7.73-7.72 (m, 1H), 7.55-7.52 (m, 1H), 7.47-7.43 (m, 1H), 7.29 (d, *J =* 8.8 Hz, 1H), 7.23 (s, 1H), 6.87-6.81 (m, 1H), 6.26-6.21 (m, 1H), 5.79-5.76 (m, 1H), 4.62-4.60 (m, 1H), 4.03 (s, 3H), 3.98-3.94 (m, 5H), 3.70-3.68 (m, 2H), 2.11-2.09 (m, 2H), 1.93-1.92 (m, 2H); MS (ESI): m/z 554 [M+H]⁺ | 19 | 6.12 94 (a) |
| 135 | 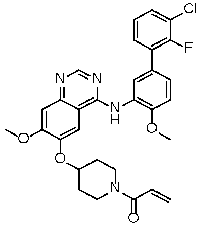 | 1-(4-((4-((3'-chloro-2'-fluoro-4-methoxy-[1,1'-biphenyl]-3-yl)amino)-7-methoxyquinaz olin-6-yl)oxy)piperi din-1-yl)prop-2-en-1-one | ¹H NMR (400MHz, FA salt, Methanol-*d*₄) δ 8.34 (s, 1H), 7.85-7.84 (m, 2H), 7.51-7.41 (m, 3H), 7.25-7.20 (m, 3H), 6.85-6.78 (m, 1H), 6.24-6.19 (m, 1H), 5.77-5.74 (m, 1H), 4.02 (s, 3H), 3.98-3.92 (m, 5H), 3.70-3.63 (m, 2H), 2.09-2.08 (m, 2H), 1.91-1.89 (m, 2H); MS (ESI): m/z 564 [M+1]⁺ | 30 | 6.86 100 (a) |
| 136 | 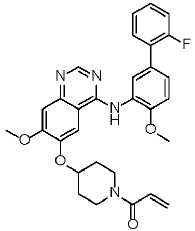 | 1-(4-((4-((2'-fluoro-4-methoxy-[1,1'- biphenyl]-3-yl)amino)-7-methoxyquinaz olin-6-yl)oxy)piperi din-1-yl)prop-2-en-1-one | ¹H NMR (400MHz, free base, Methanol-*d*₄) δ 8.30 (s, 1H), 7.84-7.83 (m, 2H), 7.54-7.48 (m, 2H), 7.34-7.30 (m, 1H), 7.26-7.14 (m, 4H), 6.85-6.78 (m, 1H), 6.24-6.19 (m, 1H), 5.77-5.74 (m, 1H), 4.01-3.91 (m, 8H), 3.69-3.64 (m, 2H), 2.08-2.07 (m, 2H), 1.90-1.89 (m, 2H); MS (ESI): m/z 529 [M+1]⁺ | 97 | 6.37 99 (a) |
| 137 | 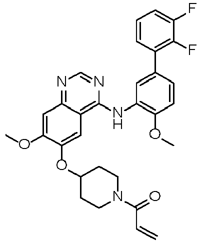 | 1-(4-((4-((2' ,3'-difluoro-4-methoxy-[1,1'- biphenyl]-3-yl)amino)-7-methoxyquinaz olin-6-yl)oxy)piperi din-1-yl)prop-2-en-1-one | ¹H NMR (400MHz, HCl salt, Methanol-*d*₄) δ 8.49 (s, 1H), 7.97 (s, 1H), 7.82 (s, 1H), 7.61-7.59 (m, 1H), 7.32-7.23 (m, 5H), 6.88-6.81 (m, 1H), 6.27-6.22 (m, 1H), 5.80-5.77 (m, 1H), 4.87-4.85 (m, 1H), 4.07 (s, 3H), 3.98-3.96 (m, 2H), 3.94 (s, 3H), 3.70-3.68 (m, 2H), 2.18-2.07 (m, 2H), 2.00-1.89 (m, 2H); MS (ESI): m/z 547 [M+1] ⁺ | 94 | 6.48 99 (a) |
| 138 | 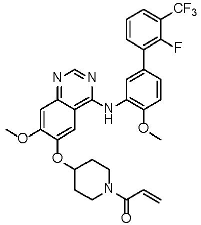 | 1-(4-((4-((2'-fluoro-4-methoxy-3'-(trifluoromet hyl)-[1,1'-biphenyl]-3-yl)amino)-7-methoxyquinaz olin-6-yl)oxy)piperi din-1-yl)prop-2-en-1-one | ¹H NMR (400 MHz, free base, Methanol-*d*₄) δ 8.34 (s, 1H), 7.90 (s, 1H), 7.85-7.81 (m, 2H), 7.69-7.66 (m, 1H), 7.55-7.52 (m, 1H), 7.46-7.42 (m, 1H), 7.29 (d, *J =* 8.4 Hz, 1H), 7.23 (s, 1H), 6.87-6.81 (m, 1H), 6.26-6.21 (m, 1H), 5.79-5.76 (m, 1H), 4.66-4.65 (m, 1H), 4.03 (s, 3H), 3.99-3.95 (m, 5H), 3.70-3.67 (m, 2H), 2.11-2.09 (m, 2H), 1.93-1.91 (m, 2H); MS (ESI): m/z 597 [M+H] ⁺ | 54 | 7.01 100 (a) |
| 139 | 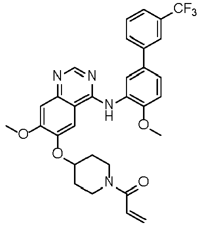 | 1-(4-((7-methoxy-4-((4-methoxy-3'-(trifluoromet hyl)-[1,1'-biphenyl]-3-y1)amino)quin azolin-6-yl)oxy)piperi din-1-yl)prop-2-en-1-one | ¹H NMR (400MHz, HCl salt, DMSO-*d*₆) δ 10.83 (s, 1H), 8.63 (s, 1H), 8.19 (s, 1H), 8.01-7.97 (m, 2H), 7.90 (d, *J* = 2.4 Hz, 1H), 7.83-7.80 (m, 1H), 7.70-7.69 (m, 2H), 7.34 (d, *J =* 8.8 Hz, 1H), 7.28 (s, 1H), 7.26 (t, *J* = 10.8 Hz, 1H), 6.88-6.82 (m, 1H), 6.14-6.09 (m, 1H), 5.71-5.67 (m, 1H), 4.88-4.86 (m, 1H), 4.05-3.91 (m, 8H), 2.08-2.07 (m, 2H), 1.75-1.69 (m, 2H); MS (ESI): m/z 579 [M+1] ⁺ | 91 | 6.88 99 (a) |
| 140 | 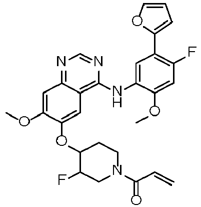 | 1-(3-fluoro-4-((4-((4-fluoro-5-(furan-2-yl)- 2-methoxyphenyl )amino)-7-methoxyquinaz olin-6-yl)oxy)piperi din-1-yl)prop-2-en-1-one | ¹H NMR (400 MHz, TFA salt, Methanol-*d*₄) δ 8.54 (d, *J* = 1.6 Hz, 1H), 8.08 (s, 1H), 7.85-7.82 (m, 1H), 7.49 (d, *J* = 2.4 Hz, 1H), 7.20 (s, 1H), 7.06 (d, *J* = 12.4 Hz, 1H), 6.77-6.69 (m, 2H), 6.49-6.48 (m, 1H), 6.19-6.14 (m, 1H), 5.73-5.70 (m, 1H), 5.01-5.00 (m, 2H), 4.02 (s, 3H), 3.99-3.97 (m, 2H), 3.94 (s, 3H), 3.79-3.77 (m, 2H), 2.22-1.91 (m, 2H); MS (ESI): m/z 537 [M+H]⁺ | 27 | 6.30, 100 (a) |
| 141 | 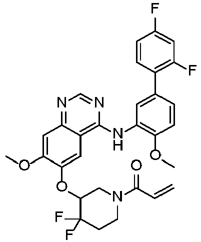 | 1-(3-((4-((2',4'-difluoro-4-methoxy-[1,1'-biphenyl]-3-yl)amino)-7-methoxyquinaz olin-6-yl)oxy)-4,4-difluoropiper idin-1-yl)prop-2-en-1-one | ¹H NMR (400MHz, FA salt, Methanol-*d*₄) δ 8.37 (s, 1H), 7.98 (s, 1H), 7.92-7.88 (m, 1H), 7.59-7.53 (m, 1H), 7.48-7.46 (m, 1H), 7.24-7.22 (m, 2H), 7.08-7.03 (m, 2H), 6.91-6.51 (m, 1H), 6.17-6.13 (m, 1H), 5.88-5.56 (m, 1H), 4.88-4.60 (m, 2H), 4.01 (s, 3H), 3.95 (s, 3H), 3.71-3.50 (m, 2H), 2.47-2.16 (m, 2H); MS (ESI): m/z 583 [M+1]⁺ | 9 | 7.03 99 (a) |
| 142 | 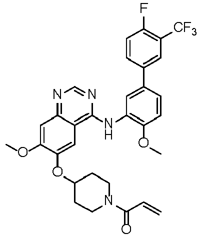 | 1-(4-((4-((4'-fluoro-4-methoxy-3'- (trifluoromet hyl)-[1,1'-biphenyl]-3-yl)amino)-7-methoxyquinaz olin-6-yl)oxy)piperi din-1-yl)prop-2-en-1-one | ¹H NMR (400 MHz, free base, Methanol-*d*₄) δ 8.35 (s, 1H), 7.99 (d, *J =* 2.0 Hz, 1H), 7.95-7.89 (m, 2H), 7.84 (s, 1H), 7.59-7.56 (m, 1H), 7.44-7.39 (m, 1H), 7.27-7.23 (m, 2H), 6.87-6.80 (m, 1H), 6.26-6.21 (m, 1H), 5.79-5.76 (m, 1H), 4.61-4.60 (m, 1H), 4.03 (s, 3H), 3.98-3.94 (m, 5H), 3.35-3.32 (m, 2H), 2.11-2.09 (m, 2H), 1.93-1.91 (m, 2H); MS (ESI): m/z 597 [M+H]⁺ | 57 | 7.12, 100 (a) |
| 143 | 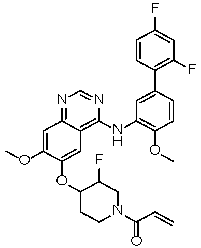 | 1-(4-((4-((2',4'-difluoro-4-methoxy-[1,1'-biphenyl]-3-yl)amino)-7-methoxyquinaz olin-6-yl)oxy)-3-fluoropiperid in-1-yl)prop-2-en-1-one | ¹H NMR (400MHz, free base, MeOD) δ 8.33 (s, 1H), 7.95-7.85 (m, 2H), 7.57-7.51 (m, 1H), 7.47-7.44 (m, 1H), 7.23-7.21 (m, 2H), 7.06-7.02 (m, 2H), 6.85-6.78 (m, 1H), 6.26 (d, *J =* 16.8 Hz, 1H), 5.81 (d, *J =* 16.8 Hz, 1H), 4.70-4.60 (m, 2H), 4.20-3.70 (m, 9H), 3.65-3.45 (m, 1H), 2.30-1.90 (m, 2H); MS (ESI): m/z 565 [M+1]⁺ | 55 | 6.52 100 |
| 144 | 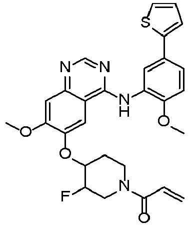 | 1-(3-fluoro-4-((7-methoxy-4-((2-methoxy-5-(thiophen-2-yl)phenyl)ami no)quinazolin -6-yl)oxy)piperi din-1-yl)prop-2-en-1-one | ¹H NMR (400MHz, FA salt, Methanol-*d*₄) δ 8.36 (s, 1H), 7.96-7.94 (m, 2H), 7.57-7.54 (m, 1H), 7.32-7.30 (m, 2H), 7.23 (s, 1H), 7.16 (d, *J* = 8.4 Hz, 1H), 7.08-7.05 (m, 1H), 6.85-6.77 (m, 1H), 6.24 (d, *J* = 16.8 Hz, 1H), 5.80-5.77 (m, 1H), 4.79 (s, 1H), 4.13-3.95 (m, 6H), 3.89 (s, 3H), 3.85-3.71 (m, 1H), 2.23-2.14 (m, 1H), 1.99-1.97 (m, 1H); MS (ESI): m/z 535 [M+1] ⁺ | 18 | 6.36 99 (a) |
| 145 | 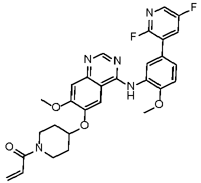 | 1-(4-((4-((5-(2,5-difluoropyrid in-3-yl)-2-methoxyphenyl )amino)-7-methoxyquinaz olin-6-yl)oxy)piperi din-1-yl)prop-2-en-1-one | ¹H NMR (400 MHz, FA salt, Methanol-*d*₄) δ 8.42 (s, 1H), 8.18-8.17 (m, 1H), 8.03 (s, 1H), 7.90 (s, 1H), 7.72 (d, *J* = 1.6 Hz, 1H), 7.33-7.24 (m, 2H), 6.87-6.80 (m, 1H), 6.26-6.21 (m, 1H), 5.80-5.77 (m, 1H), 5.02-5.01 (m, 1H), 4.05 (s, 3H), 3.96-3.95 (m, 5H), 3.69-3.68 (m, 2H), 2.11-2.10 (m, 2H), 1. 93-1. 92 (m, 2H); MS (ESI): m/z 548 [M+H] ⁺ | 36 | 5.74, 90 (NMR) (a) |
| 146 | 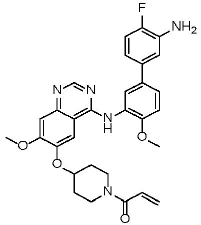 | 1-(4-((4-((3'-amino-4'-fluoro-4-methoxy-[1,1'-biphenyl]-3-yl)amino)-7-methoxyquinaz olin-6-yl)oxy)piperi din-1-yl)prop-2-en-1-one | ¹H NMR (400 MHz, TFA salt, Methanol-*d*₄) δ 8.47 (s, 1H), 7.95 (s, 1H), 7.78 (d, *J* = 1.2 Hz, 1H), 7.57-7.55 (m, 1H), 7.24-7.21 (m, 2H), 7.11-7.09 (m, 1H), 7.01-6.99 (m, 1H), 6.90-6.80 (m, 2H), 6.26-6.25 (m, 1H), 5.80-5.78 (m, 1H), 4.91-4.90 (m, 1H), 4.07 (s, 3H), 3.91-3.89 (m ,5H), 3.71-3.70 (m, 2H), 2.05-2.04 (m, 2H), 1. 99-1. 98 (m, 2H); MS (ESI): m/z 544 [M+H] ⁺ | 11 | 5.32, 98 (a) |
| 147 | 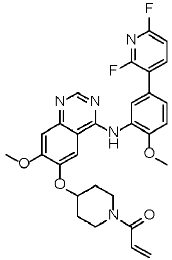 | 1-(4-((4-((5-(2,6-difluoropyrid in-3-yl)-2-methoxyphenyl )amino)-7-methoxyquinaz olin-6-yl)oxy)piperi din-1-yl)prop-2-en-1-one | ¹H NMR (400MHz, FA salt, DMSO-*d*₆) δ 9.23 (s, 1H), 8.35-8.32 (m, 1H), 8.30 (s, 1H), 7.93 (s, 1H), 7.76 (s, 1H), 7.54-7.52 (m, 1H), 7.30-7.28 (m, 2H), 7.20 (s, 1H), 6.89-6.82 (m, 1H), 6.15-6.10 (m, 1H), 5.76-5.68 (m, 1H), 4.81-4.80 (m, 1H), 3.95-3.92 (m, 5H), 3.84 (s, 1H), 3.60-3.50 (m, 2H), 2.09-2.05 (m, 2H), 1. 70-1. 69 (m, 2H); MS (ESI): m/z 548 [M+1] ⁺ | 61 | 6.07 100 (a) |
| 148 | 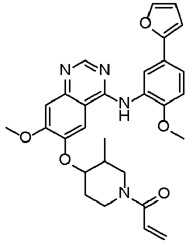 | 1-(4-((4-((5-(furan-2-yl)-2-methoxyphenyl )amino)-7-methoxyquinaz olin-6-yl)oxy)-3-methylpiperid in-1-yl)prop-2-en-1-one | ¹H NMR (400MHz, FA salt, Methanol-*d*₄) δ 8.36 (s, 1H), 7.98-7.97 (m, 1H), 7.85-7.83 (m, 1H), 7.65-7.63 (m, 1H), 7.53-7.52 (m, 1H), 7.23-7.22 (m, 1H), 7.20-7.17 (m, 1H), 6.98-6.97 (m, 1H), 6.51-6.50 (m, 1H), 6.26-6.10 (m, 1H), 5.78-5.70 (m, 1H), 4.88-4.83 (m, 1H), 4.50-4.10 (m, 1H), 4.05 (s, 3H), 3.90 (s, 3H), 3.88-3.80 (m, 1H), 3.73-3.42 (m, 2H), 2.40-2.00 (m, 2H), 1.92-1.63 (m, 1H), 1.13-1.10 (m, 3H); MS (ESI): m/z 515 [M+1]⁺ | 45 | 6.27 100 (a) |
| 149 | 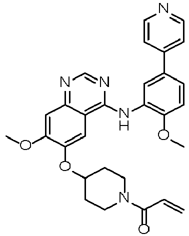 | 1-(4-((7-methoxy-4-((2-methoxy-5-(pyridin-4-yl)phenyl)ami no)quinazolin -6-yl)oxy)piperi din-1-yl)prop-2-en-1-one | ¹H NMR (400MHz, FA salt, Methanol-*d*₄) δ 8.56 (s, 2H), 8.37 (s, 1H), 8.15 (d, *J* = 2.4 Hz, 1H), 7.85 (s, 1H), 7.76-7.73 (m, 3H), 7.29 (d, *J* = 8.4 Hz, 1H), 7.22 (s, 1H), 6.86-6.79 (m, 1H), 6.24-6.20 (m, 1H), 5.78-5.75 (m, 1H), 4.02 (s, 3H), 3.99-3.93 (m, 5H), 3.72-3.64 (m, 2H), 2.09-2.08 (m, 2H), 1.94-1.91 (m, 2H); MS (ESI): m/z 512 [M+1]⁺ | 72 | 3.46 94 (a) |
| 150 | 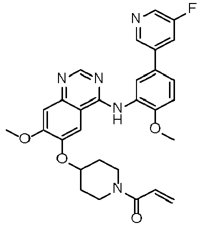 | 1-(4-((4-((5-(5-fluoropyridin -3-yl)-2-methoxyphenyl )amino)-7-methoxyquinaz olin-6-yl)oxy)piperi din-1-yl)prop-2-en-1-one | ¹H NMR (400MHz, FA salt, Methanol-*d*₄) δ 8.69 (s, 1H), 8.40 (s, 2H), 8.04 (d, *J* = 2 Hz, 1H), 7.90-7.85 (m, 2H), 7.66-7.63 (m, 1H), 7.27 (d, *J* = 8.8 Hz, 1H), 7.21 (s, 1H), 6.85-6.78 (m, 1H), 6.24-6.19 (m, 1H), 5.77-5.74 (m, 1H), 4.02 (s, 3H), 4.00-3.93 (m, 5H), 3.68-3.66 (m, 2H), 2.08 (brs, 2H), 1.94-1.91 (m, 2H); MS (ESI): m/z 530 [M+1]⁺ | 10 | 4.86 94 (a) |
| 151 | 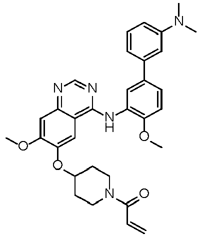 | 1-(4-((4-((3'-(dimethylamin o)-4-methoxy-[1,1'- biphenyl]-3-yl)amino)-7-methoxyquinaz olin-6-yl)oxy)piperi din-1-yl)prop-2-en-1-one | ¹H NMR (400MHz, TFA salt, Methanol-*d*₄) δ 8.41 (s, 1H), 7.89 (s, 1H), 7.67-7.66 (m, 1H), 7.55-7.52 (m, 1H), 7.18-7.12 (m, 3H), 6.87-6.84 (m, 2H), 6.75-6.68 (m, 2H), 6.15-6.10 (m, 1H), 5.68-5.65 (m, 1H), 4.85-4.75 (m, 1H), 3.97 (s, 3H), 3.90-3.81 (m, 2H), 3.80 (s, 3H), 3.62-3.50 (m, 2H), 2.88 (s, 6H), 2.10-2.00 (m, 2H), 1. 98-1. 84 (m, 2H); MS (ESI): m/z 554 [M+1]⁺ | 6 | 4.48 90 (NMR) (a) |
| 152 | 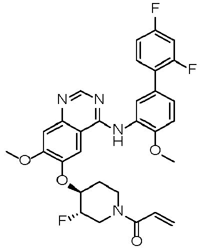 | 1-((3S,4S)-4-((4-((2',4'-difluoro-4-methoxy-[1,1'- biphenyl]-3-yl)amino)-7-methoxyquinaz olin-6-yl)oxy)-3-fluoropiperid in-1-yl)prop-2-en-1-one | ¹H NMR (400MHz, HCl salt, Methanol-*d*₄) δ 8.33 (s, 1H), 7.89 (s, 1H), 7.88 (s, 1H), 7.54-7.48 (m, 1H), 7.43-7.40 (m, 1H), 7.19-7.17 (m, 2H), 7.04-7.02 (m, 2H), 6.84-6.76 (m, 1H), 6.26 (d, *J* = 16.8 Hz, 1H), 5.80-5.77 (m, 1H), 4.88-4.77 (m, 2H), 4.14-3.53 (m, 9H), 2.22-2.10 (m, 1H), 1.97-1.95 (m, 1H); MS (ESI): m/z 565 [M+1]⁺ | 52 | 6.50 98 (a) |
| 153 | 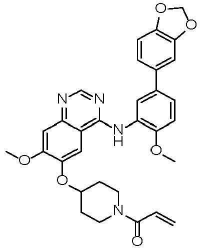 | 1-(4-((4-((5-(benzo[d][1,3 ]dioxol-5-yl)-2-methoxyphenyl )amino)-7-methoxyquinaz olin-6-yl)oxy)piperi din-1-yl)prop-2-en-1-one | ¹H NMR (400MHz, TFA salt, DMSO-*d*₆) δ 11.01 (s, 1H), 8.74 (s, 1H), 8.14 (s, 1H), 7.70-7.66 (m, 2H), 7.29-7.24 (m, 3H), 7.15-7.13 (m, 1H), 6.99 (d, *J =* 8.0 Hz, 1H), 6.88-6.81 (m, 1H), 6.14-6.10 (m, 1H), 6.09 (s, 2H), 5.71-5.65 (m, 1H), 4.01 (s, 3H), 3.89 (s, 3H), 2.33-2.32 (m, 2H), 1. 70-1. 68 (m, 2H); MS (ESI): m/z 555 [M+1]⁺ | 79 | 6.10 100 (a) |
| 154 | 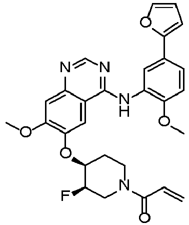 | 1-((3R,4S)-3-fluoro-4-((4-((5-(furan-2-yl)-2-methoxyphenyl )amino)-7-methoxyquinaz olin-6-yl)oxy)piperi din-1-yl)prop-2-en-1-one | ¹H NMR (400 MHz, HCl salt, Methanol-*d*₄) δ 8.54 (s, 1H), 8.04 (s, 1H), 7.86-7.85 (d, *J* = 2.14 Hz, 1H), 7.72-7.70 (d, *J* = 8.66 Hz, 1H), 7.52-7.51 (d, *J =* 1.65 Hz, 1H), 7.25-7.20 (t, 2H), 6.87-6.76 (m, 1H), 6.68-6.67 (d, *J =* 3.34 Hz, 1H), 6.51-6.50 (m, 1H), 6.25-6.21 (d, *J* = 16.63 Hz, 1H), 5.80-5.77 (d, *J* = 10.57 Hz, 1H), 5.12-5.06 (m, 1H), 4.55-4.35 (m, 2H), 4.08 (s, 3H), 3.88 (s, 3H), 3.69-3.56 (m, 1H), 3.24-3.15 (m, 1H), 2.22-2.04 (m, 2H); MS (ESI): m/z=519 [M+1]⁺ | 34 | 9.72 97 (b) |
| 155 | 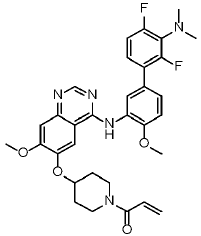 | 1-(4-((4-((3'- (dimethylamin o)-2',4'-difluoro-4-methoxy-[1,1' - biphenyl]-3-yl)amino)-7-methoxyquinaz olin-6-yl)oxy)piperi din-1-yl)prop-2-en-1-one | ¹H NMR (400 MHz, FA salt, Methanol-*d*₄) δ 8.30 (s, 1H), 7.83 (s, 1H), 7.77 (s, 1H), 7.42 (d, *J* = 8.5 Hz, 1H), 7.22-7.12 (m, 3H), 7.00-6.93 (m, 1H), 6.85-6.78 (m, 1H), 6.22 (dd, *J* = 16.8, 1.9 Hz, 1H), 5.76 (dd, *J* = 10.6, 1.9 Hz, 1H), 4.83-4.78 (m, 1H), 4.01 (s, 3H), 3.99-3.93 (m, 2H), 3.91 (s, 3H), 3.70-3.63 (m, *J* = 16.8 Hz, 2H), 2.91-2.88 (m, 6H), 2.14-2.05 (m, 2H), 1.94-1.86 (m, 2H); MS (ESI): m/z=590 [M+1]⁺ | 56 | 10.77 98 (b) |
| 156 | 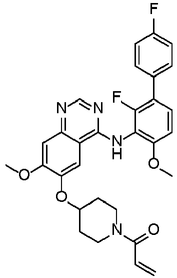 | 1-(4-((4-((2,4'-difluoro-4-methoxy-[1,1'-biphenyl]-3-yl)amino)-7-methoxyquinaz olin-6-yl)oxy)piperi din-1-yl)prop-2-en-1-one | ¹H NMR (400MHz, TFA salt, DMSO-*d*₆) δ 10.65 (s, 1H), 8.70 (s, 1H), 8.11 (s, 1H), 7.61-7.56 (m, 3H), 7.34-7.29 (m, 3H), 7.19 (d, *J* = 8.8 Hz, 1H), 6.88-6.81 (m, 1H), 6.14-6.10 (m, 1H), 5.71-5.67 (m, 1H), 5.23-5.20 (m, 1H), 4.01 (s, 3H), 3.91-3.90 (m, 2H), 3.89 (s, 3H), 2.07-2.05 (m, 2H), 1.71-1.68 (m, 2H); MS (ESI): m/z 547 [M+1] ⁺ | 64 | 6.22 98 (a) |
| 157 | 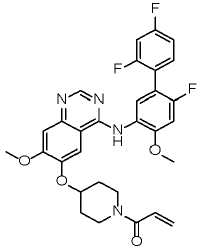 | 1-(4-((7-methoxy-4-((2',4',6-trifluoro-4-methoxy-[1,1'-biphenyl]-3-yl)amino)quin azolin-6-yl)oxy)piperi din-1-yl)prop-2-en-1-one | ¹H NMR (400MHz, TFA salt, DMSO-*d*₆) δ 11.13 (s, 1H), 8.72 (s, 1H), 8.26 (s, 1H), 7.53-7.50 (m, 2H), 7.47-7.37 (m, 1H), 7.33-7.30 (m, 2H), 7.24-7.19 (m, 1H), 6.88-6.81 (m, 1H), 6.14-6.09 (m, 1H), 5.70-5.65 (m, 1H), 4.91-4.85 (m, 1H), 3.99 (s, 3H), 3.95-3.91 (m, 2H), 3.85 (s, 3H), 2.08-2.07 (m, 2H), 1. 67-1. 65 (m, 2H); MS (ESI): m/z 565 [M+1]⁺ | 46 | 6.46 100 (a) |
| 158 | 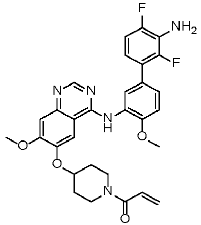 | 1-(4-((4-((3'-amino-2',4'-difluoro-4-methoxy-[1,1'-biphenyl]-3-yl)amino)-7-methoxyquinaz olin-6-yl)oxy)piperi din-1-yl)prop-2-en-1-one | ¹H NMR (400MHz, FA salt, Methanol-*d*₄) δ 8.33 (s, 1H), 7.85 (s, 1H), 7.80 (s, 1H), 7.47-7.45 (m, 1H), 7.22-7.20 (m, 2H), 6.91-6.75 (m, 3H), 6.26-6.21 (m, 1H), 5.79-5.76 (m, 1H), 4.88-4.85 (m, 1H), 4.03 (s, 3H), 4.02-3.97 (m, 2H), 3.96 (s, 3H), 3.70-3.69 (m, 2H), 2.09-1.92 (m, 4H); MS (ESI): m/z 562 [M+1] ⁺ | 37 | 8.57 99 (b) |
| 159 | 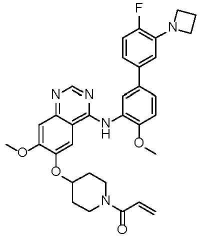 | 1-(4-((4-((3'-(azetidin-1-yl)-4'-fluoro-4-methoxy-[1,1'-biphenyl]-3-yl)amino)-7-methoxyquinaz olin-6-yl)oxy)piperi din-1-yl)prop-2-en-1-one | ¹H NMR (400 MHz, FA salt, CDCl₃) δ 8.83 (s, 1H), 8.70 (s, 1H), 8.14 (s, 1H), 7.45 (s, 1H), 7.25-7.24 (m, 1H), 7.01-6.78 (m, 2H), 6.93-6.92 (m, 1H), 6.69-6.66 (m, 1H), 6.63-6.59 (m, 1H), 6.34-6.29 (m, 1H), 5.75-5.72 (m, 1H), 4.68-4.65 (m, 1H), 4.06-4.01 (m, 14H), 3.72-3.65 (m, 2H), 2.01-1.99 (m, 4H); MS (ESI): m/z 584 [M+H]⁺ | 13 | 10.95 100 (b) |
| 160 | 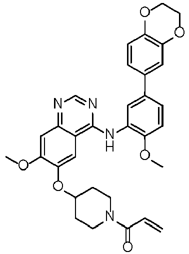 | 1-(4-((4-((5-(2,3-dihydrobenzo[ b][1,4]dioxin -6-yl)-2-methoxyphenyl )amino)-7-methoxyquinaz olin-6-yl)oxy)piperi din-1-yl)prop-2-en-1-one | ¹H NMR (400 MHz, FA salt, CDCl₃) δ 8.93-8.92 (d, *J =* 2.20 Hz, 1H), 8.73 (s, 1H), 7.89 (s, 1H), 7.30 (s, 1H), 7.25-7.23 (m, 2H), 7.17-7.12 (m, 2H), 7.01-6.93 (m, 2H), 6.66-6.59 (m, 1H), 6.34-6.29 (dd, *J* = 16.83 Hz, 1H), 5.74-5.71 (dd, *J* = 10.56 Hz, 1H), 4.70-4.65 (m, 1H), 4.31 (s, 4H), 4.01-4.00 (d, J=2.19Hz, 7H), 3.94-3.86 (m, 1H), 3.73-3.65 (m, 1H), 3.57-3.48 (m, 1H), 2.07-1.93 (m, 4H); MS (ESI): m/z=569 [M+1]⁺ | 64 | 10.13 98 (b) |
| 161 | 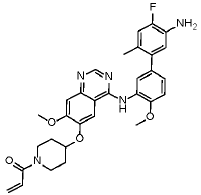 | 1-(4-((4-((5'-amino-4'-fluoro-4-methoxy-2'-methyl-[1,1'-biphenyl]-3-yl)amino)-7-methoxyquinaz olin-6-yl)oxy)piperi din-1-yl)prop-2-en-1-one | ¹H NMR (400 MHz, TFA salt, DMSO-*d*₆) δ 10.86 (s, 1H), 8.73 (s, 1H), 8.11 (s, 1H), 7.34-7.26 (m, 4H), 6.95-6.82 (m, 1H), 6.68 (d, *J* = 9.6 Hz, 1H), 6.15-6.10 (m, 1H), 5.76 (s, 1H), 5.72-5.69 (m, 1H), 4.83-4.82 (m, 1H), 4.01 (s, 3H), 3.84-3.80 (m, 5H), 3.56-3.54 (m, 2H), 2.12-2.10 (m, 5H), 1.72-1.70 (m, 2H); MS (ESI): m/z 558 [M+H] ⁺ | 99 | 5.30 57 (a) |
| 162 | 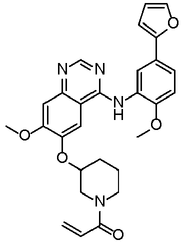 | 1-(3-((4-((5-(furan-2-yl)-2-methoxyphenyl )amino)-7-methoxyquinaz olin-6-yl)oxy)piperi din-1-yl)prop-2-en-1-one | ¹H NMR (400 MHz, TFA salt, Methanol-*d*₄) δ8.61 (s, 1H), 8.08 (d, *J =* 17.6 Hz, 1H), 7.86-7.84 (m, 1H), 7.76-7.74 (m, 1H), 7.55 (d, *J* = 1.2 Hz, 1H), 7.26-7.23 (m, 2H), 6.87-6.59 (m, 2H), 6.53-6.52 (m, 1H), 6.27-6.21 (m, 1H), 5.80-5.62 (m, 1H), 4.15-4.11 (m, 2H), 4.09 (s, 3H), 4.06 (s, 3H), 3.91-3.77 (m, 3H), 2.18-2.03 (m, 3H), 1.31-1.24 (m, 1H); MS (ESI): m/z 501 [M+H]⁺ | 5 | 6.14 100 (a) |
| 163 | 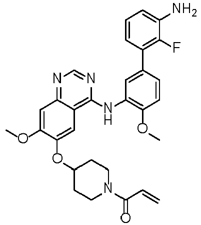 | 1-(4-((4-((3'-amino-2'-fluoro-4-methoxy-[1,1'-biphenyl]-3-yl)amino)-7-methoxyquinaz olin-6-yl)oxy)piperi din-1-yl)prop-2-en-1-one | ¹H NMR (400 MHz, TFA salt, Methanol-*d*₄) δ 8.60 (s, 1H), 8.07 (s, 1H), 7.71 (s, 1H), 7.64 (d, *J* = 8.4 Hz, 1H), 7.30-7.26 (m, 2H), 7.08-7.04 (m, 1H), 6.97-6.81 (m, 3H), 6.27-6.22 (m, 1H), 5.80-5.78 (m, 1H), 5.02-5.01 (m, 1H), 4.11 (s, 3H), 3.99-3.93 (m, 5H), 3.70-3.69 (m, 2H), 2.17-2.15 (m, 2H), 2.02-1.99 (m, 2H); MS (ESI): m/z 544 [M+H] ⁺ | 76 | 5.08 99 (a) |
| 164 | 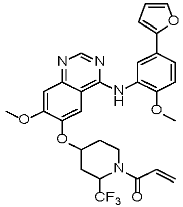 | 1-(4-((4-((5-(furan-2-yl)-2-methoxyphenyl )amino)-7-methoxyquinaz olin-6-yl)oxy)-2-(trifluoromet hyl)piperidin -1-yl)prop-2-en-1-one | ¹H NMR (400MHz, TFA salt, Methanol-*d*₄) δ 8.37 (s, 1H), 8.33 (s, 1H), 8.08 (s, 1H, formic acid), 7.79 (s, 1H), 7.63 (d, *J* = 8.8 Hz, 1H), 7.52 (s, 1H), 7.22 (s, 1H), 7.18 (d, *J* = 8.4 Hz, 1H), 6.88-6.81 (m, 1H), 6.67 (s, 1H), 6.50 (s, 1H), 6.32-6.20 (m, 1H), 5.86 (d, *J* = 10.4Hz, 1H), 4.70-4.50 (m, 2H), 4.30-4.10 (m, 1H), 4.01 (s, 3H), 3.90 (s, 3H), 3.55-3.45 (m, 1H), 2.60-2.50 (m, 1H), 2.44-2.36 (m, 1H), 2.20-1.95 (m, 2H); MS (ESI): m/z 569 [M+1]⁺ | 24 | 6.80 93 (a) |
| 165 | 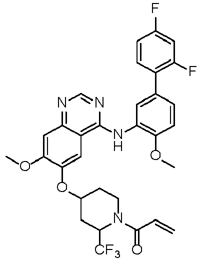 | 1-(4-((4-((2',4'-difluoro-4-methoxy-[1,1'-biphenyl]-3-yl)amino)-7-methoxyquinaz olin-6-yl)oxy)-2-(trifluoromet hyl)piperidin -1-yl)prop-2-en-1-one | ¹H NMR (400MHz, FA salt, Methanol-*d*₄) δ 8.35 (s, 1H), 8.30 (s, 1H), 7.91 (s, 1H, formic acid), 7.86 (s, 1H), 7.57-7.54 (m, 1H), 7.47-7.44 (m, 1H), 7.23-7.20 (m, 2H), 7.06-7.01 (m, 2H), 6.88-6.81 (m, 1H), 6.32-6.20 (m, 1H), 5.86 (d, *J* = 10.4 Hz, 1H), 4.70-4.50 (m, 2H), 4.30-4.10 (m, 1H), 4.01 (s, 3H), 3.93 (s, 3H), 3.55-3.45 (m, 1H), 2.60-2.50 (m, 1H), 2.44-2.36 (m, 1H), 2.20-1.95 (m, 2H); MS (ESI): m/z 615 [M+1]⁺ | 48 | 7.21 92 (a) |
| 166 | 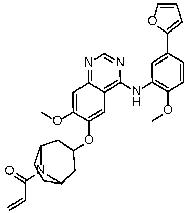 | 1-(3-((4-((5-(furan-2-yl)-2-methoxyphenyl )amino)-7-methoxyquinaz olin-6-yl)oxy)-8-azabicyclo[3. 2.1]octan-8-yl)prop-2-en-1-one | ¹H NMR (400 MHz, TFA salt, Methanol-*d*₄) δ 8.59 (s, 1H), 7.87 (s, 1H), 7.81 (s, 1H), 7.74-7.72 (m, 1H), 7.52 (s, 1H), 7.25-7.21 (m, 2H), 6.78-6.68 (m, 2H), 6.51-6.50 (m, 1H), 6.35-6.30 (m, 1H), 5.80-5.77 (m, 1H), 4.96-4.95 (m, 1H), 4.11 (s, 3H), 3.89 (s, 3H), 2.33-2.16 (m, 10H); MS (ESI): m/z 527 [M+H]⁺ | 21 | 6.29 100 (a) |
| 167 | 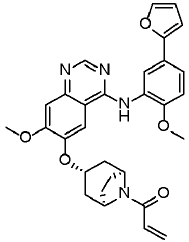 | 1-((1R,3r,5S)-3-((4-((5-(furan-2-yl)-2-methoxyphenyl )amino)-7-methoxyquinaz olin-6-yl)oxy)-8-azabicyclo[3. 2.1]octan-8-yl)prop-2-en-1-one | ¹H NMR (400MHz, TFA salt, Methanol-*d*₄) δ 8.54 (s, 1H), 7.83-7.82 (m, 2H), 7.72-7.69 (m, 1H), 7.52-7.51 (m, 1H), 7.24-7.20 (m, 2H), 6.77-6.67 (m, 2H), 6.50-6.49 (m, 1H), 6.34-6.30 (m, 1H), 5.80-5.77 (m, 1H), 4.71-4.57 (m, 2H), 4.09 (s, 3H), 3.87 (s, 3H), 2.45-1.51 (m, 8H); MS (ESI): m/z 527 [M+1]⁺ | 3 | 10.24 94 (b) |
| 168 | 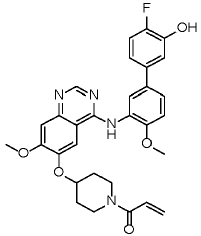 | 1-(4-((4-((4'-fluoro-3'-hydroxy-4-methoxy-[1,1'- biphenyl]-3-yl)amino)-7-methoxyquinaz olin-6-yl)oxy)piperi din-1-yl)prop-2-en-1-one | ¹H NMR (400 MHz, TFA salt, DMSO-*d*₆) δ 9.99 (s, 1H), 9.19 (s, 1H), 8.31 (s, 1H), 7.92 (s, 1H), 7.71 (s, 1H), 7.49-7.46 (m, 1H), 7.21-7.16 (m, 4H), 7.06-7.05 (m, 1H), 6.88-6.82 (m, 1H), 6.14-6.10 (m, 1H), 5.71-5.68 (m, 1H), 4.80-4.79 (m, 1H), 4.14-4.13 (m, 5H), 3.93 (s, 3H), 3.36-3.35 (m, 2H), 2.04-2.02 (m, 2H), 1. 79-1. 77 (m, 2H); MS (ESI): m/z 545 [M+H] ⁺ | 47 | 9.31 99 (b) |
| 169 | 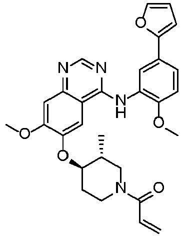 | 1-((3R,4S)-4-((4-((5-(furan-2-yl)- 2-methoxyphenyl )amino)-7-methoxyquinaz olin-6-yl)oxy)-3-methylpiperid in-1-yl)prop-2-en-1-one | ¹H NMR (400MHz, HCl salt, Methanol-*d*₄) δ 8.48 (s, 1H), 7.93 (s, 1H), 7.73-7.72 (m, 1H), 7.65-7.63 (m, 1H), 7.43-7.42 (m, 1H), 7.14-7.12 (m, 2H), 6.80-6.72 (m, 1H), 6.60-6.59 (m, 1H), 6.42-6.40 (m, 1H), 6.16-6.12 (m, 1H), 5.70-5.67 (m, 1H), 4.40-4.10 (m, 2H), 3.99 (s, 3H), 3.98-3.80 (m, 1H), 3.79 (s, 3H), 3.21-2.80 (m, 1H), 2.30-1.88 (m, 2H), 1. 63-1. 08 (m, 2H), 1.06-1.01 (m, 3H); MS (ESI): m/z 515 [M+1] ⁺ | 31 | 10.02 96 (b) |
| 170 | 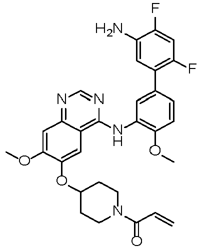 | 1-(4-((4-((5'-amino-2',4'-difluoro-4-methoxy-[1,1'-biphenyl]-3-yl)amino)-7-methoxyquinaz olin-6-yl)oxy)piperi din-1-yl)prop-2-en-1-one | ¹H NMR (400MHz, FA salt, Methanol-*d*₄) δ 8.30 (s, 1H), 7.83 (s, 1H), 7.76 (s, 1H), 7.43 (d, *J =* 7.6 Hz, 1H), 7.20 (s, 2H), 7.00-6.78 (m, 3H), 6.22 (d, *J* = 16.8 Hz, 1H), 5.76 (d, *J* = 10.4 Hz, 1H), 4.01-3.89 (m, 8H), 3.66 (s, 2H), 2.66 (s, 1H), 2.08 (s, 2H), 1.90 (s, 2H); MS (ESI): m/z 562 [M+1]⁺ | 23 | 5.46 100 (a) |
| 171 | 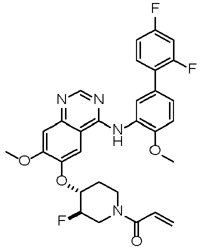 | 1-(trans-4-((4-((2',4'-difluoro-4-methoxy-[1,1'-biphenyl]-3-yl)amino)-7-methoxyquinaz olin-6-yl)oxy)-3-fluoropiperid in-1-yl)prop-2-en-1-one | ¹H NMR (400 MHz, TFA salt, Methanol-*d*₄) δ 8.55 (s, 1H), 8.13 (s, 1H), 7.74 (s, 1H), 7.58-7.52 (m, 2H), 7.30-7.27 (m, 2H), 7.09-7.05 (m, 2H), 6.87-6.80 (m, 1H), 6.28-6.24 (m, 1H), 5.83-5.79 (m, 1H), 4.81-4.80 (m, 2H), 4.12-4.03 (m, 5H), 3.92 (s, 3H), 3.88-3.50 (m, 2H), 2.31-2.17 (m, 1H), 2.05-2.01 (m, 1H); MS (ESI): m/z 565 [M+H] ⁺ | 25 | 6.88 100 (a) |
| 172 | 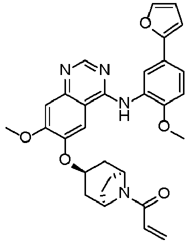 | 1-((1R,3s,5S)-3-((4-((5-(furan-2-yl)-2-methoxyphenyl )amino)-7-methoxyquinaz olin-6-yl)oxy)-8-azabicyclo[3. 2.1]octan-8-yl)prop-2-en-1-one | ¹H NMR (400MHz, TFA salt, Methanol-*d*₄) δ 8.58 (s, 1H), 7.95 (s, 1H), 7.85 (d, *J* = 2.0 Hz, 1H), 7.75-7.73 (m, 1H), 7.53 (d, *J* = 1.2 Hz, 1H), 7.26-7.21 (m, 2H), 6.78-6.67 (m, 2H), 6.51-6.50 (m, 1H), 6.37-6.32 (m, 1H), 5.82-5.79 (m, 1H), 5.13-5.10 (m, 1H), 4.77-4.64 (m, 2H), 4.10 (s, 3H), 3.90 (s, 3H), 2.44-1.72 (m, 8H); MS (ESI): m/z 527 [M+1]⁺ | 10 | 10.07 95 (b) |
| 173 | 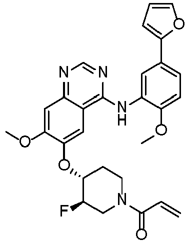 | 1-((3R,4R)-3-fluoro-4-((4-((5-(furan-2-yl)-2-methoxyphenyl )amino)-7-methoxyquinaz olin-6-yl)oxy)piperi din-1-yl)prop-2-en-1-one | ¹H NMR (400 MHz, HCl salt, Methanol-*d*₄) δ 8.60 (s, 1H), 8.17 (s, 1H), 7.87 (d, *J* = 2.4 Hz, 1H), 7.76-7.73 (m, 1H), 7.54 (d, *J =* 1.2 Hz, 1H), 7.28-7.23 (m, 2H), 6.87-6.80 (m, 1H), 6.70 (d, *J* = 3.6 Hz, 1H), 6.53-6.52 (m, 1H), 6.29-6.24 (m, 1H), 5.83-5.80 (m, 1H), 5.01-5.00 (m, 2H), 4.11-4.04 (m, 5H), 3.93-3.91 (m, 4H), 3.66-3.59 (m, 1H), 2.32-2.30 (m, 1H), 2.02-1.99 (m, 1H); MS (ESI): m/z 519 [M+H]⁺ | 82 | 9.96 99 (b) |
| 174 | 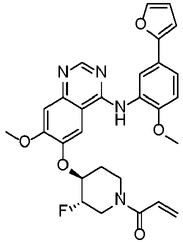 | 1-((3S,4S)-3-fluoro-4-((4-((5-(furan-2-yl)-2-methoxyphenyl )amino)-7-methoxyquinaz olin-6-yl)oxy)piperi din-1-yl)prop-2-en-1-one | ¹H NMR (400 MHz, HCl salt, DMSO-*d*₆) δ 11.02 (s, 1H), 8.74 (s, 1H), 8.33 (s, 1H), 7.75-7.71 (m, 3H), 7.31-7.28 (m, 2H), 6.90-6.83 (m, 2H), 6.59-6.58 (m, 1H), 6.17-6.12 (m, 1H), 5.75-5.72 (m, 1H), 4.94-4.75 (m, 2H), 4.08-3.99 (m, 4H), 3.83 (s, 3H), 3.68 (brs, 2H), 2.21-2.13 (m, 1H), 1.77-1.75 (m, 1H); MS (ESI): m/z=519 [M+1]⁺ | 74 | 9.97 95 (b) |
| 175 | 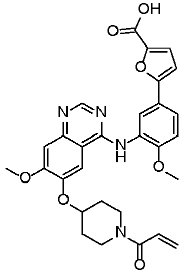 | 5-(3-((6-((1-acryloylpiper idin-4-yl)oxy)-7-methoxyquinaz olin-4-yl)amino)-4-methoxyphenyl )furan-2-carboxylic acid | ¹H NMR (400MHz, TFA salt, DMSO-*d*₆) δ 11.00 (s, 1H), 8.77 (s, 1H), 8.13 (s, 1H), 7.95-7.87 (m, 2H), 7.38-7.22 (m, 3H), 7.09 (d, *J* = 3.6 Hz, 1H), 6.97-6.82 (m, 1H), 6.15-6.12 (m, 1H), 5.77-5.75 (m, 1H), 4.84-4.82 (m, 1H), 4.02 (s, 3H), 3.97-3.84 (m, 5H), 2.34-2.32 (m, 2H), 1.07-1.07 (m, 2H); MS (ESI): m/z 545 [M+1]⁺ | 4 | 4.96 98 (a) |
| 176 | 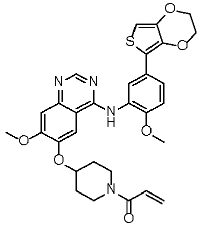 | 1-(4-((4-((5-(2,3-dihydrothieno [3,4-b][1,4]dioxin -5-yl)-2-methoxyphenyl )amino)-7-methoxyquinaz olin-6-yl)oxy)piperi din-1-yl)prop-2-en-1-one | ¹H NMR (400 MHz, free base, Methanol-*d*₄) δ 8.31 (s, 1H), 7.99 (d, *J =* 2.0 Hz, 1H), 7.82 (s, 1H), 7.64-7.61 (m, 1H), 7.22 (s, 1H), 7.15 (d, *J =* 8.8 Hz, 1H), 6.87-6.80 (m, 1H), 6.32 (s, 1H), 6.26-6.21 (m, 1H), 5.79-5.76 (m, 1H), 4.62-4.61 (m, 1H), 4.31-4.29 (m, 2H), 4.25-4.23 (m, 2H), 4.03-3.97 (m, 5H), 3.88 (s, 3H), 3.68-3.66 (m, 2H), 2.10-2.08 (m, 2H), 1.91-1.88 (m, 2H); MS (ESI): m/z 475 [M+H]⁺ | 42 | 10.16 95 (b) |
| 177 | 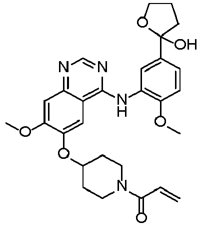 | 1-(4-((4-((5-(2-hydroxytetrah ydrofuran-2-yl)-2-methoxyphenyl )amino)-7-methoxyquinaz olin-6-yl)oxy)piperi din-1-yl)prop-2-en-1-one | ¹H NMR (400MHz, FA salt, Methanol-*d*₄) δ 8.35 (s, 1H), 8.31 (d, *J* = 2 Hz, 1H), 8.03-8.00 (m, 1H), 7.84 (S, 1H), 7.25-7.20 (m, 2H), 6.85-6.79 (m, 1H), 6.24-6.19 (m, 1H), 5.78-5.74 (m, 1H), 4.02 (s, 3H), 3.98-3.93 (m, 5H), 3.68-3.63 (m, 4H), 3.13-3.08 (m, 2H), 2.16-2.03 (m, 2H), 1.97-1.91 (m, 4H); MS (ESI): m/z 521 [M+1]⁺ | 39 | 7.78 93 (b) |
| 178 | 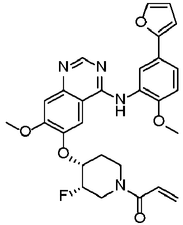 | 1-((3S,4R)-3-fluoro-4-((4-((5-(furan-2-yl)-2-methoxyphenyl )amino)-7-methoxyquinaz olin-6-yl)oxy)piperi din-1-yl)prop-2-en-1-one | ¹H NMR (400MHz, HCl salt, DMSO-*d*₆) δ 11.22 (s, 1H), 8.73 (s, 1H), 8.37 (s, 1H), 7.75-7.71 (m, 3H), 7.31 (s, 1H), 7.29 (d, *J* = 9.2 Hz, 1H), 6.89-6.79 (m, 2H), 6.58-6.57 (m, 1H), 6.16-6.15 (m, 1H), 5.71-5.70 (m, 1H), 5.00-4.95 (m, 2H), 4.38-4.31 (m, 1H), 4.01 (s, 3H), 3.77 (s, 3H), 3.07 (t, *J* = 12.0 Hz, 1H), 2.32-1.80 (m, 4H); MS (ESI): m/z 519 [M+1]⁺ | 69 | 5.79 100 (a) |
| 179 | 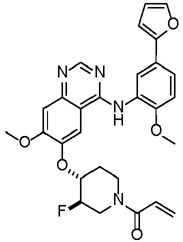 | 1-(trans-3-fluoro-4-((4-((5-(furan-2-yl)-2-methoxyphenyl )amino)-7-methoxyquinaz olin-6-yl)oxy)piperi din-1-yl)prop-2-en-1-one | ¹H NMR (400 MHz, TFA salt, Methanol-*d*₄) δ 8.63 (s, 1H), 8.19 (s, 1H), 7.85 (d, *J* = 2.0 Hz, 1H), 7.77-7.75 (m, 1H), 7.55 (s, 1H), 7.28-7.24 (m, 2H), 6.87-6.80 (m, 1H), 6.71 (d, *J* = 3.2 Hz, 1H), 6.53-6.52 (m, 1H), 6.29 (d, *J =* 16.8 Hz, 1H), 5.83-5.50 (m, 1H), 5.02-5.01 (m, 2H), 4.12 (s, 3H), 4.09-4.00 (m, 2H), 3.98 (s, 3H), 3.74-3.71 (m, 2H), 2.34-2.33 (m, 1H), 2.01-2.00 (m, 1H); MS (ESI): m/z 519 [M+H] ⁺ | 23 | 6.24 100 (a) |
| 180 | 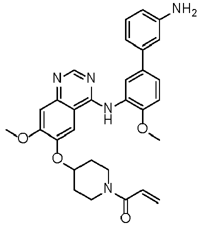 | 1-(4-((4-((3'-amino-4-methoxy-[1,1'- biphenyl]-3-yl)amino)-7-methoxyquinaz olin-6-yl)oxy)piperi din-1-yl)prop-2-en-1-one | ¹H NMR (400MHz, Free base, Methanol-*d*₄) δ 8.31 (s, 1H), 7.86 (d, *J* = 2 Hz, 1H), 7.81 (s, 1H), 7.51-7.49 (m, 1H), 7.19-7.13 (m, 3H), 6.99 (s, 1H), 6.94 (d, *J* = 7.6 Hz, 1H), 6.84-6.77 (m, 1H), 6.69-6.67 (m, 1H), 6.23-6.19 (m, 1H), 5.77-5.74 (m, 1H), 4.00-3.88 (m, 8H), 3.66-3.61 (m, 2H), 2.07-2.03 (m, 2H), 1.89 (brs, 2H); MS (ESI): m/z 527 [M+1]⁺ | 5 | 4.24 100 (a) |
| 181 | 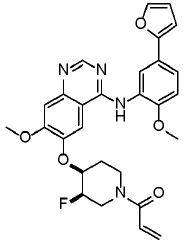 | 1-(cis-3-fluoro-4-((4-((5-(furan-2-yl)-2-methoxyphenyl )amino)-7-methoxyquinaz olin-6-yl)oxy)piperi din-1-yl)prop-2-en-1-one | ¹H NMR (400MHz, TFA salt, Methanol-*d*₄) δ 8.60 (s, 1H), 8.09 (s, 1H), 7.82 (d, *J* = 2.0 Hz, 1H), 7.74-7.72 (m, 1H), 7.52 (d, *J* = 1.2 Hz, 1H), 7.27 (s, 1H), 7.23 (d, *J* = 8.8 Hz, 1H), 6.86-6.75 (m, 1H), 6.68 (d, *J* = 3.6 Hz, 1H), 6.50-6.49 (m, 1H), 6.25 (d, *J =* 16.8 Hz, 1H), 5.80 (d, *J =* 10.8 Hz, 1H), 5.10-4.95 (m, 1H), 4.49-4.34 (m, 2H), 4.09-4.03 (m, 4H), 3.88 (s, 3H), 3.68-3.53 (m, 2H), 2.09-2.08 (m, 2H); MS (ESI): m/z 519 [M+1]⁺ | 15 | 6.09 98 (a) |
| 182 | 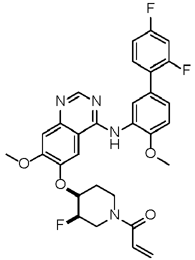 | 1-(cis-4-((4-((2',4'-difluoro-4-methoxy-[1,1'-biphenyl]-3-yl)amino)-7-methoxyquinaz olin-6-yl)oxy)-3-fluoropiperid in-1-yl)prop-2-en-1-one | ¹H NMR (400MHz, TFA salt, Methanol-*d*₄) δ 8.59 (s, 1H), 8.10 (s, 1H), 7.67 (s, 1H), 7.59-7.49 (m, 2H), 7.29 (s, 1H), 7.27 (s, 1H), 7.07-7.03 (m, 2H), 6.86-6.75 (m, 1H), 6.25 (d, *J* = 16.8 Hz, 1H), 5.80 (d, *J* = 10.4Hz, 1H), 5.10-4.96 (m, 1H), 4.51-4.34 (m, 2H), 4.09 (s, 3H), 3.90 (s, 3H), 3.68-3.48 (m, 2H), 2.19-2.09 (m, 2H); MS (ESI): m/z 564 [M+1]⁺ | 20 | 6.66 99 (a) |
| 183 | 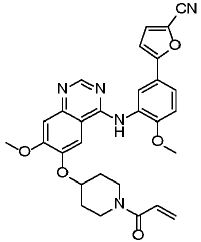 | 5-(3-((6-((1-acryloylpiper idin-4-yl)oxy)-7-methoxyquinaz olin-4-yl)amino)-4-methoxyphenyl )furan-2-carbonitrile | ¹H NMR (400 MHz, TFA salt, Methanol-*d*₄) δ8.63 (s, 1H), 8.07 (s, 1H), 7.98 (d, *J* = 2.4 Hz, 1H), 7.89-7.86 (m, 1H), 7.42 (d, *J =* 4.0 Hz, 1H), 7.34 (d, *J =* 8.8 Hz, 1H), 7.27 (s, 1H), 6.95 (d, *J* = 4.0 Hz, 1H), 6.88-6.81 (m, 1H), 6.27-6.22 (m, 1H), 5.81-5.78 (m, 1H), 5.01-5.00 (m, 1H), 4.13 (s, 3H), 3.99-3.96 (m, 5H), 3.70-3.68 (m, 2H), 2.13-2.10 (m, 2H), 1.99-1.97 (m, 2H); (ESI): m/z 526 [M+H] ⁺ | 41 | 9.69 99 (b) |
| 184 | 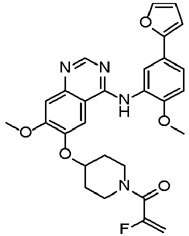 | 2-fluoro-1-(4-((4-((5-(furan-2-yl)-2-methoxyphenyl )amino)-7-methoxyquinaz olin-6-yl)oxy)piperi din-1-yl)prop-2-en-1-one | ¹H NMR (400MHz, TFA salt, Methanol-*d*₄) δ 8.32 (s, 1H), 8.00 (d, *J* = 2.4 Hz, 1H), 7.76 (s, 1H), 7.59-7.56 (m, 1H), 7.50-7.49 (m, 1H), 7.17 (s, 1H), 7.13 (d, *J* = 8.8 Hz, 1H), 6.63-6.62 (m, 1H), 6.48-6.46 (m, 1H), 5.25-5.23 (m, 1H), 5.19-5.12 (m, 1H), 4.83-4.80 (m, 1H), 3.99 (s, 3H), 3.92-3.86 (m, 5H), 3.65-3.64 (m, 2H), 2.11-2.04 (m, 2H), 1.96-1.90 (m, 2H); MS (ESI): m/z 519 [M+1]⁺ | 33 | 6.16 100 (a) |
| 185 | 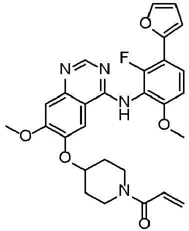 | 1-(4-((4-((2-fluoro-3-(furan-2-yl)- 6-methoxyphenyl )amino)-7-methoxyquinaz olin-6-yl)oxy)piperi din-1-yl)prop-2-en-1-one | ¹H NMR (400MHz, TFA salt, DMSO-*d*₆) δ 10.36 (s, 1H), 8.62 (s, 1H), 8.07 (s, 1H), 7.84-7.80 (m, 2H), 7.28 (s, 1H), 7.19 (d, *J* = 8.4 Hz, 1H), 6.89-6.82 (m, 1H), 6.75 (t, *J* = 3.6 Hz, 1H), 6.64-6.63 (m, 1H), 6.15-6.11 (m, 1H), 5.71-5.69 (m, 1H), 4.84-4.80 (m, 1H), 4.00-3.86 (m, 8H), 2.07-2.05 (m, 2H), 1.72-1.71 (m, 2H); MS (ESI): m/z 519 [M+1]⁺ | 67 | 5.82 96 (a) |
| 186 | 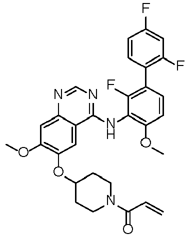 | 1-(4-((7-methoxy-4-((2,2',4'-trifluoro-4-methoxy-[1,1'-biphenyl]-3-yl)amino)quin azolin-6-yl)oxy)piperi din-1-yl)prop-2-en-1-one | ¹H NMR (400MHz, TFA salt, DMSO-*d*₆) δ 10.54 (s, 1H), 8.67 (s, 1H), 8.09 (s, 1H), 7.58-7.51 (m, 2H), 7.43-7.37 (m, 1H), 7.28-7.19 (m, 3H), 6.89-6.82 (m, 1H), 6.15-6.10 (m, 1H), 5.71-5.68 (m, 1H), 4.83-4.81 (m, 1H), 4.00-3.87 (m, 8H), 2.08-2.06 (m, 2H), 1. 72-1. 71 (m, 2H); MS (ESI): m/z 565 [M+1]⁺ | 51 | 6.26 100 (a) |
| 187 | 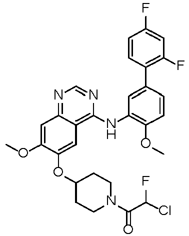 | 2-chloro-1-(4-((4-((2',4'-difluoro-4-methoxy-[1,1'- biphenyl]-3-yl)amino)-7-methoxyquinaz olin-6-yl)oxy)piperi din-1-yl)-2-fluoroethan-1-one | ¹H NMR (400 MHz, TFA salt, Methanol-*d*₄) δ 8.59 (d, *J* = 7.1 Hz, 1H), 8.05 (s, 1H), 7.68 (d, *J* = 3.5 Hz, 1H), 7.63 - 7.47 (m, 2H), 7.33 - 7.21 (m, 2H), 7.11 - 6.94 (m, 3H), 4.94 - 4.90 (m, 1H), 4.09 (d, *J* = 7.0 Hz, 3H), 3.95 - 3.56 (m, 9H), 3.17 - 3.09 (m, 1H), 2.23 - 1.90 (m, 4H); MS (ESI): m/z 587 [M+1]⁺ | 79 | 11.18 98 (b) |
| 188 | 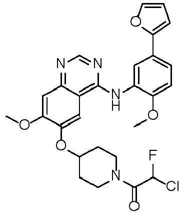 | 2-chloro-2-fluoro-1-(4-((4-((5-(furan-2-yl)-2-methoxyphenyl )amino)-7-methoxyquinaz olin-6-yl)oxy)piperi din-1-yl)ethan-1-one | ¹H NMR (400 MHz, TFA salt, CDCl₃) δ 9.66 (s, 1H), 8.53 (s, 1H), 8.30 (s, 1H), 7.59-7.56 (m, 2H), 7.43 (s, 1H), 7.39-7.37 (d, *J* = 7.05 Hz, 1H), 7.04-7.02 (d, *J* = 8.68 Hz, 1H), 6.57-6.55 (t, 1H), 6.43 (s, 1H), 4.83 (s, 1H), 3.97-3.86 (m, 8H), 3.72-3.60 (m, 2H), 2.14-2.06 (m, 2H), 2.00-1.89 (m, 2H); MS (ESI): m/z=541 [M+1]⁺ | 36 | 10.45 96 (b) |
| 189 | 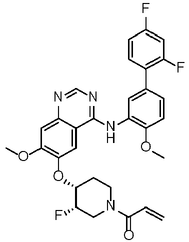 | 1-((3S,4R)-4-((4-((2',4'-difluoro-4-methoxy-[1,1'-biphenyl]-3-yl)amino)-7-methoxyquinaz olin-6-yl)oxy)-3-fluoropiperid in-1-yl)prop-2-en-1-one | ¹H NMR (400 MHz, HCl salt, Methanol-*d*₄) δ 8.56 (s, 1H), 8.03 (s, 1H), 7.72 (s, 1H), 7.60-7.52 (m, 2H), 7.31-7.26 (m, 2H), 7.10-7.05 (m, 2H), 6.85-6.82 (m, 1H), 6.27-6.23 (m, 1H), 5.82-5.79 (m, 1H), 5.24-5.21 (m, 1H), 4.62-4.59 (m, 2H), 4.41 (s, 3H), 4.11 (s, 3H), 3.67-3.49 (m, 3H), 2.12-2.10 (m, 2H); MS (ESI): m/z 565 [M+H]⁺ | 52 | 10.59 100 (b) |
| 190 | 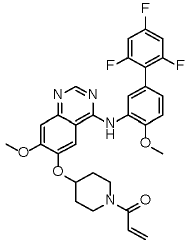 | 1-(4-((7-methoxy-4-((2',4',6'-trifluoro-4-methoxy-[1,1'-biphenyl]-3-yl)amino)quin azolin-6-yl)oxy)piperi din-1-yl)prop-2-en-1-one | ¹H NMR (400MHz, HCl salt, DMSO-*d*₆) δ 11.05 (s, 1H), 8.72 (s, 1H), 8.20 (s, 1H), 7.50-7.49 (m, 2H), 7.36-7.29 (m, 4H), 6.88-6.81 (m, 1H), 6.14-6.09 (m, 1H), 5.70-5.67 (m, 1H), 4.86-4.84 (m, 1H), 4.00-3.84 (m, 8H), 3.53-3.50 (m, 1H), 2.08-2.07 (m, 2H), 1.68-1.66 (m, 2H); MS (ESI): m/z 565 [M+1]⁺ | 78 | 10.63 100 (b) |
| 191 | 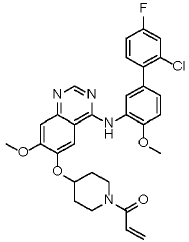 | 1-(4-((4-((2'-chloro-4'-fluoro-4-methoxy-[1,1'-biphenyl]-3-yl)amino)-7-methoxyquinaz olin-6-yl)oxy)piperi din-1-yl)prop-2-en-1-one | ¹H NMR (400MHz, HCl salt, DMSO-*d*₆) δ 11.01 (s, 1H), 8.71 (s, 1H), 8.27 (s, 1H), 7.58-7.55 (m, 1H), 7.50-7.45 (m, 3H), 7.34-7.29 (m, 3H), 6.88-6.81 (m, 1H), 6.14-6.09 (m, 1H), 5.70-5.67 (m, 1H), 4.87-4.86 (m, 1H), 3.99-3.84 (m, 8H), 3.51-3.50 (m, 1H), 2.08-2.07 (m, 2H), 1.68-1.66 (m, 2H); MS (ESI): m/z 564 [M+1]⁺ | 81 | 10.89 100 (b) |
| 192 | 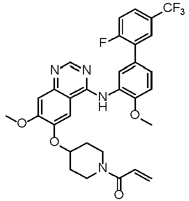 | 1-(4-((4-((2'-fluoro-4-methoxy-5'-(trifluoromet hyl)-[1,1'-biphenyl]-3-yl)amino)-7-methoxyquinaz olin-6-yl)oxy)piperi din-1-yl)prop-2-en-1-one | ¹H NMR (400MHz, HCl salt, DMSO-*d*₆) δ 11.09 (s, 1H), 8.70 (s, 1H), 8.25 (s, 1H), 7.90-7.88 (m, 1H), 7.82-7.78 (m, 1H), 7.72-7.68 (m, 2H), 7.60 (t, *J =* 9.2 Hz, 1H), 7.37 (d, *J =* 8.8 Hz, 1H), 7.30 (s, 1H), 6.88-6.81 (m, 1H), 6.14-6.09 (m, 1H), 5.70-5.67 (m, 1H), 4.88-4.86 (m,1H), 4.00-3.85 (m, 8H), 3.52-3.50 (m, 1H), 2.08-2.07 (m, 2H), 1.68-1.66 (m, 2H); MS (ESI): m/z 597 [M+1]⁺ | 75 | 11.21 100 (b) |
| 193 | 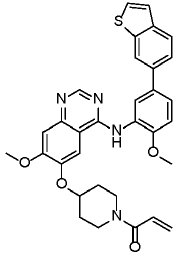 | 1-(4-((4-((5-(benzo[b]thio phen-6-yl)-2-methoxyphenyl )amino)-7-methoxyquinaz olin-6-yl)oxy)piperi din-1-yl)prop-2-en-1-one | ¹H NMR (400 MHz, FA salt, Methanol-*d*₄) δ 8.35 (s, 1H), 8.14 (s, 1H), 8.00-7.99 (d, *J* = 2.12 Hz, 1H), 7.90-7.88 (d, *J* = 8.32 Hz, 1H), 7.85 (s, 1H), 7.66-7.63 (dd, *J* = 8.38 Hz, 2H), 7.56-7.55 (d, *J =* 5.44 Hz, 2H), 7.38-7.37 (d, *J* = 5.40 Hz, 1H), 7.25-7.23 (d, *J* = 8.60 Hz, 1H) 7.21 (s, 1H) 6.85-6.78 (m, 1H), 6.24-6.20 (d, *J =* 16.89 Hz, 1H), 5.77-5.75 (d, *J* = 10.70 Hz, 1H), 4.85 (m, 1H), 4.02 (s, 3H), 3.99-3.92 (m, 6H), 3.71-3.62 (m, 2H), 2.13-2.06 (m, 2H), 1.96-1.86 (m, 2H); MS (ESI): m/z=567 [M+1]⁺ | 57 | 11.12 98 (b) |
| 194 | 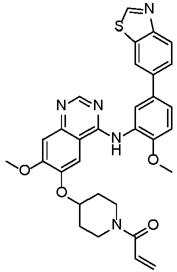 | 1-(4-((4-((5-(benzo[d]thia zol-6-yl)-2-methoxyphenyl )amino)-7-methoxyquinaz olin-6-yl)oxy)piperi din-1-yl)prop-2-en-1-one | ¹H NMR (400 MHz, FA salt, DMSO-*d*₆) δ 9.38 (s, 1H), 9.22 (s, 1H), 8.48 (d, *J* = 1.6 Hz, 1H), 8.45 (s, 1H), 8.31 (s, 1H), 8.13 (d, *J* = 8.5 Hz, 1H), 7.95 (s, 1H), 7.92 (d, *J* = 2.3 Hz, 1H), 7.85 (dd, *J =* 8.6, 1.8 Hz, 1H), 7.69 (dd, *J* = 8.6, 2.3 Hz, 1H), 7.27 (d, *J* = 8.7 Hz, 1H), 7.20 (s, 1H), 6.85 (dd, *J* = 16.7, 10.5 Hz, 1H), 6.12 (dd, *J =* 16.7, 2.4 Hz, 1H), 5.69 (dd, *J* = 10.5, 2.4 Hz, 1H), 4.83-4.76 (m, 1H), 3.97-3.87 (m, 5H), 3.84 (s, 3H), 3.53-3.44 (m, 2H), 2.11-2.01 (m, 2H), 1.75-1.62 (m, 2H); MS (ESI): m/z 568 [M+1] ⁺ | 82 | 9. 67 97 (b) |
| 195 | 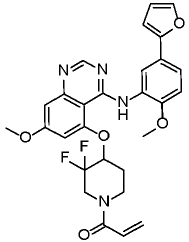 | 1-(3,3-difluoro-4-((4-((5-(furan-2-yl)-2-methoxyphenyl )amino)-7-methoxyquinaz olin-5-yl)oxy)piperi din-1-yl)prop-2-en-1-one | ¹H NMR (400 MHz, TFA salt, Methanol-*d*₄) δ 8.76 (s, 2H), 7.65-7.62 (m, 1H), 7.55 (s, 1H), 7.24-7.19 (m, 2H), 6.89-6.88 (m, 2H), 6.79 (s, 1H), 6.71 (s, 1H), 6.54-6.52 (m, 1H), 6.29-6.28 (m, 1H), 5.87-5.84 (m, 1H), 4.72-4.71 (m, 1H), 4.33-4.32 (m, 1H), 4.01 (s, 3H), 3.93 (s, 3H), 3.90-3.89 (m, 1H), 3.42-3.41 (m, 1H), 2.52-2.50 (m, 1H), 2.15-2.13 (m, 1H); MS (ESI): m/z 537 [M+H] ⁺ | 51 | 10.95 100 (b) |
| 196 | 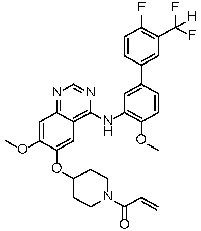 | 1-(4-((4-((3⁻-(difluorometh yl)-4'-fluoro-4-methoxy-[1,1'- biphenyl]-3-yl)amino)-7-methoxyquinaz olin-6-yl)oxy)piperi din-1-yl)prop-2-en-1-one | ¹H NMR (400MHz, TFA salt, Methanol-*d*₄) δ 8.60 (s, 1H), 8.06 (s, 1H), 7.82-7.78 (m, 3H), 7.71-7.68 (m, 1H), 7.34-7.29 (m, 2H), 7.24 (s, 1H), 7.04 (tr, *J* = 54.4 Hz, 1H), 6.85-6.79 (m, 1H), 6.25-6.20 (m, 1H), 5.78-5.75 (m, 1H)4.09 (s, 3H), 3.98-3.91 (m, 6H), 3.68 (brs, 2H), 2.16-2.11 (m, 2H), 1.92 (brs, 2H); MS (ESI): m/z 579 [M+1]⁺ | 27 | 10.73 99 (b) |
| 197 | 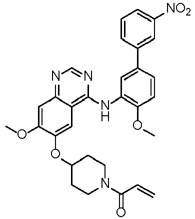 | 1-(4-((7-methoxy-4-((4-methoxy-3'-nitro-[1,1'-biphenyl]-3-yl)amino) quin azolin-6-yl)oxy)piperi din-1-yl)prop-2-en-1-one | ¹H NMR (400 MHz, FA salt, Methanol-*d*₄) δ 8.49-8.46 (m, 1H), 8.37 (s, 1H), 8.22 (s, 1H), 8.20-8.16 (m, 1H), 8.07-8.03 (m, 2H), 7.86 (s, 1H), 7.71-7.65 (m, 2H), 7.28 (d, *J* = 8.6 Hz, 1H), 7.22 (s, 1H), 6.82 (dd, *J* = 16.8, 10.7 Hz, 1H), 6.22 (dd, *J =* 16.8, 1.9 Hz, 1H), 5.76 (dd, *J* = 10.7, 1.9 Hz, 1H), 4.03 (s, 3H), 4.01-3.95 (m, 2H), 3.94 (s, 3H), 3.72-3.62 (m, 2H), 2.14-2.06 (m, 2H), 1.96-1.87 (m, 2H); MS (ESI): m/z 556 [M+1]⁺ | 48 | 10.34 95 (b) |
| 198 | 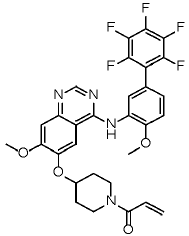 | 1-(4-((7-methoxy-4-((2',3',4',5' ,6'-pentafluoro-4-methoxy-[1,1'-biphenyl]-3-yl)amino))qui nazolin-6-yl)oxy)piperi din-1-yl)prop-2-en-1-one | ¹H NMR (400 MHz, TFA salt, Methanol-*d*₄) δ 8.61 (s, 1H), 8.05 (s, 1H), 7.87 (s, 1H), 7.58 (d, *J* = 8.8 Hz, 1H), 7.38 (d, *J* = 15.2 Hz, 1H), 7.26 (s, 1H), 6.88-6.81 (m, 1H), 6.27-6.22 (m, 1H), 5.80-5.77 (m, 1H), 4.92-4.91 (m, 1H), 4.10 (s, 3H), 3.99-3.94 (m, 5H), 3.70-3.68 (m, 2H), 2.17-2.12 (m, 2H), 1.94-1.92 (m, 2H); MS (ESI): m/z 601 [M+H]⁺ | 13 | 11.18 100 (b) |
| 199 | 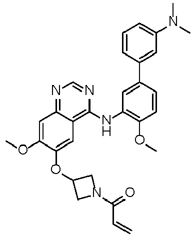 | 1-(3-((4-((3'-(dimethylamin o)-4-methoxy-[1,1'- biphenyl]-3-yl)amino)-7-methoxyquinaz olin-6-yl)oxy)azetid in-1-yl)prop-2-en-1-one | ¹H NMR (400MHz, TFA salt, Methanol-*d*₄) δ 8.64-8.63 (m, 1H), 7.88 (s, 1H), 7.79-7.68 (m, 3H), 7.64-7.61 (m, 1H), 7.49-7.47 (m, 1H), 7.35 (s, 1H), 7.33 (s, 1H), 6.45-6.40 (m, 1H), 6.38-6.29 (m, 1H), 5.81-5.79 (m, 1H), 5.31-5.30 (m, 1H), 4.89-4.88 (m, 1H), 4.66-4.62 (m, 1H), 4.49-4.46 (m, 1H), 4.17-4.13 (m, 1H), 4.12 (s, 3H), 3.97 (s, 3H), 3.27 (s, 6H); MS (ESI): m/z 525 [M+1]⁺ | 43 | 8.31 97 (b) |
| 200 | 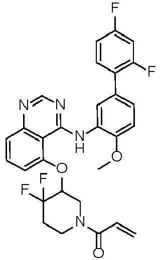 | 1-(3-((4-((2'4'-difluoro-4-methoxy-[1,1'- biphenyl]-3-yl)amino)quin azolin-5-yl)oxy)-4,4-difluoropiper idin-1-yl)prop-2-en-1-one | ¹H NMR (400 MHz, TFA salt, Methanol-*d*₄) δ 8.77 (s, 1H), 8.35 (s, 1H), 8.11-8.07 (m, 1H), 7.73-7.71 (m, 1H), 7.59-7.52 (m, 3H), 7.30 (d, *J* = 8.4 Hz, 1H), 7.12-7.09 (m, 2H), 6.78-6.76 (m, 1H), 6.06-6.01 (m, 1H), 5.65-5.42 (m, 2H), 4.23-4.22 (m, 1H), 4.09-4.00 (m, 5H), 3.96-3.90 (m, 2H), 2.40-2.38 (m, 2H); MS (ESI): m/z 553 [M+H]⁺ | 47 | 11.50 100 (b) |
| 201 | 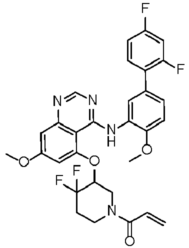 | 1-(3-((4-((2',4'-difluoro-4-methoxy-[1,1'-biphenyl]-3-yl)amino)-7-methoxyquinaz olin-5-yl)oxy)-4,4-difluoropiper idin-1-yl)prop-2-en-1-one | ¹H NMR (400 MHz, TFA salt, Methanol-*d*₄) δ 8.68 (s, 1H), 8.28 (s, 1H), 7.58-7.49 (m, 2H), 7.29-7.26 (m, 2H), 7.11-7.06 (m, 2H), 6.91 (s, 1H), 6.78-6.76 (m, 1H), 6.06-6.02 (m, 1H), 5.68-5.43 (m, 2H), 4.21-4.20 (m, 1H), 4.11-4.08 (m, 4H), 4.01 (s, 3H), 3.99-3.81 (m, 2H), 2.45-2.42 (m, 2H); MS (ESI): m/z 583 [M+H]⁺ | 47 | 11.59 100 (b) |
| 202 | 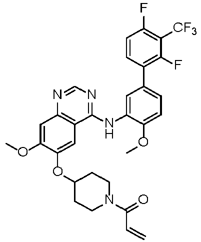 | 1-(4-((4-((2',4'-difluoro-4-methoxy-3'-(trifluoromet hyl)-[1,1'-biphenyl]-3-yl)amino)-7-methoxyquinaz olin-6-yl)oxy)piperi din-1-yl)prop-2-en-1-one | ¹H NMR (400MHz, TFA salt, Methanol-*d*₄) δ 8.60 (s, 1H), 8.05 (s, 1H), 7.84-7.78 (m, 1H), 7.71-7.00 (m, 1H), 7.61-7.58 (m, 1H), 7.34-7.25 (m, 3H), 6.86-6.79 (m, 1H), 6.25-6.20 (m, 1H), 5.79-5.76 (m, 1H), 4.90 (m, 1H), 4.09 (s, 3H) 3.98-3.93 (m, 6H), 3.68 (brs, 2H), 2.13 (brs, 2H), 1.94 (brs, 2H); MS (ESI): m/z 615 [M+1]⁺ | 83 | 11.11 100 (b) |
| 203 | 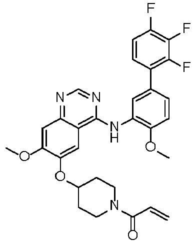 | 1-(4-((7-methoxy-4-((2',3',4'-trifluoro-4-methoxy-[1,1'- biphenyl]-3-yl)amino)quin azolin-6-yl)oxy)piperi din-1-yl)prop-2-en-1-one | ¹H NMR (400MHz, TFA salt, Methanol-*d*₄) δ 8.61 (s, 1H), 8.07 (s, 1H), 7.73-7.72 (m, 1H), 7.63-7.60 (m, 1H), 7.34-7.28 (m, 2H), 7.23-7.21 (m, 2H), 6.87-6.81 (m, 1H), 6.27-6.22 (m, 1H), 5.80-5.77 (m, 1H), 5.00-4.88 (m, 1H), 4.11 (s, 3H), 3.97-3.94 (m, 2H), 3.93 (s, 3H), 3.71-3.70 (m, 2H), 2.20-1.89 (m, 4H); MS (ESI): m/z 564 [M+1]⁺ | 50 | 10.82 100 (b) |
| 204 | 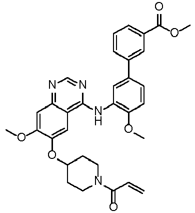 | methyl 3'-((6-((1-acryloylpiper idin-4-yl)oxy)-7-methoxyquinaz olin-4-yl)amino)-4'-methoxy-[1,1'- biphenyl]-3-carboxylate | ¹H NMR (400 MHz, free base, DMSO) δ 9.22 (s, 1H), 8.30 (s, 1H), 8.19-8.17 (m, 1H), 7.97-7.89 (m, 3H), 7.84 (d, *J =* 2.3 Hz, 1H), 7.65-7.58 (m, 2H), 7.26 (d, *J* = 8.7 Hz, 1H), 7.19 (s, 1H), 6.85 (dd, *J =* 16.7, 10.5 Hz, 1H), 6.12 (dd, *J* = 16.7, 2.4 Hz, 1H), 5.69 (dd, *J* = 10.5, 2.4 Hz, 1H), 4.83-4.76 (m, 1H), 3.98-3.93 (m, 2H), 3.93 (s, 3H), 3.88 (s, 3H), 3.83 (s, 3H), 3.55-3.42 (m, 2H), 2.09-2.01 (m, 2H), 1. 74-1. 63 (m, 2H); MS (ESI): m/z=569 [M+1]⁺ | 40 | 10.30 99 (b) |
| 205 | 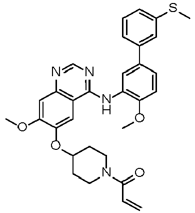 | 1-(4-((7-methoxy-4-((4-methoxy-3'-(methylthio)-[1,1'-biphenyl]-3-yl)amino)quin azolin-6-yl)oxy)piperi din-1-yl)prop-2-en-1-one | ¹H NMR (400MHz, TFA salt, Methanol-*d*₄) δ 8.58 (s, 1H), 8.06 (s, 1H), 7.75-7.74 (d, *J* = 2.4 Hz 1H), 7.67-7.65 (m, 1H), 7.47 (m, 1H), 7.37-7.21 (m, 5H), 6.82-6.78 (m, 1H), 6.24-6.19 (m, 1H), 5.78-5.74 (m, 1H), 4.90 (m, 1H), 4.07 (s, 3H), 3.98-3.91 (m, 2H), 3.89 (s, 3H), 3.66 (brs, 2H), 2.51 (s, 3H), 2.10 (brs, 2H), 1.90 (brs, 2H); MS (ESI): m/z 557 [M+1]⁺ | 69 | 10.60 99 (b) |
| 206 | 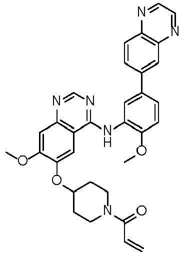 | 1-(4-((7-methoxy-4-((2-methoxy-5-(quinoxalin-6-yl)phenyl)ami no)quinazolin -6-yl)oxy)piperi din-1-yl)prop-2-en-1-one | ¹H NMR (400MHz, TFA salt, Methanol-*d*₄) δ 8.89-8.85 (m, 2H), 8.60 (s, 1H), 8.28 (s, 1H), 8.20-8.14 (m, 2H), 8.05 (s, 1H), 8.01 (d, *J* = 2.4 Hz 1H), 7.91-7.89 (m, 1H), 7.37-7.34 (d, *J* = 8.8 Hz 1H), 7.23 (d, *J* = 1.2 Hz 1H), 6.80-6.76 (m, 1H), 6.22-6.18 (m, 1H), 5.76-5.73 (m, 1H), 4.89 (m, 1H), 4.07 (s, 3H), 3.95 (brs, 2H), 3.91 (s, 3H), 3.66 (brs, 2H), 2.10 (brs, 2H), 1.90 (brs, 2H); MS (ESI): m/z 563 [M+1]⁺ | 60 | 9.02 99 (b) |
| 207 | 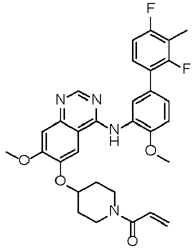 | 1-(4-((4-((2',4'-difluoro-4-methoxy-3'-methyl-[1,1'-biphenyl] -3-yl)amino)-7-methoxyquinaz olin-6-yl)oxy)piperi din-1-yl)prop-2-en-1-one | ¹H NMR (400 MHz, TFA salt, Methanol-*d*₄) δ 8.59 (s, 1H), 8.05 (s, 1H), 7.69-7.68 (m, 1H), 7.59-7.57 (m, 1H), 7.36-7.25 (m, 3H), 7.02-7.01 (m, 1H), 6.87-6.81 (m, 1H), 6.27-6.22 (m, 1H), 5.80-5.77 (m, 1H), 5.06-5.02 (m, 1H), 4.10 (s, 3H), 3.99-3.93 (m, 5H), 3.69-3.67 (m, 2H), 2.27 (s, 3H), 2.18-2.17 (m, 2H), 2.01-1.99 (m, 2H); MS (ESI): m/z 561 [M+H] ⁺ | 92 | 10.95 100 (b) |
| 208 | 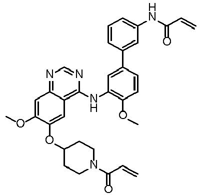 | N-(3'-((6-((1-acryloylpiper idin-4-yl)oxy)-7-methoxyquinaz olin-4-yl)amino)-4'-methoxy-[1,1'- biphenyl]-3-yl)acrylamide | ¹H NMR (400 MHz, TFA salt, Methanol-*d*₄) δ 9.22 (s, 1H), 8.31 (s, 1H), 8.02 (s, 1H), 7.93 (s, 1H), 7.59-7.53 (m, 1H), 7.40-7.38 (m, 2H), 7.26-7.24 (m, 2H), 7.20 (s, 1H), 6.90-6.80 (m, 1H), 6.42-6.41 (m, 1H), 6.30-6.29 (m, 1H), 6.15-6.14 (m, 1H), 5.71-5.70 (m, 1H), 5.68-5.67 (m, 1H), 4.85-4.75 (m, 1H), 4.00-3.94 (m, 5H), 3.83 (s, 3H), 3.54-3.51 (m, 2H), 2.55 (s, 6H), 2.20-2.00 (m, 3H), 1.80-1.60 (m, 3H), 1. 40-1. 39 (m, 5H), 1.25 (s 6H); MS (ESI): m/z 579 [M+1]⁺ | 22 | 9.44 94 (b) |
| 209 | 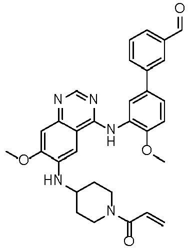 | 3'-((6-((1-acryloylpiper idin-4-yl)amino)-7-methoxyquinaz olin-4-yl)amino)-4'-methoxy-[1,1'- biphenyl]-3-carbaldehyde | ¹H NMR (400 MHz, TFA salt, DMSO-*d*₆) δ 10.10 (s, 1H), 9.23 (s, 1H), 8.31 (s, 1H), 8.21 (s, 1H), 8.04-8.02 (s, 1H), 7.95-7.85 (m, 2H), 7.71-7.70 (m, 1H), 7.69-7.66 (m, 2H), 7.29-7.27 (m, 1H), 7.20 (s, 1H), 6.89-6.85 (m, 1H), 6.15-6.10 (m, 1H), 5.71-5.68 (m, 1H), 4.81-4.80 (m, 1H), 3.98 (s, 3H), 3.82 (s, 3H), 3.60-3.30 (m, 2H), 2.05-2.00 (m, 2H), 1.80-1.60 (m, 2H); MS (ESI): m/z 538 [M+1]⁺ | 24 | 9.64 92 (b) |
| 210 | 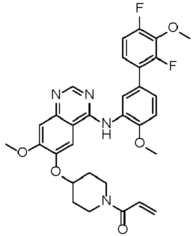 | 1-(4-((4-((2' ,4'-difluoro-3',4-dimethoxy-[1,1'- biphenyl]-3-yl)amino)-7-methoxyquinaz olin-6-yl)oxy)piperi din-1-yl)prop-2-en-1-one | ¹H NMR (400 MHz, FA salt, Methanol-*d*₄) δ 8.35 (s, 1H), 8.16 (s, 1H), 7.86 (s, 1H), 7.78 (d, *J* = 1.8 Hz, 1H), 7.49-7.44 (m, 1H), 7.25-7.16 (m, 3H), 7.08-7.02 (m, 1H), 6.82 (dd, *J* = 16.8, 10.7 Hz, 1H), 6.22 (dd, *J* = 16.8, 1.9 Hz, 1H), 5.76 (dd, *J* = 10.7, 1.9 Hz, 1H), 4.02 (s, 3H), 3.99 (s, 3H), 3.97-3.93 (m, 2H), 3.91 (s, 3H), 3.73-3.62 (m, 2H), 2.14-2.05 (m, 2H), 1. 95-1. 85 (m, 2H); MS (ESI): m/z 577 [M+1] ⁺ | 26 | 10.39 98 (b) |
| 211 | 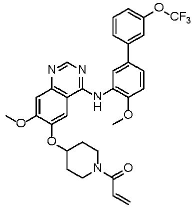 | 1-(4-((7-methoxy-4-((4-methoxy-3'-(trifluoromet hoxy)-[1,1'-biphenyl]-3-yl)amino)quin azolin-6-yl)oxy)piperi din-1-yl)prop-2-en-1-one | ¹H NMR (400MHz, TFA salt, Methanol-*d*₄) δ 8.61 (s, 1H), 8.06 (s, 1H), 7.81-7.80 (d, *J* = 2.4 Hz 1H), 7.74-7.71 (m, 1H), 7.65-7.63 (m, 1H), 7.56-7.52 (tri, *J* = 8.0 Hz 1H), 7.50 (s, 1H), 7.32-7.30 (d, *J* = 8.8 Hz 1H), 7.27-7.26 (m, 2H), 6.83-6.79 (m, 1H), 6.26-6.21 (m, 1H), 5.79-5.76 (m, 1H), 4.90 (m, 1H), 4.13 (s, 3H), 3.99-3.96 (m, 2H), 3.95 (s, 3H), 3.69 (brs, 2H), 2.12 (brs, 2H), 1.94 (brs, 2H); MS (ESI): m/z 595 [M+1]⁺ | 70 | 11.44 100 (b) |
| 212 | 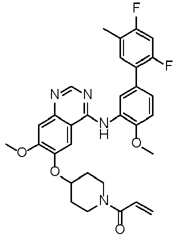 | 1-(4-((4-((2',4'-difluoro-4-methoxy-5'-methyl-[1,1'-biphenyl]-3-yl)amino)-7-methoxyquinaz olin-6-yl)oxy)piperi din-1-yl)prop-2-en-1-one | MS (ESI): m/z 561 [M+1]⁺ | | |
| 213 | 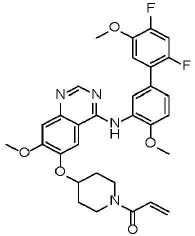 | 1-(4-((4-((2',4'-difluoro-4,5'-dimethoxy-[1,1'- biphenyl]-3-yl)amino)-7-methoxyquinaz olin-6-yl)oxy)piperi din-1-yl)prop-2-en-1-one | ¹H NMR (400 MHz, TFA salt, Methanol-*d*₄) δ 8.31 (s, 1H), 7.83 (s, 1H), 7.81 (s, 1H), 7.50-7.45 (m, 1H), 7.24-7.15 (m, 3H), 7.09-7.02 (m, 1H), 6.82 (dd, *J* = 16.8, 10.7 Hz, 1H), 6.22 (dd, *J* = 16.8, 2.0 Hz, 1H), 5.76 (dd, *J =* 10.6, 1.9 Hz, 1H), 4.84-4.82 (m, 1H), 4.01 (s, 3H), 3.99-3.93 (m, 2H), 3.91 (s, 6H), 3.72-3.61 (m, 2H), 2.13-2.05 (m, 2H), 1.96-1.84 (m, 2H); MS (ESI): m/z 576 [M]⁺ | 74 | 10.51 99 (b) |
| 214 | 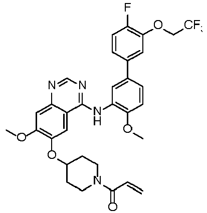 | 1-(4-((4-((4'-fluoro-4-methoxy-3'-(2,2,2-trifluoroetho xy)-[1,1'-biphenyl]-3-yl)amino)-7-methoxyquinaz olin-6-yl)oxy)piperi din-1-yl)prop-2-en-1-one | ¹H NMR (400MHz, TFA salt, Methanol-*d*₄) δ 8.60 (s, 1H), 8.06 (s, 1H), 7.79-7.77 (d, *J* = 2.4 Hz 1H), 7.71-7.68 (m, 1H), 7.42-7.39 (m, 1H), 7.33-7.21 (m, 4H), 6.84-6.79 (m, 1H), 6.26-6.21 (m, 1H), 5.79-5.76 (m, 1H), 4.90 (m, 1H), 4.72-4.65 (m, 2H), 4.10 (s, 3H), 3.99-3.93 (m, 2H), 3.91 (s, 3H), 3.69 (brs, 2H), 2.16 (brs, 2H), 1.93 (brs, 2H); MS (ESI): m/z 627 [M+1]⁺ | 35 | 11.17 99 (b) |
| 215 | 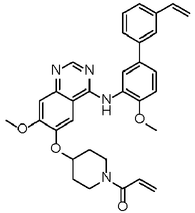 | 1-(4-((7-methoxy-4-((4-methoxy-3'-vinyl-[1,1'- biphenyl]-3-yl)amino)quin azolin-6-yl)oxy)piperi din-1-yl)prop-2-en-1-one | ¹H NMR (400 MHz, TFA salt, Methanol-*d*₄) δ 8.61 (s, 1H), 7.81 (s, 1H), 7.80 (s, 1H), 7.73-7.71 (m, 1H), 7.66 (s, 1H), 7.53 (d, *J* = 6.8 Hz, 1H), 7.43-7.41 (m, 2H), 7.31-7.27 (m, 2H), 6.84-6.79 (m, 2H), 6.26-6.22 (m, 1H), 5.89-5.77 (m, 2H), 5.31 (d, *J* = 10.8 Hz, 1H), 5.01-5.00 (m, 1H), 4.10 (s, 3H), 3.93-3.91 (m, 5H), 3.71-3.69 (m, 2H), 2.17-2.16 (m, 2H), 1.99-1.97 (m, 2H); MS (ESI): m/z 601 [M+H] ⁺ | 29 | 10.70 100 (b) |
| 216 | 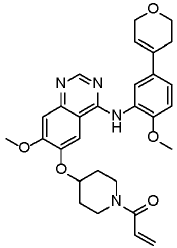 | 1-(4-((4-((5-(3,6-dihydro-2H-pyran-4-yl)-2-methoxyphenyl )amino)-7-methoxyquinaz olin-6-yl)oxy)piperi din-1-yl)prop-2-en-1-one | ¹H NMR (400MHz, FA salt, Methanol-*d*₄) δ 8.31 (s, 1H), 7.79 (s, 1H), 7.76-7.75 (m, 1H), 7.38-7.35 (m, 1H), 7.19 (s, 1H), 7.10-7.08 (m, 1H), 6.86-6.79 (m, 1H), 6.25-6.21 (m, 1H), 6.14-6.13 (m, 1H), 5.79-5.75 (m, 1H), 4.87-4.80 (m, 1H), 4.30-4.29 (m, 2H), 4.02 (s, 3H), 4.01-3.91 (m, 4H), 3.87 (s, 3H), 3.70-3.60 (m, 2H), 3.33 (m, 2H), 3.32 (s, 3H), 2.52-2.50 (m, 2H), 2.15-1. 80 (m, 4H); MS (ESI): m/z 517 [M+1]⁺ | 45 | 8.90 92 (b) |
| 217 | 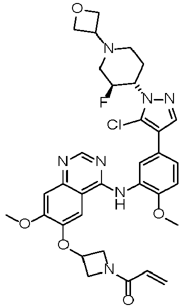 | 1-(3-((4-((5-(5-chloro-1-((3S,4S)-3-fluoro-1-(oxetan-3-yl)piperidin-4-yl)-1H-pyrazol-4-yl)-2-methoxyphenyl )amino)-7-methoxyquinaz olin-6-yl)oxy)azetid in-1-yl)prop-2-en-1-one | ¹H NMR (400 MHz, free base, Methanol-*d*₄) δ 8.36 (s, 1H), 8.00 (s, 1H), 7.89 (s, 1H), 7.56-7.53 (m, 1H), 7.46 (s, 1H), 7.24-7.19 (m, 2H), 6.45-6.38 (m, 1H), 6.32-6.28 (m, 1H), 5.81-5.76 (m, 1H), 5.27-5.25 (m, 1H), 5.12-5.11 (m, 1H), 4.61-4.40 (m, 7H), 4.19-4.18 (m, 1H), 4.05 (s, 3H), 3.96 (s, 3H), 3.72-3.69 (m, 2H), 2.92-2.91 (m, 1H), 2.20-2.15 (m, 5H); MS (ESI): m/z 665 [M+H] ⁺ | 12 | 4.27 90* |
| 218 | 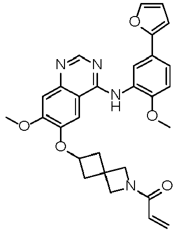 | 1-(6-((4-((5-(furan-2-yl)-2-methoxyphenyl )amino)-7-methoxyquinaz olin-6-yl)oxy)-2-azaspiro[3.3] heptan-2-yl)prop-2-en-1-one | ¹H NMR (400MHz, TFA salt, Methanol-*d*₄) δ 8.60 (s, 1H), 7.83 (s, 1H), 7.76-7.72 (m, 2H), 7.54 (d, *J* = 1.2 Hz, 1H), 7.26 (s, 1H), 7.23 (s, 1H), 6.70 (d, *J* = 3.2 Hz, 1H), 6.52-6.51 (m, 1H), 6.37-6.22 (m, 2H), 5.76-5.73 (m, 1H), 4.41-4.35 (m, 2H), 4.18-4.09 (m, 5H), 3.90 (d, *J* = 2.0 Hz, 3H), 3.01-2.87 (m, 2H), 2.51-2.46 (m, 2H); MS (ESI): m/z 513 [M+1] ⁺ | 5 | 5.80 100 (a) |
| 219 | 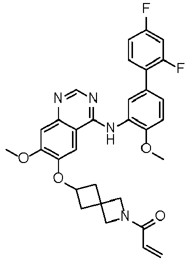 | 1-(6-((4-((2',4'-difluoro-4-methoxy-[1,1'-biphenyl]-3-yl)amino)-7-methoxyquinaz olin-6-yl)oxy)-2-azaspiro[3.3] heptan-2-yl)prop-2-en-1-one | ¹H NMR (400MHz, TFA salt, Methanol-*d*₄) δ 8.59 (s, 1H), 7.73-7.69 (m, 2H), 7.61-7.51 (m, 2H), 7.31 (d, *J* = 8.4 Hz, 1H), 7.26 (s, 1H), 7.09 (t, *J* = 8.4 Hz, 1H), 6.37-6.22 (m, 2H), 5.76-5.73 (m, 1H), 4.41-4.35 (m, 2H), 4.18-4.09 (m, 5H), 3.92 (s, 3H), 3.01-2.97 (m, 2H), 2.51-2.46 (m, 2H); MS (ESI): m/z 559 [M+1] ⁺ | 3 | 6.38 100 (a) |
| 220 | 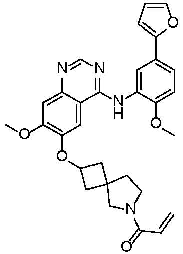 | 1-(2-((4-((5-(furan-2-yl)-2-methoxyphenyl )amino)-7-methoxyquinaz olin-6-yl)oxy)-6-azaspiro[3.4] octan-6-yl)prop-2-en-1-one | ¹H NMR (400 MHz,FA salt, Methanol-*d*₄) δ 8.38 (s, 1H), 8.22 (s, 1H), 8.00-7.95 (m, 1H), 7.66-7.60 (m, 1H), 7.52-7.45 (m, 2H), 7.19-7.13 (m, 2H), 6.68-6.53 (m, 2H), 6.50-6.47 (m, 1H), 6.30-6.23 (m, 1H), 5.77-5.70 (m, 1H), 5.01-4.94 (m, 1H), 4.02 (s, 3H), 3.88 (s, 3H), 3.72-3.50 (m, 4H), 2.75-2.67 (m, 2H), 2.33-2.25 (m, 2H), 2.16-2.02 (m, 2H); MS (ESI): m/z=527 [M+1]⁺ | 13 | 10.00 100 (b) |
| 221 | 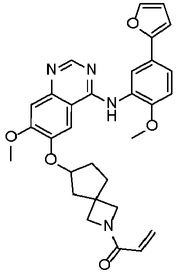 | 1-(6-((4-((5-(furan-2-yl)-2-methoxyphenyl )amino)-7-methoxyquinaz olin-6-yl)oxy)-2-azaspiro[3.4] octan-2-yl)prop-2-en-1-one | ¹H NMR (400 MHz, TFA salt, Methanol-*d*₄) δ 8.60 (s, 1H), 7.84 (s, 1H), 7.83 (s, 1H), 7.77-7.75 (m, 1H), 7.54 (s, 1H), 7.26-7.23 (m, 2H), 6.71 (d, *J =* 3.2 Hz, 1H), 6.53-6.52 (m, 1H), 6.36-6.28 (m, 2H), 5.77-5.74 (m, 1H), 5.15-5.14 (m, 1H), 4.28-4.26 (m, 2H), 4.10 (s, 3H), 4.02-4.01 (m, 2H), 3.90 (s, 3H), 2.35-2.32 (m, 4H), 2.06-2.04 (m, 2H); MS (ESI): m/z 527 [M+H]⁺ | 20 | 10.03 100 (b) |
| 222 | 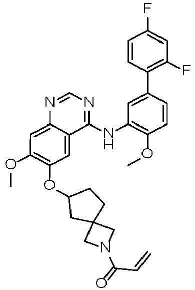 | 1-(6-((4-((2',4'-difluoro-4-methoxy-[1,1'-biphenyl]-3-yl)amino)-7-methoxyquinaz olin-6-yl)oxy)-2-azaspiro[3.4] octan-2-yl)prop-2-en-1-one | ¹H NMR (400 MHz, FA salt, Methanol-*d*₄) δ 8.32 (s, 1H), 7.87 (s, 1H), 7.65 (d, *J* = 1.2 Hz, 1H), 7.57-7.55 (m ,1H), 7.45 (d, *J* = 8.8 Hz, 1H), 7.24-7.19 (m, 2H), 7.08-7.03 (m, 2H), 6.35-6.32 (m, 1H), 6.28-6.23 (m, 1H), 5.77-5.74 (m, 1H), 5.14-5.13 (m, 1H), 5.51-5.50 (m, 1H), 4.35-4.32 (m, 1H), 4.12-4.10 (m, 5H), 4.00 (s, 3H), 2.35-2.34 (m, 4H), 2.04-2.03 (m, 2H); MS (ESI): m/z 573 [M+H]⁺ | 24 | 10.77 98 (b) |
| 223 | 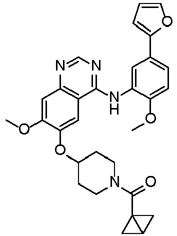 | bicyclo[1.1.0 ]butan-1-yl(4-((4-((5-(furan-2-yl)-2-methoxyphenyl )amino)-7-methoxyquinaz olin-6-yl)oxy)piperi din-1-yl)methanone | MS (ESI): m/z 527 [M+1]⁺ | | |
| 224 | 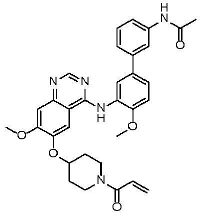 | N-(3'-((6-((1-acryloylpiper idin-4-yl)oxy)-7-methoxyquinaz olin-4-yl)amino)-4'-methoxy-[1,1'- biphenyl]-3-yl)acetamide | ¹H NMR (400 MHz, TFA salt, Methanol-*d*₄) δ 8.56 (s, 1H), 8.02 (s, 1H), 7.98-7.97 (d, *J* = 1.35 Hz, 1H), 7.80-7.79 (d, *J* = 2.28 Hz, 1H), 7.67-7.65 (dd, *J =* 8.6 Hz 1H), 7.39-7.35 (m, 3H), 7.28-7.24 (t, 2H), 6.85-6.79 (m, 1H), 6.25-6.20 (dd, *J* = 16.81 Hz, 1H), 5.78-5.75 (dd, *J =* 10.66 Hz, 1H), 4.86 (m, 1H), 4.08 (s, 3H), 3.99-3.91 (m, 5H), 3.71-3.65 (m, 2H), 2.15-2.08 (m, 5H), 1.97-1.86 (m, 2H); MS (ESI): m/z 568 [M+1]⁺ | 8 | 9.03 97 (b) |
| 225 | 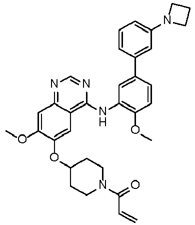 | 1-(4-((4-((3'-(azetidin-1-yl)-4-methoxy-[1,1'- biphenyl]-3-yl)amino)-7-methoxyquinaz olin-6-yl)oxy)piperi din-1-yl)prop-2-en-1-one | ¹H NMR (400 MHz, FA salt, CDCl₃) δ 8.94 (d, *J* = 2.1 Hz, 1H), 8.70 (s, 1H), 8.11 (s, 1H), 7.89 (s, 1H), 7.34-7.27 (m, 4H), 7.01 (dd, *J* = 8.3, 3.1 Hz, 2H), 6.70-6.58 (m, 2H), 6.45 (dd, *J* = 7.9, 1.8 Hz, 1H), 6.31 (dd, *J* = 16.8, 1.8 Hz, 1H), 5.72 (dd, *J =* 10.6, 1.8 Hz, 1H), 4.71-4.63 (m, 1H), 4.01 (s, 6H), 3.97-3.85 (m, 7H), 3.74-3.48 (m, 3H), 2.44-2.34 (m, 2H), 2.04-1.95 (m, 2H); MS (ESI): m/z 566 [M+1]⁺ | 14 | 9.74 97 (b) |
| 226 | 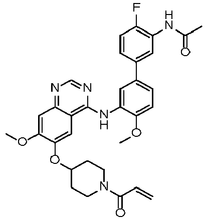 | N-(3'-((6-((1-acryloylpiper idin-4-yl)oxy)-7-methoxyquinaz olin-4-yl)amino)-4-fluoro-4'-methoxy-[1,1'- biphenyl]-3-yl)acetamide | ¹H NMR (400 MHz, TFA salt, Methanol-*d*₄) δ 8.60 (s, 1H), 8.24-8.23 (d, *J =* 7.23 Hz, 1H), 8.05 (s, 1H), 7.75 (s, 1H), 7.66-7.64 (d, *J* = 7.24 Hz, 1H), 7.41-7.38 (m, 1H), 7.28-7.20 (m, 3H), 6.85-6.79 (m, 1H), 6.25-6.21 (d, *J* = 16.75 Hz, 1H), 5.78-5.76 (d, *J* = 10.65 Hz, 1H), 4.85 (m, 1H), 4.09 (s, 3H), 3.98-3.90 (m, 5H), 3.71-3.62 (m, 2H), 2.20 (s, 3H), 2.17-2.08 (m, 2H), 1. 98-1. 85 (m, 2H); MS (ESI): m/z 586 [M+1]⁺ | 42 | 9.15 96 (b) |
| 227 | 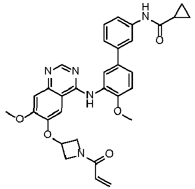 | N-(3'-((6-((1-acryloylazeti din-3-yl)oxy)-7-methoxyquinaz olin-4-yl)amino)-4'-methoxy-[1,1'- biphenyl]-3-yl)cyclopropa ncarboxamide | ¹H NMR (400MHz, TFA salt, Methanol-*d*₄) δ 8.62 (s, 1H), 8.01 (s, 1H), 7.79-7.68 (m, 3H), 7.38-7.27 (m, 5H), 6.39-6.28 (m, 2H), 5.81 (d, *J* = 11.2 Hz, 1H), 5.29 (s, 1H), 4.66-4.61 (m, 1H), 4.47-4.45 (m, 1H), 4.17-4.12 (m, 4H), 3.92 (s, 3H), 1.82-1.78 (m, 1H), 0.97-0.87 (m, 4H); MS (ESI): m/z 566 [M+1]⁺ | 30 | 5.54 100 (a) |
| 228 | 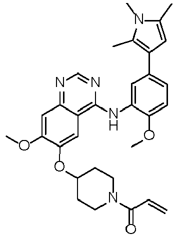 | 1-(4-((7-methoxy-4-((2-methoxy-5-(1,2,5-trimethyl-1H-pyrrol-3-yl)phenyl)ami no)quinazolin -6-yl)oxy) piperidin-1-yl)prop-2-en-1-one | MS (ESI): m/z 542 [M+1]⁺ | | |
| 229 | 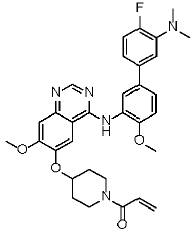 | 1-(4-((4-((3'-(dimethylamin o)-4'-fluoro-4-methoxy-[1,1'-biphenyl]-3-yl)amino)-7-methoxyquinaz olin-6-yl)oxy)piperi din-1-yl)prop-2-en-1-one | ¹H NMR (400 MHz, FA salt, CDCl₃) δ 8.92 (d, *J =* 2.4 Hz, 1H), 8.71 (s, 1H), 8.15 (s, 1H), 7.52 (s, 1H), 7.28-7.27 (m, 1H), 7.13-7.00 (m, 4H), 6.66-6.59 (m, 1H), 6.34-6.29 (m, 1H), 5.74-5.71 (m, 1H), 4.70-4.68 (m, 1H), 4.12-4.10 (m, 8H), 3.82-3.56 (m, 2H), 2.99 (s, 6H), 2.04-1.98 (m, 4H); MS (ESI): m/z 572 [M+H]⁺ | 5 | 9.84 97 (b) |
| 230 | 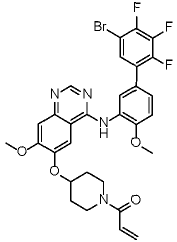 | 1-(4-((4-((5'-bromo-2'3'4'-trifluoro-4-methoxy-[1,1'- biphenyl]-3-yl)amino)-7-methoxyquinaz olin-6-yl)oxy)piperi din-1-yl)prop-2-en-1-one | MS (ESI): m/z 643 [M+H]⁺ | | |

### Example 231. Preparation of 4-(3-((6-((1-acryloylpiperidin-4-yl)oxy)-7-methoxyquinazolin-4-yl)amino)-4-methoxyphenyl)-N-(3-fluorophenethyl)thiophen-2-carboxamide

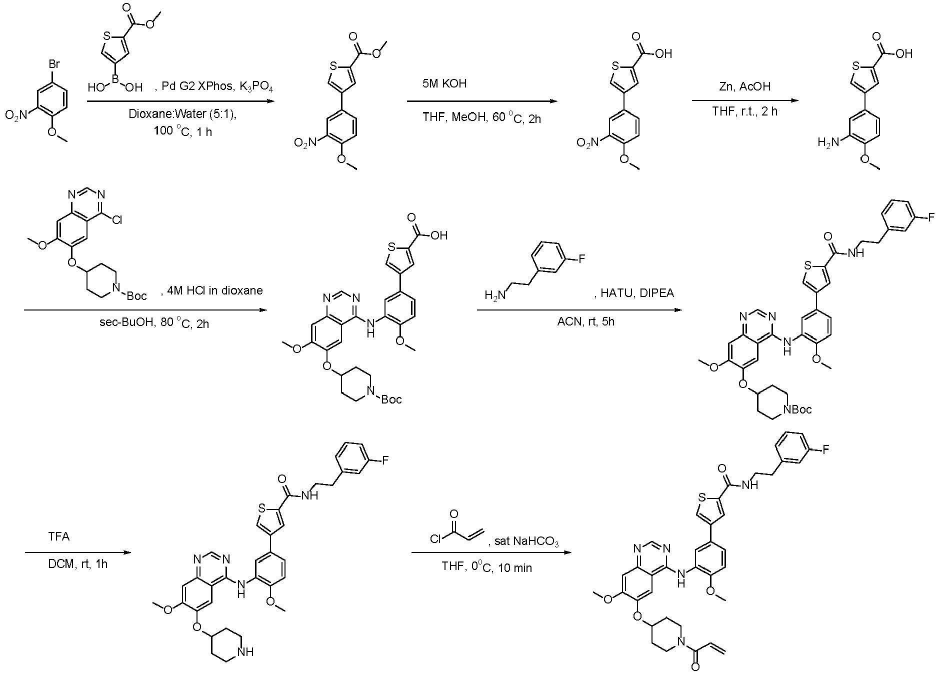

### [Step-1] Preparation of methyl 4-(4-methoxy-3-nitrophenyl)thiophen-2-carboxylate

In 1,4-dioxane (0.1 M) and water (0.5 M), 4-bromo-1-methoxy-2-nitrobenzene (1.0 equiv.), (5-(methoxycarbonyl)thiophen-3-yl)boronic acid (2.0 equiv.), and K₃PO₄ (2.2 equiv.) were dissolved, and degassed using nitro gas. Then, Xphos Pd G2 (0.13 equiv.) was added at 80 °C, and the mixture was stirred at 100 °C for 1 hour. The reaction mixture was filtered through a celite filter, and concentrated. The reaction mixture was purified by MPLC (DCM / MeOH) to obtain the target compound as a yellow solid (yield: 100%, MS (ESI): m/z 294 [M+1]⁺).

### [Step-2] Preparation of 4-(4-methoxy-3-nitrophenyl)thiophen-2-carboxylic acid

In THF (0.05 M) and MeOH (0.05 M), methyl 4-(4-methoxy-3-nitrophenyl)thiophen-2-carboxylate (1.0 equiv.) obtained in Step-1 above was dissolved, and 5M KOH (1.2 equiv.) dissolved in distilled water was further added and stirred at 60 °C for 2 hours. The reaction product was vacuum dried at 50 °C to obtain the target compound as a white solid (yield: 100%, MS (ESI): m/z 280 [M+1]⁺).

### [Step-3] Preparation of 4-(3-amino-4-methoxyphenyl)thiophen-2-carboxylic acid

In THF (0.3 M), 4-(4-methoxy-3-nitrophenyl)thiophen-2-carboxylic acid (1.0 equiv.) obtained in Step-2 above was dissolved at 0 °C, and then acetic acid (20 equiv.) and zinc (10 equiv.) were added. The reaction mixture was stirred at room temperature for 2 hours. The mixture was filtered through a celite filter and concentrated. The concentrate was neutralized by adding a saturated NaHCO₃ aqueous solution at 0 °C, the organic materials were extracted with DCM, and the collected organic layer was concentrated by removing remaining water using Na₂SO₄. The concentrate was purified by prep-HPLC to obtain the target compound as a yellow solid (yield: 84%, MS (ESI): m/z 250 [M+1]⁺).

### [Step-4] Preparation of 4-(3-((6-((1-(tert-butoxycarbonyl)piperidin-4-yl)oxy)-7-methoxyquinazolin-4-yl)amino)-4-methoxyphenyl)thiophen-2-carboxylic acid

In sec-BuOH (0.1 M), 4-(3-amino-4-methoxyphenyl)thiophen-2-carboxylic acid (1.2 equiv.) obtained in Step-3 above and tert-butyl 4-((4-chloro-7-methoxyquinazolin-6-yl)oxy)piperidine-1-carboxylate (1.0 equiv.) were dissolved. Then, 4M HCl (0.2 equiv.) dissolved in 1,4-dioxane was further added, and stirred at 80 °C for 2 hours, and then concentrated. The reaction mixture was purified by MPLC (DCM:MeOH) to obtain the target compound as a yellow solid (yield: 100%, MS (ESI): m/z 607 [M+1]⁺).

### [Step-5] Preparation of tert-butyl 4-((4-((5-(5-((3-fluorophenethyl)carbamoyl)thiophen-3-yl)-2-methoxyphenyl)amino)-7-methoxyquinazolin-6-yl)oxy)piperidine-1-carboxylate

In acetonitrile (0.1 M), 4-(3-((6-((1-(tert-butoxycarbonyl)piperidin-4-yl)oxy)-7-methoxyquinazolin-4-yl)amino)-4-methoxyphenyl)thiophen-2-carboxylic acid (1.0 equiv.) obtained in Step-4 above and 2-(3-fluorophenyl)ethan-1-amine (1.0 equiv.) were dissolved. Then, HATU (2.0 equiv.) and DIPEA (3.0 equiv.) were added, and stirred at room temperature for 5 hours, and then concentrated. The reaction mixture was purified by MPLC (DCM:MeOH) to obtain the target compound as a yellow solid (yield: 100%, MS (ESI): m/z 728 [M+1]⁺).

### [Step-6] Preparation of N-(3-fluorophenethyl)-4-(4-methoxy-3-((7-methoxy-6-(piperidin-4-yloxy)quinazolin-4-yl)amino)phenyl)thiophen-2-carboxamide

In DCM (0.1 M), tert-butyl 4-((4-((5-(5-((3-fluorophenethyl)carbamoyl)thiophen-3-yl)-2-methoxyphenyl)amino)-7-methoxyquinazolin-6-yl)oxy)piperidine-1-carboxylate (1.0 equiv.) obtained in Step-5 above was dissolved. Then, TFA (10 equiv.) was added and the reaction mixture was stirred at room temperature for 1 hour and then concentrated. The reaction mixture was purified by MPLC (DCM:MeOH) to obtain the target compound as a yellow solid (yield: 95%, MS (ESI): m/z 628 [M+1]⁺).

### [Step-7] Preparation of 4-(3-((6-((1-acryloylpiperidin-4-yl)oxy)-7-methoxyquinazolin-4-yl)amino)-4-methoxyphenyl)-N-(3-fluorophenethyl)thiophen-2-carboxamide

In THF (0.1 M), N-(3-fluorophenethyl)-4-(4-methoxy-3-((7-methoxy-6-(piperidin-4-yloxy)quinazolin-4-yl)amino)phenyl)thiophen-2-carboxamide (1.0 equiv.) obtained in Step-6 above and saturated NaHCO₃ aqueous solution (5.0 equiv.) were dissolved. Then, acryloyl chloride (1.0 equiv.) was added at 0 °C and the reaction mixture was stirred for 10 minutes and then concentrated.

The reaction mixture was purified by prep-HPLC to obtain the target compound as a pale yellow solid (yield: 13%, MS (ESI): m/z 682 [M+1]⁺).

### Preparation of Compounds of Examples 232 to 235

Compounds of Examples 232 to 235 according to the present invention were prepared in a similar manner to Example 231 above. Chemical structures, compound names, ¹H NMR, MS, HPLC data and yields of respective Examples are summarized in Table 2 below.

**[Table 2]**

| Exa mpl e No. | Structure | Compound Name | ¹H NMR; MS | Yie ld (%) | HPLC r.t. (min) Purity (%) (method) |
|---|---|---|---|---|---|
| 231 | 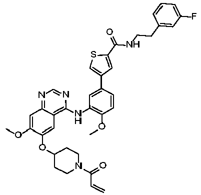 | 4-(3-((6-((1-acryloylpiper idin-4-yl)oxy)-7-methoxyquinaz olin-4-yl)amino)-4-methoxyphenyl )-N-(3-fluoropheneth yl)thiophen-2-carboxamide | ¹H NMR (400MHz, TFA salt, Methanol-*d*₄) δ 8.60 (s, 1H), 8.06 (s, 1H), 7.82-7.78 (m, 3H), 7.71-7.68 (m, 1H), 7.34-7.29 (m, 2H), 7.24 (s, 1H), 7.04 (tr, *J* = 54.4 Hz, 1H), 6.85-6.79 (m, 1H), 6.25-6.20 (m, 1H), 5.78-5.75 (m, 1H), 4.09 (s, 3H), 3.98-3.91 (m, 6H), 3.68 (brs, 2H), 2.16-2.11 (m, 2H), 1.92 (brs, 2H); MS (ESI): m/z 579 [M+1] ⁺ | 27 | 10.73 99 (b) |
| 232 | 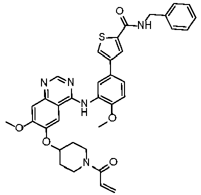 | 4-(3-((6-((1-acryloylpiper idin-4-yl)oxy)-7-methoxyquinaz olin-4-yl)amino)-4-methoxyphenyl )-N-benzylthiophe n-2-carboxamide | ¹H NMR (400MHz, TFA salt, Methanol-*d*₄) δ 8.59 (s, 1H), 8.05 (d, *J* = 1.2 Hz, 1H), 7.82-7.81 (m, 2H), 7.73-7.71 (m, 1H), 7.37-7.31 (m, 4H), 7.27-7.24 (m, 3H), 6.85-6.78 (m, 1H), 6.25-6.20 (m, 1H), 5.78-5.75 (m, 1H), 4.57 (m, 2H), 4.09 (s, 3H), 3.98-3.89 (m, 5H), 3.67 (brs, 2H), 2.11 (brs, 2H), 1.91 (brs, 2H); MS (ESI): m/z 650 [M+1] ⁺ | 21 | 6.30 100 (a) |
| 233 | 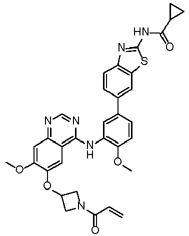 | N-(6-(3-((6-((1-acryloylazeti din-3-yl)oxy)-7-methoxyquinaz olin-4-yl)amino)-4-methoxyphenyl )benzo[d]thia zol-2-yl)cyclopropa ncarboxamide | ¹H NMR (400 MHz, free base, Methanol-*d*₄) δ 8.38 (s, 1H), 8.11 (s, 1H), 8.04 (s, 1H), 7.81 (d, *J* = 8.4 Hz, 1H), 7.74 (d, *J* = 8.0 Hz, 1H), 7.64 (d, *J* = 8.8 Hz, 1H), 7.49 (s, 1H), 7.26-7.24 (m, 2H), 6.41-6.38 (m, 1H), 6.32-6.28 (m, 1H), 5.80 (d, *J* = 10.8 Hz, 1H), 5.51-5.48 (m, 1H), 4.62-4.60 (m, 2H), 4.46-4.44 (m, 1H), 4.18-4.12 (m, 1H), 4.05 (s, 3H), 3.95 (s, 3H), 3.62-3.60 (m, 1H), 1.05-1.03 (m, 2H), 0.97-0.90 (m, 2H); MS (ESI): m/z 623 [M+H]⁺ | 22 | 5.76 100 (a) |
| 234 | 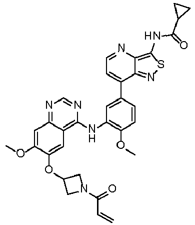 | N-(7-(3-((6-((1-acryloylazeti din-3-yl)oxy)-7-methoxyquinaz olin-4-yl)amino)-4-methoxyphenyl )isothiazolo[ 4,3-b]pyridin-3-yl)cyclopropa ncarboxamide | ¹H NMR (400MHz, FA salt, DMSO-*d*₆) δ 9.37 (s, 1H), 8.69 (d, *J* = 4.0 Hz, 1H), 8.41 (d, *J* = 2.0 Hz, 1H), 8.28 (s, 1H), 8.23-8.20 (m, 1H), 7.69 (d, *J* = 4.4 Hz, 1H), 7.56 (s, 1H), 7.33 (d, *J =* 8.8 Hz, 1H), 7.22 (s, 1H), 6.42-6.35 (m, 1H), 6.16-6.12 (m, 1H), 5.73-5.69 (m, 1H), 5.23-5.22 (m, 1H), 4.78-4.77 (m, 1H), 4.59-4.55 (m, 1H), 4.40-4.29 (m, 1H), 3.95 (s, 3H), 3.86 (s, 3H), 1.32-1.10 (m, 5H); MS (ESI): m/z 624 [M+1]⁺ | 31 | 5.17 100 (a) |
| 235 | 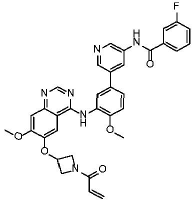 | N-(5-(3-((6-((1-acryloylazeti din-3-yl)oxy)-7-methoxyquinaz olin-4-yl)amino)-4-methoxyphenyl )pyridin-3-yl)-3-fluorobenzami de | ¹H NMR (400MHz, FA salt, Methanol-*d*₄) δ 9.18-9.10 (s, 1H), 8.85 (s, 1H), 8.80-8.70 (m, 1H), 8.67 (s, 1H), 8.01-8.00 (s, 1H), 7.88-7.84 (m, 2H), 7.80-7.79 (m, 1H), 7.77-7.74 (m, 1H), 7.61-7.60 (m, 1H), 7.41-7.40 (m, 2H), 7.33 (s, 1H), 6.41-6.38 (m, 1H), 6.34-6.30 (m, 1H), 5.82-5.80 (m, 1H), 5.32-5.25 (m, 1H), 4.94-4.91 (m, 1H), 4.90-4.89 (m, 1H), 4.58-4.47 (m, 1H), 4.15-4.14 (m, 1H), 4.13 (s, 3H), 3.98 (s, 3H); MS (ESI): m/z 621 [M+1]⁺ | 22 | 4.76 99 (a) |

### Example 236. Preparation of (R)-N-(3'-((6-((1-acryloylazetidin-3-yl)oxy)-7-methoxyquinazolin-4-yl)amino)-4'-methoxy-[1,1'-biphenyl]-3-yl)-2-oxo-4-phenyloxazolidin-3-carboxamide

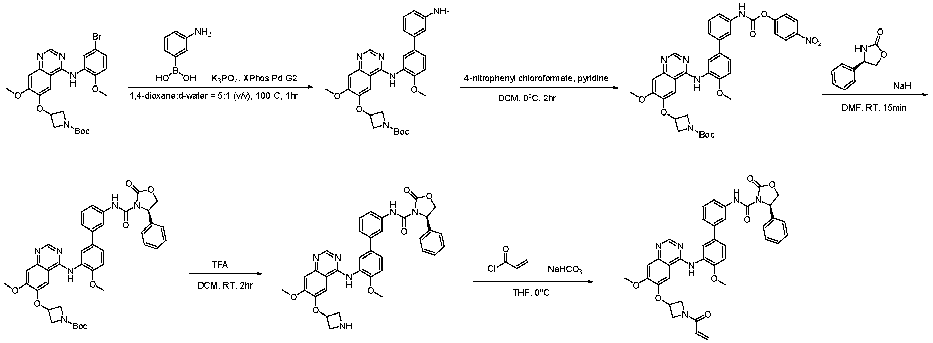

### [Step-1] Preparation of tert-butyl 3-((4-((3'-amino-4-methoxy-[1,1'-biphenyl]-3-yl)amino)-7-methoxyquinazolin-6-yl)oxy)azetidin-1-carboxylate

In 1,4-dioxane:distilled water = 5:1 (v/v) (0.1 M), tert-butyl 3-((4-((5-bromo-2-methoxyphenyl)amino)-7-methoxyquinazolin-6-yl)oxy)azetidin-1-carboxylate (1.0 equiv.), (3-aminophenyl)boronic acid (3.0 equiv.), and K₃PO₄ (2.0 equiv.) were dissolved, then degassed with nitrogen, and stirred at 100 °C for 3 minutes under nitrogen. Then, Xphos Pd G2 (0.13 equiv.) was added at 100 °C, and the reaction mixture was stirred for 1 hour and then concentrated. The reaction mixture was purified by MPLC (DCM:MeOH) to obtain the target compound as a pale yellow solid (yield: 630, MS (ESI): m/z 544 [M+1]⁺).

### [Step-2] Preparation of tert-butyl 3-((7-methoxy-4-((4-methoxy-3'-(((4-nitrophenoxy)carbonyl)amino)-[1,1'-biphenyl]-3-yl)amino)quinazolin-6-yl)oxy)azetidin-1-carboxylate

In DCM (0.1 M), tert-butyl 3-((4-((3'-amino-4-methoxy-[1,1'-biphenyl]-3-yl)amino)-7-methoxyquinazolin-6-yl)oxy)azetidin-1-carboxylate (1.0 equiv.) obtained in Step-1 above was dissolved, and then pyridine (3.0 equiv.) was added. The reaction mixture was cooled to 0 °C, then 4-nitrophenylchloroformate (1.5 equiv.) was added and stirred at room temperature for 2 hours. The target compound obtained as a brown solid was used in the next reaction without further purification (yield: 100%, MS (ESI): m/z 709 [M+1]⁺).

### [Step-3] Preparation of tert-butyl (R)-3-((7-methoxy-4-((4-methoxy-3'-(2-oxo-4-phenyloxazolidin-3-carboxamido)-[1,1'-biphenyl]-3-yl)amino)quinazolin-6-yl)oxy)azetidin-1-carboxylate

In DMF (0.1 M), (R)-4-phenyloxazolidin-2-one (2.2 equiv.) was dissolved, then NaH (2.2 equiv.) was added, and the mixture was stirred at room temperature for 20 minutes. To tert-butyl 3-((7-methoxy-4-((4-methoxy-3'-(((4-nitrophenoxy)carbonyl)amino)-[1,1'-biphenyl]-3-yl)amino)quinazolin-6-yl)oxy)azetidin-1-carboxylate obtained in Step-2 above, the reaction mixture was added and stirred at room temperature for 15 minutes. Organic materials were extracted from the reaction mixture with saturated NaHCO₃ and EA, and the collected organic layer was concentrated by removing remaining water using MgSO₄. The target compound obtained as a brown solid was used in the next reaction without further purification (yield: 100%, MS (ESI): m/z 733 [M+1]⁺).

### [Step-4] Preparation of (R)-N-(3'-((6-(azetidin-3-yloxy)-7-methoxyquinazolin-4-yl)amino)-4'-methoxy-[1,1'-biphenyl]-3-yl)-2-oxo-4-phenyloxazolidin-3-carboxamide

In a solution of DCM:TFA (10:1 v/v) (0.1 M), tert-butyl (R)-3-((7-methoxy-4-((4-methoxy-3'-(2-oxo-4-phenyloxazolidin-3-carboxamido)-[1,1'-biphenyl]-3-yl)amino)quinazolin-6-yl)oxy)azetidin-1-carboxylate (1.0 equiv.) obtained in Step-3 above was dissolved, and stirred at room temperature for 2 hours. The reaction mixture was concentrated and purified by MPLC (DCM:MeOH) to obtain the target compound as a yellow solid (yield: 38%, MS (ESI): m/z 633 [M+1]⁺).

### [Step-5] Preparation of (R)-N-(3'-((6-((1-acryloylazetidin-3-yl)oxy)-7-methoxyquinazolin-4-yl)amino)-4'-methoxy-[1,1'-biphenyl]-3-yl)-2-oxo-4-phenyloxazolidin-3-carboxamide

In THF (0.1 M), (R)-N-(3'-((6-(azetidin-3-yloxy)-7-methoxyquinazolin-4-yl)amino)-4'-methoxy-[1,1'-biphenyl]-3-yl)-2-oxo-4-phenyloxazolidin-3-carboxamide (1.0 equiv.) obtained in Step-4 above was dissolved, and cooled to 0 °C. To the mixture, 1M NaHCO₃ solution (2.0 equiv.) was added, and acryloyl chloride (1.2 equiv.) was added and stirred at 0 °C for 30 minutes. Saturated NaHCO₃ aqueous solution was added to the reaction mixture, then organic materials were extracted with EA, and the collected organic layer was concentrated by removing remaining water using MgSO₄. The reaction mixture was purified by MPLC (DCM:MeOH) to obtain the target compound as a white solid (yield: 49%, MS (ESI): m/z 687 [M+1]⁺).

### Preparation of Compound of Example 237

A compound of Example 237 according to the present invention was prepared in a similar manner to Example 236 above. Chemical structures, compound names, ¹H NMR, MS, HPLC data and yields of respective Examples are summarized in Table 3 below.

**[Table 3]**

| Exa mpl e No. | Structure | Compound Name | ¹H NMR; MS | Yie ld (%) | HPLC r.t.(min ) Purity(% ) (method) |
|---|---|---|---|---|---|
| 236 | 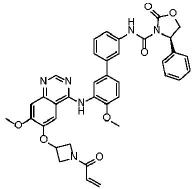 | (R)-N-(3'-((6-((1-acryloylazeti din-3-yl)oxy)-7-methoxyquinaz olin-4-yl)amino)-4'-methoxy-[1,1'- biphenyl]-3-yl)-2-oxo-4-phenyloxazoli din-3-carboxamide | ¹H NMR (400MHz, TFA salt, Methanol-*d*₄) δ 10.11 (s, 1H), 8.54 (d, *J* = 2.4 Hz, 1H), 7.77-7.64 (m, 4H), 7.42-7.23 (m, 10H), 6.38-6.28 (m, 2H), 5.81-5.78 (m, 1H), 5.56-5.53 (m, 1H), 5.25-5.24 (m, 1H), 4.63-4.59 (m, 1H), 4.44-4.42 (m, 1H), 4.30-4.26 (m, 1H), 4.13-4.10 (s, 4H), 3.89 (s, 3H); MS (ESI): m/z 687 [M+1]⁺ | 49 | 6.69 100 (a) |
| 237 | 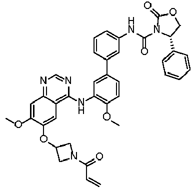 | (S)-N-(3'-((6-((1-acryloylazeti din-3-yl)oxy)-7-methoxyquinaz olin-4-yl)amino)-4'-methoxy-[1,1'- biphenyl]-3-yl)-2-oxo-4-phenyloxazoli din-3-carboxamide | ¹H NMR (400MHz, TFA salt, Methanol-*d*₄) δ 10.11 (s, 1H), 8.54 (d, *J* = 2.8 Hz, 1H), 7.77-7.64 (m, 4H), 7.42-7.23 (m, 10H), 6.42-6.28 (m, 2H), 5.81-5.78 (m, 1H), 5.57-5.53 (m, 1H), 5.25-5.24 (m, 1H), 4.63-4.59 (m, 1H), 4.44-4.42 (m, 1H), 4.30-4.27 (m, 1H), 4.14-4.10 (s, 4H), 3.89 (s, 3H); MS (ESI): m/z 687 [M+1] ⁺ | 45 | 6.68 100 (a) |

### Example 238. Preparation of 1-(4-((7-ethoxy-4-((5-(furan-2-yl)-2-methoxyphenyl)amino)quinazolin-6-yl)oxy)piperidin-1-yl)prop-2-en-1-one

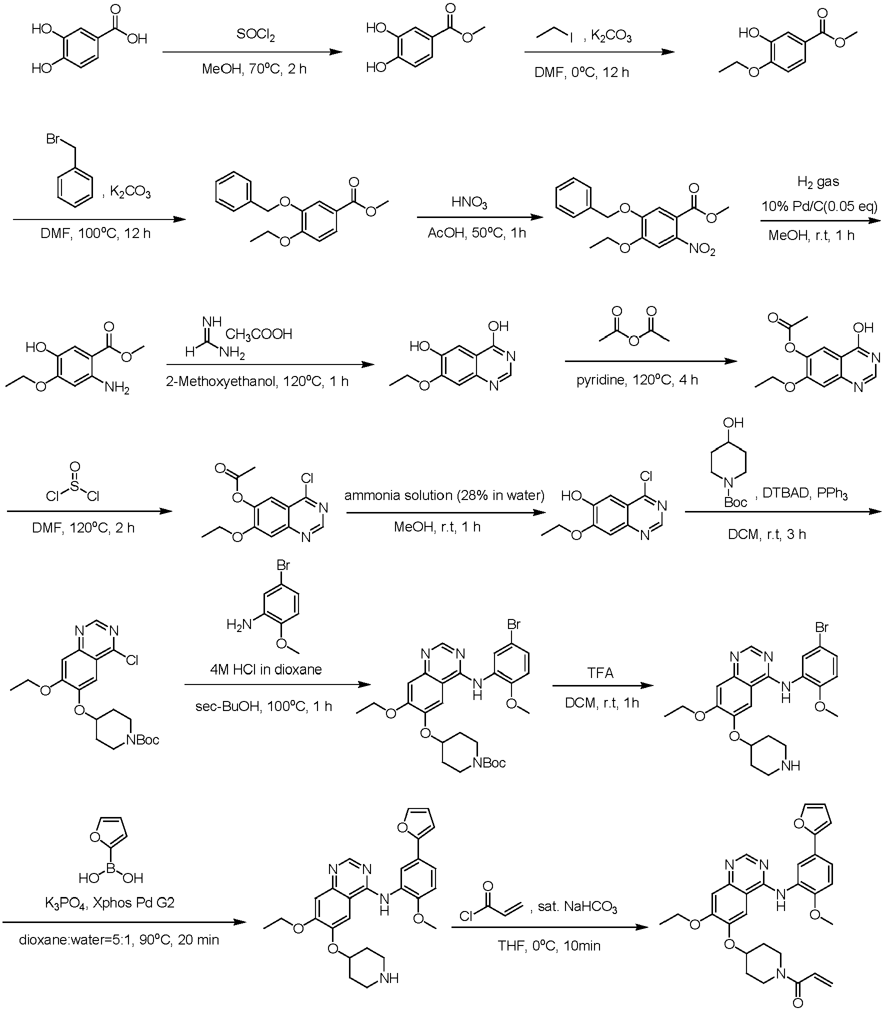

### [Step-1] Preparation of methyl 3,4-dihydroxybenzoate

3,4-Dihydroxybenzoic acid (1.0 equiv.) and thionyl chloride (1.0 equiv.) were added to methanol (0.5 M). The mixture was heated at 70 °C for 2 hours and then concentrated. The residue was diluted with ethyl acetate, and the residue was washed with sat. NaHCO₃ solution and brine. The collected organic layer was concentrated by removing remaining water using MgSO₄. The target compound obtained as a white solid was used in the next reaction without further purification (yield: 98%).

### [Step-2] Preparation of methyl 4-ethoxy-3-hydroxybenzoate

Methyl 3,4-dihydroxybenzoate (1.0 equiv.) obtained in Step-1 above and K₂CO₃ (1.0 equiv.) were added to DMF (0.2 M). The mixture was stirred at 0 °C for 20 minutes, and iodoethane (4.0 equiv.) was added dropwise. After the addition was completed, the mixture was stirred at 0 °C for an additional 12 hours. The desired product was confirmed through LC-MS. The mixture was passed through a celite filter and DMF was removed under reduced pressure. The residue was redissolved in ethyl acetate and washed with 1M HCl. The collected organic layer was concentrated by removing remaining water using MgSO₄. The target compound obtained as a white solid was used in the next reaction without further purification (yield: 37%, MS (ESI): m/z 197 [M+1]⁺).

### [Step-3] Preparation of methyl 3-(benzyloxy)-4-ethoxybenzoate

Methyl 4-ethoxy-3-hydroxybenzoate obtained in Step-2 above, K₂CO₃ (1.5 equiv.) and benzyl bromide (1.1 equiv.) were added to DMF (0.2 M). The mixture was heated at 100 °C for 12 hours. The mixture was cooled to room temperature, water was added, and organic materials were extracted with ethyl acetate. The collected organic layer was concentrated by removing remaining water using MgSO₄. The target compound obtained as a white solid was used in the next reaction without further purification (yield: 91%, MS (ESI): m/z 287 [M+1]⁺).

### [Step-4] Preparation of methyl 5-(benzyloxy)-4-ethoxy-2-nitrobenzoate

Methyl 3-(benzyloxy)-4-ethoxybenzoate (1.0 equiv.) obtained in Step-3 above was dissolved in a minimal amount of acetic acid. To this solution, concentrated nitric acid (2.9 equiv., 70%) was added. After stirring the mixture at 50 °C for 1 hour, ice water was poured to form a solid, and the resulting solid was washed with water and vacuum dried to obtain the target compound as a yellow solid (yield: 92%) .

### [Step-5] Preparation of methyl 2-amino-4-ethoxy-5-hydroxybenzoate

Methyl 5-(benzyloxy)-4-ethoxy-2-nitrobenzoate (1.0 equiv.) obtained in Step-4 above was dissolved in methanol (0.1 M), and then Pd/C (10% purity, 0.05 equiv.) was added. The mixture was stirred at room temperature for 1 hour under hydrogen. The mixture was filtered through a celite filter, then washed with methanol, and the filtrate was concentrated. The target compound obtained as a white solid was used in the next reaction without further purification (yield: 100%, MS (ESI) : m/z 212 [M+1]⁺).

### [Step-6] Preparation of 7-ethoxyquinazolin-4,6-diol

Methyl 2-amino-4-ethoxy-5-hydroxybenzoate (1.0 equiv.) obtained in Step-5 above and formamidine acetate (2.0 equiv.) were dissolved in 2-methoxyethanol (0.1 M), and the mixture was stirred at 120 °C for 1 hour. The solvent was concentrated, washed with water, and dried. The target compound obtained as a light brown solid was used in the next reaction without further purification (yield: 77%, MS (ESI): m/z 207 [M+1]⁺).

### [Step-7] Preparation of 7-ethoxy-4-hydroxyquinazolin-6-ylacetate

Pyridine (2.5 equiv.) was added to 7-ethoxyquinazolin-4,6-diol (1.0 equiv.) obtained in Step-6 above and acetic anhydride (1.1 equiv.), and the mixture was heated at 120 °C for 4 hours. Ice water was added to the reaction mixture, followed by filtration to obtain a solid, and the obtained solid was vacuum dried. The target compound obtained as a light brown solid was used in the next reaction without further purification (yield: 91%, MS (ESI): m/z 249 [M+1]⁺).

### [Step-8] Preparation of 4-chloro-7-ethoxyquinazolin-6-ylacetate

7-Ethoxy-4-hydroxyquinazolin-6-ylacetate (1.0 equiv.) obtained in Step-7 above and thionyl chloride (20.0 equiv.) were dissolved in DMF (0.3 M), and heated at 120 °C for 2 hours. Excess thionyl chloride was removed under reduced pressure, and the reaction mixture was purified using MPLC (DCM:MeOH) to obtain the target compound as a white solid (yield: 47%, MS (ESI) : m/z 267 [M+1]⁺).

### [Step-9] Preparation of 4-chloro-7-ethoxyquinazolin-6-ol

4-Chloro-7-ethoxyquinazolin-6-ylacetate (1.0 equiv.) obtained in Step-8 above and ammonia solution (65 equiv., 28% in water) were dissolved in MeOH (0.1 M), and stirred at room temperature for 1 hour. The reaction mixture was concentrated, then ethyl ether was added to obtain a solid, and the obtained solid was filtered. The obtained solid was vacuum dried at 60 °C to obtain the target compound as a white solid (yield: 52%, MS (ESI): m/z 225 [M+1]⁺).

### [Step-10] Preparation of tert-butyl 4-((4-chloro-7-ethoxyquinazolin-6-yl)oxy)piperidine-1-carboxylate

4-Chloro-7-ethoxyquinazolin-6-ol (1.0 equiv.) obtained in Step-9 above, tert-butyl 4-hydroxypiperidine-1-carboxylate (2.0 equiv.) and triphenylphosphine (1.5 equiv.) were dissolved in DCM (0.1 M). Then, DTBAD (1.5 equiv.) was added at 0 °C, followed by stirring at room temperature for 3 hours. The reaction mixture was concentrated and purified by MPLC to obtain the target compound as a white solid (yield: 73%, MS (ESI) : m/z 408 [M+1]⁺).

### [Step-11] Preparation of tert-butyl 4-((4-((5-bromo-2-methoxyphenyl)amino)-7-ethoxyquinazolin-6-yl)oxy)piperidine-1-carboxylate

tert-Butyl 4-((4-chloro-7-ethoxyquinazolin-6-yl)oxy)piperidine-1-carboxylate (1.0 equiv.) obtained in Step-10 above and 5-bromo-2-methoxyaniline (1.2 equiv.) were dissolved in sec-BuOH (0.1 M), then 4M HCl (0.2 equiv.) dissolved in dioxane was added and stirred at 100 °C for 1 hour. Ethyl ether was added to the mixture to produce a solid, and the obtained solid was filtered and washed with ethyl ether. The target compound obtained as a white solid was used in the next reaction without further purification (yield: 94%, MS (ESI) : m/z 574 [M+1]⁺).

### [Step-12] Preparation of N-(5-bromo-2-methoxyphenyl)-7-ethoxy-6-(piperidin-4-yloxy)quinazolin-4-amine

tert-Butyl 4-((4-((5-bromo-2-methoxyphenyl)amino)-7-ethoxyquinazolin-6-yl)oxy)piperidine-1-carboxylate (1.0 equiv.) obtained in Step-11 above was dissolved in DCM (0.1 M), and then TFA (10 equiv.) was added and stirred at room temperature for 1 hour. The reaction mixture was concentrated in vacuo, and purified using MPLC (DCM:MeOH) to obtain the target compound as a yellow solid (yield: 94%, MS (ESI): m/z 474 [M+1]⁺).

### [Step-13] Preparation of 7-ethoxy-N-(5-(furan-2-yl)-2-methoxyphenyl)-6-(piperidin-4-yloxy)quinazolin-4-amine

N-(5-Bromo-2-methoxyphenyl)-7-ethoxy-6-(piperidin-4-yloxy)quinazolin-4-amine (1.0 equiv.) obtained in Step-12 above, furan-2-ylboronic acid (2.2 equiv.) and K₃PO₄ (2.0 equiv.) were dissolved in dioxane (0.1 M) and water (0.5 M), then the mixture was degassed with nitro gas, Xphos Pd G2 (0.13 equiv.) was added at 80 °C, and the mixture was stirred at 90 °C for 20 minutes. The mixture was filtered through a celite filter, and the filtrate was concentrated to obtain the target compound as a brown solid, which was used in the next reaction without further purification (MS (ESI): m/z 461 [M+1]⁺).

### [Step-14] Preparation of 1-(4-((7-ethoxy-4-((5-(furan-2-yl)-2-methoxyphenyl)amino)quinazolin-6-yl)oxy)piperidin-1-yl)prop-2-en-1-one

7-Ethoxy-N-(5-(furan-2-yl)-2-methoxyphenyl)-6-(piperidin-4-yloxy)quinazolin-4-amine (1.0 equiv.) obtained in Step-13 above and saturated NaHCO₃ aqueous solution (5.0 equiv.) were dissolved in THF (0.1 M), and then acryloyl chloride (1.0 equiv.) was added at 0 °C and stirred for 10 minutes. The reaction mixture was concentrated and purified using prep-HPLC to obtain the target compound as a yellow solid (yield: 22%, MS (ESI) : m/z 515 [M+1]⁺).

### Preparation of Compound of Example 239

A compound of Example 239 according to the present invention was prepared in a similar manner to Example 238 above. Chemical structures, compound names, ¹H NMR, MS, HPLC data and yields of respective Examples are summarized in Table 4 below.

**[Table 4]**

| Exa mpl e No. | Structure | Compound Name | ¹H NMR; MS | Yie ld (%) | HPLC r.t.(min ) Purity(% ) (method) |
|---|---|---|---|---|---|
| 238 | 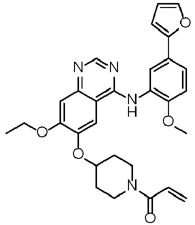 | 1-(4-((7-ethoxy-4-((5-(furan-2-yl)-2-methoxyphenyl ) amino) quinaz olin-6-yl)oxy)piperi din-1-yl)prop-2-en-1-one | ¹H NMR (400MHz, TFA salt, Methanol-*d*₄) δ 8.60 (s, 1H), 8.09 (s, 1H), 7.84 (s, 3H), 7.77-7.75 (m, 1H), 7.55 (d, *J* = 1.2 Hz, 1H), 7.26 (s, 1H), 7.24 (s, 1H), 6.88-6.81 (m, 1H), 6.71 (d, *J* = 2.8 Hz, 1H), 6.53-6.52 (m, 1H), 6.27-6.22 (m, 1H), 5.81-5.77 (m, 1H), 4.36-4.31 (m, 2H), 3.95-3.93 (m, 2H), 3.90 (s, 3H), 3.74-3.72 (m, 2H), 2.19-2.11 (m, 2H), 2.02-1.98 (m, 2H), 1. 59-1. 55 (m, 3H); MS (ESI): m/z 515 [M+1]⁺ | 22 | 6.24 100 (a) |
| 239 | 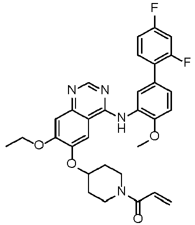 | 1-(4-((4-((2',4'-difluoro-4-methoxy-[1,1'- biphenyl]-3-yl)amino)-7-ethoxyquinazo lin-6-yl)oxy)piperi din-1-yl)prop-2-en-1-one | ¹H NMR (400 MHz, TFA salt, Methanol-*d*₄) δ8.60 (s, 1H), 8.07 (s, 1H), 7.70 (s, 1H), 7.69-7.54 (m, 2H), 7.32 (d, *J* = 8.8 Hz, 1H), 7.23 (s, 1H), 7.10-7.07 (m, 1H), 6.88-6.81 (m, 2H), 6.27-6.22 (m, 1H), 5.80-5.77 (m, 1H), 5.01-5.00 (m, 1H), 4.36-4.31 (m, 2H), 4.00-3.95 (m, 4H), 3.79-3.77 (m, 2H), 2.12-2.10 (m, 2H), 1.99-1.97 (m, 2H), 1.56-1.55 (m, 5H); MS (ESI): m/z 561 [M+H]⁺ | 13 | 10.91 100 (b) |

### Preparation Example 2. Preparation of 2-((1-methylpyrrolidin-3-yl)oxy)-5-(thiophen-2-yl)aniline

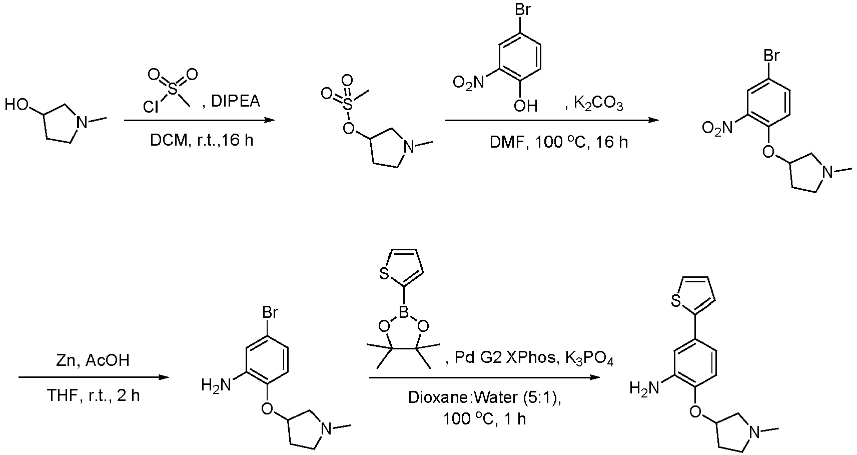

### [Step-1] Preparation of 1-methylpyrrolidin-3-ylmethanesulfonate

In DCM (1.0 M), 1-methylpyrrolidin-3-ol (1.0 equiv.) was dissolved at 0 °C, then DIPEA (1.2 equiv.) and methylsulfonyl chloride (1.1 equiv.) were added. The reaction mixture was stirred at 0 °C for 1 hour and then stirred at room temperature for 16 hours. After confirming the reaction through TLC, water was added to the reaction mixture, and organic materials were extracted with DCM. The collected organic layer was concentrated by removing remaining water using Na₂SO₄. The target compound obtained as a brown solid was used in the next reaction without further purification (yield: 80%).

### [Step-2] Preparation of 3-(4-bromo-2-nitrophenoxy)-1-methylpyrrolidine

In DMF (0.6 M), 1-methylpyrrolidin-3-ylmethanesulfonate (1.0 equiv.) obtained in Step-1 above, 4-bromo-2-nitrophenol (1.5 equiv.) and K₂CO₃ (2.0 equiv.) were dissolved, and stirred at 100 °C for 16 hours. Water was added to the reaction mixture, and organic materials were extracted with ethyl acetate. The collected organic layer was concentrated by removing remaining water using Na₂SO₄. The reaction mixture was purified by MPLC (hexane:EA) to obtain the target compound as a brown solid (yield: 80%, MS (ESI) : m/z 301 [M+1]⁺).

### [Step-3] Preparation of 5-bromo-2-((1-methylpyrrolidin-3-yl)oxy)aniline

In THF (0.3 M), 3-(4-bromo-2-nitrophenoxy)-1-methylpyrrolidine (1.0 equiv.) obtained in Step-2 was dissolved at 0 °C, and then acetic acid (20 equiv.) and zinc (10 equiv.) were added. The reaction mixture was stirred at room temperature for 2 hours. The mixture was diluted with methanol, filtered through a celite filter, and concentrated. The concentrate was neutralized by adding a saturated NaHCO₃ aqueous solution at 0 °C, and organic materials were then extracted with DCM. The collected organic layer was concentrated by removing remaining water using Na₂SO₄. The reaction mixture was purified by MPLC (DCM:MeOH) to obtain the target compound as a yellow solid (yield: 77%, MS (ESI): m/z 271 [M+1]⁺).

### [Step-4] Preparation of 2-((1-methylpyrrolidin-3-yl)oxy)-5-(thiophen-2-yl)aniline

In dioxane (0.1 M) and water (0.5 M), 5-bromo-2-((1-methylpyrrolidin-3-yl)oxy)aniline (1.0 equiv.) obtained in Step-3 above, 4,4,5,5-tetramethyl-2-(thiophen-2-yl)-1,3,2-dioxaborolane (2.0 equiv.) and K₃PO₄ (2.0 equiv.) were dissolved. The reaction mixture was degassed using nitro gas, and Xphos Pd G2 (0.13 equiv.) was added to the mixture at 80 °C, followed by stirring at 100 °C for 1 hour. The mixture was filtered through a celite filter and the filtrate was concentrated. The reaction mixture was purified by MPLC (DCM:MeOH) to obtain the target compound as a brown solid (yield: 64%, MS (ESI) : m/z 275 [M+1]⁺).

### Example 240. Preparation of 1-(3-((7-methoxy-4-((2-((1-methylpyrrolidin-3-yl)oxy)-5-(thiophen-2-yl)phenyl)amino)quinazolin-6-yl)oxy)azetidin-1-yl)prop-2-en-1-one

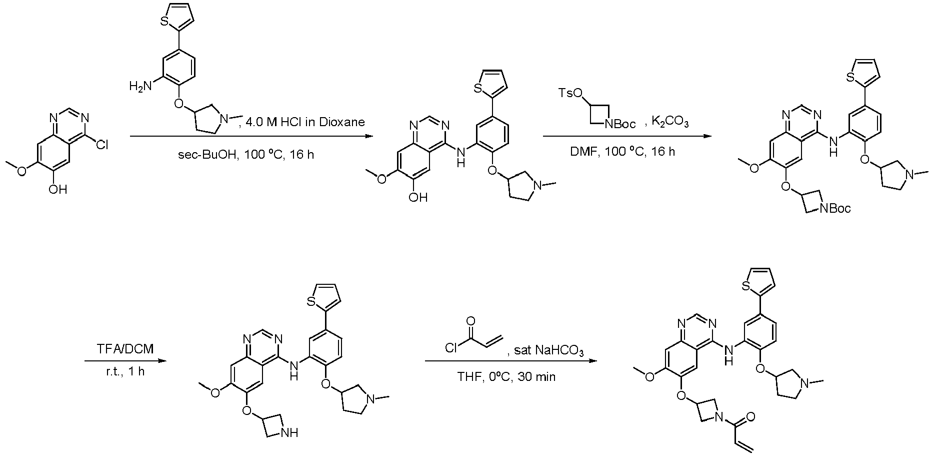

### [Step-1] Preparation of 7-methoxy-4-((2-((1-methylpyrrolidin-3-yl)oxy)-5-(thiophen-2-yl)phenyl)amino)quinazolin-6-ol

In sec-BuOH (0.1 M), 2-((1-methylpyrrolidin-3-yl)oxy)-5-(thiophen-2-yl)aniline (1.2 equiv.) obtained in Preparation Example 2 and 4-chloro-7-methoxyquinazolin-6-ol (1.0 equiv.) were dissolved, and 4M HCl (0.2 equiv.) dissolved in 1,4-dioxane was added, followed by stirring at 100 °C for 16 hours. Ethyl ether was added to the mixture, and the resulting solid was filtered and washed with ethyl ether to obtain the target compound as a yellow solid (yield: 84%, MS (ESI): m/z 449 [M+1]⁺).

### [Step-2] Preparation of tert-butyl 3-((7-methoxy-4-((2-((1-methylpyrrolidin-3-yl)oxy)-5-(thiophen-2-yl)phenyl)amino)quinazolin-6-yl)oxy)azetidin-1-carboxylate

In DMF (0.1 M), 7-methoxy-4-((2-((1-methylpyrrolidin-3-yl)oxy)-5-(thiophen-2-yl)phenyl)amino)quinazolin-6-ol (1.0 equiv.) obtained in Step-1 above, tert-butyl 3-(tosyloxy)azetidin-1-carboxylate (1.2 equiv.), and K₂CO₃ (2.0 equiv.) were dissolved, and the mixture was stirred at 100 °C for 16 hours. The solution was cooled to room temperature, and organic materials were extracted by adding DCM and brine. The collected organic layer was concentrated by removing remaining water using Na₂SO₄. The reaction mixture was purified by MPLC (DCM:MeOH) to obtain the target compound as a yellow solid (yield: 33%, MS (ESI): m/z 604 [M+1]⁺).

### [Step-3] Preparation of 6-(azetidin-3-yloxy)-7-methoxy-N-(2-((1-methylpyrrolidin-3-yl)oxy)-5-(thiophen-2-yl)phenyl)quinazolin-4-amine

In DCM (0.2 M), tert-butyl 3-((7-methoxy-4-((2-((1-methylpyrrolidin-3-yl)oxy)-5-(thiophen-2-yl)phenyl)amino)quinazolin-6-yl)oxy)azetidin-1-carboxylate (1.0 equiv.) obtained in Step-2 above was dissolved, and TFA (50.0 equiv.) was then added and stirred at room temperature for 1 hour. The reaction mixture was concentrated and purified by MPLC (DCM:MeOH) to obtain the target compound as a yellow solid (yield: 92%, MS (ESI) : m/z 504 [M+1]⁺).

### [Step-4] Preparation of 1-(3-((7-methoxy-4-((2-((1-methylpyrrolidin-3-yl)oxy)-5-(thiophen-2-yl)phenyl)amino)quinazolin-6-yl)oxy)azetidin-1-yl)prop-2-en-1-one

In THF (0.1 M), 6-(azetidin-3-yloxy)-7-methoxy-N-(2-((1-methylpyrrolidin-3-yl)oxy)-5-(thiophen-2-yl)phenyl)quinazolin-4-amine (1.0 equiv.) obtained in Step-3 above and saturated NaHCO₃ aqueous solution (5.0 equiv.) were dissolved, and then acryloyl chloride (1.0 equiv.) was added at 0 °C and stirred for 30 minutes. The reaction mixture was concentrated and purified using prep-HPLC to obtain the target compound as a yellow solid (yield: 30%, MS (ESI) : m/z 558 [M+1]⁺).

### Preparation of Compounds of Examples 241 to 261

Compounds of Examples 241 to 271 according to the present invention were prepared in a similar manner to Example 240 above. Chemical structures, compound names, ¹H NMR, MS, HPLC data and yields of respective Examples are summarized in Table 5 below.

**[Table 5]**

| Exa mpl e No. | Structure | Compound Name | ¹H NMR; MS | Yie ld (%) | HPLC r.t.(min ) Purity(% ) (method) |
|---|---|---|---|---|---|
| 240 | 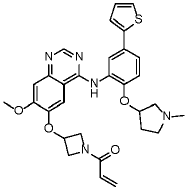 | 1-(3-((7-methoxy-4-((2-((1-methylpyrroli din-3-yl)oxy)-5-(thiophen-2-yl)phenyl)ami no)quinazolin -6-yl)oxy)azetidin-1-yl)prop-2-en-1-one | ¹H NMR (400MHz, TFA salt, Methanol-*d*₄) δ 8.68 (s, 1H), 7.73-7.71 (m, 3H), 7.41-7.38 (m, 2H), 7.33 (s, 1H), 7.24-7.22 (m, 1H), 7.12-7.10 (m, 1H), 6.42-6.38 (m, 1H), 6.34-6.29 (m, 1H), 5.83-5.80 (m, 1H), 5.32-5.30 (m, 2H), 4.87 (s, 3H), 4.70-4.61 (m, 1H), 4.58-4.48 (m, 1H), 4.15-4.14 (m, 4H), 4.13 (s, 3H), 3.38-3.32 (m, 2H), 2.96-2.88 (m, 2H); MS (ESI): m/z 558 [M+1]⁺ | 29 | 4.61 100 (a) |
| 241 | 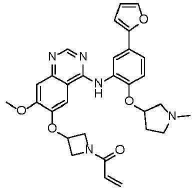 | 1-(3-((4-((5-(furan-2-yl)-2-((1-methylpyrroli din-3-yl)oxy)phenyl )amino)-7-methoxyquinaz olin-6-yl)oxy)azetid in-1-yl)prop-2en-1-one | MS (ESI): m/z 542 [M+1] ⁺ | | |
| 242 | 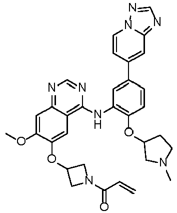 | 1-(3-((4-((5-([1,2,4]triaz olo[1,5-a]pyridin-7-yl)-2-((1-methylpyrroli din-3-yl)oxy))pheny l)amino)-7-methoxyquinaz olin-6-yl)oxy)azetid in-1-yl)prop-2-en-1-one | ¹H NMR (400MHz, FA salt, Methanol-*d*₄) δ 8.87 (d, *J* = 7.2 Hz, 1H), 8.46 (s, 1H), 8.42 (s, 1H), 8.32 (s, 1H), 8.23 (s, 1H), 8.03 (s, 1H), 7.83-7.80 (m, 1H), 7.61-7.57 (m, 2H), 7.31-7.27 (m, 2H), 6.42-6.30 (m, 2H), 5.83-5.80 (m, 1H), 5.32-5.29 (m, 2H), 4.99-4.91 (m, 1H), 4.72-4.69 (m, 2H), 4.52-4.50 (m, 2H), 4.22-4.19 (m, 1H), 4.06 (s, 3H), 3.50-3.48 (m, 1H), 3.22-3.18 (m, 1H), 2.75 (s, 3H), 2.61-2.57 (m, 1H), 2.33-2.31 (m, 1H); MS (ESI) : m/z 593 [M+1]⁺ | 9 | 97* |
| 243 | 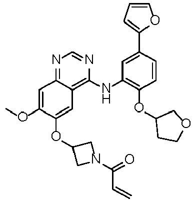 | 1-(3-((4-((5-(furan-2-yl)-2-((tetrahydrof uran-3-yl)oxy)phenyl )amino)-7-methoxyquinaz olin-6-yl)oxy)azetid in-1-yl)prop-2-en-1-one | MS (ESI): m/z 557 [M+1] ⁺ | | |
| 244 | 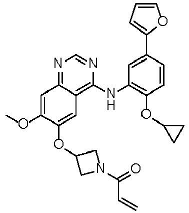 | 1-(3-((4-((2-cyclopropoxy-5-(furan-2-yl)phenyl)ami no)-7-methoxyquinaz olin-6-yl)oxy)azetid in-1-yl)prop-2-en-1-one | MS (ESI): m/z 499 [M+1] ⁺ | | |
| 245 | 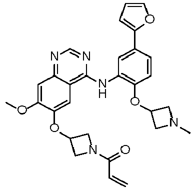 | 1-(3-((4-((5-(furan-2-yl)-2-((1-methylazetidi n-3-yl)oxy)phenyl )amino)-7-methoxyquinaz olin-6-yl)oxy)azetid in-1-yl)prop-2-en-1-one | MS (ESI): m/z 528 [M+1]⁺ | | |
| 246 | 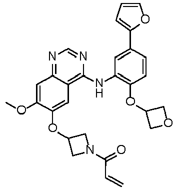 | 1-(3-((4-((5-(furan-2-yl)-2-(oxetan-3-yloxy)phenyl) amino)-7-methoxyquinaz olin-6-yl)oxy)azetid in-1-yl)prop-2-en-1-one | MS (ESI): m/z 515 [M+1] ⁺ | | |
| 247 | 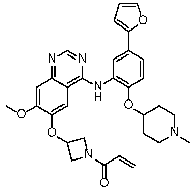 | 1-(3-((4-((5-(furan-2-yl)-2-((1-methylpiperid in-4-yl)oxy)phenyl )amino)-7-methoxyquinaz olin-6-yl)oxy)azetid in-1-yl)prop-2-en-1-one | MS (ESI): m/z 556 [M+1]⁺ | | |
| 248 | 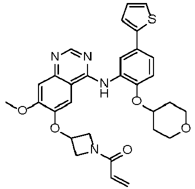 | 1-(3-((7-methoxy-4-((2-((tetrahydro-2H-pyran-4-yl)oxy)-5-(thiophen-2-yl)phenyl)ami no)quinazolin -6-yl)oxy)azetid in-1-yl)prop-2-en-1-one | ¹H NMR (400MHz, free base, Methanol-*d*₄) δ 8.38 (s, 1H), 7.99 (d, *J* = 2.4 Hz, 1H), 7.55-7.53 (m, 1H), 7.41 (s, 1H), 7.34-7.31 (m, 2H), 7.24-7.13 (m, 2H), 7.09-7.06 (m, 1H), 6.42-6.31 (m, 2H), 5.79-5.76 (m, 1H), 5.23-5.18 (m, 1H), 4.82-4.81 (m, 1H), 4.66-4.52 (m, 2H), 4.44-4.41 (m, 1H), 4.18-4.12 (m, 1H), 3.72-3.68 (m, 2H), 3.53-3.48 (m, 2H), 2.00-1.97 (m, 2H), 1. 67-1. 64 (m, 1H); MS (ESI): m/z 559 [M+1] ⁺ | 64 | 5.78 100 (a) |
| 249 | 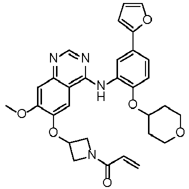 | 1-(3-((4-((5-(furan-2-yl)-2-((tetrahydro-2H-pyran-4-yl)oxy)phenyl )amino)-7-methoxyquinaz olin-6-yl)oxy)azetid in-1-yl)prop-2-en-1-one | ¹H NMR (400MHz, free base, Methanol-*d*₄) δ 8.38 (s, 1H), 8.01 (s, 1H), 7.64-7.61 (m, 1H), 7.54 (s, 1H), 7.26 (s, 1H), 7.22 (d, *J* = 8.4 Hz, 1H), 6.70 (d, *J* = 3.2 Hz, 1H), 6.52-6.51 (m, 1H), 6.41-6.32 (m, 1H), 6.31-6.28 (m, 1H), 5.81-5.78 (m, 1H), 5.28-5.26 (m, 1H), 4.86-4.84 (m, 1H), 4.78-4.75 (m, 1H), 4.74-4.69 (m, 1H), 4.45-4.40 (m, 1H), 4.26-4.21 (m, 1H), 4.06 (s, 3H), 3.71-3.69 (m, 2H), 3.54-3.50 (m, 2H), 2.01-1. 98 (m, 2H), 1.77-1.74 (m, 2H); MS (ESI): m/z 543 [M+1]⁺ | 45 | 5.68 100 (a) |
| 250 | 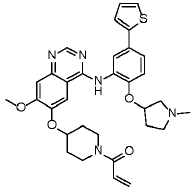 | 1-(4-((7-methoxy-4-((2-((1-methylpyrroli din-3-yl)oxy)-5-(thiophen-2-yl)phenyl)ami no)quinazolin -6-yl)oxy)piperi din-1-yl)prop-2-en-1-one | ¹H NMR (400MHz, free base, Methanol-*d*₄) δ 8.43 (s, 1H), 8.22-8.21 (m, 1H), 7.93 (s, 1H), 7.50-7.48 (m, 1H), 7.35-7.33 (m, 1H), 7.24 (s, 1H), 7.10-7.04 (m, 2H), 6.88-6.81 (m, 1H), 6.26-6.22 (m, 1H), 5.80-5.76 (m, 1H), 4.04 (s, 3H), 4.00-3.92 (m, 2H), 3.76-3.68 (m, 2H), 3.61 (s, 3H), 3.00-2.98 (m, 2H), 2.43-2.40 (m, 1H), 2.14-1. 87 (m, 8H); MS (ESI): m/z 586 [M+1]⁺ | 22 | 4.87 100 (a) |
| 251 | 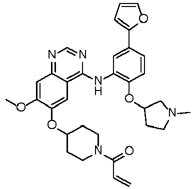 | 1-(4-((4-((5-(furan-2-yl)-2-((1-methylpyrroli din-3-yl)oxy)phenyl )amino)-7-methoxyquinaz olin-6-yl)oxy)piperi din-1-yl)prop-2-en-1-one | MS (ESI): m/z 570 [M+1] ⁺ | | |
| 252 | 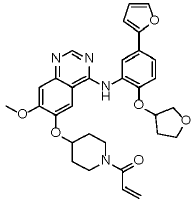 | 1-(4-((4-((5-(furan-2-yl)-2-((tetrahydrof uran-3-yl)oxy)phenyl )amino)-7-methoxyquinaz olin-6-yl)oxy)piperi din-1-yl)prop-2-en-1-one | MS (ESI): m/z 557 [M+1] ⁺ | | |
| 253 | 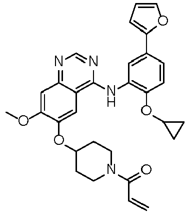 | 1-(4-((4-((2-cyclopropoxy-5-(furan-2-yl)phenyl)ami no)-7-methoxyquinaz olin-6-yl)oxy)piperi din-1-yl)prop-2-en-1-one | ¹H NMR (400MHz, FA salt, Methanol-*d*₄) δ 8.34 (s, 1H), 7.93 (d, *J* = 2 Hz ,1H), 7.76 (s, 1H), 7.63-7.60 (m, 1H), 7.51 (d, *J* = 1.6 Hz, 1H), 7.45 (d, *J* = 8.8 Hz, 1H), 7.19 (s, 1H), 6.84-6.77 (m, 1H), 6.65 (d, *J* = 3.6 Hz, 1H), 6.49-6.48 (m, 1H), 6.23-6.19 (m, 1H), 5.77-5.74 (m, 1H), 4.83-4.79 (m, 1H), 4.01 (s, 3H), 3.96-3.86 (m, 3H), 3.68-3.61 (m, 2H), 2.07-2.06 (m, 2H), 1. 90-1. 89 (m, 2H), 0.81-0.76 (m, 2H), 0.67-0.63 (m, 2H); MS (ESI): m/z=527 [M+1] ⁺ | 31 | 6.54 100 (a) |
| 254 | 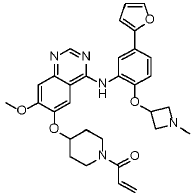 | 1-(4-((4-((5-(furan-2-yl)-2-((1-methylazetidi n-3-yl)oxy)phenyl )amino)-7-methoxyquinaz olin-6-yl)oxy)piperi din-1-yl)prop-2-en-1-one | ¹H NMR (400 MHz, free base, Methanol-*d*₄) δ 8.46 (s, 1H), 7.65 (d, *J* = 5.2Hz, 1H), 7.47 (d, *J* = 1.6 Hz, 1H), 7.11 (s, 1H), 6.85 (s, 1H), 6.77-6.70 (m, 2H), 6.50 (d, *J* = 3.2 Hz, 1H), 6.46-6.45 (m, 1H), 6.42-6.36 (m, 1H), 6.20 (s, 1H), 6.16 (s, 1H), 5.74 (d, *J* = 10.4 Hz, 1H), 4.71-4.70 (m, 1H), 4.65-4.63 (m, 1H), 4.32-4.30 (m, 1H), 3.91 (s, 3H), 3.82-3.79 (m, 3H), 3.62-3.61 (m, 1H), 3.50-3.44 (m, 3H), 3.22-3.20 (m, 2H), 1.93-1.92 (m, 2H), 1.82-1.80 (m, 2H); MS (ESI): m/z 556 [M+H] ⁺ | 6 | 6.17 94 (a) |
| 255 | 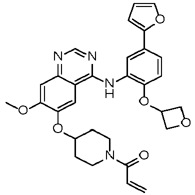 | 1-(4-((4-((5-(furan-2-yl) - 2- (oxetan-3-yloxy) phenyl) amino)-7-methoxyquinaz olin-6-yl)oxy)piperi din-1-yl)prop-2-en-1-one | ¹H NMR (400 MHz, TFA salt, Methanol-*d*₄) δ 8.67 (s, 1H), 8.07 (s, 1H), 7.87 (s, 1H), 7.73 (d, *J* = 8.8 Hz, 1H), 7.56 (s, 1H), 7.29 (s, 1H), 6.88-6.81 (m, 2H), 6.74 (d, *J* = 3.2 Hz, 1H), 6.54 (s, 1H), 6.27-6.23 (m, 1H), 5.81-5.78 (m, 1H), 5.43-5.40 (m, 1H), 5.04-5.00 (m, 2H), 4.60-4.58 (m, 2H), 4.12 (s, 3H), 3.97-3.95 (m, 2H), 3.69-3.67 (m, 2H), 3.37-3.35 (m, 1H), 2.14-2.12 (m, 2H), 1.95-1.93 (m, 2H); MS (ESI): m/z 543 [M+H]⁺ | 21 | 5.59 100 (a) |
| 256 | 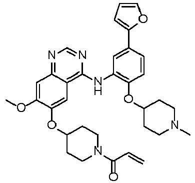 | 1-(4-((4-((5-(furan-2-yl)-2-((1-methylpiperid in-4-yl)oxy)phenyl )amino)-7-methoxyquinaz olin-6-yl)oxy)piperi din-1-yl)prop-2-en-1-one | MS (ESI): m/z=584 [M+1]⁺ | | |
| 257 | 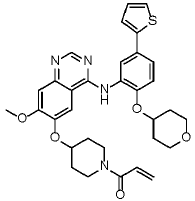 | 1-(4-((7-methoxy-4-((2-((tetrahydro-2H-pyran-4-yl)oxy)-5-(thiophen-2-yl)phenyl)ami no)quinazolin -6-yl)oxy)piperi din-1-yl)prop-2-en-1-one | ¹H NMR (400 MHz, TFA salt, Methanol-*d*₄) δ 8.62 (s, 1H), 7.99 (s, 1H), 7.78 (d, *J =* 2.4 Hz, 1H), 7.67-7.64 (m, 1H), 7.36-7.32 (m, 2H), 7.27-7.25 (m, 2H), 7.09-7.07 (m, 1H), 6.85-6.78 (m, 1H), 6.24-6.19 (m, 1H), 5.78-5.75 (m, 1H), 4.73-4.67 (m, 1H), 4.09 (s, 3H), 3.97-3.91 (m, 2H), 3.75-3.66 (m, 4H), 3.59-3.47 (m, 2H), 2.15-2.10 (m, 2H), 2.02-1.91 (M, 4H), 1.65-1.57 (m, 2H); MS (ESI): m/z=587 [M+1]⁺ | 36 | 6.17 99 (a) |
| 258 | 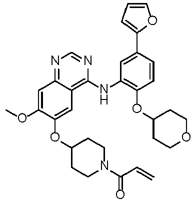 | 1-(4-((4-((5-(furan-2-yl)-2-((tetrahydro-2H-pyran-4-yl)oxy)phenyl )amino)-7-methoxyquinaz olin-6-yl)oxy)piperi din-1-yl)prop-2-en-1-one | ¹H NMR (400MHz, TFA salt, Methanol-*d*₄) δ 8.65 (s, 1H), 8.07 (s, 1H), 7.85-7.84 (m, 1H), 7.75-7.73 (m, 1H), 7.55-7.54 (m, 1H), 7.30-7.28 (m, 2H), 6.83-6.80 (m, 1H), 6.72-6.71 (m, 1H), 6.54-6.53 (m, 1H), 6.27-6.22 (m, 1H), 5.81-5.77 (m, 1H), 4.80-4.71 (m, 1H), 4.11 (s, 3H), 4.02-3.97 (m, 2H), 3.74-3.1 (m, 5H), 3.56-3.54 (m, 3H), 2.22-1.88 (m, 7H); MS (ESI): m/z 571 [M+1]⁺ | 3 | 5.94 100 (a) |
| 259 | 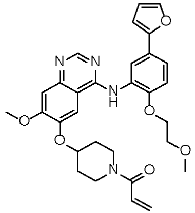 | 1-(4-((4-((5-(furan-2-yl)- 2-(2-methoxyethoxy )phenyl)amino )-7-methoxyquinaz olin-6-yl)oxy)piperi din-1-yl)prop-2-en-1-one | ¹H NMR (400 MHz, FA salt, Methanol-*d*₄) δ 8.43 (s, 1H), 8.26 (s, 1H), 7.82 (s, 1H), 7.59-7.54 (m, 2H), 7.24 (s, 1H), 7.20 (d, *J* = 8.4 Hz, 1H), 6.87-6.81 (m, 1H), 6.70 (d, *J* = 3.6 Hz, 1H), 6.53-6.51 (m, 1H), 6.26-6.21 (m, 1H), 5.79-5.76 (m, 1H), 5.01-5.00 (m, 1H), 4.28-4.25 (m, 2H), 4.04 (s, 3H), 3.97-3.95 (m, 2H), 3.72-3.70 (m, 4H), 3.32 (s, 3H), 2.12-2.09 (m, 2H), 1.99-1.97 (m, 2H); MS (ESI): m/z 545 [M+H]⁺ | 9 | 6.06 100 (a) |
| 260 | 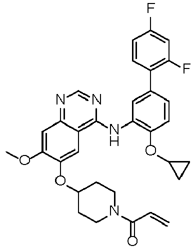 | 1-(4-((4-((4-cyclopropoxy-2',4'-difluoro-[1,1'-biphenyl]-3-yl)amino)-7-methoxyquinaz olin-6-yl)oxy)piperi din-1-yl)prop-2-en-1-one | ¹H NMR (400MHz, FA salt, DMSO-*d*₆) δ 9.11 (s, 1H), 8.29 (s, 1H), 7.87 (s, 1H), 7.68 (s, 1H), 7.63-7.56 (m, 1H), 7.50-7.42 (m, 2H), 7.38-7.32 (m, 1H), 7.21-7.16 (m, 2H), 6.88-6.82 (m, 1H), 6.14-6.09 (m, 1H), 5.70-5.67 (m, 1H), 4.80-4.76 (m, 1H), 3.97-3.93 (m, 7H), 2.04 (brs, 2H), 1.68 (brs, 2H), 0.79-0.74 (m, 2H), 0.60-0.56 (m, 2H); MS (ESI): m/z 573 [M+1]⁺ | 52 | 6.88 100 (a) |
| 261 | 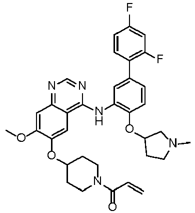 | 1-(4-((4-((2',4'-difluoro-4-((1-methylpyrroli din-3-yl)oxy)-[1,1'-biphenyl]-3-yl)amino)-7-methoxyquinaz olin-6-yl)oxy)piperi din-1-yl)prop-2-en-1-one | ¹H NMR (400 MHz, FA salt, Methanol-*d*₄) δ 8.56 (s, 1H), 7.81 (d, *J* = 8.0 Hz, 1H), 7.45-7.39 (m, 1H), 7.21 (S, 1H), 7.02-6.97 (m, 2H), 6.81-6.64 (m, 4H), 6.21 (d, *J* = 16.8 Hz, 1H), 5.74-5.72 (m, 1H), 4.52-4.51 (m ,1H), 4.29-4.28 (m, 1H), 4.18-4.11 (m, 4H), 3.86-3.85 (m, 2H), 3.71-3.67 (m, 5H), 2.39-2.35 (m, 3H), 2.06-2.05 (m, 3H), 1.87-1.85 (m, 3H); MS (ESI): m/z 616 [M+H]⁺ | 4 | 6.93, 98 (a) |

### Example 262. Preparation of 1-(4-((4-((5-(furan-2-yl)-2-methoxyphenyl)amino)-7-(2-methoxyethoxy)quinazolin-6-yl)amino)piperidin-1-yl)prop-2-en-1-one

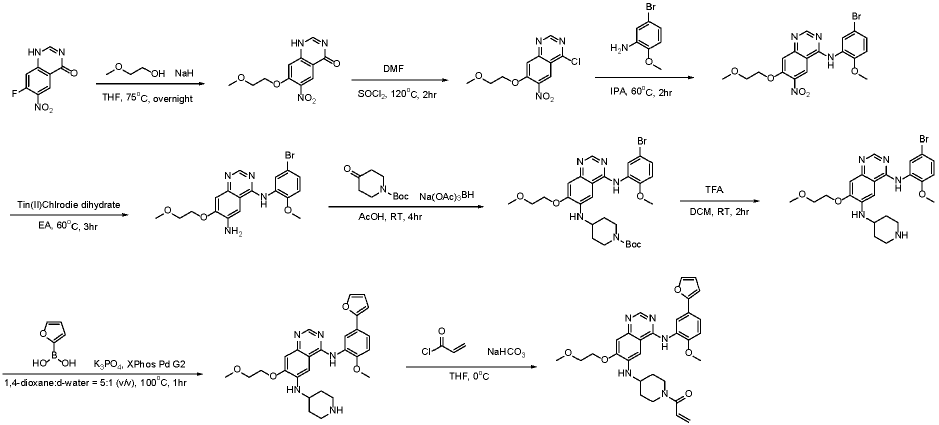

### [Step-1] Preparation of 7-(2-methoxyethoxy)-6-nitroquinazolin-4(1H)-one

In anhydrous THF (1.5 M), 2-methoxyethanol (1.0 equiv.) was dissolved, and cooled to 0 °C under nitrogen, then NaH (0.68 equiv.) was added, and the mixture was stirred at room temperature for 1 hour. To the reaction mixture, 7-fluoro-6-nitroquinazolin-4(1H)-one (0.3 equiv.) was added and stirred at 75 °C for 12 hours. The reaction mixture was adjusted to pH 7 with a saturated NaHCO₃ aqueous solution, and organic materials were extracted with chloroform. The collected organic layer was concentrated by removing remaining water using MgSO₄. The reaction mixture was purified by MPLC (DCM:MeOH) to obtain the target compound as a white solid (yield: 54%, MS (ESI): m/z 266 [M+1]⁺).

### [Step-2] Preparation of 4-chloro-7-(2-methoxyethoxy)-6-nitroquinazoline

To 7-(2-methoxyethoxy)-6-nitroquinazolin-4(1H)-one (1.0 equiv.) obtained in Step-1 above, SOCl₂ (0.4 M) and DMF (0.2 equiv.) were added and stirred at 120 °C for 2 hours. The reaction mixture was concentrated under reduced pressure to obtain the target compound as a yellow solid, which was used in the next reaction without further purification (yield: 70%, MS (ESI): m/z 284 [M+1]⁺).

### [Step-3] Preparation of N-(5-bromo-2-methoxyphenyl)-7-(2-methoxyethoxy)-6-nitroquinazolin-4-amine

In isopropyl alcohol (0.2 M), 4-chloro-7-(2-methoxyethoxy)-6-nitroquinazoline (1.0 equiv.) obtained in Step-2 above was dissolved, then 5-bromo-2-methoxyaniline (1.0 equiv.) was added and stirred at 60 °C for 2 hours. The reaction mixture was concentrated and purified by MPLC (DCM:MeOH) to obtain the target compound as a pale yellow solid (yield: 80%, MS (ESI): m/z 450 [M+1]⁺).

### [Step-4] Preparation of N⁴-(5-bromo-2-methoxyphenyl)-7-(2-methoxyethoxy)quinazolin-4,6-diamine

In EA (0.2 M), N-(5-bromo-2-methoxyphenyl)-7-(2-methoxyethoxy)-6-nitroquinazolin-4-amine (1.0 equiv.) obtained in Step-3 above was dissolved, and tin(II) chloride dihydrate (5.0 equiv.) was added and stirred at 60 °C for 3 hours. The reaction mixture was cooled to room temperature, then aqueous ammonia was added thereto, and the mixture was concentrated. The reaction mixture was purified by MPLC (DCM:MeOH) to obtain the target compound as a white solid (yield: 86%, MS (ESI): m/z 420 [M+1]⁺).

### [Step-5] Preparation of tert-butyl 4-((4-((5-bromo-2-methoxyphenyl)amino)-7-(2-methoxyethoxy)quinazolin-6-yl)amino)piperidine-1-carboxylate

In AcOH (0.2 M), N⁴-(5-bromo-2-methoxyphenyl)-7-(2-methoxyethoxy)quinazolin-4,6-diamine (1.0 equiv.) obtained in Step-4 above was dissolved, and tert-butyl 4-oxopiperidine-1-carboxylate (2.0 equiv.) was added. The mixture was stirred at room temperature for 2 hours, then sodium triacetoxyborohydride (1.2 equiv.) was added, and the mixture was stirred at room temperature for 12 hours. Saturated NaHCO₃ aqueous solution was added to the reaction mixture, then organic materials were extracted with EA, and the collected organic layer was concentrated by removing remaining water using MgSO₄. The reaction mixture was purified by MPLC (DCM:MeOH) to obtain the target compound as a pale yellow solid (yield: 58%, MS (ESI): m/z 603 [M+1]⁺).

### [Step-6] Preparation of N⁴-(5-bromo-2-methoxyphenyl)-7-(2-methoxyethoxy)-N⁶-(piperidin-4-yl)quinazolin-4,6-diamine

In DCM:TFA (10:1 v/v) (0.2 M), tert-butyl 4-((4-((5-bromo-2-methoxyphenyl)amino)-7-(2-methoxyethoxy)quinazolin-6-yl)amino)piperidine-1-carboxylate (1.0 equiv.) obtained in Step-5 above was dissolved, and stirred at room temperature for 2 hours. The reaction mixture was concentrated under reduced pressure and purified by MPLC (DCM:MeOH) to obtain the target compound as a pale yellow solid (yield: 99%, MS (ESI) : m/z 503 [M+1]⁺).

### [Step-7] Preparation of N⁴-(5-(furan-2-yl)-2-methoxyphenyl)-7-(2-methoxyethoxy)-N⁶-(piperidin-4-yl)quinazolin-4,6-diamine

In 1,4-dioxane:distilled water = 5:1 (v/v) (0.2 M), N⁴-(5-bromo-2-methoxyphenyl)-7-(2-methoxyethoxy)-N⁶-(piperidin-4-yl)quinazolin-4,6-diamine (1.0 equiv.) obtained in Step-6 above, furan-2-yl boronic acid (2.2 equiv.), and K₃PO₄ (3.0 equiv.) were dissolved, and degassed with nitrogen. The reaction mixture was heated at 100 °C for 5 minutes under nitrogen, and Xphos Pd G2 (0.1 equiv.) was added and stirred for 1 hour. The reaction mixture was concentrated and purified by MPLC (DCM:MeOH) to obtain the target compound as a white solid (yield: 98%, MS (ESI): m/z 490 [M+1]⁺).

### [Step-8] Preparation of 1-(4-((4-((5-(furan-2-yl)-2-methoxyphenyl)amino)-7-(2-methoxyethoxy)quinazolin-6-yl)amino)piperidin-1-yl)prop-2-en-1-one

In THF (0.1 M), N⁴-(5-(furan-2-yl)-2-methoxyphenyl)-7-(2-methoxyethoxy)-N⁶-(piperidin-4-yl)quinazolin-4,6-diamine (1.0 equiv.) obtained in Step-7 above was dissolved, then cooled to 0 °C, and 1M NaHCO₃ aqueous solution (1.5 equiv.) and acryloyl chloride (1.0 equiv.) were added, followed by stirring at 0 °C for 30 minutes. The reaction mixture was concentrated and purified using prep-HPLC to obtain the target compound as a yellow solid (yield: 40%, MS (ESI): m/z 544 [M+1]⁺).

### Preparation of Compounds of Examples 263 to 286

Compounds of Examples 263 to 286 were prepared in a similar manner to Example 262 above. Chemical structures, compound names, ¹H NMR, MS, HPLC data and yields of respective Examples are summarized in Table 6 below.

**[Table 6]**

| Exa mpl e No. | Structure | Compound Name | ¹H NMR; MS | Yie ld (%) | HPLC r.t.(min ) Purity(% ) (method) |
|---|---|---|---|---|---|
| 262 | 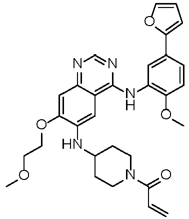 | 1-(4-((4-((5-(furan-2-yl)-2-methoxyphenyl )amino)-7-(2-methoxyethoxy )quinazolin-6-yl)amino)pipe ridin-1-yl)prop-2-en-1-one | ¹H NMR (400MHz, free base, Methanol-*d*₄) δ 8.25 (s, 1H), 8.00 (d, *J* = 2.4 Hz, 1H), 7.62-7.60 (m, 1H), 7.51 (d, *J* = 1.2 Hz 1H), 7.25 (s, 1H), 7.17 (d, *J* = 8.4 Hz, 1H), 7.09 (s, 1H), 6.85-6.78 (m, 1H), 6.66 (d, *J* = 3.6 Hz, 1H), 6.49-6.48 (m, 1H), 6.23-6.18 (m, 1H), 5.77-5.74 (m, 1H), 4.58 (d, *J* = 13.2 Hz, 1H), 4.36-4.34 (m, 2H), 4.20 (d, *J* = 12.8 Hz, 1H), 3.89-3.82 (m, 4H), 3.47 (s, 3H), 3.36-3.34 (m, 1H), 3.02-3.01 (m, 1H), 2.23-2.21 (m, 2H), 1.55-1.50 (m, 2H); MS (ESI): m/z 544 [M+1]⁺ | 40 | 6.23 100 (a) |
| 263 | 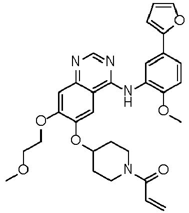 | 1-(4-((4-((5-(furan-2-yl)-2-methoxyphenyl )amino)-7-(2-methoxyethoxy )quinazolin-6-yl)oxy)piperi din-1-yl)prop-2-en-1-one | MS (ESI): m/z=545 [M+1]⁺ | | |
| 264 | 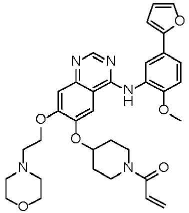 | 1-(4-((4-((5-(furan-2-yl)-2-methoxyphenyl )amino)-7-(2-morpholinoeth oxy)quinazoli n-6-yl)oxy)piperi din-1-yl)prop-2-en-1-one | MS (ESI): m/z=600 [M+1]⁺ | | |
| 265 | 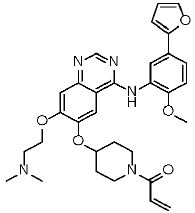 | 1- (4-((7-(2-(dimethylamin o)ethoxy)-4-((5-(furan-2-yl)-2-methoxyphenyl )amino)quinaz olin-6-yl)oxy)piperi din-1-yl)prop-2-en-1-one | MS (ESI): m/z=558 [M+1]⁺ | | |
| 266 | 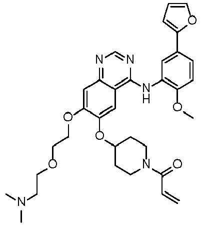 | 1-(4-((7-(2-(2-(dimethylamin o)ethoxy)etho xy)-4-((5-(furan-2-yl)-2-methoxyphenyl )amino)quinaz olin-6-yl)oxy)piperi din-1-yl)prop-2-en-1-one | MS (ESI): m/z=602 [M+1]⁺ | | |
| 267 | 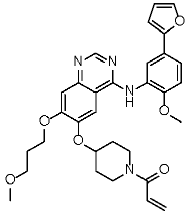 | 1-(4-((4-((5-(furan-2-yl)-2-methoxyphenyl )amino)-7-(3-methoxypropox y)quinazolin-6-yl)oxy)piperi din-1-yl)prop-2-en-1-one | MS (ESI): m/z=559 [M+1]⁺ | | |
| 268 | 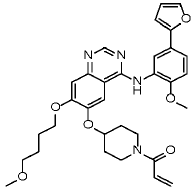 | 1-(4-((4-((5-(furan-2-yl)-2-methoxyphenyl )amino)-7-(4-methoxybutoxy ) quinazolin-6-yl)oxy)piperi din-1-yl)prop-2-en-1-one | MS (ESI): m/z=573 [M+1]⁺ | | |
| 269 | 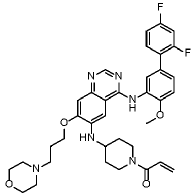 | 1-(4-((4-((2',4'-difluoro-4-methoxy-[1,1'-biphenyl]-3-yl)amino)-7-(3-morpholinopro poxy)quinazol in-6-yl)amino)pipe ridin-1-yl)prop-2-en-1-one | ¹H NMR (400 MHz, TFA salt, Methanol-*d*₄) δ8.47 (s, 1H), 7.70-7.69 (m, 1H), 7.61-7.52 (m, 3H), 7.31 (d, *J* = 8.8 Hz, 1H), 7.19 (s, 1H), 7.10-7.05 (m, 2H), 6.87-6.80 (m, 1H), 6.26-6.21 (m, 1H), 5.80-5.77 (m, 1H), 4.71-4.70 (m, 1H), 4.43-4.40 (m, 2H), 4.23-4.22 (m, 1H), 4.15-4.14 (m, 2H), 3.93-3.81 (m, 5H), 3.51-3.49 (m, 2H), 3.47-3.46 (m, 2H), 3.37-3.35 (m, 2H), 3.21-3.20 (m, 2H), 3.01-3.00 (m, 1H), 2.46-2.45 (m, 2H), 2.21-2.18 (m, 2H), 1. 66-1. 64 (m, 2H); MS (ESI): m/z 659 [M+H]⁺ | 7 | 6.02, 100 (a) |
| 270 | 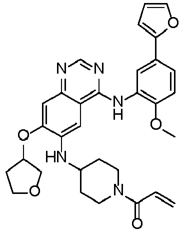 | 1-(4-((4-((5-(furan-2-yl)-2-methoxyphenyl )amino)-7-((tetrahydrof uran-3-yl)oxy)quinaz olin-6-yl)amino)pipe ridin-1-yl)prop-2-en-1-one | ¹H NMR (400 MHz, FA salt, Methanol-*d*₄) δ 8.24 (s, 1H), 8.05 (d, *J* = 2.0 Hz, 1H), 7.63-7.60 (m, 1H), 7.53 (d, *J* = 2.4Hz, 1H), 7.26 (s, 1H), 7.18 (d, *J* = 8.8, 1H), 7.05 (s, 1H), 6.87-6.80 (m, 1H), 6.68 (d, *J* = 3.2 Hz, 1H), 6.51-6.50 (m, 1H), 6.25-6.20 (m, 1H), 5.79-5.76 (m, 1H), 5.29-5.25 (m, 1H), 4.12-4.05 (m, 4H), 3.94-3.91 (m, 5H), 3.12-3.11 (m, 1H), 2.52-2.50 (m, 1H), 2.28-2.27 (m, 3H), 1. 59-1. 57 (m, 2H), 1.32-1.30 (m, 2H); MS (ESI): m/z 556 [M+H]⁺ | 3 | 6.23, 100 (a) |
| 271 | 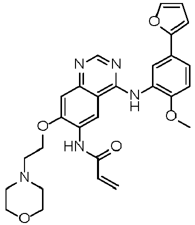 | N-(4-((5-(furan-2-yl)-2-methoxyphenyl )amino)-7-(2-morpholinoeth oxy)quinazoli n-6-yl)acrylamide | ¹H NMR (400MHz, TFA salt, Methanol-*d*₄) δ 9.21 (s, 1H), 8.67 (s, 1H), 7.93 (d, *J* = 2.4 Hz, 1H), 7.75-7.73 (m, 1H), 7.54 (d, *J* = 1.2 Hz, 1H), 7.47 (s, 1H), 7.25 (d, *J* = 8.8 Hz, 1H), 6.76-6.70 (m, 2H), 6.55-6.51 (m, 2H), 5.95-5.92 (m, 1H), 4.78 (t, *J* = 4.4 Hz, 2H), 4.00-3.85 (m, 10H), 3.52-3.49 (m, 4H) ; MS (ESI): m/z 516 [M+1]⁺ | 16 | 5.10 97 (a) |
| 272 | 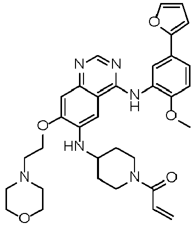 | 1-(4-((4-((5-(furan-2-yl)-2-methoxyphenyl )amino)-7-(2-morpholinoeth oxy)quinazoli n-6-yl)amino)pipe ridin-1-yl)prop-2-en-1-one | ¹H NMR (400MHz, TFA salt, Methanol-*d*₄) δ 8.34 (s, 1H), 7.92 (d, *J* = 2.4 Hz, 1H), 7.67-7.64 (m, 1H), 7.51 (d, *J* = 1.6 Hz, 1H), 7.34 (s, 1H), 7.19 (d, *J* = 8.8 Hz, 1H), 7.13 (s, 1H), 6.85-6.81 (m, 1H), 6.67-6.66 (m, 1H), 6.50-6.48 (m, 1H), 6.24-6.19 (m, 1H), 5.77-5.74 (m, 1H), 4.62 (d, *J* = 12.8 Hz, 1H), 4.43-4.40 (m, 2H), 4.23 (d, *J* = 14.4 Hz, 1H), 3.88-3.77 (m, 8H), 3.10-3.09 (m, 2H), 3.07-2.98 (m, 1H), 2.75-2.74 (m, 4H), 2.25-2.15 (m, 2H), 1.57-1.52 (m, 2H); MS (ESI): m/z 599 [M+1]⁺ | 16 | 5.32 93 (a) |
| 273 | 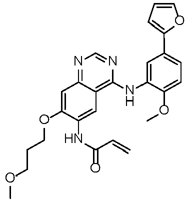 | N-(4-((5-(furan-2-yl)-2-methoxyphenyl )amino)-7-(3-methoxypropox y)quinazolin-6-yl)acrylamide | ¹H NMR (400 MHz, TFA salt, Methanol-*d*₄) δ 9.06 (s, 1H), 8.52 (s, 1H), 8.29 (s, 1H), 7.62 (d, *J* = 8.4 Hz, 1H), 7.54 (s, 1H), 7.26 (s, 1H), 7.19 (d, *J* = 8.4 Hz, 1H), 6.69-6.65 (m, 2H), 6.52-6.48 (m, 2H), 5.91-5.88 (m, 1H), 4.41-4.38 (m, 2H), 3.97 (s, 3H), 3.68-3.65 (m, 2H), 3.41 (s, 3H), 2.45-2.18 | 5 | 6.66, 100 (a) |
| | | | (m, 2H); MS (ESI): m/z 475 [M+H]⁺ | | |
| 274 | 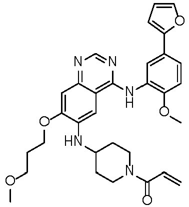 | 1-(4-((4-((5-(furan-2-yl)-2-methoxyphenyl )amino)-7-(3-methoxypropox y)quinazolin-6-yl)amino)pipe ridin-1-yl)prop-2-en-1-one | ¹H NMR (400 MHz, TFA salt, Methanol-*d*₄) δ 8.45 (s, 1H), 7.87 (d, *J* = 2.0 Hz, 1H), 7.75-7.72 (m, 1H), 7.55 (d, *J* = 1.2 Hz, 1H), 7.44 (s, 1H), 7.25 (d, *J* = 8.8 Hz, 1H), 7.13 (s, 1H), 6.87-6.81 (m, 1H), 6.71 (d, *J* = 2.4 Hz, 1H), 6.53-6.52 (m, 1H), 6.26-6.22 (m, 1H), 5.80-5.77 (m, 1H), 4.62-4.61 (m, 1H), 4.40-4.38 (m, 2H), 4.22-4.21 (m, 1H), 3.92-3.90 (m, 5H), 3.66-3.64 (m, 2H), 3.40 (s, 3H), 3.01-2.99 (m, 1H), 2.25-2.22 (m, 4H), 1.69-1.67 (m, 2H); MS (ESI): m/z 558 [M+H]⁺ | 25 | 6.46, 100 (a) |
| 275 | 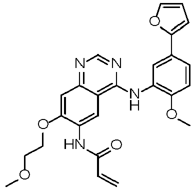 | N-(4-((5-(furan-2-yl)-2-methoxyphenyl )amino)-7-(2-methoxyethoxy )quinazolin-6-yl)acrylamide | ¹H NMR (400MHz, TFA salt, DMSO-*d*₆) δ 11.15 (s, 1H), 9.83 (s, 1H), 9.15 (s, 1H), 8.73 (s, 1H), 7.75-7.69 (m, 3H), 7.36 (s, 1H), 7.27 (d, *J* = 8.8 Hz, 1H), 6.85 (d, *J* = 3.6 Hz, 1H), 6.58-6.57 (m, 1H), 6.37-6.32 (m, 1H), 5.90-5.87 (m, 1H), 4.44-4.42 (m, 2H), 3.85-3.82 (m, 5H), 3.33 (s, 3H); MS (ESI): m/z 461 [M+1]⁺ | 32 | 6.27 96 (a) |
| 276 | 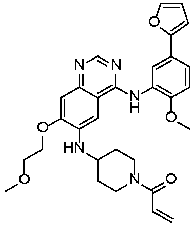 | 1-(4-((4-((5-(furan-2-yl)-2-methoxyphenyl )amino)-7-(2-methoxyethoxy ) quinazolin-6-yl)amino)pipe ridin-1-yl)prop-2-en-1-one | ¹H NMR (400MHz, free base, Methanol-*d*₄) δ 8.25 (s, 1H), 8.00 (d, *J* = 2.4 Hz, 1H), 7.62-7.60 (m, 1H), 7.51 (d, *J* = 1.2 Hz 1H), 7.25 (s, 1H), 7.17 (d, *J* = 8.4 Hz, 1H), 7.09 (s, 1H), 6.85-6.78 (m, 1H), 6.66 (d, *J* = 3.6 Hz, 1H), 6.49-6.48 (m, 1H), 6.23-6.18 (m, 1H), 5.77-5.74 (m, 1H), 4.58 (d, *J* = 13.2 Hz, 1H), 4.36-4.34 (m, 2H), 4.20 (d, *J* = 12.8 Hz, 1H), 3.89-3.82 (m, 4H), 3.47 (s, 3H), 3.36-3.34 (m, 1H), 3.02-3.01 (m, 1H), 2.23-2.21 (m, 2H), 1.55-1.50 (m, 2H); MS (ESI): m/z 544 [M+1]⁺ | 40 | 6.23 100 (a) |
| 277 | 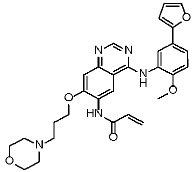 | N-(4-((5-(furan-2-yl)-2-methoxyphenyl )amino)-7-(3-morpholinopro poxy)quinazol in-6-yl)acrylamide | ¹H NMR (400 MHz, free base, Methanol-*d*₄) δ 8.96 (s, 1H), 8.44 (s, 1H), 8.43 (s, 1H), 7.53-7.51 (m, 2H), 7.19 (s, 1H), 7.13 (d, *J* = 8.8 Hz, 1H), 6.66-6.64 (m, 2H), 6.52-6.46 (m, 2H), 5.89-5.86 (m, 1H), 4.29-4.27 (m, 2H), 3.97 (s, 3H), 3.75-3.72 (m, 4H), 2.63-2.54 (m, 6H), 2.15-2.13 (m, 2H); MS (ESI): m/z 530 [M+H]⁺ | 12 | 5.45, 98 (a) |
| 278 | 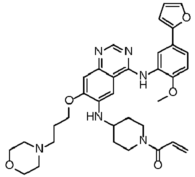 | 1-(4-((4-((5-(furan-2-yl)-2-methoxyphenyl )amino)-7-(3-morpholinopro poxy)quinazol in-6-yl)amino)pipe ridin-1-yl)prop-2-en-1-one | ¹H NMR (400 MHz, FA salt, Methanol-*d*₄) δ 8.32 (s, 1H), 8.26 (s, 1H), 7.98 (d, *J* = 2.0 Hz, 1H), 7.67-7.65 (m, 1H), 7.54 (d, *J* = 1.6 Hz, 1H), 7.34 (s, 1H), 6.88-6.81 (m, 1H), 6.69 (d, *J* = 3.6 Hz, 1H), 6.52-6.51 (m, 1H), 6.26-6.22 (m, 1H), 5.80-5.76 (m, 1H), 4.62-4.61 (m, 1H), 4.36-4.33 (m, 2H), 4.22-4.21 (m, 1H), 3.90-3.80 (m, 9H), 3.12-3.11 (m, 1H), 2.92-2.83 (m, 6H), 2.26-2.23 (m, 4H), 1.76-1.74 (m, 2H); MS (ESI): m/z 616 [M+H]⁺ | 14 | 5.29, 100 (a) |
| 279 | 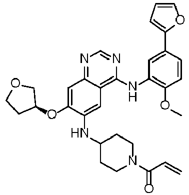 | (S)-1-(4-((4-((5-(furan-2-yl)-2-methoxyphenyl )amino)-7-((tetrahydrof uran-3-yl)oxy)quinaz olin-6-yl)amino)pipe ridin-1-yl)prop-2-en-1-one | ¹H NMR (400 MHz, TFA salt, Methanol-*d*₄) δ 8.48 (s, 1H), 7.84 (d, *J* = 2.4 Hz, 1H), 7.77-7.74 (m, 1H), 7.55 (s, 1H), 7.49 (s, 1H), 7.26 (d, *J* = 8.8 Hz, 1H), 7.10 (s, 1H), 6.87-6.80 (m, 1H), 6.71 (d, *J =* 3.6 Hz, 1H), 6.53-6.52 (m, 1H), 6.26-6.21 (m, 1H), 5.80-5.77 (m, 1H), 5.33-5.30 (m, 1H), 4.62-4.61 (m, 1H), 4.31-4.12 (m, 4H), 4.02-3.94 (m, 5H), 3.01-3.00 (m, 1H), 2.52-2.51 (m, 1H), 2.33-2.31 (m, 3H), 1. 57-1. 55 (m, 2H); MS (ESI): m/z 556 [M+H]⁺ | 20 | 6.38 96 (a) |
| 280 | 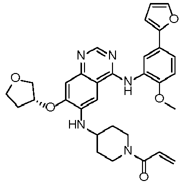 | (R)-1-(4-((4-((5-(furan-2-yl)-2-methoxyphenyl )amino)-7-((tetrahydrof uran-3-yl)oxy)quinaz olin-6-yl)amino)pipe ridin-1-yl)prop-2-en-1-one | ¹H NMR (400 MHz, TFA salt, Methanol-*d*₄) δ8.24 (s, 1H), 8.06 (d, *J* = 2.0 Hz, 1H), 7.62-7.60 (m, 1H), 7.53-7.52 (m, 1H), 7.25 (s, 1H), 7.18 (d, *J* = 8.8 Hz, 1H), 7.05 (s, 1H), 6.87-6.80 (m, 1H), 6.68-6.67 (m, 1H), 6.51-6.50 (m, 1H), 6.25-6.20 (m, 1H), 5.79-5.75 (m, 1H), 5.27-5.26 (m, 1H), 4.60-4.59 (m, 1H), 4.07-4.05 (m, 4H), 3.91-3.88 (m, 5H), 3.34-3.33 (m, 1H), 3.02-3.01 (m, 1H), 2.50-2.49 (m, 1H), 2.25-2.21 (m, 3H), 1.52-1.51 (m, 2H); (ESI): m/z 556 [M+H]⁺ | 11 | 6.27 100 (a) |
| 281 | 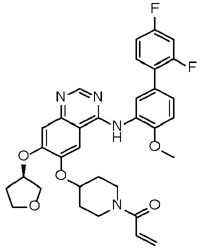 | (R)-1-(4-((4-((2',4'-difluoro-4-methoxy-[1,1'-biphenyl]-3-yl)amino)-7-((tetrahydrof uran-3-yl)oxy)quinaz olin-6-yl)oxy)piperi din-1-yl)prop-2-en-1-one | ¹H NMR (400MHz, free base, Methanol-*d*₄) δ 8.23 (s, 1H), 7.89 (s, 1H), 7.54-7.50 (m, 1H), 7.44 (d, *J* = 8.4 Hz, 1H), 7.25 (s, 1H), 7.20 (d, *J* = 8.8 Hz, 1H), 7.05-7.01 (m, 3H), 6.85-6.78 (m, 1H), 6.24-6.20 (m, 1H), 5.77-5.75 (m, 1H), 5.23-5.22 (m, 1H), 4.59 (d, *J* = 12.5 Hz, 1H), 4.19-4.01 (m, 4H), 3.94-3.83 (m, 5H), 3.03 (t, *J* = 12.0 Hz, 1H), 2.44-2.38 (m, 1H), 2.36-2.22 (m, 3H), 1.61-1.48 (m, 3H); MS (ESI): m/z 603 [M+1]⁺ | 50 | 6.78 100 (a) |
| 282 | 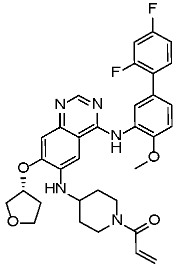 | (R)-1-(4-((4-((2',4'-difluoro-4-methoxy-[1,1'- biphenyl]-3-yl)amino)-7-((tetrahydrof uran-3-yl)oxy)quinaz olin-6-yl)amino)pipe ridin-1-yl)prop-2-en-1-one | ¹H NMR (400 MHz, TFA salt, Methanol-*d*₄) δ 8.23 (s, 1H), 7.88 (s, 1H), 7.57-7.53 (m, 1H), 7.47-7.45 (m, 1H), 7.29 (s, 1H), 7.24 (d, *J =* 8.4 Hz, 1H), 7.08-7.03 (m, 3H), 6.87-6.80 (m, 1H), 6.25-6.20 (m, 1H), 5.79-5.76 (m, 1H), 5.27-5.25 (m, 1H), 4.61-4.59 (m, 1H), 4.11-4.03 (m, 4H), 3.93-3.87 (m, 5H), 3.09-3.01 (m, 1H), 2.51-2.49 (m, 1H), 2.27-2.24 (m, 3H), 1.55-1.52 (m, 2H); MS (ESI): m/z 602 [M+H]⁺ | 15 | 6.92 100 (a) |
| 283 | 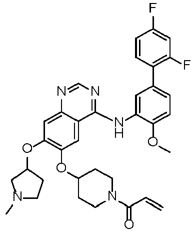 | 1-(4-((4-((2',4'-difluoro-4-methoxy-[1,1'-biphenyl]-3-yl)amino)-7-((1-methylpyrroli din-3-yl)oxy)quinaz olin-6-yl)oxy)piperi din-1-yl)prop-2-en-1-one | MS (ESI): m/z 616 [M+H]⁺ | | |
| 284 | 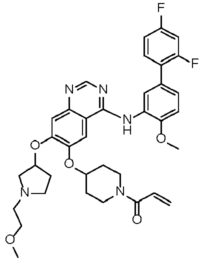 | 1-(4-((4-((2',4'-difluoro-4-methoxy-[1,1'-biphenyl]-3-yl)amino)-7-((1-(2-methoxyethyl) pyrrolidin-3-yl)oxy)quinaz olin-6-yl)oxy)piperi din-1-yl)prop-2-en-1-one | MS (ESI): m/z 660 [M+H]⁺ | | |
| 285 | 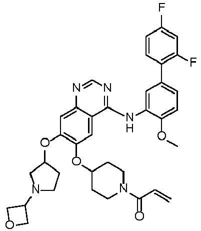 | 1-(4-((4-((2',4'-difluoro-4-methoxy-[1,1'-biphenyl]-3-yl)amino)-7-((1-(oxetan-3-yl)pyrrolidin -3-yl)oxy)quinaz olin-6-yl)oxy)piperi din-1-yl)prop-2-en-1-one | MS (ESI): m/z 658 [M+H]⁺ | | |
| 286 | 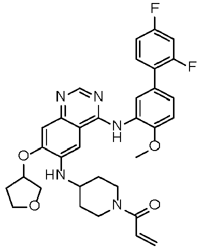 | 1-(4-((4-((2',4'-difluoro-4-methoxy-[1,1'-biphenyl]-3-yl)amino)-7-((tetrahydrof uran-3-yl)oxy)quinaz olin-6-yl)amino)pipe ridin-1-yl)prop-2-en-1-one | ¹H NMR (400MHz, TFA salt, Methanol-*d*₄) δ 8.45 (s, 1H), 7.67 (s, 1H), 7.58-7.47 (m, 3H), 7.29 (d, *J* = 8.8 Hz, 1H), 7.09-7.03 (m, 3H), 6.85-6.78 (m, 1H), 6.23-6.19 (m, 1H), 5.77-5.74 (m, 1H), 5.30-5.27 (m, 1H), 4.62 (d, *J* = 13.6 Hz, 1H), 4.23-4.03 (m, 4H), 4.02-3.85 (m, 5H), 3.00 (t, *J* = 12.4 Hz, 1H), 2.46-2.41 (m, 1H), 2.28-2.19 (m, 3H), 1. 60-1. 54 (m, 2H); MS (ESI): m/z 602 [M+1]⁺ | 15 | 6.88 100 (a) |

### Example 287. Preparation of N-(4-((5-([1,2,4]triazolo[1,5-a]pyridin-7-yl)-2-methoxyphenyl)amino)-7-methoxyquinazolin-6-yl)acrylamide

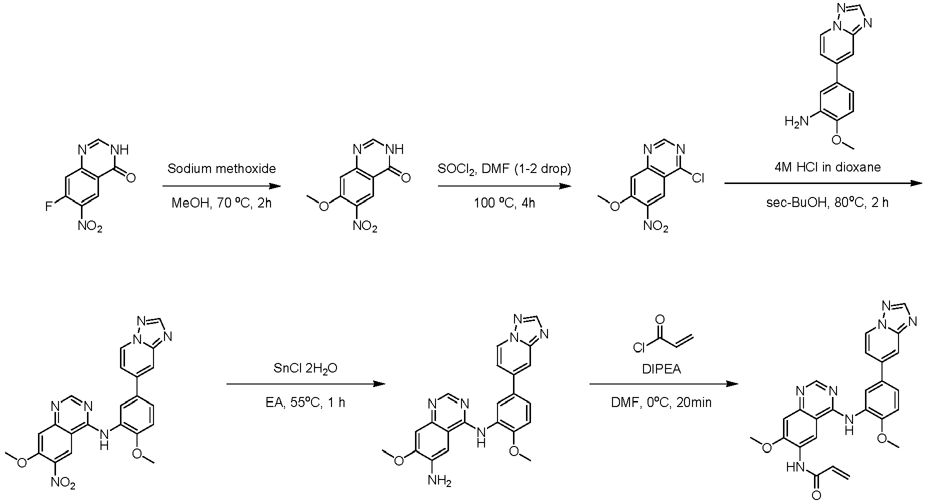

### [Step-1] Preparation of 7-methoxy-6-nitroquinazolin-4 (3H) -one

In MeOH (0.2 M), 7-fluoro-6-nitroquinazolin-4(3H)-one (1.0 equiv.) and sodium methoxide (2.0 equiv.) were dissolved, and the mixture was stirred at 70 °C for 2 hours. To the mixture, 1N HCl was added to obtain a solid, and the obtained solid was filtered. The target compound obtained as a white solid was used in the next reaction without further purification (yield: 78%, MS (ESI): m/z 222 [M+1]⁺).

### [Step-2] Preparation of 4-chloro-7-methoxy-6-nitroquinazoline

To 7-methoxy-6-nitroquinazolin-4(3H)-one (1.0 equiv.) obtained in Step-1 above, thionyl chloride (30.0 equiv.) and DMF (1 drop) were added under nitrogen. The mixture was stirred at 100 °C for 4 hours and then concentrated. The target compound obtained as a transparent oil was used in the next reaction without further purification (yield: 92%, MS (ESI): m/z 240 [M+1]⁺).

### [Step-3] Preparation of N-(5-([1,2,4]triazolo[1,5-a]pyridin-7-yl)-2-methoxyphenyl)-7-methoxy-6-nitroquinazolin-4-amine

In sec-BuOH (0.1 M), 4-chloro-7-methoxy-6-nitroquinazoline (3.0 equiv.) obtained in Step-2 above and 5-([1,2,4]triazolo[1,5-a]pyridin-7-yl)-2-methoxyaniline (1.0 equiv.) were dissolved, and 4M HCl (0.2 equiv.) dissolved in 1,4-dioxane was added and stirred at 80 °C for 2 hours. Ethyl ether was added to the reaction mixture to form a solid, and the formed solid was filtered and washed with ethyl ether. The target compound obtained as a white solid was used in the next reaction without further purification (yield: 39%, MS (ESI): m/z 444 [M+1]⁺).

### [Step-4] Preparation of N⁴-(5-([1,2,4]triazolo[1,5-a]pyridin-7-yl)-2-methoxyphenyl)-7-methoxyquinazolin-4,6-diamine

In ethyl acetate (0.1 M), N-(5-([1,2,4]triazolo[1,5-a]pyridin-7-yl)-2-methoxyphenyl)-7-methoxy-6-nitroquinazolin-4-amine (1.0 equiv.) obtained in Step-3 above and tin(II) chloride dihydrate (5.0 equiv.) were dissolved, and the mixture was stirred at 55 °C for 1 hour. The desired product was confirmed through LC-MS. The mixture was filtered through a celite filter and concentrated to obtain the target compound as a white solid, which was used in the next reaction without further purification (MS (ESI): m/z 414 [M+1]⁺).

### [Step-5] Preparation of N-(4-((5-([1,2,4]triazolo[1,5-a]pyridin-7-yl)-2-methoxyphenyl)amino)-7-methoxyquinazolin-6-yl)acrylamide

In DMF (0.1 M), N⁴-(5-([1,2,4]triazolo[1,5-a]pyridin-7-yl)-2-methoxyphenyl)-7-methoxyquinazolin-4,6-diamine (1.0 equiv.) obtained in Step-4 above and DIPEA (2.5 equiv.) were dissolved, and then acryloyl chloride (1.0 equiv.) was added at 0 °C and stirred at 0 °C for 20 minutes. The reaction mixture was concentrated in vacuo and purified using prep-HPLC to obtain the target compound as a white solid (yield: 15%, MS (ESI) : m/z 468 [M+1]⁺).

### Preparation of Compounds of Examples 288 to 296

Compounds of Examples 288 to 296 according to the present invention were prepared in a similar manner to Example 287 above. Chemical structures, compound names, ¹H NMR, MS, HPLC data and yields of respective Examples are summarized in Table 7 below.

**[Table 7]**

| Exa mple No. | Structure | Compound Name | ¹H NMR; MS | Yie ld (%) | HPLC r.t. (min ) Purity(% ) (method) |
|---|---|---|---|---|---|
| 287 | 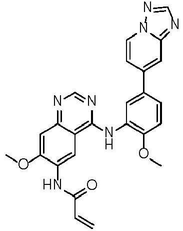 | N-(4-((5-([1,2,4]triaz olo[1,5-a]pyridin-7-yl)-2-methoxyphenyl )amino)-7-methoxyquinaz olin-6-yl)acrylamide | ¹H NMR (400MHz, TFA salt, Methanol-*d*₄) δ 9.10 (s, 1H), 8.85 (d, *J* = 7.2 Hz, 1H), 8.66 (d, *J* = 2.4 Hz, 1H), 8.54 (s, 1H), 8.44 (s, 1H), 8.02 (d, *J* = 1.2 Hz, 1H), 7.74-7.71 (m, 1H), 7.62-7.60 (m, 1H), 7.33-7.30 (m, 2H), 6.76-6.89 (m, 1H), 6.52-6.48 (m, 1H), 5.90-5.87 (m, 1H), 4.13 (s, 3H), 4.06 (s, 3H); MS (ESI): m/z 468 [M+1]⁺ | 15 | 4.42 100 (a) |
| 288 | 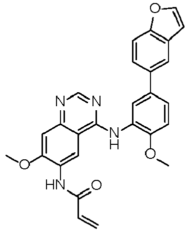 | N-(4-((5-(benzofuran-5-yl)-2-methoxyphenyl )amino)-7-methoxyquinaz olin-6-yl)acrylamide | ¹H NMR (400MHz, TFA salt, DMSO-*d*₆) δ 9.76 (s, 1H), 9.22 (s, 1H), 8.94 (s, 1H), 8.40 (s, 1H), 8.02 (d, *J* = 2.4 Hz, 2H), 7.89 (d, *J* = 1.6 Hz, 1H), 7.67-7.52 (m, 5H), 7.27 (s, 1H), 7.22 (d, *J* = 8.8 Hz, 1H), 7.00-6.99 (m, 1H), 6.80-6.73 (m, 1H), 6.34-6.30 (m, 1H), 5.82-5.78 (m, 1H), 4.02 (s, 3H), 3.85 (s, 3H); MS (ESI): m/z 467 [M+1]⁺ | 11 | 6.83 100 (a) |
| 289 | 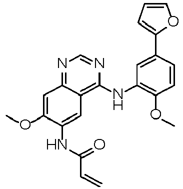 | N- (4- ((5-(furan-2-yl)-2-methoxyphenyl ) amino) -7-methoxyquinaz olin-6-yl)acrylamide | ¹H NMR (400MHz, TFA salt, Methanol-*d*₄) δ 9.28 (s, 1H), 8.66 (s, 1H), 7.97-7.95 (m, 1H), 7.75-7.72 (m, 1H), 7.55-7.54 (m, 1H), 7.31 (s, 1H), 7.24 (d, *J* = 8.8 Hz, 1H), 6.76-6.69 (m, 2H), 6.54-6.49 (m, 2H), 5.92-5.89 (m, 1H), 4.19 (s, 3H), 3.93 (s, 3H); MS (ESI): m/z 417 [M+1]⁺ | 43 | 6.16 97 (a) |
| 290 | 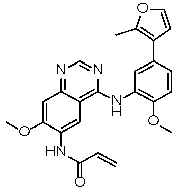 | N-(7-methoxy-4-((2-methoxy-5-(2-methylfuran-3-yl)phenyl)ami no)quinazolin -6-yl)acrylamide | ¹H NMR (400MHz, TFA salt, Methanol-*d*₄) δ 9.27 (s, 1H), 8.63 (s, 1H), 7.68 (d *J* = 2 Hz, 1H), 7.46-7.43 (m, 1H), 7.39 (d, *J* = 2 Hz, 1H), 7.29 (s, 1H), 7.22 (d, *J* = 8.4 Hz, 1H), 6.73-6.66 (m, 1H), 6.56-6.47 (m, 2H), 5.90-5.87 (m, 1H), 4.17 (s, 3H), 3.91 (s, 3H), 2.44 (s, 3H); MS (ESI): m/z 431 [M+1]⁺ | 6 | 97* |
| 291 | 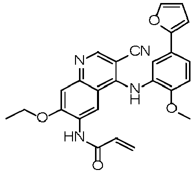 | N-(3-cyano-7-ethoxy-4-((5-(furan-2-yl)-2-methoxyphenyl ) amino) quinol in-6-yl)acrylamide | ¹H NMR (400MHz, TFA salt, CDCl₃) δ 9.40 (s, 1H), 8.62 (s, 1H), 8.57 (s, 1H), 8.25 (s, 1H), 8.03 (s, 1H), 7.77-7.74 (m, 1H), 7.63 (d, *J* = 2.0 Hz, 1H), 7.07-7.05 (m, 1H), 7.44 (d, *J* = 2.0 Hz, 1H), 7.07-7.05 (m, 1H), 6.59-6.48 (m, 2H), 6.47-6.38 (m, 1H), 5.95-5.93 (m, 1H), 4.46-4.44 (m, 2H), 3.87 (s, 3H), 1.60 (t, *J* = 6.8, 7.2 Hz, 3H); MS (ESI): m/z 455 [M+1]⁺ | 7 | 7.27 100 (a) |
| 292 | 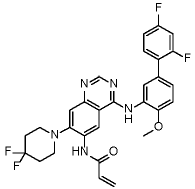 | N-(4-((2',4'-difluoro-4-methoxy-[1,1'-biphenyl]-3-yl)amino)-7-(4,4-difluoropiper idin-1-yl)quinazolin -6-yl)acrylamide | ¹H NMR (400MHz, TFA salt, CDCl-*d*₃) δ 9.16 (s, 1H), 8.87-8.86 (m, 1H), 8.73-8.35 (m, 2H), 7.66 (s, 1H), 7.51-7.45 (m, 1H), 7.28-7.25 (m, 1H), 7.04 (d, *J* = 8.8 Hz, 1H), 6.99-6.89 (m, 2H), 6.53-6.48 (m, 1H), 6.39-6.32 (m, 1H), 5.92-5.89 (m, 1H), 4.06 (s, 3H), 3.16 (t, *J* = 5.6 Hz, 4H), 2.29-2.17 (m, 4H); MS (ESI): m/z 552 [M+1]⁺ | 56 | 11.97 100 (b) |
| 293 | 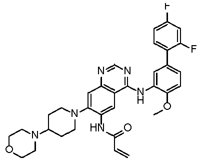 | N-(4-((2',4'-difluoro-4-methoxy-[1,1'-biphenyl]-3-yl)amino)-7-(4-morpholinopip eridin-1-yl)quinazolin -6-yl)acrylamide | MS (ESI): m/z 601 [M+1]⁺ | | |
| 294 | 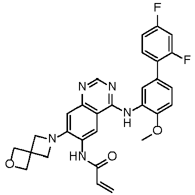 | N-(4-((2',4'-difluoro-4-methoxy-[1,1'-biphenyl]-3-yl)amino)-7-(2-oxa-6-azaspiro[3.3] heptan-6-yl)quinazolin -6-yl)acrylamide | MS (ESI): m/z 530 [M+1]⁺ | | |
| 295 | 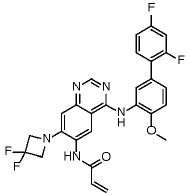 | N-(4-((2',4'-difluoro-4-methoxy-[1,1'-biphenyl]-3-yl)amino)-7-(3,3-difluoroazeti din-1-yl)quinazolin -6-yl)acrylamide | ¹H NMR (400 MHz, TFA salt, DMSO-*d*₆) δ 9.82 (s, 1H), 9.28 (s, 1H), 8.34 (s, 1H), 8.31 (s, 1H), 7.70 (s, 1H), 7.61-7.53 (m, 1H), 7.42-7.30 (m, 2H), 7.23-7.13 (m, 2H), 6.82 (s, 1H), 6.54 (dd, *J* = 17.2, 10.0 Hz, 1H), 6.31 (d, *J* = 17.2 Hz, 1H), 5.82 (d, *J* = 10.4 Hz, 1H), 4.43 (t, *J* = 12.4 Hz, 4H), 3.83 (s, 3H); MS (ESI): m/z 523 [M]⁺ | 17 | 10.93 99 (b) |
| 296 | 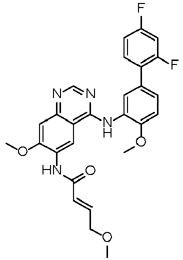 | (E)-N-(4-((2' ,4'-difluoro-4-methoxy-[1,1'-biphenyl]-3-yl)amino)-7-methoxyquinaz olin-6-yl)-4-methoxybut-2-enamide | MS (ESI): m/z 507 [M]⁺ | | |

### Example 297. Preparation of 1-(4-((4-((2',4'-difluoro-4-methoxy-[1,1'-biphenyl]-3-yl)amino)-7-(difluoromethoxy)quinazolin-6-yl)amino)piperidin-1-yl)prop-2-en-1-one

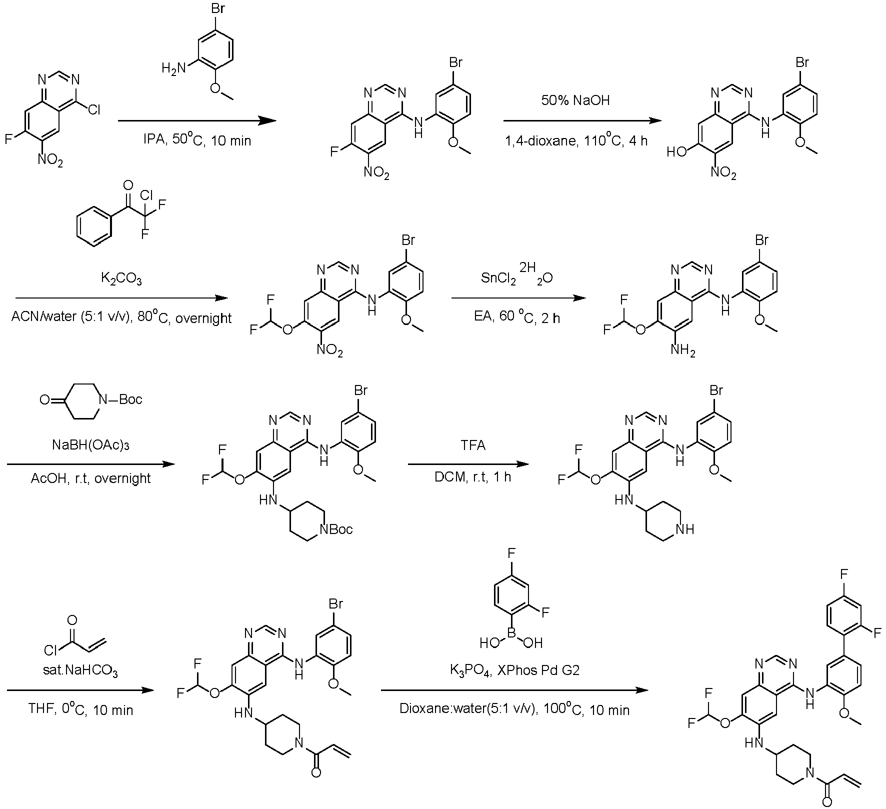

### [Step-1] Preparation of N-(5-bromo-2-methoxyphenyl)-7-fluoro-6-nitroquinazolin-4-amine

In isopropyl alcohol (0.2 M), 4-chloro-7-fluoro-6-nitroquinazoline (1.0 equiv.) and 5-bromo-2-methoxyaniline (1.0 equiv.) were dissolved, and stirred at 50 °C for 10 minutes. Ethyl ether was added to produce a solid compound, and the resulting solid was filtered to obtain the target compound as a yellow solid (yield: 72%, MS (ESI): m/z 393 [M+1]⁺).

### [Step-2] Preparation of 4-((5-bromo-2-methoxyphenyl)amino)-6-nitroquinazolin-7-ol

In 1,4-dioxane, N-(5-bromo-2-methoxyphenyl)-7-fluoro-6-nitroquinazolin-4-amine (1.0 equiv.) obtained in Step-1 above was dissolved, then 50% NaOH aqueous solution (12.5 equiv.) was added and stirred at 110 °C for 4 hours. The mixture was acidified by adding 10% sulfuric acid aqueous solution, and organic materials were extracted with EA. The collected organic layer was concentrated by removing remaining water using Na₂SO₄. The target compound obtained as a yellow solid was used in the next reaction without further purification (yield: 74%, MS (ESI): m/z 391 [M+1]⁺).

### [Step-3] Preparation of N-(5-bromo-2-methoxyphenyl)-7-(difluoromethoxy)-6-nitroquinazolin-4-amine

In ACN:H₂O (5:1), 4-((5-bromo-2-methoxyphenyl)amino)-6-nitroquinazolin-7-ol (1.0 equiv.) obtained in Step-2 above, 2-chloro-2,2-difluoro-1-phenylethan-1-one (2.0 equiv.) and K₂CO₃ (2.0 equiv.) were dissolved, and the mixture was stirred at 80 °C for 16 hours. Water was added thereto, and organic materials were extracted with EA. The collected organic layer was concentrated by removing remaining water using Na₂SO₄. The reaction mixture was purified by MPLC (DCM:MeOH) to obtain the target compound as a yellow solid (yield: 37%, MS (ESI) : m/z 441 [M+1]⁺).

### [Step-4] Preparation of N⁴-(5-bromo-2-methoxyphenyl)-7-(difluoromethoxy)quinazolin-4,6-diamine

In ethyl acetate (0.1 M), N-(5-bromo-2-methoxyphenyl)-7-(difluoromethoxy)-6-nitroquinazolin-4-amine (1.0 equiv.) obtained in Step-3 above and tin(II) chloride dihydrate (5.0 equiv.) were dissolved, and the mixture was stirred at 60 °C for 2 hours. An aqueous solution of ammonia was added to the reaction mixture and concentrated, then the concentrate was purified using MPLC (DCM:MeOH) to obtain the target compound as a yellow solid (yield: 49%, MS (ESI): m/z 411 [M+1]⁺).

### [Step-5] Preparation of tert-butyl 4-((4-((5-bromo-2-methoxyphenyl)amino)-7-(difluoromethoxy)quinazolin-6-yl)amino)piperidine-1-carboxylate

In acetic acid (0.1 M), N⁴-(5-bromo-2-methoxyphenyl)-7-(difluoromethoxy)quinazolin-4,6-diamine (1.0 equiv.) obtained in Step-4 above and tert-butyl 4-oxopiperidine-1-carboxylate (3.0 equiv.) were dissolved, and stirred at room temperature for 2 hours. Sodium triacetoxyborohydride (3.0 equiv.) was added and the mixture was stirred at room temperature for 16 hours. To the mixture, 2M NaOH aqueous solution was added and organic materials were extracted with DCM. The collected organic layer was concentrated by removing remaining water using MgSO₄. The target compound obtained as a yellow solid was used in the next reaction without further purification (yield: 100%, MS (ESI): m/z 594 [M+1]⁺).

### [Step-6] Preparation of N⁴-(5-bromo-2-methoxyphenyl)-7-(difluoromethoxy)-N⁶-(piperidin-4-yl)quinazolin-4,6-diamine

In DCM, tert-butyl 4-((4-((5-bromo-2-methoxyphenyl)amino)-7-(difluoromethoxy)quinazolin-6-yl)amino)piperidine-1-carboxylate (1.0 equiv.) obtained in Step-5 above was dissolved, then TFA (50.0 equiv.) was added, and the mixture was stirred at room temperature for 1 hour. The reaction mixture was concentrated and purified by MPLC (DCM:MeOH) to obtain the target compound as a yellow solid (yield: 97%, MS (ESI) : m/z 494 [M+1]⁺).

### [Step-7] Preparation of 1-(4-((4-((5-bromo-2-methoxyphenyl)amino)-7-(difluoromethoxy)quinazolin-6-yl)amino)piperidin-1-yl)prop-2-en-1-one

In THF, N⁴-(5-bromo-2-methoxyphenyl)-7-(difluoromethoxy)-N⁶-(piperidin-4-yl)quinazolin-4,6-diamine (1.0 equiv.) obtained in Step-6 above and saturated NaHCO₃ aqueous solution (5.0 equiv.) were dissolved, and then acryloyl chloride (1.0 equiv.) was added at 0 °C and stirred for 10 minutes. The reaction mixture was concentrated to obtain the target compound as a brown solid, which was used in the next reaction without further purification (yield: 100%, MS (ESI): m/z 548 [M+1]⁺).

### [Step-8] Preparation of 1-(4-((4-((2',4'-difluoro-4-methoxy-[1,1'-biphenyl]-3-yl)amino)-7-(difluoromethoxy)quinazolin-6-yl)amino)piperidin-1-yl)prop-2-en-1-one

In 1,4-dioxane:water (5:1), 1-(4-((4-((5-bromo-2-methoxyphenyl)amino)-7-(difluoromethoxy)quinazolin-6-yl)amino)piperidin-1-yl)prop-2-en-1-one (1.0 equiv.) obtained in Step-7 above, (2,4-difluorophenyl)boronic acid (2.2 equiv.), and K₃PO₄ (2.0 equiv.) were dissolved. The reaction mixture was degassed using nitro gas, and Xphos Pd G2 (0.1 equiv.) was added at 100 °C, followed by stirring at 100 °C for 10 minutes. The mixture was filtered through a celite filter and the filtrate was concentrated. The reaction mixture was purified by prep-HPLC to obtain the target compound as a yellow solid (yield: 7%, MS (ESI): m/z 582 [M+1]⁺).

### Preparation of Compounds of Examples 298 to 303

Compounds of Examples 298 to 303 according to the present invention were prepared in a similar manner to Example 297 above. Chemical structures, compound names, ¹H NMR, MS, HPLC data and yields of respective Examples are summarized in Table 8 below.

**[Table 8]**

| Exa mpl e No. | Structure | Compound Name | ¹H NMR; MS | Yie ld (%) | HPLC r.t.(min ) Purity(% ) (method) |
|---|---|---|---|---|---|
| 297 | 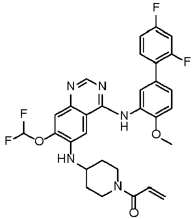 | 1-(4-((4-((2',4'-difluoro-4-methoxy-[1,1'-biphenyl]-3-yl)amino)-7-(difluorometh oxy)quinazoli n-6-yl)amino)pipe ridin-1-yl)prop-2-en-1-one | ¹H NMR (400 MHz, FA salt, Methanol-*d*₄) δ 8.24 (s, 1H), 7.85-7.84 (m, 1H), 7.59-7.47 (m, 3H), 7.44 (d, *J* = 19.2 Hz, 1H), 7.29-7.23 (m, 1H), 7.11-7.03 (m, 2H), 6.93-6.80 (m, 1H), 6.25-6.20 (m, 1H), 5.79-5.76 (m, 1H), 4.63-4.59 (m, 1H), 4.23-4.20 (m, 1H), 3.93-3.87 (m, 4H), 3.37-3.36 (m, 1H), 3.05-2.98 (m, 1H), 2.26-2.23 (m, 2H), 1.60-1.55 (m, 2H); MS (ESI): m/z 581 [M+H]⁺ | 7 | 10.97 99 (b) |
| 298 | 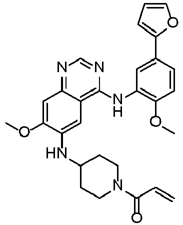 | 1-(4-((4-((5-(furan-2-yl)-2-methoxyphenyl )amino)-7-methoxyquinaz olin-6-yl)amino)pipe ridin-1-yl)prop-2-en-1-one | ¹H NMR (400 MHz, FA salt, Methanol-*d*₄) δ 8.24 (s, 1H), 8.06 (d, *J* = 2.0 Hz, 1H), 7.62-7.59 (m, 1H), 7.53 (d, *J* = 1.6 Hz, 1H), 7.22 (s, 1H), 7.18 (d, *J* = 8.4 Hz, 1H), 7.09 (s, 1H), 6.87-6.80 (m, 1H), 6.68 (d, *J* = 3.6 Hz, 1H), 6.51-6.50 (m, 1H), 6.25-6.20 (m, 1H), 5.79-5.75 (m, 1H), 4.22-4.21 (m, 1H), 4.05 (s, 3H), 3.91 (s, 3H), 3.59-3.58 (m, 1H), 3.09-3.07 (m, 1H), 2.25-2.18 (m, 2H), 1.53-1.50 (m, 2H), 1.31-1.29 (m, 2H); MS (ESI): m/z 500 [M+H]⁺ | 3 | 6.14, 98 (a) |
| 299 | 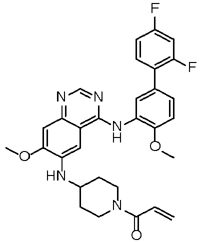 | 1-(4-((4-((2',4'-difluoro-4-methoxy-[1,1'-biphenyl]-3-yl)amino)-7-methoxyquinaz olin-6-yl)amino)pipe ridin-1-yl)prop-2-en-1-one | ¹H NMR (400 MHz, TFA salt, Methanol-*d*₄) δ 8.22 (s, 1H), 7.89 (s, 1H), 7.58-7.54 (m, 1H), 7.47-7.44 (m, 1H), 7.25-7.22 (m, 2H), 7.09-7.03 (m, 3H), 6.87-6.80 (m, 1H), 6.25-6.20 (m, 1H), 5.79-5.76 (m, 1H), 4.60-4.59 (m, 1H), 4.07-4.06 (m, 1H), 4.05 (s, 3H), 3.94 (s, 3H), 3.88-3.87 (m, 1H), 3.37-3.35 (m, 1H), 3.02-3.01 (m, 1H), 2.25-2.22 (m, 2H), 1. 51-1. 50 (m, 2H); MS (ESI): m/z 546 [M+H]⁺ | 19 | 6.79, 100 (a) |
| 300 | 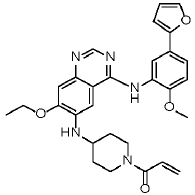 | 1-(4-((7-ethoxy-4-((5-(furan-2-yl)-2-methoxyphenyl )amino) quinaz olin-6-yl)amino)pipe ridin-1-yl)prop-2-en-1-one | ¹H NMR (400 MHz, TFA salt, Methanol-*d*₄) δ 8.47 (s, 1H), 7.84 (s, 1H), 7.76-7.73 (m, 1H), 7.54 (s, 1H), 7.47 (s, 1H), 7.26 (d, *J* = 8.8 Hz, 1H), 7.11 (s, 1H), 6.87-6.80 (m, 1H), 6.71-6.70 (m, 1H), 6.53-6.52 (m, 1H), 6.23-6.21 (m, 1H), 5.80-5.76 (m, 1H), 4.99-4.98 (m, 1H), 4.67-4.66 (m, 1H), 4.40-4.35 (m, 2H), 4.12-4.11 (m, 1H), 3.99-3.97 (m, 4H), 3.01-2.99 (m, 1H), 2.29-2.27 (m, 2H), 1. 61-1. 57 (m, 5H); MS (ESI): m/z 514 [M+H]⁺ | 68 | 10.45 99 (b) |
| 301 | 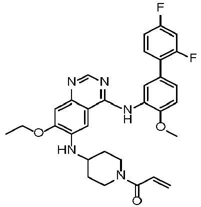 | 1-(4-((4-((2',4'-difluoro-4-methoxy-[1,1'-biphenyl] -3-yl)amino)-7-ethoxyquinazo lin-6-yl)amino)pipe ridin-1-yl)prop-2-en-1-one | ¹H NMR (400 MHz, TFA salt, Methanol-*d*₄) δ 8.46 (s, 1H), 7.70 (s, 1H), 7.60-7.54 (m, 2H), 7.48 (s, 1H), 7.31 (d, *J* = 8.8 Hz, 1H), 7.10-7.05 (m, 3H), 6.87-6.80 (m, 1H), 6.26-6.21 (m, 1H), 5.80-5.77 (m, 1H), 4.72-4.71 (m, 1H), 4.38-4.35 (m, 2H), 4.23-4.21 (m, 1H), 3.93-3.90 (m, 4H), 3.36-3.35 (m, 1H), 3.11-3.10 (m, 1H), 2.21-2.19 (m, 2H), 1.60-1.56 (m, 5H); MS (ESI): m/z 560 [M+H]⁺ | 53 | 11.24 100 (b) |
| 302 | 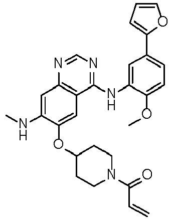 | 1-(4-((4-((5-(furan-2-yl)-2-methoxyphenyl )amino) -7-(methylamino) quinazolin-6-yl)oxy)piperi din-1-yl)prop-2-en-1-one | MS (ESI): m/z 500 [M+1]⁺ | | |
| 303 | 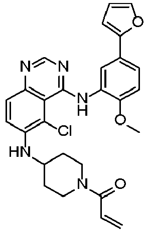 | 1-(4-((5-chloro-4-((5-(furan-2-yl)-2-methoxyphenyl )amino)quinaz olin-6-yl)amino)pipe ridin-1-yl)prop-2-en-1-one | ¹H NMR (400 MHz, TFA salt, Methanol-*d*₄) δ 8.99 (d, *J* = 2.1 Hz, 1H), 8.57 (s, 1H), 7.66 (s, 2H), 7.54 (d, *J* = 1.2 Hz, 1H), 7.51 (dd, *J* = 8.5, 2.1 Hz, 1H), 7.13 (d, *J* = 8.6 Hz, 1H), 6.82 (dd, *J* = 16.8, 10.7 Hz, 1H), 6.67 (d, *J* = 2.8 Hz, 1H), 6.52 (dd, *J* = 3.3, 1.8 Hz, 1H), 6.22 (dd, *J* = 16.8, 2.0 Hz, 1H), 5.77 (dd, *J* = 10.7, 2.0 Hz , 1H), 4.59 (d, *J* = 13.0 Hz, 1H), 4.18 (d, *J* = 13.6 Hz, 1H), 3.92-3.83 (m, 1H), 3.35-3.33 (m, 1H), 2.98 (t, *J* = 11.9 Hz, 1H), 2.16-2.08 (m, 2H), 1. 65-1. 55 (m, 3H); MS (ESI): m/z 503 [M] + | 16 | 11.10 86 (b) |

### Example 304. Preparation of (R,E)-N-(4-((2',4'-difluoro-4-methoxy-[1,1'-biphenyl]-3-yl)amino)-7-methoxyquinazolin-6-yl)-2-fluoro-3-(1-methylpyrrolidin-2-yl)acrylamide

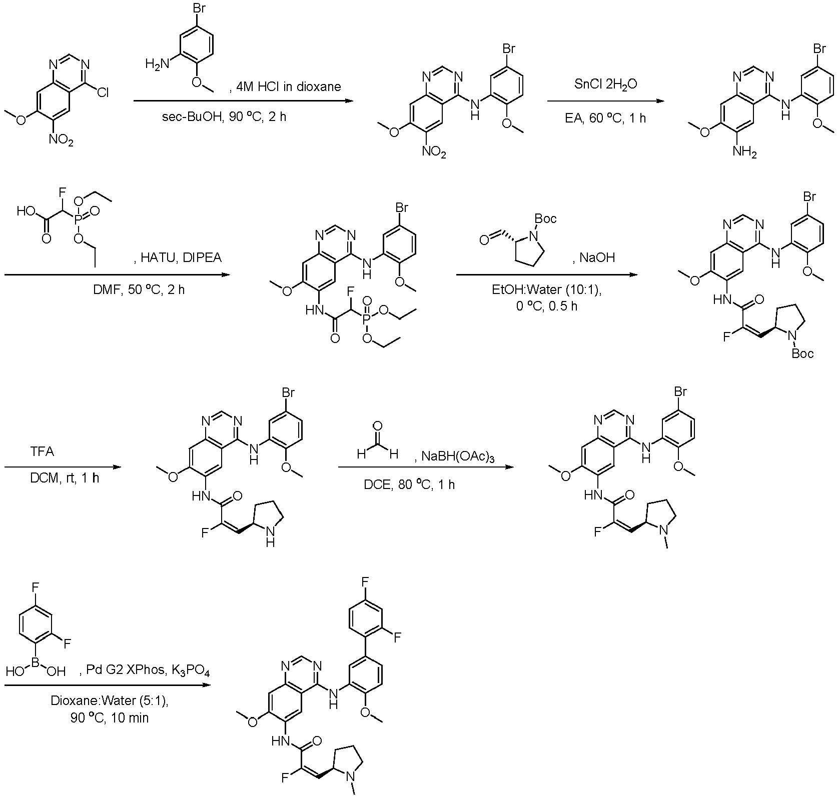

### [Step-1] Preparation of N-(5-bromo-2-methoxyphenyl)-7-methoxy-6-nitroquinazolin-4-amine

In sec-BuOH (0.2 M), 4-chloro-7-methoxy-6-nitroquinazoline (1.0 equiv.) and 5-bromo-2-methoxyaniline (1.0 equiv.) were dissolved, and then 4M HCl (0.2 equiv.) dissolved in 1,4-dioxane was added and stirred at 90 °C for 2 hours. Ethyl ether was added to the reaction mixture, and the resulting solid was filtered and washed with ethyl ether to obtain the target compound as a yellow solid (yield: 99%, MS (ESI) : m/z 406 [M+1]⁺).

### [Step-2] Preparation of N⁴-(5-bromo-2-methoxyphenyl)-7-methoxyquinazolin-4,6-diamine

In ethyl acetate (0.1 M), N-(5-bromo-2-methoxyphenyl)-7-methoxy-6-nitroquinazolin-4-amine (1.0 equiv.) obtained in Step 1 above and tin(II) chloride dihydrate (5.0 equiv.) were dissolved, and the mixture was stirred at 60 °C for 1 hour. The mixture was filtered through a celite filter, and the target compound as a brown solid obtained after concentrating the filtrate was used in the next reaction without further purification (MS (ESI): m/z 376 [M+1]⁺).

### [Step-3] Preparation of diethyl (2-((4-((5-bromo-2-methoxyphenyl)amino)-7-methoxyquinazolin-6-yl)amino)-1-fluoro-2-oxoethyl)phosphonate

In DMF (0.5 M), 2-(diethoxyphosphoryl)-2-fluoroacetic acid (2.0 equiv.) was dissolved, and then HATU (3.0 equiv.) was added and stirred at 50 °C for 1 hour. To the reaction mixture, N⁴-(5-bromo-2-methoxyphenyl)-7-methoxyquinazolin-4,6-diamine (1.0 equiv.) obtained in Step 2 above and DIPEA (3.0 equiv.) were added and stirred at 50 °C for 1 hour. The solid obtained by adding ice water was filtered and washed with distilled water to obtain the target compound as a yellow solid (yield: 30%, MS (ESI): m/z 572 [M+1]⁺).

### [Step-4] Preparation of tert-butyl (R,E)-2-(3-((4-((5-bromo-2-methoxyphenyl)amino)-7-methoxyquinazolin-6-yl)amino)-2-fluoro-3-oxoprop-1-en-1-yl)pyrrolidine-1-carboxylate

To diethyl (2-((4-((5-bromo-2-methoxyphenyl)amino)-7-methoxyquinazolin-6-yl)amino)-1-fluoro-2-oxoethyl)phosphonate (1.0 equiv.) obtained in Step-3 above, NaOH (8.0 equiv.) dissolved in ethanol (0.02 M) and distilled water (0.2 M) were added and stirred for 30 minutes. To the reaction mixture, tert-butyl (R)-2-formylpyrrolidine-1-carboxylate (2.0 equiv.) was added at 0 °C and stirred at room temperature for 30 minutes. Water was added to the reaction mixture, and organic materials were extracted with EA. The collected organic layer was concentrated by removing remaining water using Na₂SO₄. The target compound obtained as a yellow solid was used in the next reaction without further purification (yield: 91%, MS (ESI): m/z 616 [M+1]⁺).

### [Step-5] Preparation of (R,E)-N-(4-((5-bromo-2-methoxyphenyl)amino)-7-methoxyquinazolin-6-yl)-2-fluoro-3-(pyrrolidin-2-yl)acrylamide

In DCM (0.1 M), tert-butyl (R,E)-2-(3-((4-((5-bromo-2-methoxyphenyl)amino)-7-methoxyquinazolin-6-yl)amino)-2-fluoro-3-oxoprop-1-en-1-yl)pyrrolidine-1-carboxylate (1.0 equiv.) obtained in Step 4 above was dissolved, and TFA (10.0 equiv.) was added and stirred at room temperature for 1 hour. The reaction mixture was concentrated and purified by MPLC (DCM:MeOH) to obtain the target compound as a yellow solid (yield: 97%, MS (ESI) : m/z 516 [M+1]⁺).

### [Step-6] Preparation of (R,E)-N-(4-((5-bromo-2-methoxyphenyl)amino)-7-methoxyquinazolin-6-yl)-2-fluoro-3-(1-methylpyrrolidin-2-yl)acrylamide

In DCE (0.1 M), (R,E)-N-(4-((5-bromo-2-methoxyphenyl)amino)-7-methoxyquinazolin-6-yl)-2-fluoro-3-(pyrrolidin-2-yl)acrylamide (1.0 equiv.) obtained in Step 5 above was dissolved, and 37% formaldehyde (5.0 equiv.) dissolved in distilled water was added and stirred at room temperature for 1 hour. Then, NaBH(OAc)₃ (7.0 equiv.) was added and the mixture was stirred at 80 °C for 1 hour. The reaction mixture was concentrated and purified by MPLC (DCM:MeOH) to obtain the target compound as a yellow solid (yield: 56%, MS (ESI) : m/z 530 [M+1]⁺).

### [Step-7] Preparation of (R,E)-N-(4-((2',4'-difluoro-4-methoxy-[1,1'-biphenyl]-3-yl)amino)-7-methoxyquinazolin-6-yl)-2-fluoro-3-(1-methylpyrrolidin-2-yl)acrylamide

In 1,4-dioxane (0.1 M) and water (0.5 M), (R,E)-N-(4-((5-Bromo-2-methoxyphenyl)amino)-7-methoxyquinazolin-6-yl)-2-fluoro-3-(1-methylpyrrolidin-2-yl)acrylamide (1.0 equiv.) obtained in Step 6 above, (2,4-difluorophenyl)boronic acid (2.2 equiv.), and K₃PO₄ (2.0 equiv.) were dissolved. The reaction mixture was degassed using nitro gas, and Xphos Pd G2 (0.13 equiv.) was added at 80 °C, followed by stirring at 90 °C for 10 minutes. The reaction mixture was concentrated and purified by prep-HPLC to obtain the target compound as a pale yellow solid (yield: 22%, MS (ESI) : m/z 564 [M+1]⁺).

### Preparation of Compounds of Examples 305 to 313

Compounds of Examples 305 to 313 were prepared in a similar manner to Example 304 above. Chemical structures, compound names, ¹H NMR, MS, HPLC data and yields of respective Examples are summarized in Table 9 below.

**[Table 9]**

| Exa mpl e No. | Structure | Compound Name | ¹H NMR; MS | Yie ld (%) | HPLC r.t.(min ) Purity(% ) (method) |
|---|---|---|---|---|---|
| 304 | 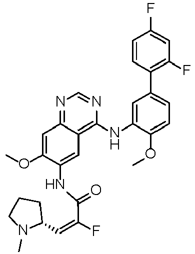 | (R,E)-N-(4-((2',4'-difluoro-4-methoxy-[1,1'-biphenyl]-3-yl)amino)-7-methoxyquinaz olin-6-yl)-2-fluoro-3-(1-methylpyrroli din-2-yl)acrylamide | ¹H NMR (400 MHz, TFA salt, Methanol-*d*₄) δ 9.19 (s, 1H), 8.67 (s, 1H), 7.77 (s, 1H), 7.61-7.52 (m, 2H), 7.36 (s, 1H), 7.32 (d, *J* = 8.8 Hz, 1H), 7.11-7.06 (m, 2H), 6.42-6.32 (m, 1H), 4.52-4.50 (m, 1H), 4.20 (s, 3H), 3.94 (s, 3H), 3.83-3.82 (m, 1H), 3.51-3.50 (m, 1H), 3.15 (s, 3H), 2.09-2.01 (m, 4H); MS (ESI): m/z 564 [M+H]⁺ | 26 | 8.84 100 (b) |
| 305 | 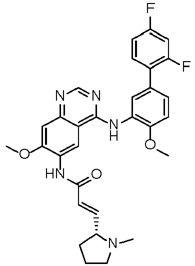 | (R,E)-N-(4-((2',4'-difluoro-4-methoxy-[1,1'-biphenyl]-3-yl)amino)-7-methoxyquinaz olin-6-yl)-3-(1-methylpyrroli din-2-yl)acrylamide | ¹H NMR (400 MHz, HCl salt, Methanol-*d*₄) δ 9.02 (s, 1H), 8.41 (s, 1H), 8.23 (s, 1H), 7.59-7.49 (m, 1H), 7.40-7.34 (m, 1H), 7.21 (s, 1H), 7.18 (d, *J* = 8.6 Hz, 1H), 7.07-6.99 (m, 2H), 6.88 (dd, *J* = 15.2, 8.4 Hz, 1H), 6.53 (d, *J* = 15.2 Hz, 1H), 4.07 (s, 3H), 3.97 (s, 3H), 3.19-3.11 (m, 1H), 2.92 (q, *J* = 8.3 Hz, 1H), 2.38-2.29 (m, 4H), 2.18-2.07 (m, 1H), 1. 95-1. 85 (m, 2H), 1.82-1.70 (m, 1H); MS (ESI): m/z 546 [M+1]⁺ | 39 | 8.73 99 (b) |
| 306 | 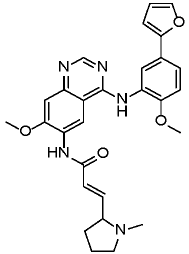 | (R,E)-N-(4-((5-(furan-2-yl)-2-methoxyphenyl )amino)-7-methoxyquinaz olin-6-yl)-3-(1-methylpyrroli din-2-yl) acrylamide | ¹H NMR (400 MHz, TFA salt, Methanol-*d*₄) δ 9.09 (s, 1H), 8.50 (s, 1H), 8.37 (d, *J* = 2.0 Hz, 1H), 7.59-7.56 (m, 1H), 7.53 (d, *J* = 1.2 Hz, 1H), 7.27 (s, 1H), 7.17 (d, *J* = 8.8 Hz, 1H), 6.96-6.90 (m, 1H), 6.86 (d, *J* = 15.2 Hz, 1H), 6.67 (d, *J* = 3.6 Hz, 1H), 6.51-6.50 (m, 1H), 4.11-4.09 (m, 4H), 3.96 (s, 3H), 3.49-3.48 (m, 1H), 2.90 (s, 3H), 2.46-2.44 (m, 1H), 2.24-2.09 (m, 4H); MS (ESI): m/z 500 [M+H]⁺ | 51 | 8.01 99 (b) |
| 307 | 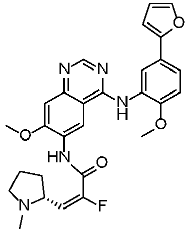 | (R,E)-2-fluoro-N-(4-((5-(furan-2-yl)-2-methoxyphenyl )amino)-7-methoxyquinaz olin-6-yl)-3-(1-methylpyrroli din-2-yl)acrylamide | ¹H NMR (400 MHz, free base, Methanol-*d*₄) δ 8.96 (s, 1H), 8.48 (s, 1H), 8.42 (d, *J* = 2.0 Hz, 1H), 7.57-7.54 (m, 2H), 7.27 (s, 1H), 7.16 (d, *J* = 8.4 Hz, 1H), 6.68-6.67 (m, 1H), 6.52-6.51 (m, 1H), 6.27-6.16 (m, 1H), 4.12 (s, 3H), 3.97 (s, 3H), 3.36-3.35 (m, 1H), 3.17-3.15 (m, 1H), 2.40-2.39 (m, 4H), 2.17-2.16 (m, 1H), 1.95-1.93 (m, 2H), 1.88-1.87 (m, 1H); MS (ESI): m/z 526 [M+H]⁺ | 47 | 7.99 99 (1H NMR) (b) |
| 308 | 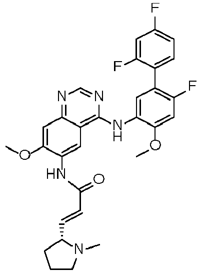 | (R,E)-N-(7-methoxy-4-((2',4',6-trifluoro-4-methoxy-[1,1'-biphenyl]-3-yl)amino) quin azolin-6-yl)-3-(1-methylpyrroli din-2-yl)acrylamide | ¹H NMR (400MHz, HCl salt, Methanol-*d*₄) δ 9.24 (s, 1H), 8.60 (s, 1H), 7.64 (d, *J* = 7.6 Hz, 1H), 7.49-7.43 (m, 1H), 7.30 (s, 1H), 7.13 (s, 1H), 7.10-7.05 (m, 2H), 7.04-6.84 (m, 2H), 4.17-4.10 (m, 4H), 3.92 (s, 3H), 3.80-3.79 (m, 1H), 2.95 (s, 3H), 2.49-2.47 (m, 1H), 2.29-2.07 (m, 3H); MS (ESI): m/z 564 [M+1]⁺ | 60 | 8.91 100 (b) |
| 309 | 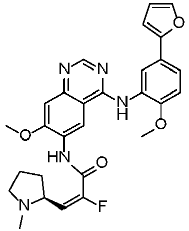 | (S,E)-2-fluoro-N-(4-((5-(furan-2-yl)-2-methoxyphenyl )amino)-7-methoxyquinaz olin-6-yl)-3-(1-methylpyrroli din-2-yl)acrylamide | ¹H NMR (400 MHz, HCl salt, Methanol-*d*₄) δ 9.19 (s, 1H), 8.69 (s, 1H), 7.91 (s, 1H), 7.77-7.74 (m, 1H), 7.55 (s, 1H), 7.38 (s, 1H), 7.26 (d, *J* = 8.8 Hz, 1H), 6.71 (d, *J* = 3.2 Hz, 1H), 6.53-6.32 (m, 2H), 4.52-4.50 (m, 1H), 4.12 (s, 3H), 3.92 (s, 3H), 3.84-3.82 (m, 1H), 2.99 (s, 3H), 2.57-2.55 (m, 1H), 2.49-2.40 (m, 4H); MS (ESI): m/z 518 [M+H]⁺ | 91 | 8.07 99 (b) |
| 310 | 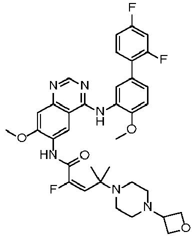 | (E)-N-(4-((2',4'-difluoro-4-methoxy-[1,1'-biphenyl]-3-yl)amino)-7-methoxyquinaz olin-6-yl)-2-fluoro-4-methyl-4-(4-(oxetan-3-yl)piperazin-1-yl)pent-2-enamide | MS (ESI): m/z 663 [M+1]⁺ | | |
| 311 | 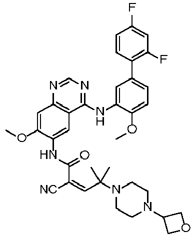 | (Z)-2-cyano-N-(4-((2',4'-difluoro-4-methoxy-[1,1'-biphenyl]-3-yl)amino)-7-methoxyquinaz olin-6-yl)-4-methyl-4-(4-(oxetan-3-yl)piperazin-1-yl)pent-2-enamide | MS (ESI): m/z 670 [M+1]⁺ | | |
| 312 | 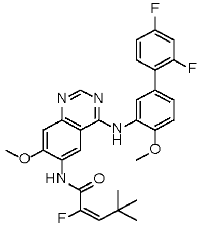 | (E)-N-(4-((2'4'-difluoro-4-methoxy-[1,1'-biphenyl]-3-yl)amino)-7-methoxyquinaz olin-6-yl)-2-fluoro-4,4-dimethylpent-2-enamide | MS (ESI): m/z 537 [M+1]⁺ | | |
| 313 | 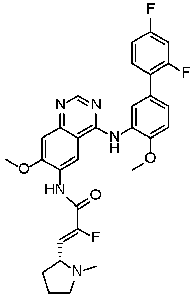 | (R,Z)-N-(4-((2'4'-difluoro-4-methoxy-[1,1'-biphenyl]-3-yl)amino)-7-methoxyquinaz olin-6-yl)-2-fluoro-3-(1-methylpyrroli din-2-yl)acrylamide | MS (ESI): m/z 564 [M+1]⁺ | | |

### Example 314. (E)-N-(4-((5-(furan-2-yl)-2-methoxyphenyl)amino)-7-methoxyquinazolin-6-yl)-4-(4-methylpiperazin-1-yl)but-2-enamide

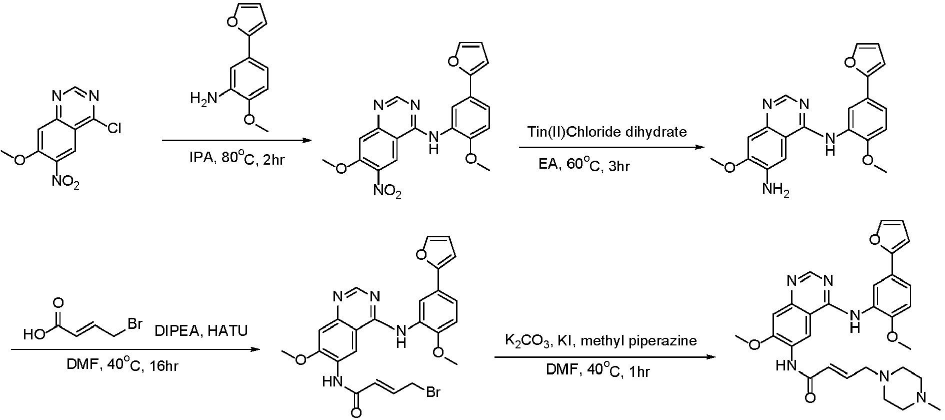

### [Step-1] Preparation of N-(5-(furan-2-yl)-2-methoxyphenyl)-7-methoxy-6-nitroquinazolin-4-amine

In isopropyl alcohol (0.1 M), 4-chloro-7-methoxy-6-nitroquinazoline (1.0 equiv.) and 5-(furan-2-yl)-2-methoxyaniline (1.1 equiv.) were dissolved, and stirred at 80 °C for 2 hours. The reaction mixture was cooled to room temperature, then the obtained solid was filtered and washed with ethyl ether. The target compound obtained as a yellow solid was used in the next reaction without further purification (yield: 96%, MS (ESI): m/z 393 [M+1]⁺).

### [Step-2] Preparation of N⁴-(5-(furan-2-yl)-2-methoxyphenyl)-7-methoxyquinazolin-4,6-diamine

In EA (0.1 M), N-(5-(furan-2-yl)-2-methoxyphenyl)-7-methoxy-6-nitroquinazolin-4-amine (1.0 equiv.) obtained in Step-1 above was dissolved, and tin(II) chloride dihydrate (6.0 equiv.) was added and stirred at 60 °C for 3 hours. The reaction mixture was cooled to room temperature, and aqueous ammonia was added until the reaction solution turned white. The reaction mixture was concentrated and purified by MPLC (DCM:MeOH) to obtain the target compound as a white solid (yield: 68%, MS (ESI): m/z 363 [M+1]⁺).

### [Step-3] Preparation of (E)-4-bromo-N-(4-((5-(furan-2-yl)-2-methoxyphenyl)amino)-7-methoxyquinazolin-6-yl)but-2-enamide

In DMF (0.2 M), N⁴-(5-(furan-2-yl)-2-methoxyphenyl)-7-methoxyquinazolin-4,6-diamine (1.0 equiv.) obtained in Step-2 above, (E)-4-bromobut-2-enoic acid (2.0 equiv.), DIPEA (4.5 equiv.), and HATU (3.0 equiv.) were dissolved, and the mixture was stirred at 40 °C for 16 hours. Water and brine were added to the mixture, organic materials were extracted with DCM, and the collected organic layer was concentrated by removing remaining water using Na₂SO₄. The reaction mixture was purified by MPLC (DCM:MeOH) to obtain the target compound as a yellow solid (yield: 7%, MS (ESI): m/z 510 [M+1]⁺).

### [Step-4] Preparation of (E)-N-(4-((5-(furan-2-yl)-2-methoxyphenyl)amino)-7-methoxyquinazolin-6-yl)-4-(4-methylpiperazin-1-yl)but-2-enamide

In DMF (0.1 M), (E)-4-bromo-N-(4-((5-(furan-2-yl)-2-methoxyphenyl)amino)-7-methoxyquinazolin-6-yl)but-2-enamide obtained in Step-3 above was dissolved, then K₂CO₃ (2.3 equiv.), KI (1.8 equiv.), and 1-methylpiperazine (4.0 equiv.) were added and stirred at 40 °C for 1 hour. Water and brine were added to the mixture, organic materials were extracted with DCM, and the collected organic layer was concentrated by removing remaining water using MgSO₄. The reaction mixture was purified by prep-HPLC to obtain the target compound as a white solid (yield: 19%, MS (ESI): m/z 529 [M+1]⁺).

### Preparation of Compounds of Examples 315 to 319

Compounds of Examples 315 to 319 were prepared in a similar manner to Example 314 above. Chemical structures, compound names, ¹H NMR, MS, HPLC data and yields of respective Examples are summarized in Table 10 below.

**[Table 10]**

| Exa mpl e No. | Structure | Compound Name | ¹H NMR; MS | Yie ld (%) | HPLC r.t. (min ) Purity(% ) (method) |
|---|---|---|---|---|---|
| 314 | 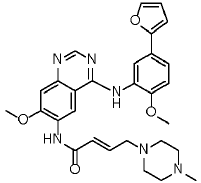 | (E)-N-(4-((5-(furan-2-yl)-2-methoxyphenyl )amino)-7-methoxyquinaz olin-6-yl)-4-(4-methylpiperaz in-1-yl)but-2-enamide | ¹H NMR (400MHz, TFA salt, Methanol-*d*₄) δ 9.02 (s, 1H), 8.46 (s, 1H), 8.41 (d, *J* = 2.0 Hz, 1H), 7.56-7.52 (m, 2H), 7.23 (s, 1H), 7.15 (d, *J* = 8.8 Hz, 1H), 6.99-6.94 (m, 1H), 6.67 (d, *J* = 3.2 Hz, 1H), 6.61 (d, *J* = 15.6 Hz, 1H), 6.50-6.49 (m, 1H), 4.08 (s, 3H), 3.96 (s, 3H), 3.07-3.05 (m, 4H), 2.69-2.60 (m, 7H); MS (ESI): m/z 529 [M+1]⁺ | 19 | 7.85 96 (b) |
| 315 | 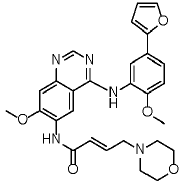 | (E)-N-(4-((5-(furan-2-yl)-2-methoxyphenyl )amino)-7-methoxyquinaz olin-6-yl)-4-morpholinobut -2-enamide | ¹H NMR (400 MHz, TFA salt, Methanol-*d*₄) δ 9.32 (s, 1H), 8.66 (s, 1H), 7.79 (s, 1H), 7.62-7.54 (m, 2H), 7.33-7.30 (m, 2H), 7.12-7.01 (m, 3H), 6.89 (d, *J* = 15.2 Hz, 1H), 4.20-3.95 (m, 12H), 3.51-3.49 (m, 4H); MS (ESI): m/z 562 [M+H]⁺ | 29 | 8.72 100 (b) |
| 316 | 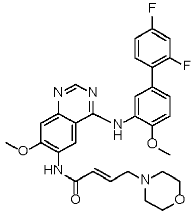 | (E)-N-(4-((2',4'-difluoro-4-methoxy-[1,1'-biphenyl]-3-yl)amino)-7-methoxyquinaz olin-6-yl)-4-morpholinobut -2-enamide | ¹H NMR (400 MHz, TFA salt, Methanol-*d*₄) δ 9.32 (s, 1H), 8.66 (s, 1H), 7.79 (s, 1H), 7.62-7.54 (m, 2H), 7.33-7.30 (m, 2H), 7.12-7.01 (m, 3H), 6.89 (d, *J* = 15.2 Hz, 1H), 4.20-3.95 (m, 12H), 3.51-3.49 (m, 4H); MS (ESI): m/z 562 [M+H]⁺ | 29 | 8.72 100 (b) |
| 317 | 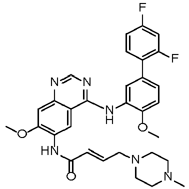 | (E)-N-(4-((2',4'-difluoro-4-methoxy-[1,1'-biphenyl]-3-yl)amino)-7-methoxyquinaz olin-6-yl)-4-(4-methylpiperaz in-1-yl)but-2-enamide | ¹H NMR (400 MHz, TFA salt, Methanol-*d*₄) δ 9.29 (s, 1H), 8.65 (s, 1H), 7.79 (s, 1H), 7.61-7.56 (m, 2H), 7.33-7.29 (m, 2H), 7.11-7.01 (m, 3H), 6.69 (d, *J* = 15.2 Hz, 1H), 4.19 (s, 3H), 3.98 (s, 3H), 3.48-3.46 (m, 6H), 2.99-2.96 (m, 7H); MS (ESI): m/z 575 [M+H]⁺ | 19 | 8.64 100 (b) |
| 318 | 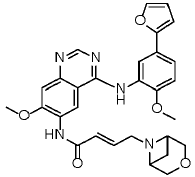 | (E)-4-(3-oxa-6-azabicyclo[3. 1.1]heptan-6-yl)-N-(4-((5-(furan-2-yl)-2-methoxyphenyl )amino)-7-methoxyquinaz olin-6-yl)but-2-enamide | ¹H NMR (400 MHz, FA salt, Methanol-*d*₄) δ 9.01 (s, 1H), 8.46 (s, 1H), 8.43-8.37 (m, 2H), 7.55-7.50 (m, 2H), 7.21 (s, 1H), 7.13 (d, *J* = 8.6 Hz, 1H), 7.02-6.93 (m, 1H), 6.69-6.61 (m, 2H), 6.51-6.48 (m, 1H), 4.32 (d, *J* = 11.6 Hz, 2H), 4.08 (s, 3H), 4.00-3.84 (m, 9H), 3.01-2.84 (m, 1H), 2.10-2.01 (m, 1H); MS (ESI): m/z 528 [M+1]⁺ | 8.1 9 | 7.97 100 (b) |
| 319 | 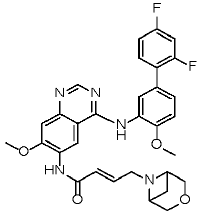 | (E)-4-(3-oxa-6-azabicyclo[3. 1.1]heptan-6-yl)-N-(4-((2',4'-difluoro-4-methoxy-[1,1')-biphenyl]-3-yl)amino)-7-methoxyquinaz olin-6-yl)but-2-enamide | ¹H NMR (400 MHz, FA salt, Methanol-*d*₄) δ 9.06 (s, 1H), 8.44 (s, 1H), 8.26 (s, 3H), 8.22 (s, 1H), 7.57-7.52 (m, 1H), 7.41-7.38 (m, 1H), 7.26-7.25 (d, *J* = 2.2 Hz, 1H), 7.28-7.19 (dd, *J* = 8.4 Hz, 1H), 7.06-6.93 (m, 3H), 6.76-6.72 (d, *J* = 16.32 Hz, 1H), 4.39-4.35 (dd, *J* = 12.02 Hz, 2H), 4.24-3.91 (m, 12H), 2.98 (s, 1H), 2.20 (s, 1H); MS (ESI): m/z 574 [M+1]⁺ | 19 | 8.75 99 (b) |

### Example 320. Preparation of (E)-N-(5-chloro-4-((2',4'-difluoro-4-methoxy-[1,1'-biphenyl]-3-yl)amino)quinazolin-6-yl)-4-(dimethylamino)but-2-enamide

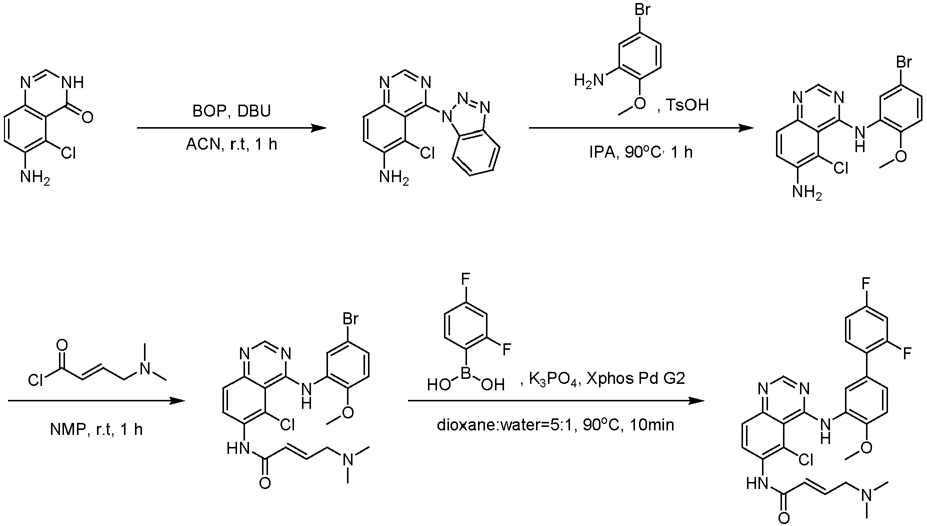

### [Step-1] Preparation of 4-(1H-benzo[d][1,2,3]triazol-1-yl)-5-chloroquinazolin-6-amine

In acetonitrile (0.1 M), 6-amino-5-chloroquinazolin-4(3H)-one (1.0 equiv.) and BOP (1.5 equiv.) were dissolved, then DBU (2.0 equiv.) was added and stirred for 1 hour at room temperature. The reaction mixture was concentrated and purified by MPLC (DCM:MeOH) to obtain the target compound as a yellow solid (yield: 100%, MS (ESI): m/z 296 [M+1]⁺).

### [Step-2] Preparation of N⁴-(5-bromo-2-methoxyphenyl)-5-chloroquinazolin-4,6-diamine

In isopropyl alcohol, 4-(1H-benzo[d][1,2,3]triazol-1-yl)-5-chloroquinazolin-6-amine (1.0 equiv.) obtained in Step-1 above and 5-bromo-2-methoxyaniline (1.2 equiv.) were dissolved, and stirred at 90 °C for 10 minutes, and then p-toluenesulfonic acid monohydrate (0.1 equiv.) was added and stirred at 90 °C for 1 hour. The reaction mixture was cooled to room temperature and water was added to form a solid. The resulting solid was filtered through a filter to obtain the target compound as a yellow solid (yield: 39%, MS (ESI): m/z 379 [M+1]⁺).

### [Step-3] Preparation of (E)-N-(4-((5-bromo-2-methoxyphenyl)amino)-5-chloroquinazolin-6-yl)-4-(dimethylamino)but-2-enamide

In NMP (0.1 M), N⁴-(5-bromo-2-methoxyphenyl)-5-chloroquinazolin-4,6-diamine (1.0 equiv.) obtained in Step-2 above was dissolved, then (E)-4-(dimethylamino)but-2-enoyl chloride (1.0 equiv.) was added under nitrogen and stirred at room temperature for 1 hour. The reaction mixture was concentrated and purified by MPLC (DCM:MeOH) to obtain the target compound as a yellow solid (yield: 44%, MS (ESI): m/z 490 [M+1]⁺).

### [Step-4] Preparation of (E)-N-(5-chloro-4-((2',4'-difluoro-4-methoxy-[1,1'-biphenyl]-3-yl)amino)quinazolin-6-yl)-4-(dimethylamino)but-2-enamide

In dioxane: water (5:1, 0.1 M), (E)-N-(4-((5-Bromo-2-methoxyphenyl)amino)-5-chloroquinazolin-6-yl)-4-(dimethylamino)but-2-enamide (1.0 equiv.) obtained in Step-3 above, (2,4-difluorophenyl)boronic acid (2.2 equiv.), and K₃PO₄ (2.0 equiv.) were dissolved. The reaction mixture was degassed using nitro gas, and Xphos Pd G2 (0.13 equiv.) was added at 80 °C, followed by stirring at 90 °C for 10 minutes. The reaction mixture was filtered through a celite filter, concentrated, and purified by prep-HPLC to obtain the target compound as a yellow solid (yield: 8%, MS (ESI): m/z 524 [M+1]⁺).

### Preparation of Compound of Example 321

A compound of Example 321 according to the present invention was prepared in a similar manner to Example 320 above. Chemical structures, compound names, ¹H NMR, MS, HPLC data and yields of respective Examples are summarized in Table 11 below.

**[Table 11]**

| Exa mpl e No. | Structure | Compound Name | ¹H NMR; MS | Yie ld (%) | HPLC r.t. (min ) Purity(% ) (method) |
|---|---|---|---|---|---|
| 320 | 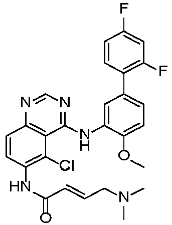 | (E)-N-(5-chloro-4-((2',4'-difluoro-4-methoxy-[1,1'-biphenyl]-3-yl) amino) quin azolin-6-yl)-4-(dimethylamin o)but-2-enamide | ¹H NMR (400 MHz, FA salt, Methanol-*d*₄) δ 9.01 (s, 1H), 8.65 (s, 1H), 8.20 (d, *J* = 9.2 Hz, 1H), 7.81 (d, *J* = 8.8 Hz, 1H), 7.58-7.53 (m, 1H), 7.35-7.32 (m, 1H), 7.21 (d, *J* = 8.4 Hz, 1H), 7.09-7.04 (m, 3H), 6.98-6.60 (m, 1H), 4.05 (s, 3H), 3.63 (d, *J* = 6.8 Hz, 2H), 2.68 (s, 6H); MS (ESI): m/z 524 [M+H]⁺ | 8 | 9.61 98 (b) |
| 321 | 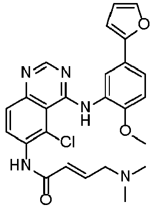 | (E)-N-(5-chloro-4-((5-(furan-2-yl)-2-methoxyphenyl ) amino) quinaz olin-6-yl)-4-(dimethylamin o)but-2-enamide | ¹H NMR (400 MHz, TFA salt, DMSO-*d*₆) δ 10.40 (s, 1H), 10.31 (s, 1H), 8.93 (s, 1H), 8.76 (s, 1H), 8.15 (d, *J* = 9.2 Hz, 1H), 7.86 (d, *J* = 9.2 Hz, 1H), 7.75 (s, 1H), 7.55-7.53 (m, 1H), 7.25 (d, *J* = 2.0 Hz, 1H), 6.88-6.81 (m, 2H), 6.71-6.59 (m, 2H), 4.00-3.99 (m, 5H), 2.91 (s, 6H); MS (ESI): m/z 478 [M+H]⁺ | 21 | 8.86 100 (b) |

### Example 322. Preparation of 1-(2-(4-((2',4'-difluoro-4-methoxy-[1,1'-biphenyl]-3-yl)amino)-7-methoxyquinazolin-6-yl)-2,6-diazaspiro[3.5]nonan-6-yl)prop-2-en-1-one

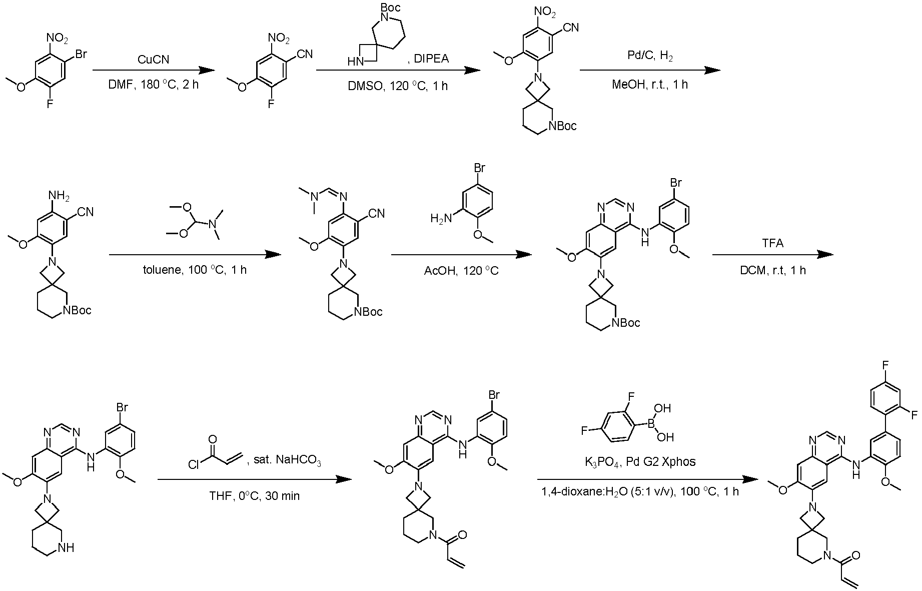

### [Step-1] Preparation of 5-fluoro-4-methoxy-2-nitrobenzonitrile

In DMF (0.2 M), 1-bromo-5-fluoro-4-methoxy-2-nitrobenzene (1.0 equiv.) and CuCN (1.5 equiv.) were dissolved, and stirred at 180 °C for 2 hours. Ice water was added to the reaction mixture to produce a solid, and the formed solid was filtered to obtain the target compound as a yellow solid (yield: 90%).

### [Step-2] Preparation of tert-butyl 2-(5-cyano-2-methoxy-4-nitrophenyl)-2,6-diazaspiro[3.5]nonane-6-carboxylate

In DMSO (0.1 M), 5-fluoro-4-methoxy-2-nitrobenzonitrile (1 equiv.) obtained in Step-1 above, tert-butyl 2,6-diazaspiro[3.5]nonane-6-carboxylate (1.0 equiv.), and DIPEA (4.0 equiv.) were dissolved, and stirred at 120 °C for 1 hour. Water was added to the reaction mixture to form a solid compound, and the solid compound was filtered through a filter to obtain the target compound as a yellow solid (yield: 51%, MS (ESI): m/z 347 [M+1]⁺).

### [Step-3] Preparation of tert-butyl 2-(4-amino-5-cyano-2-methoxyphenyl)-2,6-diazaspiro[3.5]nonane-6-carboxylate

In MeOH (0.1 M), tert-butyl 2-(5-cyano-2-methoxy-4-nitrophenyl)-2,6-diazaspiro[3.5]nonane-6-carboxylate (1.0 equiv.) obtained in Step-2 above was dissolved, then Pd/C (10% purity, 0.1 equiv.) was added, and the reaction mixture was stirred at room temperature for 1 hour under hydrogen. The stirred mixture was filtered through celite, and the filtrate was concentrated. The target compound obtained as a yellow solid was used in the next reaction without further purification (yield: 98%, MS (ESI): m/z 373 [M+1]⁺).

### [Step-4] Preparation of tert-butyl (Z)-2-(5-cyano-4-(((dimethylamino)methylene)amino)-2-methoxyphenyl)-2,6-diazaspiro[3.5]nonane-6-carboxylate

In toluene (0.1 M), tert-butyl 2-(4-amino-5-cyano-2-methoxyphenyl)-2,6-diazaspiro[3.5]nonane-6-carboxylate (1 equiv.) obtained in Step-3 above and N,N-dimethylformamide dimethylacetal (5.0 equiv.) were dissolved, and the mixture was stirred at 100 °C for 1 hour and then concentrated. The reaction mixture was purified by MPLC (DCM:MeOH) to obtain the target compound as a yellow solid (yield: 81%, MS (ESI): m/z 428 [M+1]⁺).

### [Step-5] Preparation of tert-butyl 2-(4-((5-bromo-2-methoxyphenyl)amino)-7-methoxyquinazolin-6-yl)-2,6-diazaspiro[3.5]nonane-6-carboxylate

In acetic acid (0.1 M), tert-butyl (Z)-2-(5-cyano-4-(((dimethylamino)methylene)amino)-2-methoxyphenyl)-2,6-diazaspiro[3.5]nonane-6-carboxylate (1.0 equiv.) obtained in Step-4 above and 5-bromo-2-methoxyaniline (1.1 equiv.) were dissolved, and stirred at 120 °C for 1 hour. After cooling the mixture to room temperature, saturated NaHCO₃ aqueous solution was added to the reaction mixture to a pH of 8, and the organic layer was extracted with DCM. The collected organic layer was concentrated by removing remaining water using MgSO₄. The target compound obtained as a brown solid was used in the next reaction without further purification (yield: 30%, MS (ESI): m/z 584 [M+1]⁺).

### [Step-6] Preparation of N-(5-bromo-2-methoxyphenyl)-7-methoxy-6-(2,6-diazaspiro[3.5]nonan-2-yl)quinazolin-4-amine

In DCM, tert-butyl 2-(4-((5-bromo-2-methoxyphenyl)amino)-7-methoxyquinazolin-6-yl)-2,6-diazaspiro[3.5]nonane-6-carboxylate (1.0 equiv.) obtained in Step-5 above was dissolved and an excess of TFA was added. The reaction mixture was stirred at room temperature for 1 hour. The desired product was confirmed through LC-MS, concentrated, and purified by MPLC (DCM:MeOH) to obtain the target compound as a white solid (yield: 100%, MS (ESI): m/z 484 [M+1]⁺).

### [Step-7] Preparation of 1-(2-(4-((5-bromo-2-methoxyphenyl)amino)-7-methoxyquinazolin-6-yl)-2,6-diazaspiro[3.5]nonan-6-yl)prop-2-en-1-one

In THF (0.1 M), N-(5-bromo-2-methoxyphenyl)-7-methoxy-6-(2,6-diazaspiro[3.5]nonan-2-yl)quinazolin-4-amine (1.0 equiv.) obtained in Step-6 above and saturated NaHCO₃ aqueous solution (5.0 equiv.) were dissolved, and then acryloyl chloride (1.0 equiv.) was added at 0 °C and stirred for 30 minutes. The reaction mixture was concentrated, water was added, and the organic layer was extracted with DCM. The collected organic layer was concentrated by removing remaining water using MgSO₄. The target compound obtained as a white solid was used in the next reaction without further purification (yield: 42%, MS (ESI): m/z 538 [M+1]⁺).

### [Step-8] Preparation of 1-(2-(4-((2',4'-difluoro-4-methoxy-[1,1'-biphenyl]-3-yl)amino)-7-methoxyquinazolin-6-yl)-2,6-diazaspiro[3.5]nonan-6-yl)prop-2-en-1-one

In dioxane:water (5:1, 0.006 M), 1-(2-(4-((5-bromo-2-methoxyphenyl)amino)-7-methoxyquinazolin-6-yl)-2,6-diazaspiro[3.5]nonan-6-yl)prop-2-en-1-one (1.0 equiv.) obtained in Step-7 above, (2,4-difluorophenyl)boronic acid (1.1 equiv.), and K₃PO₄ (2.5 equiv.) were dissolved. The reaction mixture was degassed using nitro gas, and Xphos Pd G2 (0.1 equiv.) was added to the mixture at 80 °C, followed by stirring at 100 °C for 1 hour. The mixture was filtered through celite, and the filtrate was concentrated and purified using prep-HPLC to obtain the target compound as a yellow solid (yield: 64%, MS (ESI): m/z 572 [M+1]⁺).

### Preparation of Compounds of Examples 323 to 330

Compounds of Examples 323 to 330 were prepared in a similar manner to Example 322 above. Chemical structures, compound names, ¹H NMR, MS, HPLC data and yields of respective Examples are summarized in Table 12 below.

**[Table 12]**

| Example No. | Structure | Compound Name | ¹H NMR; MS | Yie ld (%) | HPLC r.t. (min ) Purity(% ) (method) |
|---|---|---|---|---|---|
| 322 | 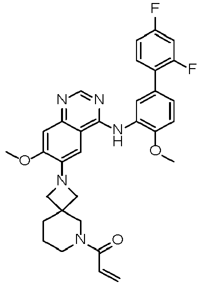 | 1-(2-(4-((2',4'-difluoro-4-methoxy-[1,1'-biphenyl]-3-yl)amino)-7-methoxyquinaz olin-6-yl)-2,6-diazaspiro[3. 5]nonan-6-yl)prop-2-en-1-one | ¹H NMR (400MHz, FA salt, Methanol-*d*₄) δ 8.19 (s, 1H), 7.92-7.90 (m, 1H), 7.82 (s, 1H), 7.63-7.61 (m, 1H), 7.51-7.48 (m, 1H), 7.31 (s, 1H), 7.16-7.09 (m, 3H), 7.00-6.79 (m, 1H), 6.32-6.19 (m, 1H), 5.80-5.75 (m, 1H), 4.05 (s, 3H), 3.98 (s, 3H), 3.97-3.96 (m, 2H), 3.93-3.87 (m, 4H), 3.85-3.37 (m, 2H), 1.98-1.96 (m, 2H), 1.75-1.68 (m, 2H); MS (ESI): m/z 572 [M+1]⁺ | 64 | 11.35 100 (b) |
| 323 | 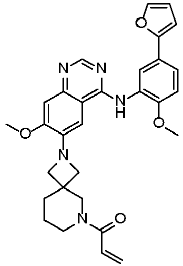 | 1-(2-(4-((5-(furan-2-yl)- 2-methoxyphenyl )amino)-7-methoxyquinaz olin-6-yl)-2,6-diazaspiro[3. 5]nonan-6-yl)prop-2-en-1-one | ¹H NMR (400MHz, FA salt, Methanol-*d*₄) δ 8.08 (s, 1H), 7.96-7.94 (m, 1H), 7.86-7.85 (m, 1H), 7.50-7.49 (m, 1H), 7.35-7.33 (m, 1H), 7.21 (s, 1H), 7.06 (s, 1H), 6.90-6.80 (m, 1H), 6.73-6.72 (m, 1H), 6.46-6.45 (m, 1H), 6.25-6.08 (m, 1H), 5.78-5.65 (m, 1H), 3.96 (s, 3H), 3.88-3.75 (m, 9H), 3.56-3.55 (m, 2H), 1.88-1.87 (m, 2H), 1. 63-1.50 (m, 2H); MS (ESI): m/z 526 [M+1]⁺ | 26 | 10.71 100 (b) |
| 324 | 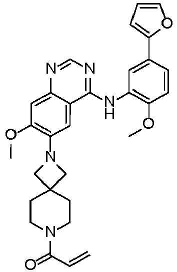 | 1-(2-(4-((5-(furan-2-yl)- 2-methoxyphenyl )amino)-7-methoxyquinaz olin-6-yl)-2,7-diazaspiro[3. 5]nonan-7-yl)prop-2-en-1-one | ¹H NMR (400 MHz, TFA salt, Methanol-*d*₄) δ 8.49 (s, 1H), 7.86 (s, 1H), 7.73 (d, *J* = 2.4 Hz, 1H), 7.50 (s, 1H), 7.24-7.21 (m, 2H), 7.11 (s, 1H), 6.82-6.80 (m, 1H), 6.70-6.69 (m, 1H), 6.53-6.52 (m, 1H), 6.25-6.20 (m, 1H), 5.79-5.76 (m, 1H), 4.06 (s, 3H), 4.01 (s, 4H), 3.90 (s, 3H), 3.70-3.69 (m, 4H), 1.94-1.91 (m, 4H); MS (ESI): m/z 526 [M+H]⁺ | 51 | 10.56 100 (b) |
| 325 | 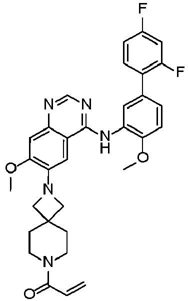 | 1-(2-(4-((2',4'-difluoro-4-methoxy-[1,1'-biphenyl]-3-yl)amino)-7-methoxyquinaz olin-6-yl)-2,7-diazaspiro[3. 5]nonan-7-yl)prop-2-en-1-one | ¹H NMR (400 MHz, TFA salt, Methanol-*d*₄) δ 8.48 (s, 1H), 7.72-7.71 (m, 1H), 7.60-7.52 (m, 2H), 7.30 (d, *J =* 8.8 Hz, 1H), 7.22 (s, 1H), 7.11-7.04 (m, 3H), 6.85-6.79 (m, 1H), 6.25-6.20 (m, 1H), 5.79-5.76 (m, 1H), 4.06 (s, 3H), 4.01 (s, 4H), 3.99 (s, 3H), 3.69-3.68 (m, 4H), 1.94-1.91 (m, 4H); MS (ESI): m/z 572 [M+H]⁺ | 26 | 11.15 100 (b) |
| 326 | 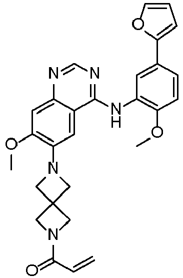 | 1-(6-(4-((5-(furan-2-yl)-2-methoxyphenyl )amino)-7-methoxyquinaz olin-6-yl)-2,6-diazaspiro[3. 3]heptan-2-yl)prop-2-en-1-one | ¹H NMR (400 MHz, FA salt, Methanol-*d*₄) δ 8.31 (s, 1H), 8.11 (s, 1H), 7.62 (d, *J* = 8.0 Hz, 1H), 7.53 (s, 1H), 7.18 (d, *J* = 8.8 Hz, 1H), 7.10 (s, 1H), 7.07 (s, 1H), 6.68 (d, *J* = 3.2 Hz, 1H), 6.51-6.50 (m, 1H), 6.38-6.29 (m, 2H), 5.79 (d, *J* = 11.2 Hz, 1H), 4.54 (s, 2H), 4.28 (s, 2H), 4.26 (s, 4H), 4.01 (s, 3H), 3.92 (s, 3H); MS (ESI): m/z 498 [M+H]⁺ | 34 | 9.86 100 (b) |
| 327 | 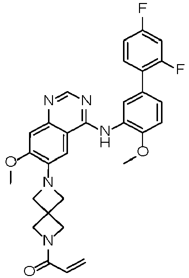 | 1-(6-(4-((2',4'-difluoro-4-methoxy-[1,1'-biphenyl]-3-yl)amino)-7-methoxyquinaz olin-6-yl)-2,6-diazaspiro [3. 3]heptan-2-yl)prop-2-en-1-one | ¹H NMR (400 MHz, FA salt, Methanol-*d*₄) δ 8.30 (s, 1H), 7.92 (s, 1H), 7.56-7.54 (m, 1H), 7.47 (d, *J* = 8.8 Hz, 1H), 7.24 (d, *J* = 8.4 Hz, 1H), 7.09-7.03 (m, 4H), 6.37-3.29 (m, 2H), 5.79-5.76 (m, 1H), 4.54 (s, 2H), 4.28 (s, 2H), 4.26 (s, 4H), 4.01 (s, 3H), 3.94 (s, 3H); MS (ESI): m/z 498 [M+H]⁺ | 34 | 10.64 100 (b) |
| 328 | 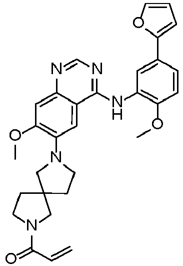 | 1-(7-(4-((5-(furan-2-yl)-2-methoxyphenyl )amino)-7-methoxyquinaz olin-6-yl)-2,7-diazaspiro[4. 4]nonan-2-yl)prop-2-en-1-one | ¹H NMR (400 MHz, TFA salt, Methanol-*d*₄) δ 8.39 (s, 1H), 7.74 (s, 1H), 7.65-7.62 (m, 1H), 7.46-7.44 (m, 1H), 7.21 (s, 1H), 7.14 (d, *J* = 8.8 Hz, 1H), 7.06 (d, *J* = 1.6 Hz, 1H), 6.60-6.53 (m, 2H), 6.43-6.42 (m, 1H), 6.22-6.21 (m, 1H), 5.72-5.71 (m, 1H), 3.99 (s, 3H), 3.77 (s, 3H), 3.69-3.25 (m, 10H), 2.08-1.94 (m, 2H); MS (ESI): m/z 526 [M+H]⁺ | 21 | 10.41 95 (b) |
| 329 | 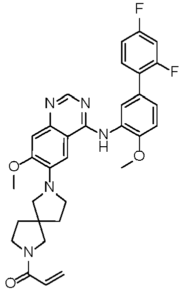 | 1-(7-(4-((2',4'-difluoro-4-methoxy-[1,1'-biphenyl]-3-yl)amino)-7-methoxyquinaz olin-6-yl)-2,7-diazaspiro[4. 4]nonan-2-yl)prop-2-en-1-one | ¹H NMR (400 MHz, TFA salt, Methanol-*d*₄) δ 8.25 (s, 1H), 7.91 (s, 1H), 7.57-7.55 (m, 1H), 7.46-7.43 (m, 1H), 7.24-7.21 (m, 2H), 7.13 (s, 1H), 7.08-7.03 (m, 2H), 6.67-6.60 (m, 1H), 6.33-6.28 (m, 1H), 5.79-5.74 (m, 1H), 4.01 (s, 3H), 3.93 (s, 3H), 3.80-3.62 (m, 4H), 3.52-3.49 (m, 4H), 2.09-2.04 (m, 4H); MS (ESI): m/z 572 [M+H]⁺ | 11 | 11.13 97 (b) |
| 330 | 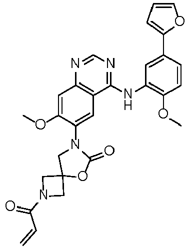 | 2-acryloyl-7-(4-((5-(furan-2-yl)-2-methoxyphenyl )amino)-7-methoxyquinaz olin-6-yl)-5-oxa-2,7-diazaspiro[3. 4]octan-6-one | ¹H NMR (400MHz, FA salt, Methanol-*d*₄) δ 8.48 (s, 1H), 8.38 (s, 1H), 8.21-8.20 (m, 1H), 7.61-7.60 (m, 1H), 7.54-7.53 (m, 1H), 7.33 (s, 1H), 7.19-7.17 (m, 1H), 6.69-6.68 (m, 1H), 6.52-6.41 (m, 1H), 6.38-6.34 (m, 2H), 5.84-5.81 (m, 1H), 4.72-4.70 (m, 2H), 4.45-4.44 (m, 2H), 4.37-4.36 (m, 2H), 3.93 (s, 3H), 3.35 (s, 3H); MS (ESI): m/z 528 [M+1]⁺ | 6 | 5.3 98 (a) |

| | | | | | |
|---|---|---|---|---|---|
| (*: wherein "*" marked to the right side of the numerical value in the last column means NMR purity) | | | | | |

### <Experimental Example 1> Evaluation of HER2, EGFR enzyme inhibitory activity

Biochemical kinase assays for HER2 and EGFR were performed by Reaction Biology Corp (San Diego, USA). Compounds were diluted 3-fold from 10 µM under the condition of ATP (10 µM) for HER2 and EGFR enzymes, and IC₅₀ values were determined in 10-dose. Results thereof are shown in Table 13 below.

**[Table 13]**

| Example No. | HER2 | EGFR |
|---|---|---|
| 68 | A | A |
| 80 | A | - |
| 90 | A | - |
| 157 | A | - |
| 304 | A | A |
| 305 | A | A |
| 306 | A | A |

| | | |
|---|---|---|
| (A: GI₅₀ ≤ 50 nM, B: 50 < GI₅₀ ≤ 100 nM, C: 100 < GI₅₀ ≤ 500 nM, D: 500 nM < GI₅₀) | | |

### <Experimental Example 2> Evaluation of SK-BR-3, BT-474, N-87, and HaCaT cell proliferation inhibitory activity

To evaluate the cell proliferation inhibitory activity of the compound according to the present invention on HER2 and EGFR kinases, the following method was performed using HER2 overexpressing cell lines (SK-BR-3, BT-474, and N-87) and EGFR expressing cell line (HaCaT).

McCOY'S 5A supplemented with 10% FBS was used for SK-BR-3 cells, RPMI-1640 medium supplemented with 20% FBS was used for BT-474 cancer cells, and RPMI-1640 medium supplemented with 10% FBS was used for N-87 cancer cells. DMEM supplemented with 10% FBS was used for HaCaT cells.

For SK-BR-3 cells, 5000 cells were aliquoted into each well of a white clear bottom 96 well plate (Corning) 48 hours before treatment with the compounds of Examples. For BT-474 cells, 5000 cells were aliquoted into each well of a white clear bottom 96 well plate (Corning) 48 hours before treatment with the compounds of Examples. For N-87 cells, 5000 cells were aliquoted into each well of a white clear bottom 96 well plate (Corning) 24 hours before treatment with the compounds of Examples. For HaCaT cells, 3000 cells were aliquoted into each well of a white clear bottom 96 well plate (Corning) 24 hours before treatment with the compounds of Examples. For SK-BR-3 cells and BT-474 cells, the compounds of Examples were diluted in dimethyl sulfoxide (three-fold dilution, a total of 11 concentrations) and injected each in an amount of 1 µl to a final concentration of 0.2 nM to 10 µM. For N-87 cells, the compounds of Examples were diluted in dimethyl sulfoxide (five-fold dilution, a total of 11 concentrations) and injected each in an amount of 0.5 µl to a final concentration of 0.5 pM to 5 µM. For HaCaT cells, the compounds of Examples were diluted in dimethyl sulfoxide (three-fold dilution, a total of 11 concentrations) and injected each in an amount of 0.5 µl to a final concentration of 0.8 nM to 45 µM. For the measurement of living cells, the cells were stored at room temperature for 10 minutes using CellTiter-Glo cell viability reagent (Promega) after a certain period of time (SK-BR-3: 120 hours, BT-474: 120 hours, N-87: 72 hours, and HaCaT: 72 hours) post treatment with the compounds of Examples, and the luminescence intensity thereof was measured using a reader (SynergyNeo, Biotek). Each test was repeated three times.

The result values were calculated as a cell growth rate (%) compared to those of the control group. A graph was created using the GraphPad Prism version 5.0 program, and GI₅₀ values were calculated. Results thereof are shown in Table 14 below.

**[Table 14]**

| Example No. | GI₅₀, nM | | | |
|---|---|---|---|---|
| | SK-BR-3 | BT-474 | N-87 | HaCaT |
| 1 | - | A | A | C |
| 2 | - | A | A | C |
| 3 | - | A | A | B |
| 4 | - | A | A | C |
| 5 | A | - | - | - |
| 6 | B | - | - | - |
| 7 | A | - | - | - |
| 8 | A | - | - | A |
| 9 | B | - | - | B |
| 10 | A | - | - | A |
| 11 | B | - | - | A |
| 12 | A | - | - | A |
| 13 | A | - | - | A |
| 14 | A | - | - | A |
| 15 | A | - | - | A |
| 17 | A | A | A | B |
| 18 | A | - | - | A |
| 19 | A | - | - | A |
| 20 | A | - | - | A |
| 21 | A | A | A | A |
| 22 | A | A | A | A |
| 23 | B | - | - | A |
| 24 | A | A | A | A |
| 25 | A | - | - | A |
| 26 | A | - | - | A |
| 27 | B | - | - | B |
| 28 | B | - | - | B |
| 29 | A | - | - | A |
| 30 | B | - | - | - |
| 31 | B | - | - | C |
| 32 | B | - | - | - |
| 34 | C | - | - | B |
| 35 | A | - | - | B |
| 36 | B | - | - | B |
| 37 | A | - | - | - |
| 39 | B | - | - | B |
| 40 | B | - | - | A |
| 41 | A | - | - | A |
| 42 | B | - | - | - |
| 43 | A | - | - | - |
| 44 | A | - | - | - |
| 45 | A | - | - | - |
| 46 | B | - | - | - |
| 47 | A | - | - | - |
| 51 | A | - | - | A |
| 52 | A | - | - | A |
| 53 | - | A | - | A |
| 54 | A | - | - | A |
| 55 | A | - | - | A |
| 56 | B | - | - | C |
| 57 | B | - | - | B |
| 58 | B | - | - | - |
| 59 | B | - | - | C |
| 60 | A | - | - | B |
| 61 | B | - | - | C |
| 63 | B | - | - | - |
| 64 | B | - | - | - |
| 65 | A | - | - | - |
| 66 | B | - | - | B |
| 67 | A | A | A | B |
| 68 | A | A | A | A |
| 69 | B | - | - | - |
| 70 | B | - | - | C |
| 71 | B | - | - | C |
| 73 | A | - | - | A |
| 75 | B | - | - | C |
| 77 | B | - | - | B |
| 78 | A | - | - | - |
| 79 | A | A | A | A |
| 80 | A | A | - | A |
| 81 | B | - | - | - |
| 82 | A | - | - | A |
| 83 | B | - | - | B |
| 84 | A | - | - | A |
| 85 | A | - | - | A |
| 86 | - | - | - | B |
| 88 | - | A | | A |
| 92 | - | B | | B |
| 95 | B | - | - | A |
| 96 | B | - | - | A |
| 99 | - | A | | A |
| 100 | - | A | | A |
| 104 | A | - | - | A |
| 105 | A | - | - | A |
| 111 | A | - | - | A |
| 112 | A | A | A | A |
| 113 | A | A | A | A |
| 114 | B | - | - | A |
| 115 | B | - | - | A |
| 116 | B | - | - | A |
| 117 | A | - | - | A |
| 118 | A | - | - | A |
| 119 | B | - | - | A |
| 120 | B | - | - | - |
| 121 | C | - | - | A |
| 125 | B | - | - | B |
| 128 | B | - | - | B |
| 129 | - | A | A | B |
| 130 | B | - | - | B |
| 131 | B | - | - | B |
| 132 | C | - | - | B |
| 133 | B | - | - | B |
| 134 | B | - | - | B |
| 135 | B | - | - | B |
| 136 | A | - | - | A |
| 137 | A | - | - | A |
| 138 | A | A | A | B |
| 139 | A | A | A | B |
| 140 | A | A | A | A |
| 141 | C | - | - | B |
| 142 | A | A | A | B |
| 143 | A | A | A | A |
| 144 | A | - | - | A |
| 145 | A | - | - | A |
| 146 | A | - | A | C |
| 147 | B | - | - | B |
| 148 | A | - | - | A |
| 149 | B | - | - | B |
| 151 | A | A | A | B |
| 152 | - | B | - | B |
| 153 | - | B | - | C |
| 154 | - | A | A | A |
| 155 | - | A | A | A |
| 157 | - | A | A | A |
| 158 | - | A | A | A |
| 159 | - | A | A | B |
| 161 | A | A | A | A |
| 162 | C | | | B |
| 163 | A | B | A | B |
| 164 | B | - | - | A |
| 165 | C | - | - | B |
| 166 | A | - | - | A |
| 167 | - | A | - | A |
| 168 | - | A | A | B |
| 169 | - | A | A | A |
| 170 | - | A | A | A |
| 171 | A | A | A | A |
| 172 | - | A | - | A |
| 173 | - | A | A | A |
| 174 | - | A | - | A |
| 176 | - | A | A | A |
| 178 | - | A | | A |
| 179 | A | - | - | A |
| 180 | B | - | - | C |
| 181 | A | A | A | A |
| 182 | A | A | A | A |
| 183 | - | B | - | B |
| 184 | - | C | - | B |
| 185 | - | A | - | A |
| 187 | - | B | - | A |
| 188 | - | B | - | A |
| 189 | - | A | A | A |
| 190 | - | A | A | A |
| 191 | - | A | A | A |
| 192 | - | A | A | A |
| 193 | - | B | - | A |
| 195 | - | A | - | A |
| 196 | - | A | - | B |
| 197 | - | B | - | C |
| 198 | - | B | - | C |
| 199 | - | A | - | B |
| 200 | - | A | - | A |
| 201 | - | A | - | A |
| 202 | - | A | - | C |
| 203 | - | A | - | B |
| 204 | - | A | - | B |
| 205 | - | B | - | C |
| 207 | - | A | - | B |
| 208 | - | B | - | C |
| 209 | - | A | - | B |
| 210 | - | A | - | B |
| 211 | - | A | - | B |
| 213 | - | A | - | A |
| 214 | - | A | - | C |
| 215 | - | A | A | B |
| 216 | - | B | - | B |
| 218 | - | C | - | B |
| 220 | - | B | - | B |
| 221 | - | C | - | B |
| 224 | - | A | A | A |
| 225 | - | B | A | C |
| 226 | - | A | A | C |
| 227 | B | - | - | C |
| 229 | - | A | A | B |
| 234 | B | - | - | B |
| 236 | B | - | - | C |
| 237 | B | - | - | C |
| 238 | A | - | - | A |
| 239 | - | A | - | A |
| 240 | A | - | - | A |
| 241 | A | - | - | A |
| 242 | B | - | - | A |
| 248 | A | - | - | A |
| 249 | B | - | - | A |
| 250 | A | - | - | A |
| 251 | A | - | - | A |
| 253 | A | - | - | A |
| 255 | A | - | - | A |
| 257 | B | - | - | A |
| 258 | B | - | - | A |
| 259 | A | - | - | A |
| 260 | A | A | A | A |
| 262 | A | - | - | A |
| 269 | A | - | - | A |
| 270 | A | A | A | A |
| 271 | A | - | - | A |
| 272 | A | - | - | A |
| 273 | A | - | - | A |
| 274 | A | - | - | A |
| 275 | A | - | - | A |
| 277 | A | - | - | A |
| 278 | A | A | A | A |
| 279 | A | - | - | A |
| 280 | A | - | - | A |
| 281 | A | - | A | A |
| 282 | A | - | - | A |
| 286 | A | A | A | A |
| 287 | A | - | - | C |
| 288 | B | - | - | C |
| 289 | A | - | - | A |
| 290 | B | - | - | A |
| 291 | C | - | - | A |
| 297 | - | A | - | A |
| 298 | A | A | A | A |
| 299 | A | A | A | A |
| 300 | - | A | A | A |
| 301 | - | A | A | A |
| 304 | - | A | A | A |
| 305 | A | A | A | A |
| 306 | A | A | A | A |
| 307 | A | A | A | A |
| 308 | - | A | - | A |
| 309 | - | A | - | A |
| 314 | - | A | - | A |
| 315 | - | A | - | A |
| 316 | - | A | - | A |
| 317 | - | A | - | A |
| 318 | - | A | - | A |
| 319 | - | A | - | A |
| 320 | - | B | - | B |
| 321 | - | C | - | B |
| 324 | - | C | - | B |
| 325 | - | B | - | B |
| 326 | - | C | - | B |
| 330 | B | - | - | B |

| | | | | |
|---|---|---|---|---|
| (A: GI₅₀ ≤ 150 nM, B: 150 < GI₅₀ ≤ 1000 nM, C: 1000 nM < GI₅₀) | | | | |

### <Experimental Example 3> Evaluation of transduced Ba/F3 cell proliferation inhibitory activity

For Ba/F3 cells, RPMI-1640 supplemented with 10% FBS and 5 ng/ml IL-3 (R&D Systems) was used. Transduced Ba/F3 cells were cultured by adding 1 ug/ml puromycin (Invitrogen) to the same medium.

3000 to 5000 cells were aliquoted into each well of a white clear bottom 96 well plate (Corning) 24 hours before treatment with the compounds of Examples. The compounds of Examples were diluted in dimethyl sulfoxide (three-fold dilution, a total of 12 concentrations) and injected each in an amount of 1 µl to a final concentration of 0.2 nM to 5 µM. For the measurement of living cells, the cells were stored at room temperature for 10 minutes using CellTiter-Glo luminescentcell-viability reagent (Promega) in 72 hours after treatment with the compounds of Examples, and the luminescence intensity thereof was measured using a reader (SynergyNeo, Biotek). Each test was repeated three times. The result values were calculated as a cell growth rate (%) compared to those of the control group. A graph was created using the GraphPad Prism version 8.3.0 program, and GI₅₀ values were calculated.

Table 15 below shows the results of evaluating the Ba/F3 cell proliferation inhibitory activity of L869R, L756S, T798I, and T862A expressing HER2 rare or uncommon and drug-resistant mutations and G719A, L861Q, S768I, G719A/S768I, and Del19/T790M expressing EGFR rare or uncommon and drug-resistant mutations.

**[Table 15]**

| Exam ple No. | Ba/F3 (GI₅₀, µM) | | | | | | | | | | |
|---|---|---|---|---|---|---|---|---|---|---|---|
| | HER2 | | | | | EGFR | | | | | |
| | WT | L869 R | L755 S | T798 I | T862 A | WT | G719 A | L861 Q | S768 I | G719 A/ S768 I | Dell 9/ T790 M |
| 80 | A | A | A | A | A | A | A | A | A | A | A |
| 78 | - | A | - | A | - | - | - | - | - | - | - |
| 68 | A | A | A | C | A | B | A | A | B | A | A |
| 112 | - | A | A | - | A | | | | | | |
| 143 | - | A | A | - | A | - | - | - | - | - | - |
| 300 | A | - | A | C | A | - | - | - | - | - | - |
| 305 | A | - | A | A | A | - | - | - | - | - | - |
| 306 | - | - | - | - | - | A | - | - | A | - | - |
| 307 | - | - | - | - | - | A | - | - | A | - | - |

| | | | | | | | | | | | |
|---|---|---|---|---|---|---|---|---|---|---|---|
| **[416]** (A: GI₅₀ ≤ 50 nM, B: 50 nM < GI₅₀ ≤ 100 nM, C: 100 nM < GI₅₀ ≤ 500 nM, D: 500 nM < GI₅₀) | | | | | | | | | | | |

## Claims

1. A compound represented by the following Chemical Formula 1, an optical isomer thereof, or a pharmaceutically acceptable salt thereof: 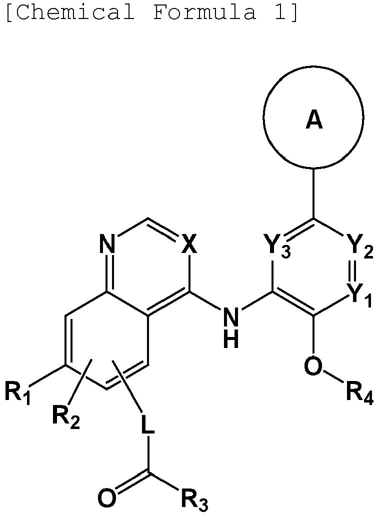 in Chemical Formula 1 above,
X is N or CR_{X1};
R₁ is -H, -OR_{X2}, or -NR_{X3}R_{X4};
R_{X1} is -H, -C₁₋₆alkyl, -C₁₋₆haloalkyl, -CN, or -halo;
R_{X2} is -H, -C₁₋₆alkyl, -C₁₋₆hydroxyalkyl, -C₁₋₆aminoalkyl, -C₁₋₆haloalkyl, -C₁₋₆alkyl-O(C₁₋₆alkyl), -C₁₋₆alkyl-O(C₁₋₆alkyl)-C₁₋₆aminoalkyl, -(CH₂)n-cycloalkyl, or -(CH₂)n-heterocycloalkyl {wherein at least one H of the -(CH₂)n-cycloalkyl or -(CH₂)n-heterocycloalkyl ring may be substituted with -C₁₋₆alkyl, -C₁₋₆hydroxyalkyl, -C₁₋₆aminoalkyl, -C₁₋₆haloalkyl, -C₁₋₆alkyl-O(C₁₋₆alkyl), -CN, -NH₂, -NH-C₁₋₆alkyl, -N(C₁₋₆alkyl)(C₁₋₆alkyl), -NO₂, -OH, -halo, or heterocycloalkyl};
n is 0, 1, 2, 3, or 4;
R_{X3} and R_{X4} are each independently -H or -C₁₋₆alkyl, or R_{X3} and R_{X4} are linked to each other to form 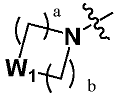 or 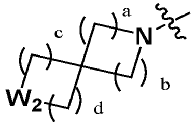 ring together with an N atom, and the W₁ and W₂ are each independently CH₂, NH, O, or S {wherein at least one H of the 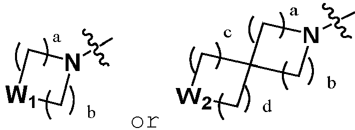 heterocycloalkyl ring may be substituted with -C₁₋₆alkyl, -C₁₋₆hydroxyalkyl, -C₁₋₆aminoalkyl, -C₁₋₆haloalkyl, -CN, -NH₂, -NH-C₁₋₆alkyl, -N(C₁₋₆alkyl)(C₁-₆alkyl), -NO₂, -OH, -halo, or heterocycloalkyl};
a to d are each independently 1, 2, or 3;
R₂ is -H, -C₁₋₆alkyl, -C₁₋₆haloalkyl, -CN, or -halo;
L is -NH-, 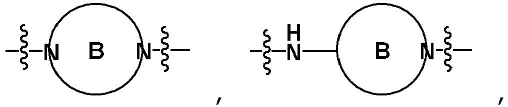 or 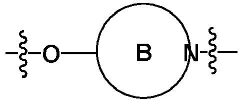 {wherein the ring B is a single ring or multiple rings containing an N atom in the ring, and at least one H of the ring B may be substituted with -C₁₋₆alkyl, -C₁₋₆hydroxyalkyl, -C₁₋₆aminoalkyl, -C₁₋₆haloalkyl, -CN, -NH₂, - NH-C₁₋₆alkyl, -N(C₁₋₆alkyl)(C₁-₆alkyl), -NO₂, -OH, =O, or - halo};
R₃ is -CZ₁=Z₂Z₃, -C₁₋₆alkyl, -C₁₋₆haloalkyl, or cycloalkyl;
Z₁ is -H, -C₁₋₆alkyl, -C₁₋₆aminoalkyl, -C₁₋₆hydroxyalkyl, -C₁₋₆haloalkyl, -CN, or -halo;
Z₂ and Z₃ are each independently -H, -C₁₋₆alkyl, -C₁₋₆hydroxyalkyl, -C₁₋₆aminoalkyl, -C₁₋₆haloalkyl, -C₁₋₆alkyl-O(C₁₋₆alkyl), -halo, cycloalkyl, heterocycloalkyl, -C₁₋₆alkylcycloalkyl, or -C₁₋₆alkyl-heterocycloalkyl {wherein at least one H of the cycloalkyl, heterocycloalkyl, -C₁₋₆alkylcycloalkyl, or -C₁₋₆alkyl-heterocycloalkyl may be substituted with -C₁₋₆alkyl, -C₁₋₆hydroxyalkyl, -C₁₋₆aminoalkyl, -C₁₋₆haloalkyl, -CN, -NH₂, -NH-C₁₋₆alkyl, -N(C₁₋₆alkyl)(C₁₋₆alkyl), -NO₂, -OH, =O, or heterocycloalkyl};
Y₁ to Y₃ are each independently N or CR_{Y};
R_{Y} is -H, -C₁₋₆alkyl, -C₁₋₆aminoalkyl, -C₁₋₆hydroxyalkyl, -C₁₋₆haloalkyl, -CN, -NH₂, -NH-C₁₋₆alkyl, -N(C₁₋₆alkyl)(C₁₋₆alkyl), -NO₂, -OH, or -halo;
R₄ is -C₁₋₆alkyl, -C₁₋₆alkyl-O-C₁₋₆alkyl, -C₁₋₆haloalkyl, cycloalkyl, heterocycloalkyl, -C₁₋₆alkyl-cycloalkyl, or -C₁₋₆alkyl-heterocycloalkyl {wherein at least one H of the cycloalkyl, heterocycloalkyl, -C₁₋₆alkyl-cycloalkyl, or -C₁₋₆alkyl-heterocycloalkyl may be substituted with -C₁₋₆alkyl};
ring A is aryl, heteroaryl, cycloalkyl, cycloalkenyl, heterocycloalkyl, or heterocycloalkenyl {wherein at least one H of the aryl, heteroaryl, cycloalkyl, cycloalkenyl, heterocycloalkyl, or heterocycloalkenyl ring may be substituted with -C₁₋₆alkyl, -C₁₋₆aminoalkyl, -C₁₋₆hydroxyalkyl, -C₁₋₆haloalkyl, -C₁₋₆alkenyl, -CN, -C(=O)-R_{A1}, -NO₂, -NR_{A2}R_{A3}, - OR_{A4}, -S-C₁₋₆alkyl, -halo, or heterocycloalkyl [here, at least one H of the heterocycloalkyl ring may be substituted with -C₁₋₆alkyl, -C₁₋₆haloalkyl, -halo, or heterocycloalkyl, and substituents on the aryl or heteroaryl ring may be linked to each other to form a 5-6 membered cycloalkyl or a 5-6 membered heterocycloalkyl};
R_{A1} is -H, -C₁₋₆alkyl, -NH₂, -NH-C₁₋₆alkyl, -N(C₁₋₆alkyl)(C₁₋₆alkyl), -NH-(CH₂)m-aryl, -OH, or -O-C₁₋₆alkyl {wherein at least one H of the -NH-(CH₂)m-aryl ring may be substituted with -C₁₋₆alkyl, -C₁₋₆haloalkyl, or -halo};
m is 0, 1, 2, 3, or 4;
R_{A2} and R_{A3} are each independently -H, -C₁₋₆alkyl, -C(=O)-C₁₋₆alkyl, -C(=O)-C₁₋₆alkenyl, -C(=O)-cycloalkyl, -C(=O)-heterocycloalkyl, or -C(=O)-aryl {wherein at least one H of the -C(=O)-cycloalkyl, -C(=O)-heterocycloalkyl, or -C(=O)-aryl ring may be substituted with -C₁₋₆alkyl, -C₁₋₆haloalkyl, =O, -halo, or aryl}; and
R_{A4} is -H, -C₁₋₆alkyl, or -C₁₋₆haloalkyl.

2. The compound represented by Chemical Formula 1, the optical isomer thereof, or the pharmaceutically acceptable salt thereof of claim 1, wherein
X is N or CR_{X1};
R₁ is -H, -OR_{X2}, or -NR_{X3}R_{X4};
R_{X1} is -H or -CN;
R_{X2} is -C₁₋₆alkyl, -C₁₋₆aminoalkyl, -C₁₋₆haloalkyl, -C₁₋₆alkyl-O(C₁₋₆alkyl), -C₁₋₆alkyl-O(C₁₋₆alkyl)-C₁₋₆aminoalkyl, or - (CH₂)n-heterocycloalkyl {wherein at least one H of the - (CH₂)n-heterocycloalkyl ring may be substituted with -C₁₋₆alkyl, -C₁₋₆alkyl-O(C₁₋₆alkyl), or heterocycloalkyl};
n is 0, 1, 2, 3, or 4;
R_{X3} and R_{X4} are each independently -H or -C₁₋₆alkyl, or R_{X3} and R_{X4} are linked to each other to form 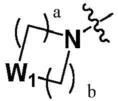 or 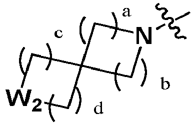 ring together with an N atom, and W₁ and W₂ are each independently CH₂, NH, or O {wherein at least one H of the 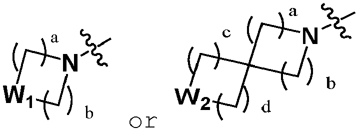 ring may be substituted with - halo, or heterocycloalkyl};
a to d are each independently 1 or 2;
R₂ is -H or -halo; 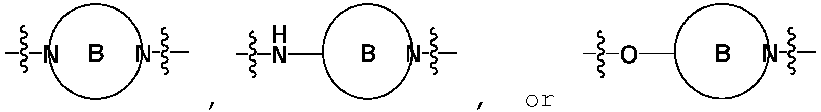
{wherein the ring B is a single ring or multiple rings containing an N atom in the ring, and at least one H of the ring B may be substituted with -C₁₋₆alkyl, -C₁₋₆haloalkyl, =O, or -halo};
R₃ is -CZ₁=Z₂Z₃, -C₁₋₆haloalkyl, or cycloalkyl;
Z₁ is -H, -C₁₋₆aminoalkyl, -CN, or -halo;
Z₂ and Z₃ are each independently -H, -C₁₋₆alkyl, -C₁₋₆aminoalkyl, -C₁₋₆alkyl-O(C₁₋₆alkyl), -halo, -heterocycloalkyl, or -C₁₋₆alkyl-heterocycloalkyl {wherein at least one H of the -heterocycloalkyl, or -C₁₋₆alkyl-heterocycloalkyl may be substituted with -C₁₋₆alkyl or -heterocycloalkyl};
Y₁ to Y₃ are each independently N or CR_{Y};
R_{Y} is -H, -C₁₋₆alkyl, or -halo;
R₄ is -C₁₋₆alkyl, -C₁₋₆alkyl-O-C₁₋₆alkyl, -C₁₋₆haloalkyl, cycloalkyl, or heterocycloalkyl {wherein at least one H of the cycloalkyl or heterocycloalkyl may be substituted with -C₁₋₆alkyl } ;
ring A is aryl, heteroaryl, heterocycloalkyl, or heterocycloalkenyl {wherein at least one H of the aryl, heteroaryl, cycloalkyl, cycloalkenyl, heterocycloalkyl, or heterocycloalkenyl ring may be substituted with -C₁₋₆alkyl, -C₁₋₆haloalkyl, -C₁₋₆alkenyl, -CN, -C(=O)-R_{A1}, -NO₂, -NR_{A2}R_{A3}, - OR_{A4}, -S-C₁₋₆alkyl, -halo, or heterocycloalkyl [here, at least one H of the heterocycloalkyl ring may be substituted with -C₁₋₆alkyl, -C₁₋₆haloalkyl, -halo, or heterocycloalkyl , and substituents on the aryl or heteroaryl ring may be linked with each other to form a 5-6 membered cycloalkyl or a 5-6 membered heterocycloalkyl};
R_{A1} is -H, -NH-(CH₂)m-aryl, -OH, or -O-C₁₋₆alkyl {wherein at least one H of the -NH-(CH₂)m-aryl ring may be substituted with -halo};
m is 0, 1, 2, 3, or 4;
R_{A2} and R_{A3} are each independently -H, -C₁₋₆alkyl, -C(=O)-C₁₋₆alkyl, -C(=O)-C₁₋₆alkenyl, -C(=O)-cycloalkyl, -C(=O)-heterocycloalkyl, or -C(=O)-aryl {wherein at least one H of the -C(=O)-cycloalkyl, -C(=O)-heterocycloalkyl, or -C(=O)-aryl ring may be substituted with =O, -halo, or aryl}; and
R_{A4} is -H, -C₁₋₆alkyl, or -C₁₋₆haloalkyl.

3. The compound represented by Chemical Formula 1, the optical isomer thereof, or the pharmaceutically acceptable salt thereof of claim 1, wherein
X is N or CR_{X1};
R₁ is -H, -OR_{X2}, or -NR_{X3}R_{X4};
R_{X1} is -H or -CN;
R_{X2} is -C₁₋₆alkyl, -C₁₋₆aminoalkyl, -C₁₋₆haloalkyl, -C₁₋₆alkyl-O(C₁₋₆alkyl), -C₁₋₆alkyl-O(C₁₋₆alkyl)-C₁₋₆aminoalkyl, or -(CH₂)n-heterocycloalkyl {wherein the -(CH₂)n-heterocycloalkyl ring is 4-6 membered, and at least one H of the -(CH₂)n-heterocycloalkyl ring may be substituted with - C₁₋₆alkyl, -C₁₋₆alkyl-O(C₁₋₆alkyl), or 4-6 membered heterocycloalkyl};
n is 0, 1, 2, or 3;
R_{X3} and R_{X4} are each independently -H or -C₁₋₆alkyl, or R_{X3} and R_{X4} are linked to each other to form 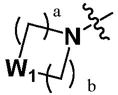 or 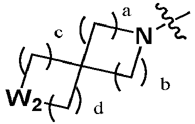 together with an N atom, and W₁ and W₂ are each independently CH₂ or O {wherein at least one H of the 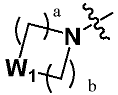 or 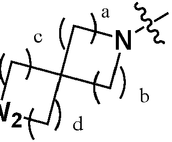 ring may be substituted with -halo, or 4-6 membered heterocycloalkyl};
a to d are each independently 1 or 2; and
R₂ is -H or -halo.

4. The compound represented by Chemical Formula 1, the optical isomer thereof, or the pharmaceutically acceptable salt thereof of claim 1, wherein
L is -NH-, 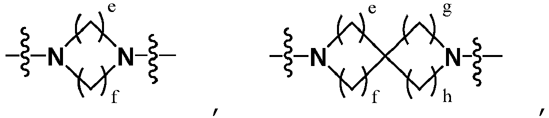 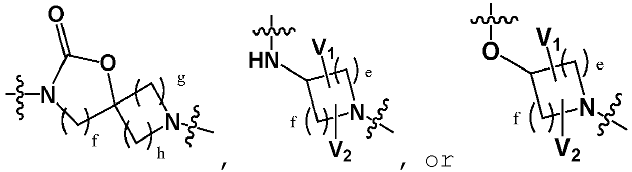 {wherein at least one H of the 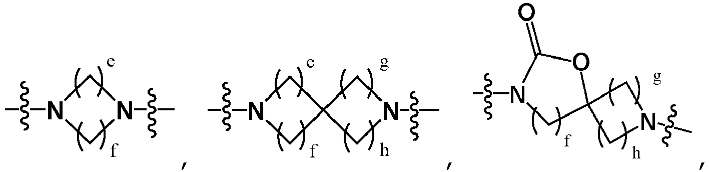 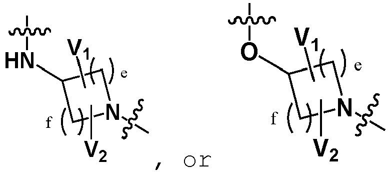 ring may be substituted with -C₁₋₆alkyl, -C₁₋₆haloalkyl, =O, or -halo};
V₁ and V₂ are linked together by a single bond, C₁alkyl, C₂alkyl, or C₃alkyl to form a bridged double ring or nothing (null); and
e to h are each independently 1, 2, or 3.

5. The compound represented by Chemical Formula 1, the optical isomer thereof, or the pharmaceutically acceptable salt thereof of claim 1, wherein
R₃ is -CZ₁=Z₂Z₃, -C₁₋₆haloalkyl, or cycloalkyl {wherein the cycloalkyl ring is a single ring or multiple rings};
Z₁ is -H, -C₁₋₆aminoalkyl, -CN, or -halo;
Z₂ and Z₃ are each independently -H, -C₁₋₆alkyl, -C₁₋₆aminoalkyl, -C₁₋₆alkyl-O(C₁₋₆alkyl), -halo, -heterocycloalkyl, or -C₁₋₆alkyl-heterocycloalkyl {wherein the heterocycloalkyl or -C₁₋₆alkyl-heterocycloalkyl ring is 4-6 membered, and at least one H of the heterocycloalkyl, -C₁₋₆alkyl-cycloalkyl, or -C₁₋₆alkyl-heterocycloalkyl may be substituted with -C₁₋₆alkyl, or 4-6 membered heterocycloalkyl}.

6. The compound represented by Chemical Formula 1, the optical isomer thereof, or the pharmaceutically acceptable salt thereof of claim 1, wherein
Y₁ to Y₃ are each independently N or CR_{Y};
R_{Y} is -H, -C₁₋₆alkyl, or -halo; and
R₄ is -C₁₋₆alkyl, -C₁₋₆alkyl-O-C₁₋₆alkyl, -C₁₋₆haloalkyl, 3-7 membered cycloalkyl, or 4-6 membered heterocycloalkyl {wherein at least one H of the 3-7 membered cycloalkyl or 4-6 membered heterocycloalkyl may be substituted with -C₁₋₆alkyl}.

7. The compound represented by Chemical Formula 1, the optical isomer thereof, or the pharmaceutically acceptable salt thereof of claim 1, wherein
ring A is phenyl, 5-10 membered heteroaryl, 4-6 membered heterocycloalkyl, or 4-6 membered heterocycloalkenyl {wherein at least one H of the phenyl, 5-10 membered heteroaryl, 4-6 membered heterocycloalkyl, or 4-6 membered heterocycloalkenyl ring may be substituted with -C₁₋₆alkyl, -C₁₋₆haloalkyl, -C₁₋₆alkenyl, -CN, -C(=O)-R_{A1}, -NO₂, -NR_{A2}R_{A3}, - OR_{A4}, -S-C₁₋₆alkyl, -halo, or 4-6 membered heterocycloalkyl [here, at least one H of the 4-6 membered heterocycloalkyl ring may be substituted with -C₁₋₆alkyl, -C₁₋₆haloalkyl, -halo, or heterocycloalkyl], and substituents on the phenyl or 5-10 membered heteroaryl ring may be linked to each other to form a 5-6 membered cycloalkyl or a 5-6 membered heterocycloalkyl};
R_{A1} is -H, -NH-(CH₂)m-phenyl, -OH, or -O-C₁₋₆alkyl {wherein at least one H of the -NH-(CH₂)m-phenyl ring may be substituted with -halo};
m is 0, 1, or 2;
R_{A2} and R_{A3} are each independently -H, -C₁₋₆alkyl, -C(=O)-C₁₋₆alkyl, -C(=O)-C₁₋₆alkenyl, -C(=O)-cycloalkyl, -C(=O)-heterocycloalkyl, or -C(=O)-phenyl {wherein at least one H of the -C(=O)-cycloalkyl, -C(=O)-heterocycloalkyl, or - C(=O)-phenyl ring may be substituted with -C₁₋₆alkyl, -C₁₋₆haloalkyl, =O, -halo, or phenyl}; and
R_{A4} is -H, -C₁₋₆alkyl, or -C₁₋₆haloalkyl.

8. The compound, the optical isomer thereof, or the pharmaceutically acceptable salt thereof of claim 1, wherein
the compound represented by Chemical Formula 1 above is selected from the group consisting of the following compounds:
| | |
|---|---|
| 1 | N-(3'-((6-((1-acryloylpiperidin-4-yl)oxy)-7-methoxyquinazolin-4-yl)amino)-2,4-difluoro-4'-methoxy-[1,1'-biphenyl]-3-yl)acetamide |
| 2 | N-(3'-((6-((1-acryloylpiperidin-4-yl)oxy)-7-methoxyquinazolin-4-yl)amino)-2,4-difluoro-4'-methoxy-[1,1'-biphenyl]-3-yl)cyclopropancarboxamide |
| 3 | N-(3'-((6-((1-acryloylazetidin-3-yl)oxy)-7-methoxyquinazolin-4-yl)amino)-2,4-difluoro-4'-methoxy-[1,1'-biphenyl]-3-yl)cyclopropancarboxamide |
| 4 | N-(3'-((6-((1-acryloylpiperidin-4-yl)oxy)-7-methoxyquinazolin-4-yl)amino)-4-fluoro-4'-methoxy-[1,1'-biphenyl]-3-yl)cyclopropancarboxamide |
| 5 | 1-(3-((7-methoxy-4-((4-methoxy-[1,1'-biphenyl]-3-yl)amino)quinazolin-6-yl)oxy)azetidin-1-yl)prop-2-en-1-one |
| 6 | 1-(3-((7-methoxy-4-((2-methoxy-5-(pyridin-3-yl)phenyl)amino)quinazolin-6-yl)oxy)azetidin-1-yl)prop-2-en-1-one |
| 7 | 1-(3-((4-((4'-fluoro-4-methoxy-[1,1'-biphenyl]-3-yl)amino)-7-methoxyquinazolin-6-yl)oxy)azetidin-1-yl)prop-2-en-1-one |
| 8 | 1-(3-((4-((3'-fluoro-4-methoxy-[1,1'-biphenyl]-3-yl)amino)-7-methoxyquinazolin-6-yl)oxy)azetidin-1-yl)prop-2-en-1-one |
| 9 | 1-(3-((7-methoxy-4-((4-methoxy-3'-(trifluoromethyl)-[1,1'-biphenyl]-3-yl)amino)quinazolin-6-yl)oxy)azetidin-1-yl)prop-2-en-1-one |
| 10 | 1-(3-((4-((2',5'-difluoro-4-methoxy-[1,1'-biphenyl]-3-yl)amino)-7-methoxyquinazolin-6-yl)oxy)azetidin-1-yl)prop-2-en-1-one |
| 11 | 1-(3-((4-((3',5'-difluoro-4-methoxy-[1,1'-biphenyl]-3-yl)amino)-7-methoxyquinazolin-6-yl)oxy)azetidin-1-yl)prop-2-en-1-one |
| 12 | 3'-((6-((1-acryloylazetidin-3-yl)oxy)-7-methoxyquinazolin-4-yl)amino)-4'-methoxy-[1,1'-biphenyl]-3-carbonitrile |
| 13 | 1-(3-((4-((2',4'-difluoro-4-methoxy-[1,1'-biphenyl]-3-yl)amino)-7-methoxyquinazolin-6-yl)oxy)azetidin-1-yl)prop-2-en-1-one |
| 14 | 3'-((6-((1-acryloylazetidin-3-yl)oxy)-7-methoxyquinazolin-4-yl)amino)-4-fluoro-4'-methoxy-[1,1'-biphenyl]-3-carbonitrile |
| 15 | 1-(3-((4-((4'-chloro-2'-fluoro-4-methoxy-[1,1'-biphenyl]-3-yl)amino)-7-methoxyquinazolin-6-yl)oxy)azetidin-1-yl)prop-2-en-1-one |
| 16 | 3'-((6-((1-acryloylazetidin-3-yl)oxy)-7-methoxyquinazolin-4-yl)amino)-3-fluoro-4'-methoxy-[1,1'-biphenyl]-4-carbonitrile |
| 17 | 1-(3-((4-((2'-fluoro-4-methoxy-3'-(trifluoromethyl)-[1,1'-biphenyl]-3-yl)amino)-7-methoxyquinazolin-6-yl)oxy)azetidin-1-yl)prop-2-en-1-one |
| 18 | 1-(3-((4-((2'-chloro-3'-fluoro-4-methoxy-[1,1'-biphenyl]-3-yl)amino)-7-methoxyquinazolin-6-yl)oxy)azetidin-1-yl)prop-2-en-1-one |
| 19 | 1-(3-((4-((2',3'-difluoro-4-methoxy-[1,1'-biphenyl]-3-yl)amino)-7-methoxyquinazolin-6-yl)oxy)azetidin-1-yl)prop-2-en-1-one |
| 20 | 1-(3-((4-((3'-chloro-2'-fluoro-4-methoxy-[1,1'-biphenyl]-3-yl)amino)-7-methoxyquinazolin-6-yl)oxy)azetidin-1-yl)prop-2-en-1-one |
| 21 | 3'-((6-((1-acryloylazetidin-3-yl)oxy)-7-methoxyquinazolin-4-yl)amino)-2-fluoro-4'-methoxy-[1,1'-biphenyl]-3-carbonitrile |
| 22 | 1-(3-((4-((5-(6-fluoropyridin-3-yl)-2-methoxyphenyl)amino)-7-methoxyquinazolin-6-yl)oxy)azetidin-1-yl)prop-2-en-1-one |
| 23 | 1-(3-((4-((3'-fluoro-4-methoxy-2'-methyl-[1,1'-biphenyl]-3-yl)amino)-7-methoxyquinazolin-6-yl)oxy)azetidin-1-yl)prop-2-en-1-one |
| 24 | 1-(3-((4-((2'-fluoro-4-methoxy-[1,1'-biphenyl]-3-yl)amino)-7-methoxyquinazolin-6-yl)oxy)azetidin-1-yl)prop-2-en-1-one |
| 25 | 1-(3-((4-((4'-fluoro-4-methoxy-2'-methyl-[1,1'-biphenyl]-3-yl)amino)-7-methoxyquinazolin-6-yl)oxy)azetidin-1-yl)prop-2-en-1-one |
| 26 | 3'-((6-((1-acryloylazetidin-3-yl)oxy)-7-methoxyquinazolin-4-yl)amino)-4'-methoxy-[1,1'-biphenyl]-4-carbonitrile |
| 27 | 1-(3-((4-((5-(6-chloro-5-fluoropyridin-3-yl)-2-methoxyphenyl)amino)-7-methoxyquinazolin-6-yl)oxy)azetidin-1-yl)prop-2-en-1-one |
| 28 | 1-(3-((4-((4'-chloro-3'-fluoro-4-methoxy-[1,1'-biphenyl]-3-yl)amino)-7-methoxyquinazolin-6-yl)oxy)azetidin-1-yl)prop-2-en-1-one |
| 29 | 1-(3-((4-((3',4'-difluoro-4-methoxy-[1,1'-biphenyl]-3-yl)amino)-7-methoxyquinazolin-6-yl)oxy)azetidin-1-yl)prop-2-en-1-one |
| 30 | 1-(3-((4-((5-([1,2,4]triazolo[1,5-a]pyridin-7-yl)-2-methoxyphenyl)amino)-7-methoxyquinazolin-6-yl)oxy)azetidin-1-yl)prop-2-en-1-one |
| 31 | 1-(3-((4-((5-([1,2,4]triazolo[1,5-a]pyridin-6-yl)-2-methoxyphenyl)amino)-7-methoxyquinazolin-6-yl)oxy)azetidin-1-yl)prop-2-en-1-one |
| 32 | 1-(3-((7-methoxy-4-((2-methoxy-5-(pyrazolo[1,5-a]pyrimidin-6-yl)phenyl)amino)quinazolin-6-yl)oxy)azetidin-1-yl)prop-2-en-1-one |
| 33 | 1-(3-((7-methoxy-4-((2-methoxy-5-(1-methyl-1H-pyrazolo[3,4-b]pyridin-5-yl)phenyl)amino)quinazolin-6-yl)oxy)azetidin-1-yl)prop-2-en-1-one |
| 34 | 1-(3-((7-methoxy-4-((2-methoxy-5-(1-methyl-1H-indol-6-yl)phenyl)amino)quinazolin-6-yl)oxy)azetidin-1-yl)prop-2-en-1-one |
| 35 | 1-(3-((4-((5-(imidazo[1,2-a]pyridin-8-yl)-2-methoxyphenyl)amino)-7-methoxyquinazolin-6-yl)oxy)azetidin-1-yl)prop-2-en-1-one |
| 36 | 1-(3-((4-((5-(benzo[d]thiazol-7-yl)-2-methoxyphenyl)amino)-7-methoxyquinazolin-6-yl)oxy)azetidin-1-yl)prop-2-en-1-one |
| 37 | 1-(3-((4-((5-(benzofuran-5-yl)-2-methoxyphenyl)amino)-7-methoxyquinazolin-6-yl)oxy)azetidin-1-yl)prop-2-en-1-one |
| 38 | 1-(3-((4-((5-(benzofuran-2-yl)-2-methoxyphenyl)amino)-7-methoxyquinazolin-6-yl)oxy)azetidin-1-yl)prop-2-en-1-one |
| 39 | 1-(3-((7-methoxy-4-((2-methoxy-5-(pyrazolo[1,5-a]pyrimidin-3-yl)phenyl)amino)quinazolin-6-yl)oxy)azetidin-1-yl)prop-2-en-1-one |
| 40 | 1-(3-((4-((5-(benzofuran-3-yl)-2-methoxyphenyl)amino)-7-methoxyquinazolin-6-yl)oxy)azetidin-1-yl)prop-2-en-1-one |
| 41 | 1-(3-((4-((5-(benzo[b]thiophen-3-yl)-2-methoxyphenyl)amino)-7-methoxyquinazolin-6-yl)oxy)azetidin-1-yl)prop-2-en-1-one |
| 42 | 1-(3-((7-methoxy-4-((2-methoxy-5-(1-methyl-1H-pyrazol-5-yl)phenyl)amino)quinazolin-6-yl)oxy)azetidin-1-yl)prop-2-en-1-one |
| 43 | 1-(3-((7-methoxy-4-((2-methoxy-5-(thiophen-2-yl)phenyl)amino)quinazolin-6-yl)oxy)azetidin-1-yl)prop-2-en-1-one |
| 44 | 1-(3-((4-((5-(furan-2-yl)-2-methoxyphenyl)amino)-7-methoxyquinazolin-6-yl)oxy)azetidin-1-yl)prop-2-en-1-one |
| 45 | 1-(3-((4-((5-(furan-3-yl)-2-methoxyphenyl)amino)-7-methoxyquinazolin-6-yl)oxy)azetidin-1-yl)prop-2-en-1-one |
| 46 | 1-(3-((7-methoxy-4-((2-methoxy-5-(thiazol-5-yl)phenyl)amino)quinazolin-6-yl)oxy)azetidin-1-yl)prop-2-en-1-one |
| 47 | 1-(3-((7-methoxy-4-((2-methoxy-5-(thiophen-3-yl)phenyl)amino)quinazolin-6-yl)oxy)azetidin-1-yl)prop-2-en-1-one |
| 48 | 1-(3-((7-methoxy-4-((2-methoxy-5-(2-methylthiazol-5-yl))amino)quinazolin-6-yl)oxy)azetidin-1-yl)prop-2-en-1-one |
| 49 | 1-(3-((7-methoxy-4-((2-methoxy-5-(5-methylthiophen-3-yl)phenyl)amino)quinazolin-6-yl)oxy)azetidin-1-yl)prop-2-en-1-one |
| 50 | 1-(3-((7-methoxy-4-((2-methoxy-5-(5-methylfuran-2-yl)phenyl)amino)quinazolin-6-yl)oxy)azetidin-1-yl)prop-2-en-1-one |
| 51 | 1-(3-((7-methoxy-4-((2-methoxy-5-(2-methylfuran-3-yl)phenyl)amino)quinazolin-6-yl)oxy)azetidin-1-yl)prop-2-en-1-one |
| 52 | 1-(3-((7-methoxy-4-((2-methoxy-4-methyl-5-(thiophen-2-yl)phenyl)amino)quinazolin-6-yl)oxy)azetidin-1-yl)prop-2-en-1-one |
| 53 | 1-(4,4-difluoro-3-((4-((5-(furan-2-yl)-2-methoxyphenyl)amino)-7-methoxyquinazolin-5-yl)oxy)piperidin-1-yl)prop-2-en-1-one |
| 54 | 1-(3-((4-((2-ethoxy-5-(thiophen-2-yl)phenyl)amino)-7-methoxyquinazolin-6-yl)oxy)azetidin-1-yl)prop-2-en-1-one |
| 55 | 1-(3-((4-((2-ethoxy-5-(furan-2-yl)phenyl)amino)-7-methoxyquinazolin-6-yl)oxy)azetidin-1-yl)prop-2-en-1-one |
| 56 | 1-(3-((4-((5-([1,2,4]triazolo[1,5-a]pyridin-7-yl)-2-ethoxyphenyl)amino)-7-methoxyquinazolin-6-yl)oxy)azetidin-1-yl)prop-2-en-1-one |
| 57 | 1-(3-((4-((5-(benzofuran-5-yl)-2-ethoxyphenyl)amino)-7-methoxyquinazolin-6-yl)oxy)azetidin-1-yl)prop-2-en-1-one |
| 58 | 1-(3-((7-methoxy-4-((4-methoxy-[1,1'-biphenyl]-3-yl)amino)quinazolin-6-yl)oxy)pyrrolidin-1-yl)prop-2-en-1-one |
| 59 | 1-(3-((7-methoxy-4-((2-methoxy-5-(pyridin-3-yl)phenyl)amino)quinazolin-6-yl)oxy)pyrrolidin-1-yl)prop-2-en-1-one |
| 60 | 1-(3-((4-((4'-fluoro-4-methoxy-[1,1'-biphenyl]-3-yl)amino)-7-methoxyquinazolin-6-yl)oxy)pyrrolidin-1-yl)prop-2-en-1-one |
| 61 | 1-(3-((7-methoxy-4-((2-methoxy-5-(1-methyl-1H-pyrazol-5-yl)phenyl)amino)quinazolin-6-yl)oxy)pyrrolidin-1-yl)prop-2-en-1-one |
| | |
|---|---|
| 62 | 1-(3-((7-methoxy-4-((2-methoxy-5-(thiazol-5-yl)phenyl)amino)quinazolin-6-yl)oxy)pyrrolidin-1-yl)prop-2-en-1-one |
| 63 | 1-(3-((7-methoxy-4-((2-methoxy-5-(thiophen-2-yl)phenyl)amino)quinazolin-6-yl)oxy)pyrrolidin-1-yl)prop-2-en-1-one |
| 64 | 1-(3-((4-((5-(furan-3-yl)-2-methoxyphenyl)amino)-7-methoxyquinazolin-6-yl)oxy)pyrrolidin-1-yl)prop-2-en-1-one |
| 65 | 1-(4-((7-methoxy-4-((4-methoxy-[1,1'-biphenyl]-3-yl)amino)quinazolin-6-yl)oxy)piperidin-1-yl)prop-2-en-1-one |
| 66 | 1-(4-((7-methoxy-4-((2-methoxy-5-(pyridin-3-yl)phenyl)amino)quinazolin-6-yl)oxy)piperidin-1-yl)prop-2-en-1-one |
| 67 | 1-(4-((4-((4'-fluoro-4-methoxy-[1,1'-biphenyl]-3-yl)amino)-7-methoxyquinazolin-6-yl)oxy)piperidin-1-yl)prop-2-en-1-one |
| 68 | 1-(4-((4-((2',4'-difluoro-4-methoxy-[1,1'-biphenyl]-3-yl)amino)-7-methoxyquinazolin-6-yl)oxy)piperidin-1-yl)prop-2-en-1-one |
| 69 | 1-(4-((4-((5-([1,2,4]triazolo[1,5-a]pyridin-7-yl)-2-methoxyphenyl)amino)-7-methoxyquinazolin-6-yl)oxy)piperidin-1-yl)prop-2-en-1-one |
| 70 | 1-(4-((4-((5-(imidazo[1,2-a]pyridin-8-yl)-2-methoxyphenyl)amino)-7-methoxyquinazolin-6-yl)oxy)piperidin-1-yl)prop-2-en-1-one |
| 71 | 1-(4-((4-((5-(benzofuran-5-yl)-2-methoxyphenyl)amino)-7-methoxyquinazolin-6-yl)oxy)piperidin-1-yl)prop-2-en-1-one |
| 72 | 1-(4-((4-((5-(benzofuran-2-yl)-2-methoxyphenyl)amino)-7-methoxyquinazolin-6-yl)oxy)piperidin-1-yl)prop-2-en-1-one |
| 73 | 1-(4-((4-((5-(benzofuran-3-yl)-2-methoxyphenyl)amino)-7-methoxyquinazolin-6-yl)oxy)piperidin-1-yl)prop-2-en-1-one |
| 74 | 1-(4-((4-((5-(2-hydroxytetrahydro-2H-pyran-3-yl)-2-methoxyphenyl)amino)-7-methoxyquinazolin-6-yl)oxy)piperidin-1-yl)prop-2-en-1-one |
| 75 | 1-(4-((7-methoxy-4-((2-methoxy-5-(1-methyl-1H-pyrazol-5-yl)phenyl)amino)quinazolin-6-yl)oxy)piperidin-1-yl)prop-2-en-1-one |
| 76 | 1-(4-((7-methoxy-4-((2-methoxy-5-(1-methyl-1H-pyrazol-3-yl)phenyl)amino)quinazolin-6-yl)oxy)piperidin-1-yl)prop-2-en-1-one |
| 77 | 1-(4-((7-methoxy-4-((2-methoxy-5-(thiazol-5-yl)phenyl)amino)quinazolin-6-yl)oxy)piperidin-1-yl)prop-2-en-1-one |
| 78 | 1-(4-((7-methoxy-4-((2-methoxy-5-(thiophen-2-yl)phenyl)amino)quinazolin-6-yl)oxy)piperidin-1-yl)prop-2-en-1-one |
| 79 | 1-(4-((7-methoxy-4-((2-methoxy-5-(thiophen-3-yl)phenyl)amino)quinazolin-6-yl)oxy)piperidin-1-yl)prop-2-en-1-one |
| 80 | 1-(4-((4-((5-(furan-2-yl)-2-methoxyphenyl)amino)-7-methoxyquinazolin-6-yl)oxy)piperidin-1-yl)prop-2-en-1-one |
| 81 | 1-(4-((4-((5-(furan-3-yl)-2-methoxyphenyl)amino)-7-methoxyquinazolin-6-yl)oxy)piperidin-1-yl)prop-2-en-1-one |
| 82 | 1-(4-((7-methoxy-4-((2-methoxy-5-(3-methylthiophen-2-yl)phenyl)amino)quinazolin-6-yl)oxy)piperidin-1-yl)prop-2-en-1-one |
| 83 | 1-(4-((7-methoxy-4-((2-methoxy-5-(4-methylthiophen-2-yl)phenyl)amino)quinazolin-6-yl)oxy)piperidin-1-yl)prop-2-en-1-one |
| 84 | 1-(4-((7-methoxy-4-((2-methoxy-5-(2-methylfuran-3-yl)phenyl)amino)quinazolin-6-yl)oxy)piperidin-1-yl)prop-2-en-1-one |
| 85 | 1-(4-((4-((5-(isoxazol-4-yl)-2-methoxyphenyl)amino)-7-methoxyquinazolin-6-yl)oxy)piperidin-1-yl)prop-2-en-1-one |
| 86 | 1-(4-((7-methoxy-4-((2-methoxy-5-(5-methylfuran-2-yl)phenyl)amino)quinazolin-6-yl)oxy)piperidin-1-yl)prop-2-en-1-one |
| 87 | 1-(4-((4-((5-(5-ethylfuran-2-yl)-2-methoxyphenyl)amino)-7-methoxyquinazolin-6-yl)oxy)piperidin-1-yl)prop-2-en-1-one |
| 88 | 1-(4-((4-((5-(5-fluorofuran-2-yl)-2-methoxyphenyl)amino)-7-methoxyquinazolin-6-yl)oxy)piperidin-1-yl)prop-2-en-1-one |
| 89 | 1-(4-((7-methoxy-4-((2-methoxy-5-(3-methylfuran-2-yl)phenyl)amino)quinazolin-6-yl)oxy)piperidin-1-yl)prop-2-en-1-one |
| 90 | 1-(4-((7-methoxy-4-((2-methoxy-5-(4-methylfuran-2-yl)phenyl)amino)quinazolin-6-yl)oxy)piperidin-1-yl)prop-2-en-1-one |
| 91 | 1-(4-((4-((5-(3,5-dimethylfuran-2-yl)-2-methoxyphenyl)amino)-7-methoxyquinazolin-6-yl)oxy)piperidin-1-yl)prop-2-en-1-one |
| 92 | 5-(3-((6-((1-acryloylpiperidin-4-yl)oxy)-7-methoxyquinazolin-4-yl)amino)-4-methoxyphenyl)furan-2-carbonitrile |
| 93 | 1-(4-((7-methoxy-4-((2-methoxy-5-(tetrahydrofuran-2-yl)phenyl)amino)quinazolin-6-yl)oxy)piperidin-1-yl)prop-2-en-1-one |
| 94 | 1-(4-((7-methoxy-4-((2-methoxy-5-(tetrahydro-2H-pyran-2-yl)phenyl)amino)quinazolin-6-yl)oxy)piperidin-1-yl)prop-2-en-1-one |
| 95 | 1-(4-((7-methoxy-4-((2-methoxy-4-methyl-5-(thiophen-2-yl))amino)quinazolin-6-yl)oxy)piperidin-1-yl)prop-2-en-1-one |
| 96 | 1-(4-((4-((5-(furan-2-yl)-2-methoxy-4-methylphenyl)amino)-7-methoxyquinazolin-6-yl)oxy)piperidin-1-yl)prop-2-en-1-one |
| 97 | 1-(4-((4-((3-(furan-2-yl)-6-methoxy-2,4-dimethylphenyl)amino)-7-methoxyquinazolin-6-yl)oxy)piperidin-1-yl)prop-2-en-1-one |
| 98 | 1-(4-((4-((2-fluoro-3-(furan-2-yl)-6-methoxy-4-methylphenyl)amino)-7-methoxyquinazolin-6-yl)oxy)piperidin-1-yl)prop-2-en-1-one |
| 99 | 1-(3,3-difluoro-4-((4-((5-(furan-2-yl)-2-methoxyphenyl)amino)-7-methoxyquinazolin-5-yl)oxy)piperidin-1-yl)prop-2-en-1-one |
| 100 | 1-(3-((4-((2',4'-difluoro-4-methoxy-[1,1'-biphenyl]-3-yl)amino)-7-methoxyquinazolin-5-yl)oxy)-4,4-difluoropiperidin-1-yl)prop-2-en-1-one |
| 101 | 1-(4-((4-((3-fluoro-5-(furan-2-yl)-2-methoxyphenyl)amino)-7-methoxyquinazolin-6-yl)oxy)piperidin-1-yl)prop-2-en-1-one |
| 102 | 1-(4-((4-((4-fluoro-5-(furan-2-yl)-2-methoxyphenyl)amino)-7-methoxyquinazolin-6-yl)oxy)piperidin-1-yl)prop-2-en-1-one |
| 103 | 1-(4-((4-((2-fluoro-3-(furan-2-yl)-6-methoxyphenyl)amino)-7-methoxyquinazolin-6-yl)oxy)piperidin-1-yl)prop-2-en-1-one |
| 104 | 1-(4-((4-((5-(furan-2-yl)-2-methoxypyridin-3-yl)amino)-7-methoxyquinazolin-6-yl)oxy)piperidin-1-yl)prop-2-en-1-one |
| 105 | 1-(4-((4-((2-(furan-2-yl)-5-methoxypyridin-4-yl)amino)-7-methoxyquinazolin-6-yl)oxy)piperidin-1-yl)prop-2-en-1-one |
| 106 | 1-(4-((4-((2-(furan-2-yl)-5-methoxypyrimidin-4-yl)amino)-7-methoxyquinazolin-6-yl)oxy)piperidin-1-yl)prop-2-en-1-one |
| 107 | 1-(4-((4-((6-(furan-2-yl)-3-methoxypyridin-2-yl)amino)-7-methoxyquinazolin-6-yl)oxy)piperidin-1-yl)prop-2-en-1-one |
| 108 | 1-(4-((4-((6-(furan-2-yl)-3-methoxypyrazin-2-yl)amino)-7-methoxyquinazolin-6-yl)oxy)piperidin-1-yl)prop-2-en-1-one |
| 109 | 1-(4-((4-((6-(furan-2-yl)-3-methoxypyridazin-4-yl)amino)-7-methoxyquinazolin-6-yl)oxy)piperidin-1-yl)prop-2-en-1-one |
| 110 | 1-(4-((4-((5-(furan-2-yl)-2-methoxyphenyl)amino)-7-methoxyquinazolin-6-yl)oxy)-2-methylpiperidin-1-yl)prop-2-en-1-one |
| 111 | 1-(3,3-difluoro-4-((4-((5-(furan-2-yl)-2-methoxyphenyl)amino)-7-methoxyquinazolin-6-yl)oxy)piperidin-1-yl)prop-2-en-1-one |
| 112 | 1-(3-fluoro-4-((4-((5-(furan-2-yl)-2-methoxyphenyl)amino)-7-methoxyquinazolin-6-yl)oxy)piperidin-1-yl)prop-2-en-1-one |
| 113 | 1-(4,4-difluoro-3-((4-((5-(furan-2-yl)-2-methoxyphenyl)amino)-7-methoxyquinazolin-6-yl)oxy)piperidin-1-yl)prop-2-en-1-one |
| 114 | 1-(4-((4-((5-(furan-2-yl)-2-methoxyphenyl)amino)-7-methoxyquinazolin-6-yl)oxy)-2-(trifluoromethyl)piperidin-1-yl)prop-2-en-1-one |
| 115 | 1-(5-((4-((5-(furan-2-yl)-2-methoxyphenyl)amino)-7-methoxyquinazolin-6-yl)oxy)-2-azabicyclo[2.2.1]heptan-2-yl)prop-2-en-1-one |
| 116 | 1-(4-((4-((5-(furan-2-yl)-2-(trifluoromethoxy)phenyl)amino)-7-methoxyquinazolin-6-yl)oxy)piperidin-1-yl)prop-2-en-1-one |
| 117 | 1-(4-((4-((2-ethoxy-5-(thiophen-2-yl)phenyl)amino)-7-methoxyquinazolin-6-yl)oxy)piperidin-1-yl)prop-2-en-1-one |
| 118 | 1-(4-((4-((2-ethoxy-5-(furan-2-yl)phenyl)amino)-7-methoxyquinazolin-6-yl)oxy)piperidin-1-yl)prop-2-en-1-one |
| 119 | (E)-4-(dimethylamino)-1-(4-((7-methoxy-4-((2-methoxy-5-(thiophen-2-yl)phenyl)amino)quinazolin-6-yl)oxy)piperidin-1-yl)but-2-en-1-one |
| 120 | (E)-4-(dimethylamino)-1-(4-((4-((5-(furan-2-yl)-2-methoxyphenyl)amino)-7-methoxyquinazolin-6-yl)oxy)piperidin-1-yl)but-2-en-1-one |
| 121 | 2-((dimethylamino)methyl)-1-(4-((4-((5-(furan-2-yl)-2-methoxyphenyl)amino)-7-methoxyquinazolin-6-yl)oxy)piperidin-1-yl)prop-2-en-1-one |
| 122 | (E)-3-fluoro-1-(4-((4-((5-(furan-2-yl)-2-methoxyphenyl)amino)-7-methoxyquinazolin-6-yl)oxy)piperidin-1-yl)prop-2-en-1-one |
| 123 | 2-(4-((4-((5-(furan-2-yl)-2-methoxyphenyl)amino)-7-methoxyquinazolin-6-yl)oxy)piperidin-1-carbonyl) acrylonitrile |
| 124 | (E)-1-(4-((4-((5-(furan-2-yl)-2-methoxyphenyl)amino)-7-methoxyquinazolin-6-yl)oxy)piperidin-1-yl)-3-(1-methylpyrrolidin-2-yl)prop-2-en-1-one |
| 125 | 1-(4-((4-((5-(furan-2-yl)-2-methoxyphenyl)amino)-7-methoxyquinolin-6-yl)oxy)piperidin-1-yl)prop-2-en-1-one |
| 126 | 6-((1-acryloylpiperidin-4-yl)oxy)-4-((5-(furan-2-yl)-2-methoxyphenyl)amino)-7-methoxyquinolin-3-carbonitrile |
| 127 | 1-(4-((4-((2-(furan-2-yl)-5-methoxypyrimidin-4-yl)amino)-7-methoxyquinolin-6-yl)oxy)piperidin-1-yl)prop-2-en-1-one |
| 128 | 1-(3-((4-((5-([1,2,4]triazolo[1,5-a]pyridin-5-yl)-2-methoxyphenyl)amino)-7-methoxyquinazolin-6-yl)oxy)azetidin-1-yl)prop-2-en-1-one |
| 129 | 1-(4-((4-((3'-(3,3-difluoroazetidin-1-yl)-4'-fluoro-4-methoxy-[1,1'-biphenyl]-3-yl)amino)-7-methoxyquinazolin-6-yl)oxy)piperidin-1-yl)prop-2-en-1-one |
| 130 | 1-(4-((7-methoxy-4-((2-methoxy-5-(oxazol-5-yl)phenyl)amino)quinazolin-6-yl)oxy)piperidin-1-yl)prop-2-en-1-one |
| 131 | 1-(4-((4-((2'-fluoro-4-methoxy-4'-methyl-[1,1'-biphenyl]-3-yl)amino)-7-methoxyquinazolin-6-yl)oxy)piperidin-1-yl)prop-2-en-1-one |
| 132 | (S)-1-(3-((4-((5-(furan-2-yl)-2-methoxyphenyl)amino)-7-methoxyquinazolin-6-yl)oxy)pyrrolidin-1-yl)prop-2-en-1-one |
| 133 | 1-(4-((4-((5-(6-fluoropyridin-3-yl)-2-methoxyphenyl)amino)-7-methoxyquinazolin-6-yl)oxy)piperidin-1-yl)prop-2-en-1-one |
| 134 | 3'-((6-((1-acryloylpiperidin-4-yl)oxy)-7-methoxyquinazolin-4-yl)amino)-2-fluoro-4'-methoxy-[1,1'-biphenyl]-3-carbonitrile |
| 135 | 1-(4-((4-((3'-chloro-2'-fluoro-4-methoxy-[1,1'-biphenyl]-3-yl)amino)-7-methoxyquinazolin-6-yl)oxy)piperidin-1-yl)prop-2-en-1-one |
| 136 | 1-(4-((4-((2'-fluoro-4-methoxy-[1,1'-biphenyl]-3-yl)amino)-7-methoxyquinazolin-6-yl)oxy)piperidin-1-yl)prop-2-en-1-one |
| 137 | 1-(4-((4-((2',3'-difluoro-4-methoxy-[1,1'-biphenyl]-3-yl)amino)-7-methoxyquinazolin-6-yl)oxy)piperidin-1-yl)prop-2-en-1-one |
| 138 | 1-(4-((4-((2'-fluoro-4-methoxy-3'-(trifluoromethyl)-[1,1'-biphenyl]-3-yl)amino)-7-methoxyquinazolin-6-yl)oxy)piperidin-1-yl)prop-2-en-1-one |
| 139 | 1-(4-((7-methoxy-4-((4-methoxy-3'-(trifluoromethyl)-[1,1'-biphenyl]-3-yl)amino)quinazolin-6-yl)oxy)piperidin-1-yl)prop-2-en-1-one |
| 140 | 1-(3-fluoro-4-((4-((4-fluoro-5-(furan-2-yl)-2-methoxyphenyl)amino)-7-methoxyquinazolin-6-yl)oxy)piperidin-1-yl)prop-2-en-1-one |
| 141 | 1-(3-((4-((2',4'-difluoro-4-methoxy-[1,1'-biphenyl]-3-yl)amino)-7-methoxyquinazolin-6-yl)oxy)-4,4-difluoropiperidin-1-yl)prop-2-en-1-one |
| 142 | 1-(4-((4-((4'-fluoro-4-methoxy-3'-(trifluoromethyl)-[1,1'-biphenyl]-3-yl)amino)-7-methoxyquinazolin-6-yl)oxy)piperidin-1-yl)prop-2-en-1-one |
| 143 | 1-(4-((4-((2',4'-difluoro-4-methoxy-[1,1'-biphenyl]-3-yl)amino)-7-methoxyquinazolin-6-yl)oxy)-3-fluoropiperidin-1-yl)prop-2-en-1-one |
| 144 | 1-(3-fluoro-4-((7-methoxy-4-((2-methoxy-5-(thiophen-2-yl)phenyl)amino)quinazolin-6-yl)oxy)piperidin-1-yl)prop-2-en-1-one |
| 145 | 1-(4-((4-((5-(2,5-difluoropyridin-3-yl)-2-methoxyphenyl)amino)-7-methoxyquinazolin-6-yl)oxy)piperidin-1-yl)prop-2-en-1-one |
| 146 | 1-(4-((4-((3'-amino-4'-fluoro-4-methoxy-[1,1'-biphenyl]-3-yl)amino)-7-methoxyquinazolin-6-yl)oxy)piperidin-1-yl)prop-2-en-1-one |
| 147 | 1-(4-((4-((5-(2,6-difluoropyridin-3-yl)-2-methoxyphenyl)amino)-7-methoxyquinazolin-6-yl)oxy)piperidin-1-yl)prop-2-en-1-one |
| 148 | 1-(4-((4-((5-(furan-2-yl)-2-methoxyphenyl)amino)-7-methoxyquinazolin-6-yl)oxy)-3-methylpiperidin-1-yl)prop-2-en-1-one |
| 149 | 1-(4-((7-methoxy-4-((2-methoxy-5-(pyridin-4-yl)phenyl)amino)quinazolin-6-yl)oxy)piperidin-1-yl)prop-2-en-1-one |
| 150 | 1-(4-((4-((5-(5-fluoropyridin-3-yl)-2-methoxyphenyl)amino)-7-methoxyquinazolin-6-yl)oxy)piperidin-1-yl)prop-2-en-1-one |
| 151 | 1-(4-((4-((3'-(dimethylamino)-4-methoxy-[1,1'-biphenyl]-3-yl)amino)-7-methoxyquinazolin-6-yl)oxy)piperidin-1-yl)prop-2-en-1-one |
| 152 | 1-((3S,4S)-4-((4-((2',4'-difluoro-4-methoxy-[1,1'-biphenyl]-3-yl)amino)-7-methoxyquinazolin-6-yl)oxy)-3-fluoropiperidin-1-yl)prop-2-en-1-one |
| 153 | 1-(4-((4-((5-(benzo[d][1,3]dioxol-5-yl)-2-methoxyphenyl)amino)-7-methoxyquinazolin-6-yl)oxy)piperidin-1-yl)prop-2-en-1-one |
| 154 | 1-((3R,4S)-3-fluoro-4-((4-((5-(furan-2-yl)-2-methoxyphenyl)amino)-7-methoxyquinazolin-6-yl)oxy)piperidin-1-yl)prop-2-en-1-one |
| 155 | 1-(4-((4-((3'-(dimethylamino)-2',4'-difluoro-4-methoxy-[1,1'-biphenyl]-3-yl)amino)-7-methoxyquinazolin-6-yl)oxy)piperidin-1-yl)prop-2-en-1-one |
| 156 | 1-(4-((4-((2,4'-difluoro-4-methoxy-[1,1'-biphenyl]-3-yl)amino)-7-methoxyquinazolin-6-yl)oxy)piperidin-1-yl)prop-2-en-1-one |
| 157 | 1-(4-((7-methoxy-4-((2',4',6-trifluoro-4-methoxy-[1,1'-biphenyl]-3-yl) amino) quinazolin-6-yl)oxy)piperidin-1-yl)prop-2-en-1-one |
| 158 | 1-(4-((4-((3'-amino-2',4'-difluoro-4-methoxy-[1,1'-biphenyl]-3-yl)amino)-7-methoxyquinazolin-6-yl)oxy)piperidin-1-yl)prop-2-en-1-one |
| 159 | 1-(4-((4-((3'-(azetidin-1-yl)-4'-fluoro-4-methoxy-[1,1'-biphenyl]-3-yl)amino)-7-methoxyquinazolin-6-yl)oxy)piperidin-1-yl)prop-2-en-1-one |
| 160 | 1-(4-((4-((5-(2,3-dihydrobenzo[b][1,4]dioxin-6-yl)-2-methoxyphenyl)amino)-7-methoxyquinazolin-6-yl)oxy)piperidin-1-yl)prop-2-en-1-one |
| 161 | 1-(4-((4-((5'-amino-4'-fluoro-4-methoxy-2'-methyl-[1,1'-biphenyl]-3-yl)amino)-7-methoxyquinazolin-6-yl)oxy)piperidin-1-yl)prop-2-en-1-one |
| 162 | 1-(3-((4-((5-(furan-2-yl)-2-methoxyphenyl)amino)-7-methoxyquinazolin-6-yl)oxy)piperidin-1-yl)prop-2-en-1-one |
| 163 | 1-(4-((4-((3'-amino-2'-fluoro-4-methoxy-[1,1'-biphenyl]-3-yl)amino)-7-methoxyquinazolin-6-yl)oxy)piperidin-1-yl)prop-2-en-1-one |
| 164 | 1-(4-((4-((5-(furan-2-yl)-2-methoxyphenyl)amino)-7-methoxyquinazolin-6-yl)oxy)-2-(trifluoromethyl)piperidin-1-yl)prop-2-en-1-one |
| 165 | 1-(4-((4-((2',4'-difluoro-4-methoxy-[1,1'-biphenyl]-3-yl)amino)-7-methoxyquinazolin-6-yl)oxy)-2-(trifluoromethyl)piperidin-1-yl)prop-2-en-1-one |
| 166 | 1-(3-((4-((5-(furan-2-yl)-2-methoxyphenyl)amino)-7-methoxyquinazolin-6-yl)oxy)-8-azabicyclo[3.2.1]octan-8-yl)prop-2-en-1-one |
| 167 | 1-((1R,3r,5S)-3-((4-((5-(furan-2-yl)-2-methoxyphenyl)amino)-7-methoxyquinazolin-6-yl)oxy)-8-azabicyclo[3.2.1]octan-8-yl)prop-2-en-1-one |
| 168 | 1-(4-((4-((4'-fluoro-3'-hydroxy-4-methoxy-[1,1'-biphenyl]-3-yl)amino)-7-methoxyquinazolin-6-yl)oxy)piperidin-1-yl)prop-2-en-1-one |
| 169 | 1-((3R,4S)-4-((4-((5-(furan-2-yl)-2-methoxyphenyl)amino)-7-methoxyquinazolin-6-yl)oxy)-3-methylpiperidin-1-yl)prop-2-en-1-one |
| 170 | 1-(4-((4-((5'-amino-2',4'-difluoro-4-methoxy-[1,1'-biphenyl]-3-yl)amino)-7-methoxyquinazolin-6-yl)oxy)piperidin-1-yl)prop-2-en-1-one |
| 171 | 1-(trans-4-((4-((2',4'-difluoro-4-methoxy-[1,1'-biphenyl]-3-yl)amino)-7-methoxyquinazolin-6-yl)oxy)-3-fluoropiperidin-1-yl)prop-2-en-1-one |
| 172 | 1-((1R,3s,5S)-3-((4-((5-(furan-2-yl)-2-methoxyphenyl)amino)-7-methoxyquinazolin-6-yl)oxy)-8-azabicyclo[3.2.1]octan-8-yl)prop-2-en-1-one |
| 173 | 1-((3R,4R)-3-fluoro-4-((4-((5-(furan-2-yl)-2-methoxyphenyl)amino)-7-methoxyquinazolin-6-yl)oxy)piperidin-1-yl)prop-2-en-1-one |
| 174 | 1-((3S,4S)-3-fluoro-4-((4-((5-(furan-2-yl)-2-methoxyphenyl)amino)-7-methoxyquinazolin-6-yl)oxy)piperidin-1-yl)prop-2-en-1-one |
| 175 | 5-(3-((6-((1-acryloylpiperidin-4-yl)oxy)-7-methoxyquinazolin-4-yl)amino)-4-methoxyphenyl)furan-2-carboxylic acid |
| 176 | 1-(4-((4-((5-(2,3-dihydrothieno[3,4-b][1,4]dioxin-5-yl)-2-methoxyphenyl)amino)-7-methoxyquinazolin-6-yl)oxy)piperidin-1-yl)prop-2-en-1-one |
| 177 | 1-(4-((4-((5-(2-hydroxytetrahydrofuran-2-yl)-2-methoxyphenyl)amino)-7-methoxyquinazolin-6-yl)oxy)piperidin-1-yl)prop-2-en-l-one |
| 178 | 1-((3S,4R)-3-fluoro-4-((4-((5-(furan-2-yl)-2-methoxyphenyl)amino)-7-methoxyquinazolin-6-yl)oxy)piperidin-1-yl)prop-2-en-l-one |
| 179 | 1-(trans-3-fluoro-4-((4-((5-(furan-2-yl)-2-methoxyphenyl)amino)-7-methoxyquinazolin-6-yl)oxy)piperidin-1-yl)prop-2-en-l-one |
| 180 | 1-(4-((4-((3'-amino-4-methoxy-[1,1'-biphenyl]-3-yl)amino)-7-methoxyquinazolin-6-yl)oxy)piperidin-1-yl)prop-2-en-1-one |
| 181 | 1-(cis-3-fluoro-4-((4-((5-(furan-2-yl)-2-methoxyphenyl)amino)-7-methoxyquinazolin-6-yl)oxy)piperidin-1-yl)prop-2-en-l-one |
| 182 | 1-(cis-4-((4-((2',4'-difluoro-4-methoxy-[1,1'-biphenyl]-3-yl)amino)-7-methoxyquinazolin-6-yl)oxy)-3-fluoropiperidin-1-yl)prop-2-en-1-one |
| 183 | 5-(3-((6-((1-acryloylpiperidin-4-yl)oxy)-7-methoxyquinazolin-4-yl)amino)-4-methoxyphenyl)furan-2-carbonitrile |
| 184 | 2-fluoro-1-(4-((4-((5-(furan-2-yl)-2-methoxyphenyl)amino)-7-methoxyquinazolin-6-yl)oxy)piperidin-1-yl)prop-2-en-l-one |
| 185 | 1-(4-((4-((2-fluoro-3-(furan-2-yl)-6-methoxyphenyl)amino)-7-methoxyquinazolin-6-yl)oxy)piperidin-1-yl)prop-2-en-1-one |
| 186 | 1-(4-((7-methoxy-4-((2,2',4'-trifluoro-4-methoxy-[1,1'-biphenyl]-3-yl) amino) quinazolin-6-yl)oxy)piperidin-1-yl)prop-2-en-1-one |
| 187 | 2-chloro-1-(4-((4-((2',4'-difluoro-4-methoxy-[1,1'-biphenyl]-3-yl)amino)-7-methoxyquinazolin-6-yl)oxy)piperidin-1-yl)-2-fluoroethan-1-one |
| 188 | 2-chloro-2-fluoro-1-(4-((4-((5-(furan-2-yl)-2-methoxyphenyl)amino)-7-methoxyquinazolin-6-yl)oxy)piperidin-1-yl)ethan-1-one |
| 189 | 1-((3S,4R)-4-((4-((2',4'-difluoro-4-methoxy-[1,1'-biphenyl]-3-yl)amino)-7-methoxyquinazolin-6-yl)oxy)-3-fluoropiperidin-1-yl)prop-2-en-1-one |
| 190 | 1-(4-((7-methoxy-4-((2',4',6'-trifluoro-4-methoxy-[1,1'-biphenyl]-3-yl) amino) quinazolin-6-yl)oxy)piperidin-1-yl)prop-2-en-1-one |
| 191 | 1-(4-((4-((2'-chloro-4'-fluoro-4-methoxy-[1,1'-biphenyl]-3-yl)amino)-7-methoxyquinazolin-6-yl)oxy)piperidin-1-yl)prop-2-en-1-one |
| 192 | 1-(4-((4-((2'-fluoro-4-methoxy-5'-(trifluoromethyl)-[1,1'-biphenyl]-3-yl)amino)-7-methoxyquinazolin-6-yl)oxy)piperidin-1-yl)prop-2-en-1-one |
| 193 | 1-(4-((4-((5-(benzo[b]thiophen-6-yl)-2-methoxyphenyl)amino)-7-methoxyquinazolin-6-yl)oxy)piperidin-1-yl)prop-2-en-1-one |
| 194 | 1-(4-((4-((5-(benzo[d]thiazol-6-yl)-2-methoxyphenyl)amino)-7-methoxyquinazolin-6-yl)oxy)piperidin-1-yl)prop-2-en-1-one |
| 195 | 1-(3,3-difluoro-4-((4-((5-(furan-2-yl)-2-methoxyphenyl)amino)-7-methoxyquinazolin-5-yl)oxy)piperidin-1-yl)prop-2-en-1-one |
| 196 | 1-(4-((4-((3'-(difluoromethyl)-4'-fluoro-4-methoxy-[1,1'-biphenyl]-3-yl)amino)-7-methoxyquinazolin-6-yl)oxy)piperidin-1-yl)prop-2-en-1-one |
| 197 | 1-(4-((7-methoxy-4-((4-methoxy-3'-nitro-[1,1'-biphenyl]-3-yl)amino)quinazolin-6-yl)oxy)piperidin-1-yl)prop-2-en-1-one |
| 198 | 1-(4-((7-methoxy-4-((2',3',4',5',6'-pentafluoro-4-methoxy-[1,1'-biphenyl]-3-yl)amino))quinazolin-6-yl)oxy)piperidin-1-yl)prop-2-en-1-one |
| 199 | 1-(3-((4-((3'-(dimethylamino)-4-methoxy-[1,1'-biphenyl]-3-yl) amino)-7-methoxyquinazolin-6-yl)oxy) azetidin-1-yl)prop-2-en-1-one |
| 200 | 1-(3-((4-((2',4'-difluoro-4-methoxy-[1,1'-biphenyl]-3-yl) amino) quinazolin-5-yl)oxy)-4,4-difluoropiperidin-1-yl)prop-2-en-1-one |
| 201 | 1-(3-((4-((2',4'-difluoro-4-methoxy-[1,1'-biphenyl]-3-yl)amino)-7-methoxyquinazolin-5-yl)oxy)-4,4-difluoropiperidin-1-yl)prop-2-en-1-one |
| 202 | 1-(4-((4-((2',4'-difluoro-4-methoxy-3'-(trifluoromethyl)-[1,1'-biphenyl]-3-yl)amino)-7-methoxyquinazolin-6-yl)oxy)piperidin-1-yl)prop-2-en-1-one |
| 203 | 1-(4-((7-methoxy-4-((2',3',4'-trifluoro-4-methoxy-[1,1'-biphenyl]-3-yl)amino)quinazolin-6-yl)oxy)piperidin-1-yl)prop-2-en-1-one |
| 204 | methyl3'-((6-((1-acryloylpiperidin-4-yl)oxy)-7-methoxyquinazolin-4-yl)amino)-4'-methoxy-[1,1'-biphenyl]-3-carboxylate |
| 205 | 1-(4-((7-methoxy-4-((4-methoxy-3'-(methylthio)-[1,1'-biphenyl]-3-yl)amino)quinazolin-6-yl)oxy)piperidin-1-yl)prop-2-en-1-one |
| 206 | 1-(4-((7-methoxy-4-((2-methoxy-5-(quinoxalin-6-yl)phenyl)amino)quinazolin-6-yl)oxy)piperidin-1-yl)prop-2-en-1-one |
| 207 | 1-(4-((4-((2',4'-difluoro-4-methoxy-3'-methyl-[1,1'-biphenyl]-3-yl)amino)-7-methoxyquinazolin-6-yl)oxy)piperidin-1-yl)prop-2-en-1-one |
| 208 | N-(3'-((6-((1-acryloylpiperidin-4-yl)oxy)-7-methoxyquinazolin-4-yl)amino)-4'-methoxy-[1,1'-biphenyl]-3-yl)acrylamide |
| 209 | 3'-((6-((1-acryloylpiperidin-4-yl)amino)-7-methoxyquinazolin-4-yl)amino)-4'-methoxy-[1,1'-biphenyl]-3-carbaldehyde |
| 210 | 1-(4-((4-((2',4'-difluoro-3',4-dimethoxy-[1,1'-biphenyl]-3-yl)amino)-7-methoxyquinazolin-6-yl)oxy)piperidin-1-yl)prop-2-en-1-one |
| 211 | 1-(4-((7-methoxy-4-((4-methoxy-3'-(trifluoromethoxy)-[1,1'-biphenyl]-3-yl)amino)quinazolin-6-yl)oxy)piperidin-1-yl)prop-2-en-1-one |
| 212 | 1-(4-((4-((2',4'-difluoro-4-methoxy-5'-methyl-[1,1'-biphenyl]-3-yl)amino)-7-methoxyquinazolin-6-yl)oxy)piperidin-1-yl)prop-2-en-1-one |
| 213 | 1-(4-((4-((2',4'-difluoro-4,5'-dimethoxy-[1,1'-biphenyl]-3-yl)amino)-7-methoxyquinazolin-6-yl)oxy)piperidin-1-yl)prop-2-en-1-one |
| 214 | 1-(4-((4-((4'-fluoro-4-methoxy-3'-(2,2,2-trifluoroethoxy)-[1,1'-biphenyl]-3-yl)amino)-7-methoxyquinazolin-6-yl)oxy)piperidin-1-yl)prop-2-en-1-one |
| 215 | 1-(4-((7-methoxy-4-((4-methoxy-3'-vinyl-[1,1'-biphenyl]-3-yl)amino)quinazolin-6-yl)oxy)piperidin-1-yl)prop-2-en-1-one |
| 216 | 1-(4-((4-((5-(3,6-dihydro-2H-pyran-4-yl)-2-methoxyphenyl)amino)-7-methoxyquinazolin-6-yl)oxy)piperidin-1-yl)prop-2-en-1-one |
| 217 | 1-(3-((4-((5-(5-chloro-1-((3S,4S)-3-fluoro-1-(oxetan-3-yl)piperidin-4-yl)-1H-pyrazol-4-yl)-2-methoxyphenyl)amino)-7-methoxyquinazolin-6-yl)oxy)azetidin-1-yl)prop-2-en-1-one |
| 218 | 1-(6-((4-((5-(furan-2-yl)-2-methoxyphenyl)amino)-7-methoxyquinazolin-6-yl)oxy)-2-azaspiro[3.3]heptan-2-yl)prop-2-en-1-one |
| 219 | 1-(6-((4-((2',4'-difluoro-4-methoxy-[1,1'-biphenyl]-3-yl)amino)-7-methoxyquinazolin-6-yl)oxy)-2-azaspiro[3.3]heptan-2-yl)prop-2-en-1-one |
| 220 | 1-(2-((4-((5-(furan-2-yl)-2-methoxyphenyl)amino)-7-methoxyquinazolin-6-yl)oxy)-6-azaspiro[3.4]octan-6-yl)prop-2-en-1-one |
| 221 | 1-(6-((4-((5-(furan-2-yl)-2-methoxyphenyl)amino)-7-methoxyquinazolin-6-yl)oxy)-2-azaspiro[3.4]octan-2-yl)prop-2-en-1-one |
| 222 | 1-(6-((4-((2',4'-difluoro-4-methoxy-[1,1'-biphenyl]-3-yl)amino)-7-methoxyquinazolin-6-yl)oxy)-2-azaspiro[3.4]octan-2-yl)prop-2-en-1-one |
| 223 | bicyclo[1.1.0]butan-1-yl(4-((4-((5-(furan-2-yl)-2-methoxyphenyl)amino)-7-methoxyquinazolin-6-yl)oxy)piperidin-1-yl)methanone |
| 224 | N-(3'-((6-((1-acryloylpiperidin-4-yl)oxy)-7-methoxyquinazolin-4-yl)amino)-4'-methoxy-[1,1'-biphenyl]-3-yl) acetamide |
| 225 | 1-(4-((4-((3'-(azetidin-1-yl)-4-methoxy-[1,1'-biphenyl]-3-yl)amino)-7-methoxyquinazolin-6-yl)oxy)piperidin-1-yl)prop-2-en-1-one |
| 226 | N-(3'-((6-((1-acryloylpiperidin-4-yl)oxy)-7-methoxyquinazolin-4-yl)amino)-4-fluoro-4'-methoxy-[1,1'-biphenyl]-3-yl) acetamide |
| 227 | N-(3'-((6-((1-acryloylazetidin-3-yl)oxy)-7-methoxyquinazolin-4-yl) amino)-4'-methoxy-[1,1'-biphenyl]-3-yl)cyclopropancarboxamide |
| 228 | 1-(4-((7-methoxy-4-((2-methoxy-5-(1,2,5-trimethyl-1H-pyrrol-3-yl)phenyl) amino) quinazolin-6-yl)oxy) piperidin-1-yl)prop-2-en-1-one |
| 229 | 1-(4-((4-((3'-(dimethylamino)-4'-fluoro-4-methoxy-[1,1'-biphenyl]-3-yl)amino)-7-methoxyquinazolin-6-yl)oxy)piperidin-1-yl)prop-2-en-1-one |
| 230 | 1-(4-((4-((5'-bromo-2',3',4'-trifluoro-4-methoxy-[1,1'-biphenyl]-3-yl)amino)-7-methoxyquinazolin-6-yl)oxy)piperidin-1-yl)prop-2-en-1-one |
| 231 | 4-(3-((6-((1-acryloylpiperidin-4-yl)oxy)-7-methoxyquinazolin-4-yl)amino)-4-methoxyphenyl)-N-(3-fluorophenethyl)thiophen-2-carboxamide |
| 232 | 4-(3-((6-((1-acryloylpiperidin-4-yl)oxy)-7-methoxyquinazolin-4-yl)amino)-4-methoxyphenyl)-N-benzylthiophen-2-carboxamide |
| 233 | N-(6-(3-((6-((1-acryloylazetidin-3-yl)oxy)-7-methoxyquinazolin-4-yl)amino)-4-methoxyphenyl)benzo[d]thiazol-2-yl)cyclopropancarboxamide |
| 234 | N-(7-(3-((6-((1-acryloylazetidin-3-yl)oxy)-7-methoxyquinazolin-4-yl)amino)-4-methoxyphenyl)isothiazolo[4,3-b]pyridin-3-yl)cyclopropancarboxamide |
| 235 | N-(5-(3-((6-((1-acryloylazetidin-3-yl)oxy)-7-methoxyquinazolin-4-yl)amino)-4-methoxyphenyl)pyridin-3-yl)-3-fluorobenzamide |
| 236 | (R)-N-(3'-((6-((1-acryloylazetidin-3-yl)oxy)-7-methoxyquinazolin-4-yl)amino)-4'-methoxy-[1,1'-biphenyl]-3-yl)-2-oxo-4-phenyloxazolidin-3-carboxamide |
| 237 | (S)-N-(3'-((6-((1-acryloylazetidin-3-yl)oxy)-7-methoxyquinazolin-4-yl)amino)-4'-methoxy-[1,1'-biphenyl]-3-yl)-2-oxo-4-phenyloxazolidin-3-carboxamide |
| 238 | 1-(4-((7-ethoxy-4-((5-(furan-2-yl)-2-methoxyphenyl) amino) quinazolin-6-yl)oxy)piperidin-1-yl)prop-2-en-1-one |
| 239 | 1-(4-((4-((2',4'-difluoro-4-methoxy-[1,1'-biphenyl]-3-yl)amino)-7-ethoxyquinazolin-6-yl)oxy)piperidin-1-yl)prop-2-en-1-one |
| 240 | 1-(3-((7-methoxy-4-((2-((1-methylpyrrolidin-3-yl)oxy)-5-(thiophen-2-yl)phenyl)amino)quinazolin-6-yl)oxy)azetidin-1-yl)prop-2-en-1-one |
| 241 | 1-(3-((4-((5-(furan-2-yl)-2-((1-methylpyrrolidin-3-yl)oxy)phenyl)amino)-7-methoxyquinazolin-6-yl)oxy)azetidin-1-yl)prop-2en-1-one |
| 242 | 1-(3-((4-((5-([1,2,4]triazolo[1,5-a]pyridin-7-yl)-2-((1-methylpyrrolidin-3-yl)oxy))phenyl)amino)-7-methoxyquinazolin-6-yl)oxy)azetidin-1-yl)prop-2-en-1-one |
| 243 | 1-(3-((4-((5-(furan-2-yl)-2-((tetrahydrofuran-3-yl)oxy)phenyl)amino)-7-methoxyquinazolin-6-yl)oxy)azetidin-1-yl)prop-2-en-1-one |
| 244 | 1-(3-((4-((2-cyclopropoxy-5-(furan-2-yl)phenyl)amino)-7-methoxyquinazolin-6-yl)oxy)azetidin-1-yl)prop-2-en-1-one |
| 245 | 1-(3-((4-((5-(furan-2-yl)-2-((1-methylazetidin-3-yl)oxy)phenyl)amino)-7-methoxyquinazolin-6-yl)oxy)azetidin-1-yl)prop-2-en-1-one |
| 246 | 1-(3-((4-((5-(furan-2-yl)-2-(oxetan-3-yloxy)phenyl)amino)-7-methoxyquinazolin-6-yl)oxy)azetidin-1-yl)prop-2-en-1-one |
| 247 | 1-(3-((4-((5-(furan-2-yl)-2-((1-methylpiperidin-4-yl)oxy)phenyl)amino)-7-methoxyquinazolin-6-yl)oxy)azetidin-1-yl)prop-2-en-1-one |
| 248 | 1-(3-((7-methoxy-4-((2-((tetrahydro-2H-pyran-4-yl)oxy)-5-(thiophen-2-yl)phenyl)amino)quinazolin-6-yl)oxy)azetidin-1-yl)prop-2-en-1-one |
| 249 | 1-(3-((4-((5-(furan-2-yl)-2-((tetrahydro-2H-pyran-4-yl)oxy)phenyl)amino)-7-methoxyquinazolin-6-yl)oxy)azetidin-1-yl)prop-2-en-1-one |
| 250 | 1-(4-((7-methoxy-4-((2-((1-methylpyrrolidin-3-yl)oxy)-5-(thiophen-2-yl)phenyl)amino)quinazolin-6-yl)oxy)piperidin-1-yl)prop-2-en-1-one |
| 251 | 1-(4-((4-((5-(furan-2-yl)-2-((1-methylpyrrolidin-3-yl)oxy)phenyl)amino)-7-methoxyquinazolin-6-yl)oxy)piperidin-1-yl)prop-2-en-1-one |
| 252 | 1-(4-((4-((5-(furan-2-yl)-2-((tetrahydrofuran-3-yl)oxy)phenyl)amino)-7-methoxyquinazolin-6-yl)oxy)piperidin-1-yl)prop-2-en-1-one |
| 253 | 1-(4-((4-((2-cyclopropoxy-5-(furan-2-yl)phenyl)amino)-7-methoxyquinazolin-6-yl)oxy)piperidin-1-yl)prop-2-en-1-one |
| 254 | 1-(4-((4-((5-(furan-2-yl)-2-((1-methylazetidin-3-yl)oxy)phenyl)amino)-7-methoxyquinazolin-6-yl)oxy)piperidin-1-yl)prop-2-en-1-one |
| 255 | 1-(4-((4-((5-(furan-2-yl)-2-(oxetan-3-yloxy)phenyl)amino)-7-methoxyquinazolin-6-yl)oxy)piperidin-1-yl)prop-2-en-1-one |
| 256 | 1-(4-((4-((5-(furan-2-yl)-2-((1-methylpiperidin-4-yl)oxy)phenyl)amino)-7-methoxyquinazolin-6-yl)oxy)piperidin-1-yl)prop-2-en-1-one |
| 257 | 1-(4-((7-methoxy-4-((2-((tetrahydro-2H-pyran-4-yl)oxy)-5-(thiophen-2-yl)phenyl)amino)quinazolin-6-yl)oxy)piperidin-1-yl)prop-2-en-1-one |
| 258 | 1-(4-((4-((5-(furan-2-yl)-2-((tetrahydro-2H-pyran-4-yl)oxy)phenyl)amino)-7-methoxyquinazolin-6-yl)oxy)piperidin-1-yl)prop-2-en-1-one |
| 259 | 1-(4-((4-((5-(furan-2-yl)-2-(2-methoxyethoxy)phenyl)amino)-7-methoxyquinazolin-6-yl)oxy)piperidin-1-yl)prop-2-en-1-one |
| 260 | 1-(4-((4-((4-cyclopropoxy-2',4'-difluoro-[1,1'-biphenyl]-3-yl)amino)-7-methoxyquinazolin-6-yl)oxy)piperidin-1-yl)prop-2-en-1-one |
| 261 | 1-(4-((4-((2',4'-difluoro-4-((1-methylpyrrolidin-3-yl)oxy)-[1,1'-biphenyl]-3-yl)amino)-7-methoxyquinazolin-6-yl)oxy)piperidin-1-yl)prop-2-en-1-one |
| 262 | 1-(4-((4-((5-(furan-2-yl)-2-methoxyphenyl)amino)-7-(2-methoxyethoxy)quinazolin-6-yl)amino)piperidin-1-yl)prop-2-en-1-one |
| 263 | 1-(4-((4-((5-(furan-2-yl)-2-methoxyphenyl)amino)-7-(2-methoxyethoxy)quinazolin-6-yl)oxy)piperidin-1-yl)prop-2-en-1-one |
| 264 | 1-(4-((4-((5-(furan-2-yl)-2-methoxyphenyl)amino)-7-(2-morpholinoethoxy)quinazolin-6-yl)oxy)piperidin-1-yl)prop-2-en-1-one |
| 265 | 1-(4-((7-(2-(dimethylamino)ethoxy)-4-((5-(furan-2-yl)-2-methoxyphenyl) amino) quinazolin-6-yl)oxy)piperidin-1-yl)prop-2-en-1-one |
| 266 | 1-(4-((7-(2-(2-(dimethylamino)ethoxy)ethoxy)-4-((5-(furan-2-yl)-2-methoxyphenyl) amino) quinazolin-6-yl)oxy)piperidin-1-yl)prop-2-en-1-one |
| 267 | 1-(4-((4-((5-(furan-2-yl)-2-methoxyphenyl)amino)-7-(3-methoxypropoxy)quinazolin-6-yl)oxy)piperidin-1-yl)prop-2-en-1-one |
| 268 | 1-(4-((4-((5-(furan-2-yl)-2-methoxyphenyl)amino)-7-(4-methoxybutoxy)quinazolin-6-yl)oxy)piperidin-1-yl)prop-2-en-1-one |
| 269 | 1-(4-((4-((2',4'-difluoro-4-methoxy-[1,1'-biphenyl]-3-yl)amino)-7-(3-morpholinopropoxy)quinazolin-6-yl)amino)piperidin-1-yl)prop-2-en-1-one |
| 270 | 1-(4-((4-((5-(furan-2-yl)-2-methoxyphenyl)amino)-7-((tetrahydrofuran-3-yl)oxy)quinazolin-6-yl)amino)piperidin-1-yl)prop-2-en-1-one |
| 271 | N-(4-((5-(furan-2-yl)-2-methoxyphenyl)amino)-7-(2-morpholinoethoxy) quinazolin-6-yl) acrylamide |
| 272 | 1-(4-((4-((5-(furan-2-yl)-2-methoxyphenyl)amino)-7-(2-morpholinoethoxy)quinazolin-6-yl)amino)piperidin-1-yl)prop-2-en-1-one |
| 273 | N-(4-((5-(furan-2-yl)-2-methoxyphenyl)amino)-7-(3-methoxypropoxy) quinazolin-6-yl) acrylamide |
| 274 | 1-(4-((4-((5-(furan-2-yl)-2-methoxyphenyl)amino)-7-(3-methoxypropoxy)quinazolin-6-yl)amino)piperidin-1-yl)prop-2-en-1-one |
| 275 | N-(4-((5-(furan-2-yl)-2-methoxyphenyl)amino)-7-(2-methoxyethoxy) quinazolin-6-yl) acrylamide |
| 276 | 1-(4-((4-((5-(furan-2-yl)-2-methoxyphenyl)amino)-7-(2-methoxyethoxy)quinazolin-6-yl)amino)piperidin-1-yl)prop-2-en-1-one |
| 277 | N-(4-((5-(furan-2-yl)-2-methoxyphenyl)amino)-7-(3-morpholinopropoxy) quinazolin-6-yl) acrylamide |
| 278 | 1-(4-((4-((5-(furan-2-yl)-2-methoxyphenyl)amino)-7-(3-morpholinopropoxy)quinazolin-6-yl)amino)piperidin-1-yl)prop-2-en-1-one |
| 279 | (S)-1-(4-((4-((5-(furan-2-yl)-2-methoxyphenyl)amino)-7-((tetrahydrofuran-3-yl)oxy)quinazolin-6-yl)amino)piperidin-1-yl)prop-2-en-1-one |
| 280 | (R)-1-(4-((4-((5-(furan-2-yl)-2-methoxyphenyl)amino)-7-((tetrahydrofuran-3-yl)oxy)quinazolin-6-yl)amino)piperidin-1-yl)prop-2-en-1-one |
| 281 | (R)-1-(4-((4-((2',4'-difluoro-4-methoxy-[1,1'-biphenyl]-3-yl)amino)-7-((tetrahydrofuran-3-yl)oxy)quinazolin-6-yl)oxy)piperidin-1-yl)prop-2-en-1-one |
| 282 | (R)-1-(4-((4-((2',4'-difluoro-4-methoxy-[1,1'-biphenyl]-3-yl)amino)-7-((tetrahydrofuran-3-yl)oxy)quinazolin-6-yl)amino)piperidin-1-yl)prop-2-en-1-one |
| 283 | 1-(4-((4-((2',4'-difluoro-4-methoxy-[1,1'-biphenyl]-3-yl)amino)-7-((1-methylpyrrolidin-3-yl)oxy)quinazolin-6-yl)oxy)piperidin-1-yl)prop-2-en-1-one |
| 284 | 1-(4-((4-((2',4'-difluoro-4-methoxy-[1,1'-biphenyl]-3-yl)amino)-7-((1-(2-methoxyethyl) pyrrolidin-3-yl)oxy)quinazolin-6-yl)oxy)piperidin-1-yl)prop-2-en-1-one |
| 285 | 1-(4-((4-((2',4'-difluoro-4-methoxy-[1,1'-biphenyl]-3-yl)amino)-7-((1-(oxetan-3-yl)pyrrolidin-3-yl)oxy)quinazolin-6-yl)oxy)piperidin-1-yl)prop-2-en-1-one |
| 286 | 1-(4-((4-((2',4'-difluoro-4-methoxy-[1,1'-biphenyl]-3-yl)amino)-7-((tetrahydrofuran-3-yl)oxy)quinazolin-6-yl)amino)piperidin-1-yl)prop-2-en-1-one |
| 287 | N-(4-((5-([1,2,4]triazolo[1,5-a]pyridin-7-yl)-2-methoxyphenyl) amino)-7-methoxyquinazolin-6-yl)acrylamide |
| 288 | N-(4-((5-(benzofuran-5-yl)-2-methoxyphenyl)amino)-7-methoxyquinazolin-6-yl)acrylamide |
| 289 | N-(4-((5-(furan-2-yl)-2-methoxyphenyl)amino)-7-methoxyquinazolin-6-yl)acrylamide |
| 290 | N-(7-methoxy-4-((2-methoxy-5-(2-methylfuran-3-yl)phenyl)amino)quinazolin-6-yl)acrylamide |
| 291 | N-(3-cyano-7-ethoxy-4-((5-(furan-2-yl)-2-methoxyphenyl)amino)quinolin-6-yl)acrylamide |
| 292 | N-(4-((2',4'-difluoro-4-methoxy-[1,1'-biphenyl]-3-yl)amino)-7-(4,4-difluoropiperidin-1-yl)quinazolin-6-yl)acrylamide |
| 293 | N-(4-((2',4'-difluoro-4-methoxy-[1,1'-biphenyl]-3-yl)amino)-7-(4-morpholinopiperidin-1-yl)quinazolin-6-yl)acrylamide |
| 294 | N-(4-((2',4'-difluoro-4-methoxy-[1,1'-biphenyl]-3-yl)amino)-7-(2-oxa-6-azaspiro[3.3]heptan-6-yl)quinazolin-6-yl)acrylamide |
| 295 | N-(4-((2',4'-difluoro-4-methoxy-[1,1'-biphenyl]-3-yl)amino)-7-(3,3-difluoroazetidin-1-yl)quinazolin-6-yl)acrylamide |
| 296 | (E)-N-(4-((2',4'-difluoro-4-methoxy-[1,1'-biphenyl]-3-yl)amino)-7-methoxyquinazolin-6-yl)-4-methoxybut-2-enamide |
| 297 | 1-(4-((4-((2',4'-difluoro-4-methoxy-[1,1'-biphenyl]-3-yl)amino)-7-(difluoromethoxy)quinazolin-6-yl)amino)piperidin-1-yl)prop-2-en-1-one |
| 298 | 1-(4-((4-((5-(furan-2-yl)-2-methoxyphenyl)amino)-7-methoxyquinazolin-6-yl)amino)piperidin-1-yl)prop-2-en-1-one |
| 299 | 1-(4-((4-((2',4'-difluoro-4-methoxy-[1,1'-biphenyl]-3-yl) amino)-7-methoxyquinazolin-6-yl) amino)piperidin-1-yl)prop-2-en-1-one |
| 300 | 1-(4-((7-ethoxy-4-((5-(furan-2-yl)-2-methoxyphenyl)amino)quinazolin-6-yl)amino)piperidin-1-yl)prop-2-en-1-one |
| 301 | 1-(4-((4-((2',4'-difluoro-4-methoxy-[1,1'-biphenyl]-3-yl) amino)-7-ethoxyquinazolin-6-yl) amino)piperidin-1-yl)prop-2-en-1-one |
| 302 | 1-(4-((4-((5-(furan-2-yl)-2-methoxyphenyl)amino)-7-(methylamino)quinazolin-6-yl)oxy)piperidin-1-yl)prop-2-en-1-one |
| 303 | 1-(4-((5-chloro-4-((5-(furan-2-yl)-2-methoxyphenyl)amino)quinazolin-6-yl)amino)piperidin-1-yl)prop-2-en-1-one |
| 304 | (R,E)-N-(4-((2',4'-difluoro-4-methoxy-[1,1'-biphenyl]-3-yl)amino)-7-methoxyquinazolin-6-yl)-2-fluoro-3-(1-methylpyrrolidin-2-yl)acrylamide |
| 305 | (R,E)-N-(4-((2',4'-difluoro-4-methoxy-[1,1'-biphenyl]-3-yl)amino)-7-methoxyquinazolin-6-yl)-3-(1-methylpyrrolidin-2-yl)acrylamide |
| 306 | (R,E)-N-(4-((5-(furan-2-yl)-2-methoxyphenyl)amino)-7-methoxyquinazolin-6-yl)-3-(1-methylpyrrolidin-2-yl) acrylamide |
| 307 | (R,E)-2-fluoro-N-(4-((5-(furan-2-yl)-2-methoxyphenyl)amino)-7-methoxyquinazolin-6-yl)-3-(1-methylpyrrolidin-2-yl)acrylamide |
| 308 | (R,E)-N-(7-methoxy-4-((2',4',6-trifluoro-4-methoxy-[1,1'-biphenyl]-3-yl)amino)quinazolin-6-yl)-3-(1-methylpyrrolidin-2-yl)acrylamide |
| 309 | (S,E)-2-fluoro-N-(4-((5-(furan-2-yl)-2-methoxyphenyl)amino)-7-methoxyquinazolin-6-yl)-3-(1-methylpyrrolidin-2-yl)acrylamide |
| 310 | (E)-N-(4-((2',4'-difluoro-4-methoxy-[1,1'-biphenyl]-3-yl)amino)-7-methoxyquinazolin-6-yl)-2-fluoro-4-methyl-4-(4-(oxetan-3-yl)piperazin-1-yl)pent-2-enamide |
| 311 | (Z)-2-cyano-N-(4-((2',4'-difluoro-4-methoxy-[1,1'-biphenyl]-3-yl)amino)-7-methoxyquinazolin-6-yl)-4-methyl-4-(4-(oxetan-3-yl)piperazin-1-yl)pent-2-enamide |
| 312 | (E)-N-(4-((2',4'-difluoro-4-methoxy-[1,1'-biphenyl]-3-yl)amino)-7-methoxyquinazolin-6-yl)-2-fluoro-4,4-dimethylpent-2-enamide |
| 313 | (R,Z)-N-(4-((2',4'-difluoro-4-methoxy-[1,1'-biphenyl]-3-yl)amino)-7-methoxyquinazolin-6-yl)-2-fluoro-3-(1-methylpyrrolidin-2-yl)acrylamide |
| 314 | (E)-N-(4-((5-(furan-2-yl)-2-methoxyphenyl)amino)-7-methoxyquinazolin-6-yl)-4-(4-methylpiperazin-1-yl)but-2-enamide |
| 315 | (E)-N-(4-((5-(furan-2-yl)-2-methoxyphenyl)amino)-7-methoxyquinazolin-6-yl)-4-morpholinobut-2-enamide |
| 316 | (E)-N-(4-((2',4'-difluoro-4-methoxy-[1,1'-biphenyl]-3-yl)amino)-7-methoxyquinazolin-6-yl)-4-morpholinobut-2-enamide |
| 317 | (E)-N-(4-((2',4'-difluoro-4-methoxy-[1,1'-biphenyl]-3-yl)amino)-7-methoxyquinazolin-6-yl)-4-(4-methylpiperazin-1-yl)but-2-enamide |
| 318 | (E)-4-(3-oxa-6-azabicyclo[3.1.1]heptan-6-yl)-N-(4-((5-(furan-2-yl)-2-methoxyphenyl)amino)-7-methoxyquinazolin-6-yl)but-2-enamide |
| 319 | (E)-4-(3-oxa-6-azabicyclo[3.1.1]heptan-6-yl)-N-(4-((2',4'-difluoro-4-methoxy-[1,1')-biphenyl]-3-yl)amino)-7-methoxyquinazolin-6-yl)but-2-enamide |
| 320 | (E)-N-(5-chloro-4-((2',4'-difluoro-4-methoxy-[1,1'-biphenyl]-3-yl)amino)quinazolin-6-yl)-4-(dimethylamino)but-2-enamide |
| 321 | (E)-N-(5-chloro-4-((5-(furan-2-yl)-2-methoxyphenyl)amino)quinazolin-6-yl)-4-(dimethylamino)but-2-enamide |
| 322 | 1-(2-(4-((2',4'-difluoro-4-methoxy-[1,1'-biphenyl]-3-yl)amino)-7-methoxyquinazolin-6-yl)-2,6-diazaspiro[3.5]nonan-6-yl)prop-2-en-1-one |
| 323 | 1-(2-(4-((5-(furan-2-yl)-2-methoxyphenyl)amino)-7-methoxyquinazolin-6-yl)-2,6-diazaspiro[3.5]nonan-6-yl)prop-2-en-1-one |
| 324 | 1-(2-(4-((5-(furan-2-yl)-2-methoxyphenyl)amino)-7-methoxyquinazolin-6-yl)-2,7-diazaspiro[3.5]nonan-7-yl)prop-2-en-1-one |
| 325 | 1-(2-(4-((2',4'-difluoro-4-methoxy-[1,1'-biphenyl]-3-yl)amino)-7-methoxyquinazolin-6-yl)-2,7-diazaspiro[3.5]nonan-7-yl)prop-2-en-1-one |
| 326 | 1-(6-(4-((5-(furan-2-yl)-2-methoxyphenyl)amino)-7-methoxyquinazolin-6-yl)-2,6-diazaspiro[3.3]heptan-2-yl)prop-2-en-1-one |
| 327 | 1-(6-(4-((2',4'-difluoro-4-methoxy-[1,1'-biphenyl]-3-yl)amino)-7-methoxyquinazolin-6-yl)-2,6-diazaspiro[3.3]heptan-2-yl)prop-2-en-1-one |
| 328 | 1-(7-(4-((5-(furan-2-yl)-2-methoxyphenyl)amino)-7-methoxyquinazolin-6-yl)-2,7-diazaspiro[4.4]nonan-2-yl)prop-2-en-1-one |
| 329 | 1-(7-(4-((2',4'-difluoro-4-methoxy-[1,1'-biphenyl]-3-yl)amino)-7-methoxyquinazolin-6-yl)-2,7-diazaspiro[4.4]nonan-2-yl)prop-2-en-1-one |
| 330 | 2-acryloyl-7-(4-((5-(furan-2-yl)-2-methoxyphenyl)amino)-7-methoxyquinazolin-6-yl)-5-oxa-2,7-diazaspiro[3.4]octan-6-one |

9. A pharmaceutical composition for preventing or treating cancer, comprising the compound according to any one of claims 1 to 8, the optical isomer thereof, or the pharmaceutically acceptable salt thereof as an active ingredient.

10. The pharmaceutical composition of claim 9, wherein
the pharmaceutical composition inhibits HER2 and/or EGFR.

11. The pharmaceutical composition of claim 10, wherein
the pharmaceutical composition inhibits at least one selected from the group consisting of HER2 L869R, HER2 L755S, HER2 T798I, HER2 T862A, EGFR G719A, EGFR L861Q, EGFR S768I, EGFR G719A/S768I, and EGFR Del19/T790M.

12. The pharmaceutical composition of claim 9, wherein
the cancer is one or more selected from the group consisting of pseudomyxoma, intrahepatic cholangiocarcinoma, hepatoblastoma, liver cancer, thyroid cancer, colon cancer, testicular cancer, myelodysplastic syndrome, glioblastoma, oral cancer, lip cancer, mycosis fungoides, acute myeloid leukemia, acute lymphoblastic leukemia, basal cell carcinoma, ovarian epithelial cancer, ovarian germ cell tumor, male breast cancer, brain cancer, pituitary adenoma, multiple myeloma, gallbladder cancer, biliary tract cancer, colorectal cancer, chronic myelogenous leukemia, chronic lymphocytic leukemia, retinoblastoma, choroidal melanoma, ampulla of vater cancer, bladder cancer, peritoneal cancer, parathyroid cancer, adrenal cancer, sinonasal cancer, non-small cell lung cancer, tongue cancer, astrocytoma, small cell lung cancer, pediatric brain cancer, pediatric lymphoma, pediatric leukemia, small bowel cancer, meningioma, esophageal cancer, glioma, renal pelvis cancer, renal cancer, heart cancer, duodenal cancer, malignant soft tissue tumor, malignant bone tumor, malignant lymphoma, malignant mesothelioma, malignant melanoma, eye cancer, vulvar cancer, ureteral cancer, urethral cancer, cancer of unknown primary site, gastric lymphoma, gastric cancer, gastric carcinoid tumor, gastrointestinal stromal tumor, Wilms' tumor, breast cancer, sarcoma, penile cancer, pharyngeal cancer, gestational choriocarcinoma, cervical cancer, endometrial cancer, uterine sarcoma, prostate cancer, metastatic bone cancer, metastatic brain cancer, mediastinal cancer, rectal cancer, rectal carcinoid tumor, vaginal cancer, spinal cord cancer, vestibular schwannoma, pancreatic cancer, salivary gland cancer, Kaposi's sarcoma, Paget's disease, tonsil cancer, squamous cell carcinoma, lung adenocarcinoma, lung cancer, lung squamous cell carcinoma, skin cancer, anal cancer, rhabdomyosarcoma, laryngeal cancer, pleura cancer, hematological cancer, and thymic carcinoma.

13. Use of the compound, the optical isomer thereof, or the pharmaceutically acceptable salt thereof according to any one of claims 1 to 8 for use in preparation of a medicament to treat or prevent cancer.

14. A method for treating or preventing cancer, comprising administering to a subject in need thereof a therapeutically effective amount of the compound, the optical isomer thereof, or the pharmaceutically acceptable salt thereof according to any one of claims 1 to 8.
